# EUROPÄISCHE PATENTSCHRIFT

(11) **EP 0 981 530 B1**
(45) Veröffentlichungstag und Bekanntmachung des Hinweises auf die Patenterteilung: **19.02.2003**
(21) Anmeldenummer: 98924299.5
(22) Anmeldetag: 11.05.1998
(51) Int. Cl.: C07D 519/00, C08G 73/06, C08K 5/353, C08K 5/52, C08K 5/15

(54) **VERBINDUNGEN AUF BASIS VON POLYALKYL-1-OXA-DIAZASPIRODECAN-VERBINDUNGEN**
COMPOUNDS ON THE BASIS OF POLYALKYL-1-OXA-DIAZASPIRODECANE COMPOUNDS
COMPOSES A BASE DE COMPOSES DE POLYALKYL-1-OXA-DIAZASPIRODECANE

(30) Priorität: 13.05.1997 DE 19719944
(43) Veröffentlichungstag der Anmeldung: 01.03.2000
(73) Patentinhaber: Clariant GmbH, 65929 Frankfurt am Main (DE)
(72) Erfinder: STÄHRFELDT, Thomas, D-86356 Neusä (DE); MEHRER, Mathias, D-86456 Gablingen (DE); ZÄH, Matthias, D-86368 Gersthofen (DE); PFAHLER, Gerhard, D-86169 Augsburg (DE)
(86) Internationale Anmeldenummer: EP9802738
(87) Internationale Veröffentlichungsnummer: WO98051690

(56) Entgegenhaltungen:
- EP-A- 0 028 318
- EP-A- 0 057 885
- EP-A- 0 402 889
- EP-A- 0 690 060
- EP-A- 0 709 426
- EP-A- 0 723 990
- DE-A- 4 423 055

## Beschreibung

Die Stabilisierung von polymerem Material gegen den zerstörerischen Einfluß energiereicher Strahlung ist Gegenstand intensiver Forschungen. In den letzten Jahren wurde eine Vielzahl von Lichtschutzmitteln auf Basis sterisch gehinderter Amine entwickelt, die die Stabilisierung von polymerem Material in ausgezeichneter Art und Weise gewährleisten. Dennoch leiden gerade niedermolekulare Stabilisatoren dieser Substanzklasse unter dem Nachteil der leichten Extrahierbarkeit sowie hoher Flüchtigkeit aus dem zu stabilisierenden polymeren Material, so daß nach einiger Anwendungszeit unerwünschte Zersetzung des Materials eintritt. Aus diesem Grund wurde eine beträchtliche Anzahl polymerer Stabilisatoren auf Basis sterisch gehinderter Amine hergestellt, die die dargestellten Nachteile nicht oder nur in geringerem Maße besitzen. Als exemplarische Beispiele seien Stabilisatoren erwähnt, wie sie z. B. in EP-A-93693, DE-A-2719131, EP-A-343717 und EP-A-402889 beschrieben sind. Es besteht jedoch ein steter Bedarf an neuen leistungsfähigeren Stabilisatoren, welche über verbesserte Lichtschutz- oder zusätzliche bessere Anwendungseigenschaften verfügen.

EP-A-28318 beschreibt die Herstellung und die Polymerisation, EP-A-402889 beschreibt die verbesserte Polymerisation von Verbindungen der allgemeinen Formel 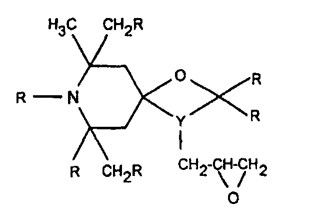 zu oligo- bzw. polymeren Stabilisatoren, während die deutsche Patentanmeldung Nr. 19608163.7 einen effektiven, hochausbeutigen und umweltfreundlichen Weg beschreibt, solche Stabilisatoren herzustellen.

Die Europäische Patentanmeldung 95 109 777.3 und EP-A-57885 (vgl. dort Beispiel 11) beschreiben Derivate von Verbindungen der allgemeinen Formel, 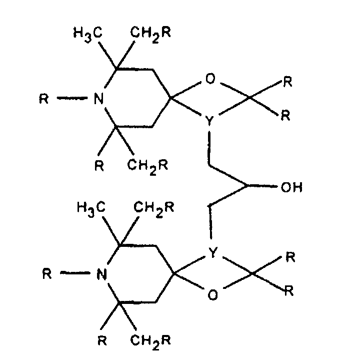 bei welchen es sich um leistungsfähige, neue Lichtschutzmittel handelt.

Überraschenderweise wurde nun gefunden, daß man durch Umsetzung von Verbindungen der Formel (I) mit Verbindungen der Formel (II) 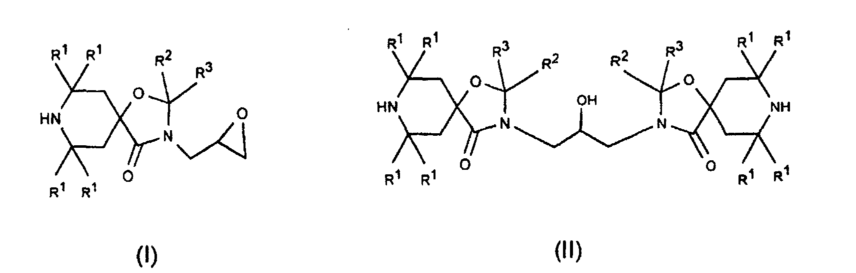 zu neuen Stabilisatoren der Formel (III) gelangt.

Gegenstand der Erfindung sind daher neue Verbindungen der allgemeinen Formel (III). 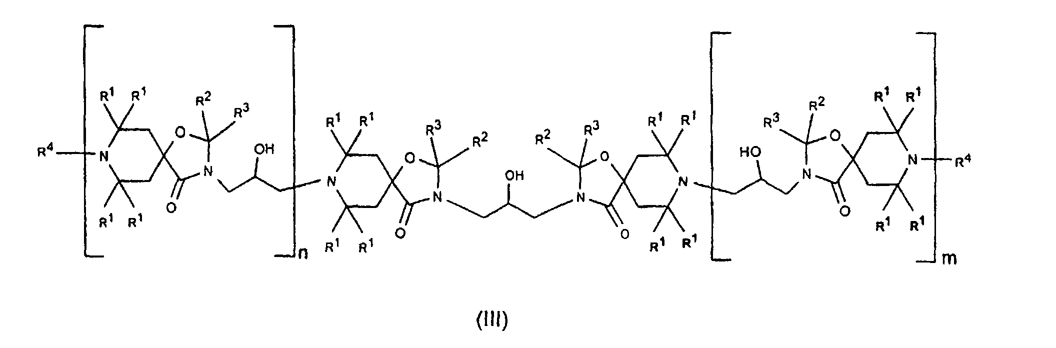 worin
- n und m: unabhängig voneinander eine Zahl von 0 bis 100 sind, wobei n und m nicht gleichzeitig 0 sein können,
- R¹: Wasserstoff, C₅-C₇-Cycloalkyl, oder eine C₁-C₁₂ -Alkylgruppe,
- R² und R³: unabhängig voneinander ein Wasserstoffatom, eine C₁-C₁₈-Alkylgruppe oder zusammen mit dem sie verbindenden C-Atom einen Ring der Ringgröße 5 bis 13 oder zusammen mit dem sie bindenden C-Atom eine Gruppe der Formel (IV), 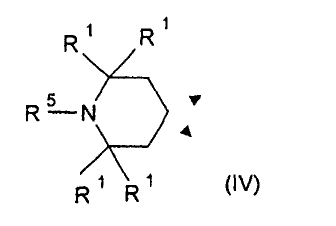
- R⁴ und R⁵: unabhängig voneinander entweder Wasserstoff oder eine C₁-C₂₂-Alkylgruppe, ein Sauerstoffradikal O*, -OH, -NO, -CH₂CN, Benzyl, Allyl, eine C₁-C₃₀-Alkyloxygruppe, eine C₅-C₁₂-Cycloalkyloxygruppe, eine C₆-C₁₀-Aryloxygruppe, wobei der Arylrest zusätzlich noch substituiert sein kann, eine C₇-C₂₀-Arylalkyloxygruppe, wobei der Arylrest zusätzlich noch substituiert sein kann, eine C₃-C₁₀- Alkenylgruppe, eine C₃-C₆-Alkinylgruppe, eine C₁-C₁₀-Acylgruppe, Halogen, unsubstituiertes oder am Phenylring durch C₁-C₄-Alkyl substituiertes C₇-C₉-Phenylalkyl bedeuten.

Gut geeignet sind auch Verbindungen der Formel (III), worin
- n und m: unabhängig voneinander eine Zahl von 0 bis 10 sind, wobei n und m nicht gleichzeitig 0 sein können,
- R¹: Wasserstoff, C₆-Cycloalkyl, oder eine C₁-C₄-Alkylgruppe,
- R² und R³: unabhängig voneinander ein Wasserstoffatom, eine C₁-C₈- Alkylgruppe oder zusammen mit dem sie verbindenden C-Atom einen Ring der Ringgröße 6 bis 12, oder zusammen mit dem sie bindenden C-Atom eine Gruppe der Formel (IV),
- R⁴ und R⁵: unabhängig voneinander entweder Wasserstoff oder eine C₁-C₅-Alkylgruppe, ein Sauerstoffradikal O*, -OH, -NO, -CH₂CN, Benzyl, Allyl, eine C₁-C₁₀-Alkyloxygruppe, eine C₅-C₆-Cycloalkyloxygruppe, eine C₆-C₇-Aryloxygruppe, wobei der Arylrest zusätzlich noch substituiert sein kann, eine C₇-C₁₀-Arylalkyloxygruppe, wobei der Arylrest zusätzlich noch substituiert sein kann, eine C₃-C₆-Alkenylgruppe, eine C₃-C₆-Alkinylgruppe, eine C₁-C₄-Acylgruppe, Halogen, unsubstituiertes oder am Phenylring durch C₁-C₂-Alkyl substituiertes C₇-C₉-Phenylalkyl bedeuten.

Besonders bevorzugt sind Verbindungen der Formel (III), worin
- n und m: unabhängig voneinander eine Zahl von 0 bis 5 sind, wobei n und m nicht gleichzeitig 0 sein können,
- R¹: Methyl,
- R² und R³: zusammen mit dem sie verbindenden C-Atom einen Ring der Ringgröße 12, oder zusammen mit dem sie verbindenden C-Atom eine Gruppe der Formel (IV),
- R⁴ und R⁵: unabhängig voneinander Wasserstoff, Methyl, Acetyl, Octyloxy oder Cyclohexyloxy, bedeuten.

Die Erfindung bezieht sich auch auf ein allgemeines Herstellverfahren von Verbindungen der Formel (III), sowie deren Verwendung zur Stabilisierung von organischem Material, sowie ein mit Verbindungen der Formet (III) stabilisiertes organisches, vorzugsweise polymeres Material, insbesondere von Kunststoffen, Anstrichmitteln, Lacken und Ölen oder ihren Vorprodukten.

Die Herstellung von Verbindungen der allgemeinen Formel (III) mit R⁴ = Wasserstoff erfolgt durch Umsetzung von Verbindungen der allgemeinen Formel (I) mit Verbindungen der allgemeinen Formel (II). Für die Herstellung von (III) kann auf an sich bereits bekannte Verfahren zurückgegriffen werden, wie sie z. B. aus *C. Ferri, Reaktionen der organischen Synthese, 1978, Seite 504 oder Houben-Weyt, Bd.* *VI*/*3, Seite 456 oder Bd. XI*/*1, Seite 311* beschrieben sind. Die Umsetzung kann mit oder ohne Lösemittel erfolgen; als Lösemittel eignen sich bevorzugt hochsiedende, aprotische Lösemittel, wie z. B. Toluol, Xylol, Mesitylen oder Decalin. Die Umsetzung kann mit oder ohne Katalysator (insbesonders thermisch) durchgeführt werden, als Katalysatoren eignen sich Basen, vorzugsweise Metallsalze, insbesondere Alkalimetallhydroxide, wie z. B. NaOH oder KOH. Ein bevorzugtes Herstellverfahren ist die Umsetzung von (I) und (II) direkt in Substanz, ohne Verwendung eines Lösemittels und ohne Katalysator. Hierbei werden (I) und (II) im Molverhältnis im Bereich von 1:1 (äquimolar) bis 100:1 im Temperaturbereich von 100 bis 300°C, vorzugsweise von 120 bis 250°C, insbesondere von 160 bis 220°C vorzugsweise unter Ausschluß von Sauerstoff polymerisiert. Insbesonders eignet sich eine Polymerisation ohne Lösungsmittel im Vakuum. Es entsteht hierbei nach Aufarbeitung ein farbloses, festes, amorphes Harz. Das Molgewicht des resultierenden Oligo- oder Polymers läßt sich in Abhängigkeit von der Temperatur, dem verwendeten Molverhältnis der eingesetzten Edukte und/ oder der Reaktionszeit steuern. Die Methoden zur nachträglichen Modifizierung von R⁴ (R⁴ nicht Wasserstoff) sind aus dem Stand der Technik hinreichend bekannt, z.B. aus EP-A-0 705 896 oder aus EP-A-0 690 060. Von herausragender Bedeutung sind vor allem die Derivate der Verbindung (III) mit R⁴ = H oder R⁴ = Methyl.

Die erfindungsgemäßen Verbindungen eignen sich in hervorragender Weise zum Stabilisieren von organischem Material gegen die Einwirkung von Licht, Sauerstoff und Wärme. Sie können dem zu stabilisierenden organischen Material vor, während oder nach der Polymerisation in fester, geschmolzener, in Lösungsmitteln gelöster Form oder auch als Masterbatch zugegeben werden. Die Lösungen können den neuen Stabilisator z. B. in 5 - 80 %iger Konzentration enthalten; ein Masterbatch eignet sich besonders gut, wenn es den neuen Stabilisator in einer Konzentration von 1 bis 80 %, vorzugsweise aber von 5 - 30 % enthält, der Rest im Masterbatch ist ein mit dem zu stabilisierenden Polymer verträgliches Polymer. Sowohl die Lösung, als auch das Masterbatch können zusätzlich noch weitere Stabilisatoren oder Effektstoffe, z. B. UV-Absorber, Antioxidantien, Pigmente, Säurefänger oder Füllstoffe, enthalten. Die neuen Stabilisatoren werden vorzugsweise so eingesetzt, daß sie im zu stabilisierenden Polymer in einer Konzentration von 0,001 bis 5 Gew.-%, vorzugsweise von 0,02 bis 2 Gew.-%, bezogen auf das organische Material, entweder alleine oder in Kombination mit weiteren Additiven enthalten sind. Unter organischem Material sind beispielsweise Vorprodukte für Kunststoffe, Anstrichmittel, Lacke und Öle, insbesondere jedoch Kunststoffe, Anstrichmittel, Lacke und Öle selbst zu verstehen.

Im besonderen eignen sich die Verbindungen der allgemeinen Formel (III) zum Stabilisieren von Folien, Fasern, Bändchen, Multifilamenten, Geweben, Extrusions-, Blasform-, Spritzguß-, Tiefziehartikeln, Pulverlacken, Druckfarben, Tonerfarben, photographischem Material, Pigmenten, Holzbeizen, Leder, Anstrichfarben für Gebäude, Schutzanstrichen für Stahlkonstruktionen, Schmierölen, Maschinenölen, Bitumen oder Asphalt und zur Stabilisierung von spontan zur Polymerisation neigenden Verbindungen.
Die erfindungsgemäßen Verbindungen der Formel (III) können auch in vorteilhafter Weise in Kombinationen mit weiteren Stabilisatoren eingesetzt werden. Das Resultat dieser neuen Kombinationen sind Mischungen mit einem verbesserten Eigenschaftsprofil gegenüber den Einzelkomponenten, wie z. B. synergistische Effekte in der Lichtschutzwirkung.
Besonders vorteilhaft ist die Kombination der Verbindungen der Formel (III) mit monomeren HALS-Stabilisatoren im Gewichtsverhältnis von 10:1 bis 1:10. Kombinationen von polymeren mit monomeren HALS-Stabilisatoren werden z. B. in EP-A-80431 und EP-A-632092 beschrieben. Besonders vorteilhaft ist die erfindungsgemäße Kombination von (III) mit Verbindungen der Formeln A1 bis A10. 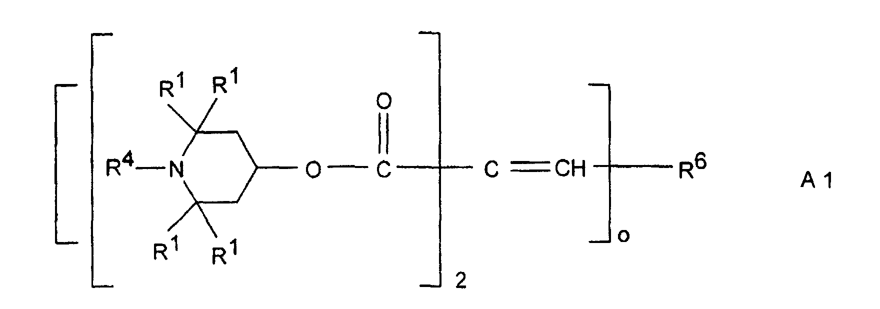 worin
- R¹ und R⁴: die weiter oben angeführten Bedeutungen haben,
- R⁶: einen ein oder mehrfach durch Wasserstoff, C₁ - C₄-Alkyl, C₁ - C₄- Alkoxy, Halogen, Cyan, Carboxy, Nitro, Amino, C₁ - C₄ -Alkylamino, C₁ - C₄-Dialkylamino, oder Acyl substituierten aromatischen Rest,
- o: 1 oder 2 bedeutet, 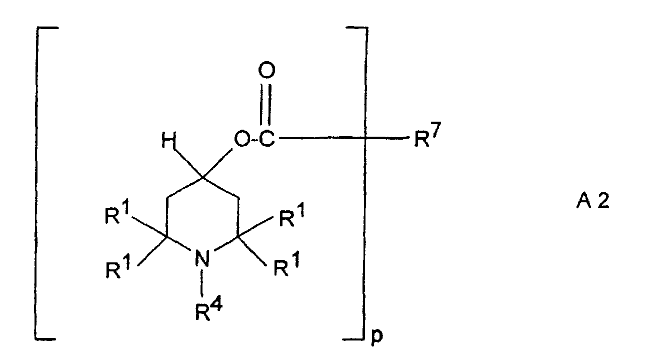
worin
R¹ und R⁴ die in Anspruch 1 angeführten Bedeutungen haben,
p = 1 oder 2 und
für p = 1
R⁷ C₁-C₂₂-Alkyl, C₂-C₁₈-Oxaalkyl, C₂-C₁₈-Thiaalkyl, C₂-C₁₈-Azaalkyl oder C₂-C₈-Alkenyl,
für p = 2
R⁷ C₁-C₂₂-Alkylen, C₂-C₁₈-Oxaalkylen, C₂-C₁₈-Thiaalkylen, C₂-C₁₈-Azaalkylen oder C₂-C₈-Alkenylen, 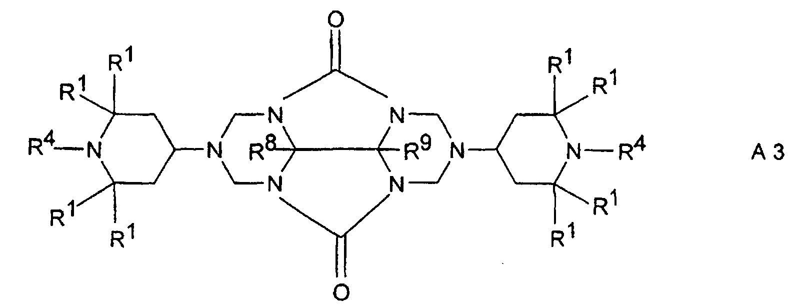 worin
- R¹ und R⁴: die in Anspruch 1 angeführten Bedeutungen haben,
- R⁸ und R⁹: unabhängig voneinander Wasserstoff, C₁-C₆-Alkyl, C₇-C₁₂-Aralkyl, -Aryl oder Carbonester,
- R⁸ und R⁹: zusammen eine Tetra- oder Pentamethylgruppe bedeuten, 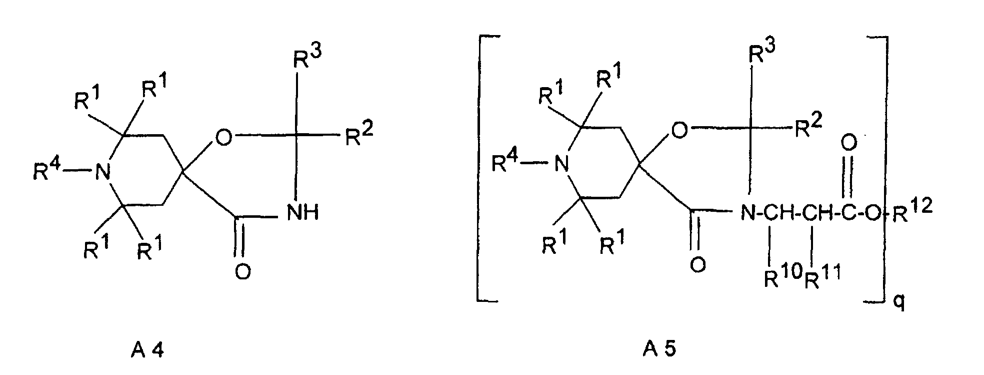
worin
- R¹, R², R³ und R⁴: die in Anspruch 1 angeführten Bedeutungen haben,
- q: eine Zahl von 1 oder 2,
- R¹⁰: Wasserstoff, Methyl, Phenyl oder Carb-C₁-C₂₁-Alkoxy,
- R¹¹: Wasserstoff oder Methyl,
- R¹²: für q = 1, Wasserstoff, C₁-C₂₁-Alkyl, C₂-C₂₂-Alkenyl, C₅-C₁₂-Cycloalkyl, ein Radikal der Formel 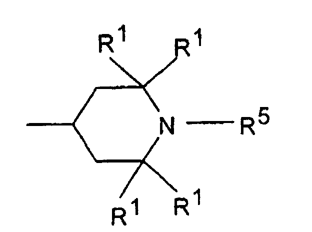
bedeutet,
wobei
- R¹ und R⁵: die in Anspruch 1 angeführte Bedeutung haben, und
- R¹²: für q = 2, C₁-C₁₈-Alkylen, C₅-C₉-Cycloalkylen oder Arylen bedeutet, 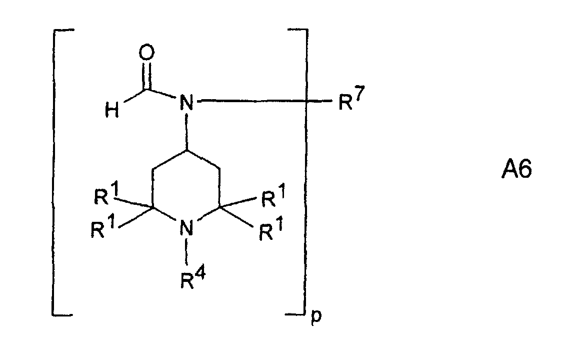 wobei R¹, R⁴, R⁷ und p die oben genannten Definitionen besitzen, 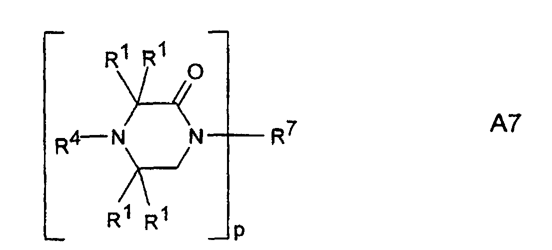 wobei R¹, R⁴, R⁷ und p die oben genannten Definitionen besitzen, 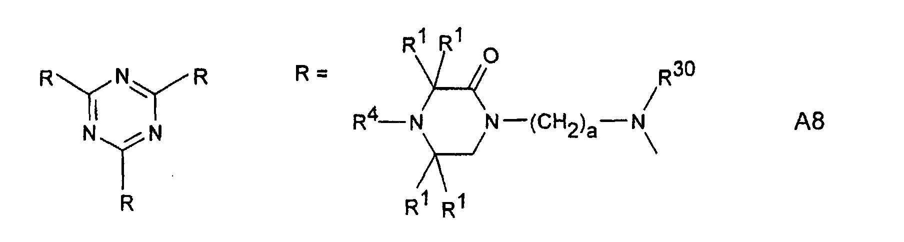
wobei R¹, R⁴ die oben genannten Definitionen besitzen,
- R³⁰: Wasserstoff, C₁-C₁₂-Alkyl, C₅-C₁₂-Cycloalkyl, Phenyl oder C₇-C₉-Phenylalkyl, und
- a: eine Zahl von 1 bis 10 bedeutet, 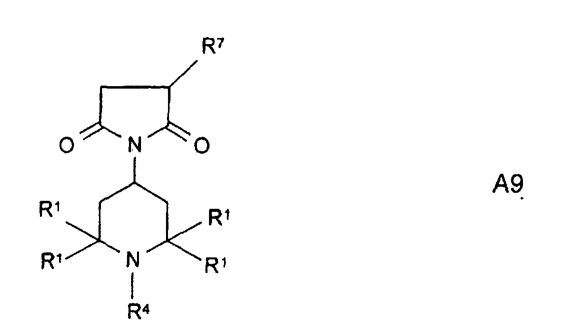
wobei R¹ und R⁴ die obige Bedeutung hat und R⁷ die in der Formel A2 für p=1 definierte Bedeutung hat;
ein Produkt A10 erhältlich durch Umsetzung eines Polyamins der Formel A10a mit Formel A10b: 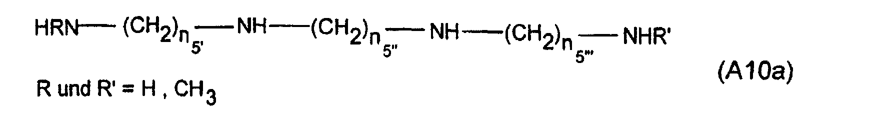 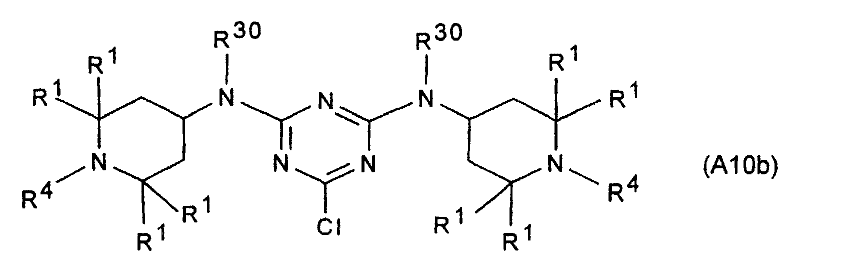 wobei
- R¹,R⁴ und R³⁰: die oben angegebene Bedeutung haben,
- n₅, n_{5"} und n_{5"'}: unabhängig voneinander eine Zahl von 2 bis 12 sind,

Bevorzugt sind Gemische von Verbindungen der Formel (III) mit Verbindungen der Formeln A1 bis A10, worin
n und m unabhängig voneinander eine Zahl von 0 bis 10 sind, wobei n und m nicht gleichzeitig 0 sein können,
R¹ Wasserstoff oder eine C₁-C₄-Alkylgruppe,
R² und R³ unabhängig voneinander ein Wasserstoffatom, eine C₁-C₈-Alkylgruppe oder zusammen mit dem sie verbindenden C-Atom einen Ring der Ringgröße 6 bis 12, oder zusammen mit dem sie bindenden C-Atom eine Gruppe der Formel (IV),
R⁴ und R⁵ unabhängig voneinander entweder Wasserstoff oder eine C₁-C₅-Alkylgruppe, ein Sauerstoffradikal O*, -OH, -NO, -CH₂CN, Benzyl, Allyl, eine C₁-C₁₀-Alkyloxygruppe, eine C₅-C₆-Cycloalkyloxygruppe, eine C₆-C₇-Aryloxygruppe, wobei der Arylrest zusätzlich noch substituiert sein kann; eine C₇-C₁₀-Arylalkyloxygruppe, wobei der Arylrest zusätzlich noch substituiert sein kann, eine C₃-C₆-Alkenylgruppe, eine C₃-C₆-Alkinylgruppe, eine C₁-C₄-Acylgruppe, Halogen, unsubstituiertes oder am Phenylring durch C₁-C₂-Alkyl substituiertes C₇-C₉-Phenylalkyl,
R⁷ ein geradkettiges C₁-C₁₀-Alkylen (für p = 2); C₁-C₁₂-Alkyl (für p = 1),
R⁸ und R⁹ unabhängig voneinander Wasserstoff, C₁-C₂-Alkyl, C₇-C₈-Arylalkyl, Aryl- oder Carbonsäureester,
R¹⁰ Wasserstoff, Methyl, Phenyl oder C₁-C₂-Alkoxy,
R¹¹ Wasserstoff oder Methyl,
R¹² für q = 1 Wasserstoff, C₁-C₁₆-Alkyl, C₂-C₁₆-Alkenyl, C₅-C₆-Cycloalkyl, ein Radikal der Formel 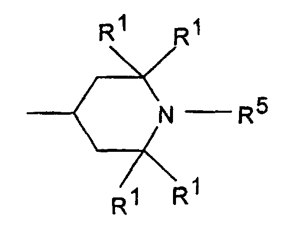 R¹² für q = 2 , C₁-C₁₆-Alkylen, C₅-C₆-Cycloalkylen oder Arylen bedeutet,
R³⁰ Wasserstoff, C₁-C₈-Alkyl, C₅-C₇ - Cycloalkyl, Phenyl oder C₇-C₈-Phenylalkyl,
a 1 bis 5,
o 1 und
p 2 bis 5 bedeutet.

Ganz besonders bevorzugt sind Gemische, worin
n und m unabhängig voneinander eine Zahl von 0-5 ist, wobei n und m nicht gleichzeitig 0 sein können,
R¹ Methyl,
R² und R³ zusammen mit dem sie verbindenden C-Atom einen Ring der Ringgröße 12, oder zusammen mit dem sie bindenden C-Atom eine Gruppe der Formel (IV),
R⁴ und R⁵ unabhängig voneinander Wasserstoff, Methyl, Acetyl, Octyloxy oder Cyctohexyloxy,
R⁶ p-Methoxyphenyl,
R⁷ Octamethylen, Hexamethylen oder Ethylen (für p = 2), Dodecyl (für p =1),
R⁸ und R⁹ Wasserstoff,
R¹⁰ Wasserstoff,
R¹¹ Wasserstoff,
R¹² Dodecamethylen oder Tetradecamethylen,
R³⁰ Cyclohexyl oder n-Butyl,
a gleich 2,
o gleich 1,
p gleich 2
und q gleich 1 ist.

Ganz besonders eignen sich die folgenden Verbindungen in Mischung mit Verbindungen der Formel (III): 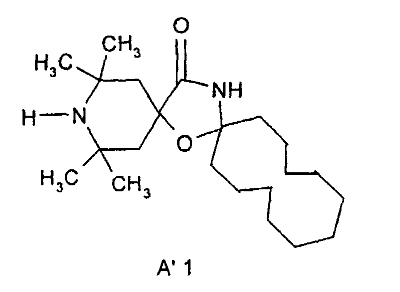 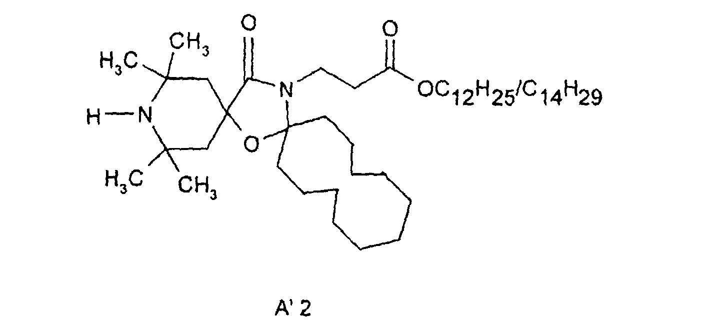 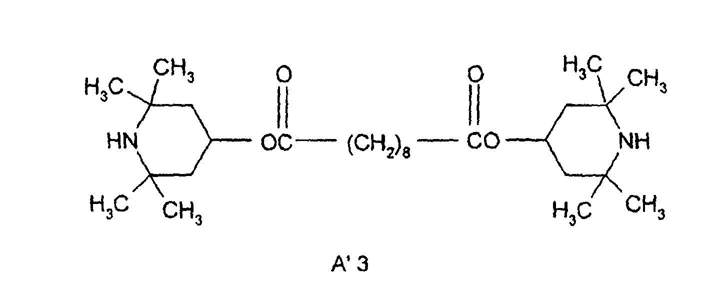 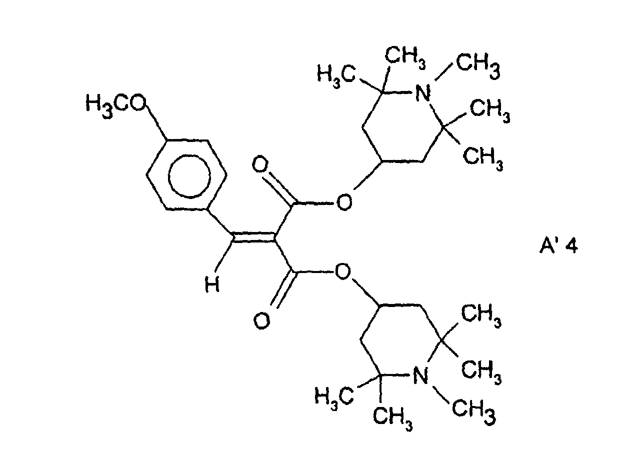 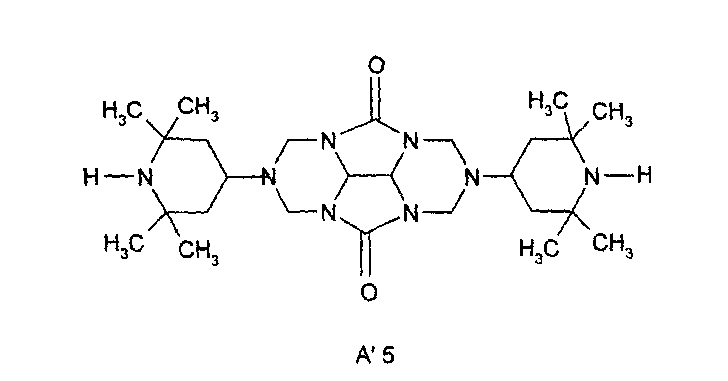 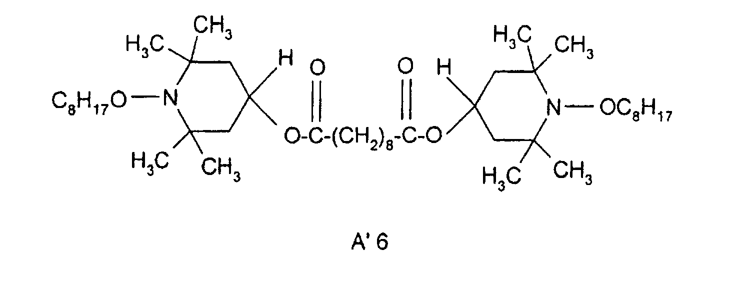 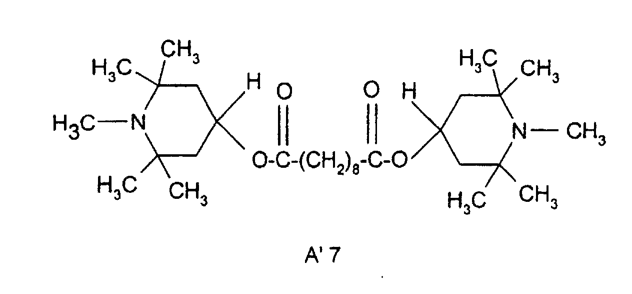 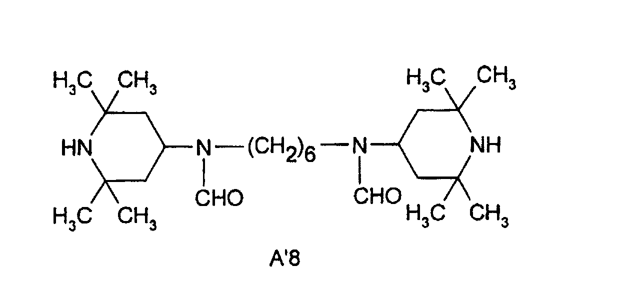 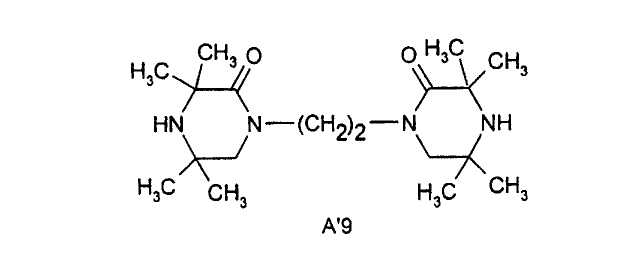 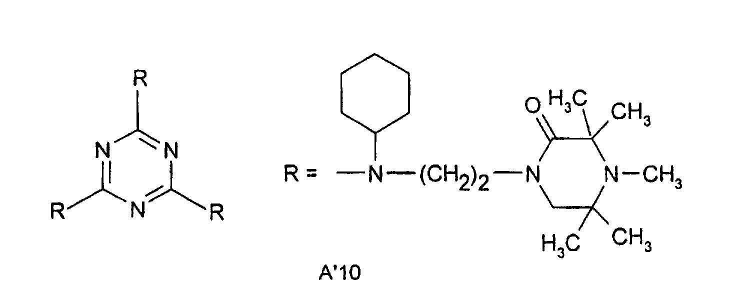 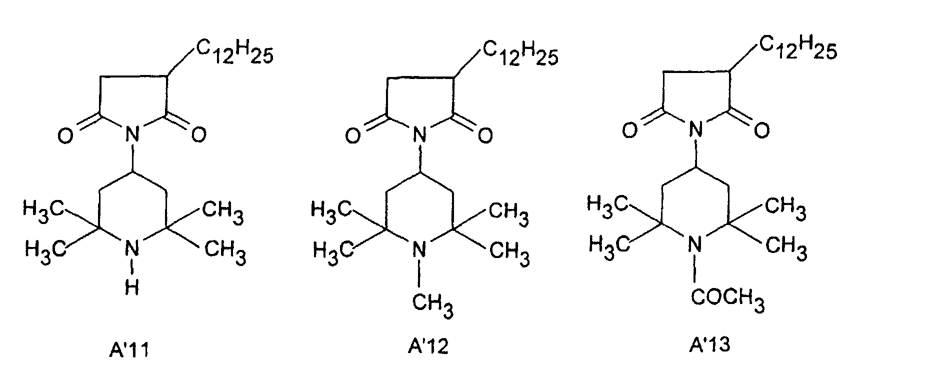 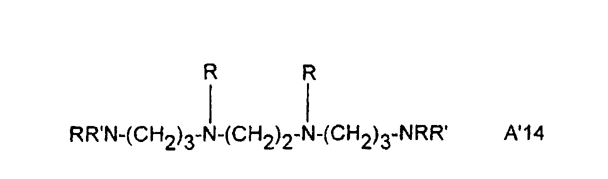 wobei 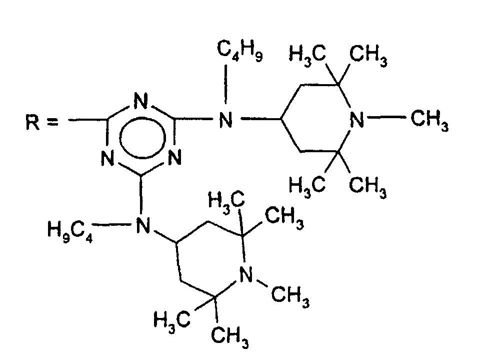 und

R' = H, CH₃

Eine besonders geeignete Ausführungsform der Erfindung sind Gemische aus einer Verbindung der allgemeinen Formel (III) mit einem oder mehreren Stabilisatoren auf Basis sterisch gehinderter Amine, wobei es sich bei der (den) Mischungskomponente(n) um ®Tinuvin 770, ®Tinuvin 765, ®Tinuvin 123, ®Hostavin N 20, ®Hostavin N 24, ®Uvinul 4049, ®Sanduvor PR 31, ®Uvinul 4050, ®Good-rite UV 3034 oder ®Good-rite 3150, ®Sanduvor 3055, ®Sanduvor 3056, ®Sanduvor 3058, ®Chimassorb 119 und ®Chimassorb 905 handelt.

Die erfindungsgemäße Verbindung der allgemeinen Formel (III) kann auch in vorteilhafter Form mit polymeren HALS-Stabilisatoren im Gewichtsverhältnis von 10:1 bis 1:10 angewendet werden. Das Resultat dieser neuen Kombinationen sind Mischungen, welche ein verbessertes Eigenschaftsprofil gegenüber den Einzelkomponenten aufweisen, z. B. synergistische Effekte in der Lichtschutzwirkung zeigen. Kombinationen polymerer HALS-Stabilisatoren werden z. B. in EP-A-252877, EP-A-709426, Research disclosure Jan. 1993 Nr. 34549, EP-A-723990 beschrieben.

Als besonders bevorzugte Ausführungsform in diesem Sinne sind die Kombinationen der Verbindung (III) mit polymeren HALS-Verbindungen der Formeln B1 bis B7 zu verstehen: 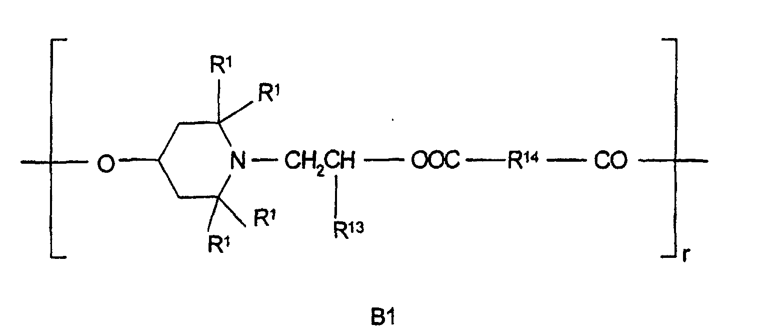 worin
- R¹: Wasserstoff, C₅-C₇-Cycloalkyl oder eine C₁-C₁₂-Alkylgruppe,
- R¹³: Wasserstoff oder Methyl,
- R¹⁴: eine direkte Bindung oder C₁-C₁₀-Alkylen und
- r: eine Zahl von 2 bis 50 bedeutet, 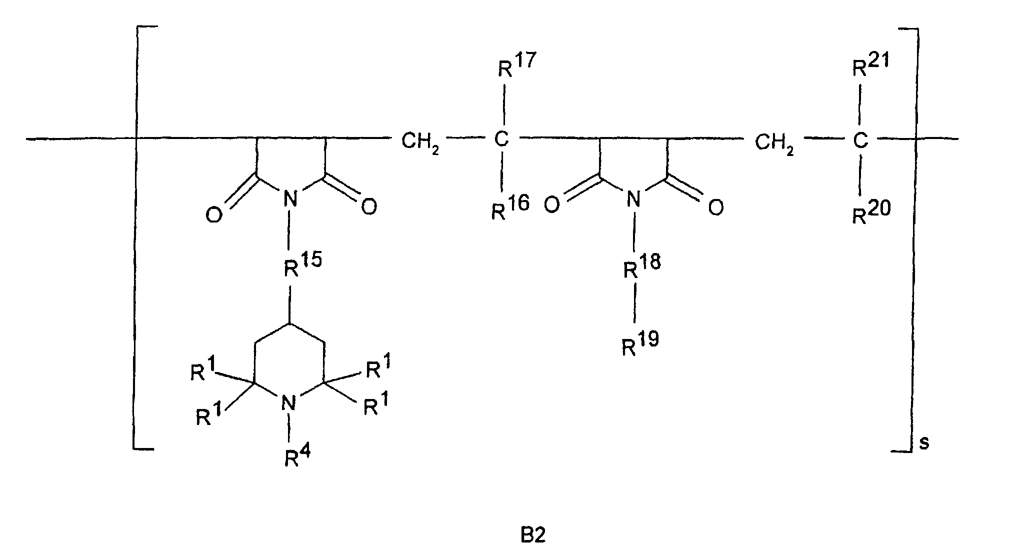
wobei
- R¹ und R⁴: die in Formel (III) angegebenen Bedeutungen haben,
- R¹⁵ und R¹⁸: unabhängig voneinander eine direkte Bindung oder eine Gruppe -N(R²²)-CO- R²³-CO-N(R²⁴)-,
- R²² und R²⁴: unabhängig voneinander Wasserstoff, C₁-C₈-Alkyl, C₅-C₁₂-Cycloalkyl, Phenyl, C₇-C₉-Phenylalkyl, oder eine Gruppe der Formel 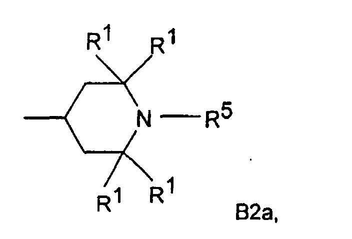
- R²³: eine direkte Bindung oder C₁-C₄-Alkylen,
- R¹⁶, R¹⁷, R²⁰, R²¹: unabhängig voneinander Wasserstoff, C₁-C₃₀-Alkyl, C₅-C₁₂-Cycloalkyl, Phenyl, oder eine Gruppe der Formel B2a,
- R¹⁹: Wasserstoff, C₁-C₃₀-Alkyl, C₅-C₁₂-Cycloalkyl, C₇-C₉-Phenylalkyl, Phenyl oder eine Gruppe der Formel B2a bedeuten und
- s: eine Zahl von 1 bis 50 ist, 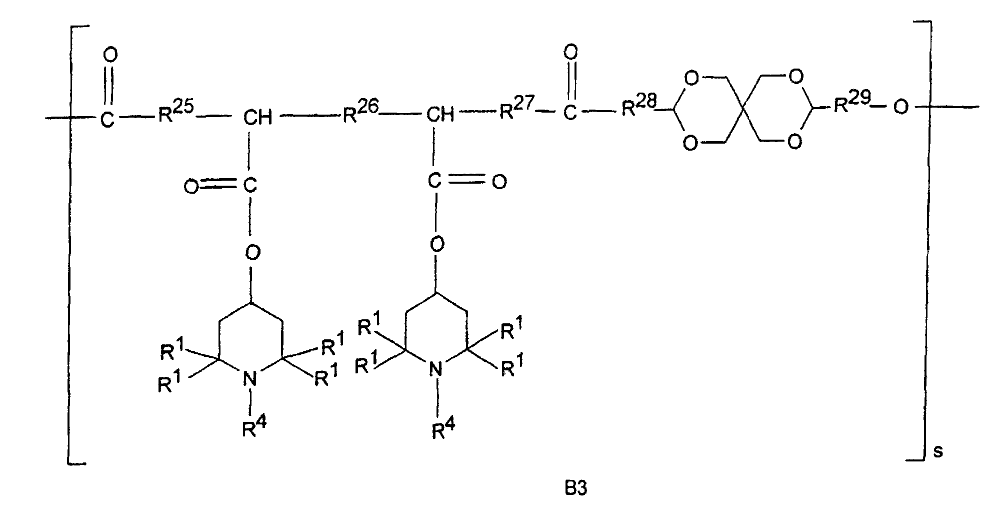
wobei
- R¹, R⁴ und s: die oben angegebenen Bedeutungen haben,
- R²⁵, R²⁶, R²⁷, R²⁸ und R²⁹: unabhängig voneinander eine direkte Bindung oder C₁-C₁₀-Alkylen sind,
ein Produkt B4 erhältlich durch Umsetzung eines durch Reaktion eines Polyamins der Formel B4a mit Cyanursäurechlorid erhaltenen Produktes mit einer Verbindung der Formel B4b, 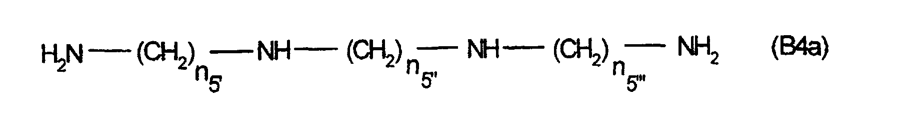 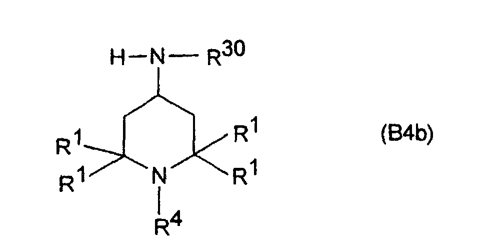 wobei
R¹ und R⁴ die in Formel (III) angegebenen Bedeutungen haben,
n_{5'}, n_{5"} und n_{5"'} unabhängig voneinander eine Zahl von 2 bis 12 sind,
R³⁰ die oben angegebene Bedeutung hat; wobei B4 eine Verbindung der Formel B4-1, B4-2, B4-3 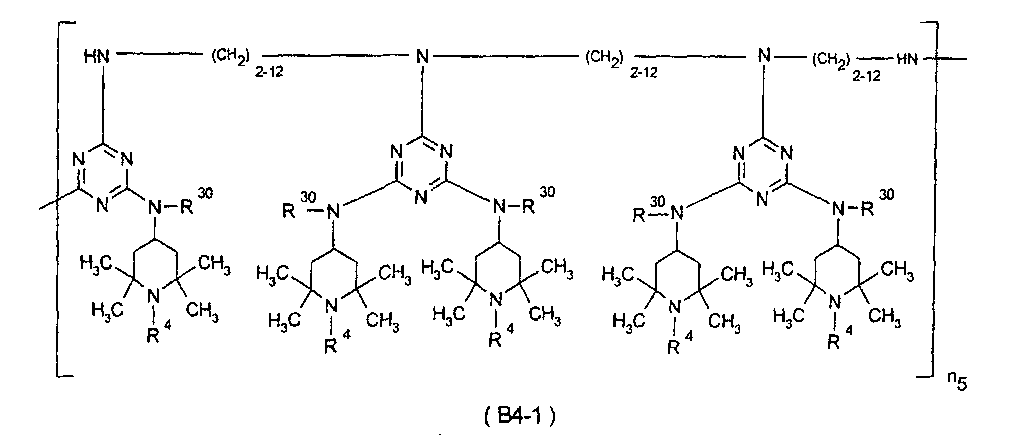 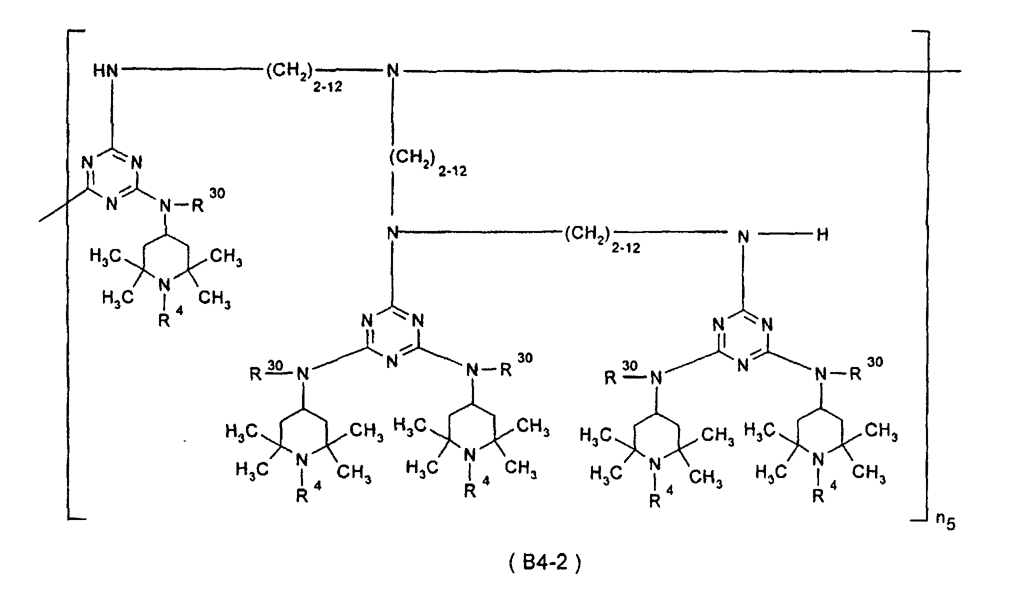 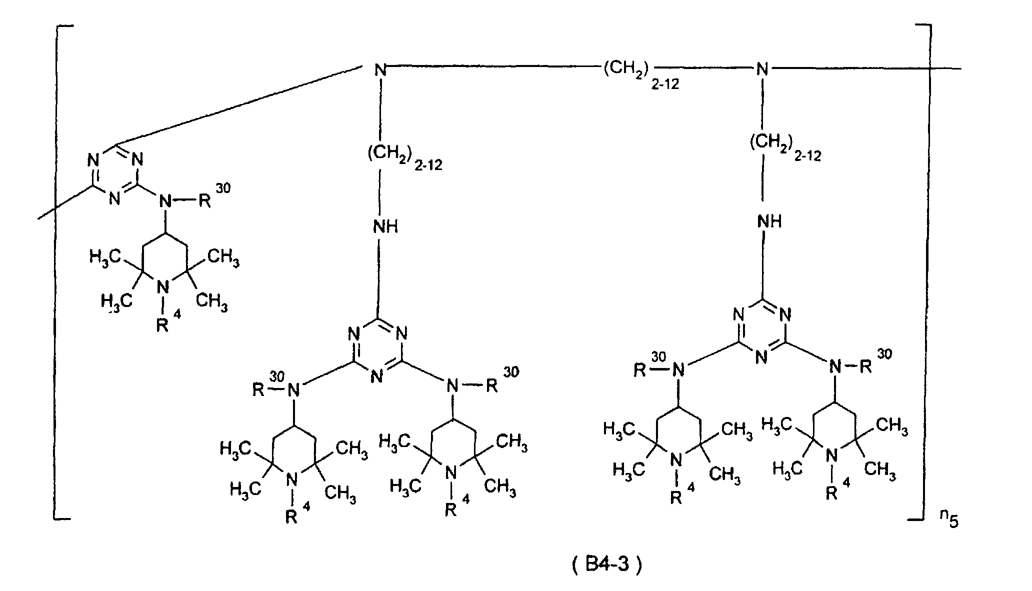 oder ein Gemisch daraus darstellt, worin
- n₅: 1 bis 20,
- R⁴ und R³⁰: die oben angegebene Bedeutung haben, 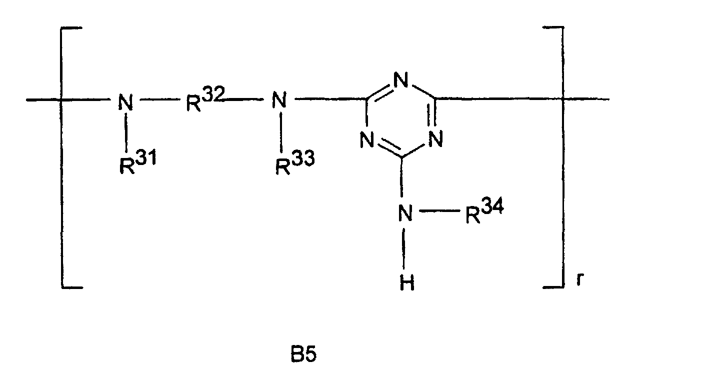
wobei
- r: die bei Formel B1 angegebene Bedeutung hat,
- R³¹, R³³ und R³⁴: xunabhängig voneinander Wasserstoff, C₁-C₁₂-Alkyl, C₅-C₁₂-Cycloalkyl, durch C₁-C₄-Alkyl substituiertes C₅-C₁₂-Cycloalkyl, Phenyl, durch -OH und/oder C₁-C₁₀-Alkyl substituiertes Phenyl, C₇-C₉-Phenylalkyl, am Phenylrest durch -OH und/oder C₁-C₁₀-Alkyl substituiertes C₇-C₉-Phenylkalkyl oder eine Gruppe der Formel B5a bedeuten, 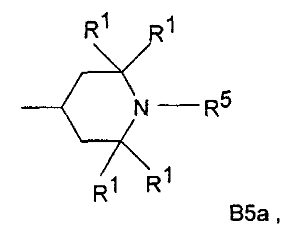
wobei
- R¹ und R⁵: die oben angegebenen Bedeutungen haben, und
- R³²: C₂-C₁₈-Alkylen, C₅-C₇-Cycloalkylen oder C₁-C₄-Alkylendi(C₅-C₇-Cyloalkylen) ist oder die Reste R³¹, R³² und R³³ zusammen mit den Stickstoffatomen, an die sie gebunden sind, einen 5- bis 10-gliedrigen heterocyclischen Ring bilden, und wobei mindestens einer der Reste R³¹, R³³ und R³⁴ eine Gruppe der Formel B5a darstellt; 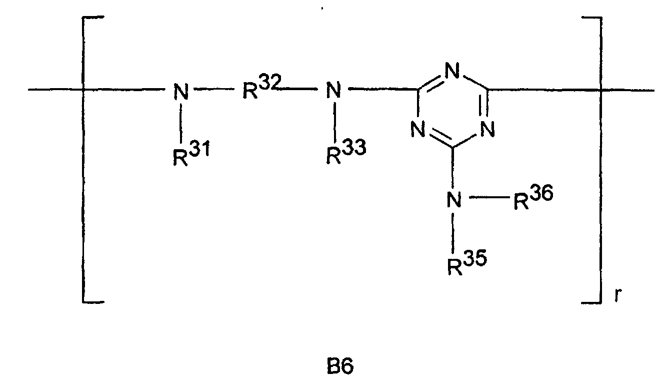
worin
- R³¹, R³², R³³ und r: die oben angegebenen Bedeutungen haben,
- R³⁵ und R³⁶: unabhängig voneinander die Definition von R³⁴ haben kann, oder R³⁵ und R³⁶ zusammen mit dem Stickstoffatom, an das sie gebunden sind, einen 5- bis 10-gliedrigen heterozyklischen Ring bilden, wobei dieser zusätzlich zum Stickstoff-Heteroatom noch ein oder mehrere Heteroatome, vorzugsweise ein Sauerstoffatom, enthalten kann und mindestens einer der Reste R³¹, R³³, R³⁵ und/oder R³⁶ eine Gruppe der Formel (B5a) darstellt, 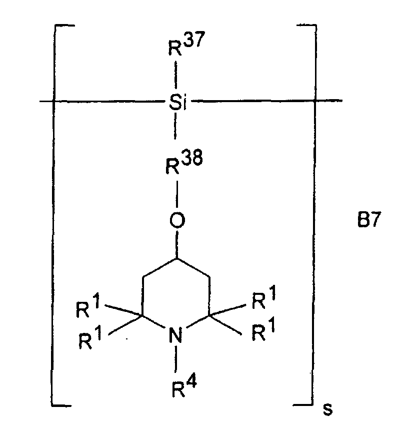
wobei
- R¹ und R⁴: die in Anspruch 1 angegebene Bedeutung haben,
- s: die in Formel B3 angegebenen Bedeutung hat,
- R³⁷: C₁-C₁₀-Alkyl, C₅-C₁₂-Cycloalkyl, durch C₁-C₄-Alkyl substituiertes C₅-C₁₂-Cycloalkyl, Phenyl oder durch C₁-C₁₀-Alkyl substituiertes Phenyl ist, und
- R³⁸: C₃-C₁₀-Alkylen darstellt.

Die als Komponenten B1 bis B4 beschriebenen Verbindungen sind im wesentlichen bekannt (teilweise im Handel erhältlich) und können nach bekannten Verfahren, zum Beispiel wie in US 4,233,412, US 4,340,534, US 4,857,595, DD-A-262 439 (Derwent 89-122 983/17, Chemical Abstracts 111:58 964u), DE-A-4 239 437 (Derwent 94-177 274/22), US 4,529,760, US 4,477,615 und Chemical Abstracts - CAS No. 136 504-96-6 beschrieben, hergestellt werden.

Die Komponente B4 kann in Analogie zu bekannten Verfahren zum Beispiel durch Umsetzung von einem Polyamin der Formel B4a mit Cyanursäurechlorid in einem molaren Verhältnis von 1:2 bis 1:4 in Gegenwart von wasserfreiem Lithium-, Natrium- oder Kaliumcarbonat in einem organischen Lösungsmittel wie 1,2-Dichlorethan, Toluol, Xylol, Benzol, Dioxan oder tert-Amylalkohol bei einer Temperatur von -20°C bis +10°C, bevorzugt -10°C bis +10°C, insbesondere 0°C bis +10°C, während 2 bis 8 Stunden und anschließender Reaktion des erhaltenen Produktes mit einem 2,2,6,6-Tetramethyl-4-piperidylamin der Formel B4b hergestellt werden. Das molare Verhältnis von 2,2,6,6-Tetramethyl-4-piperidylamin zu eingesetztem Polyamin der Formel B4a beträgt beispielsweise 4:1 bis 8:1. Die Menge an 2,2,6,6-Tetramethyl-4-piperidylamin kann auf einmal oder in mehreren Portionen im Abstand von einigen Stunden zugegeben werden.

Bevorzugt beträgt das Verhältnis Polyamin der Formel B4a : Cyanursäurechlorid: 2,2,6,6-Tetramethyl-4-piperidylamin der Formel B4b 1:3:5 bis 1:3:6.

Folgendes Beispiel gibt eine Möglichkeit für die Herstellung der bevorzugten Komponente B4 an.

Beispiel: 23,6 g (0,128 Mol) Cyanursäurechlorid, 7,43 g (0,0426 Mol) N,N'-Bis[3-aminopropyl]ethylendiamin und 18 g (0,13 Mol) wasserfreies Kaliumcarbonat werden in 250 ml 1,2-Dichlorethan bei 5°C unter Rühren während 3 Stunden umgesetzt. Die Mischung wird weitere 4 Stunden auf Raumtemperatur erwärmt. 27,2 g (0,128 Mol) N-(2,2,6,6-tetramethyl-4-piperidyl)butylamin werden hinzugegeben und das erhaltene Gemisch 2 Stunden auf 60°C erwärmt. Es werden nochmals 18 g (0,13 Mol) wasserfreies Kaliumcarbonat hinzugegeben und das Gemisch weitere 6 Stunden bei 60°C erwärmt. Das Lösungsmittel wird unter leichtem Vakuum (200 mbar) abdestilliert und durch Xylol ersetzt. 18,2 g (0,085 Mol) N-(2,2,6,6-tetramethyl-4-piperidyl)butylamin und 5,2 g (0,13 Mol) Natriumhydroxid (ground sodium hydroxide) werden hinzugefügt und das Gemisch am Rückfluß 2 Stunden erhitzt und weitere 12 Stunden wird das bei der Reaktion entstehende Wasser durch azeotrope Destillation entfernt. Das Gemisch wird filtriert. Die Lösung wird mit Wasser gewaschen und über Na₂SO₄ getrocknet. Das Lösungsmittel wird verdunstet und der Rückstand bei 120-130°C im Vakuum (0,1 mbar) getrocknet. Die Komponente B4 wird als farbloses Harz erhalten.

Allgemein kann die Komponente B4 zum Beispiel durch eine Verbindung der Formel B4-1, B4-2 oder B4-3 wiedergegeben werden. Sie kann auch als Gemisch dieser drei Verbindungen vorliegen.

Eine bevorzugte Bedeutung der Formel B4-1 ist

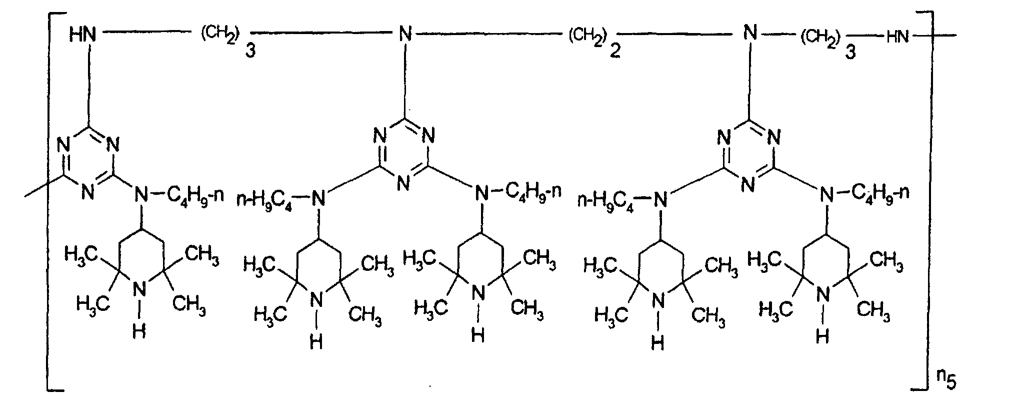

Eine bevorzugte Bedeutung der Formel B4-2 ist

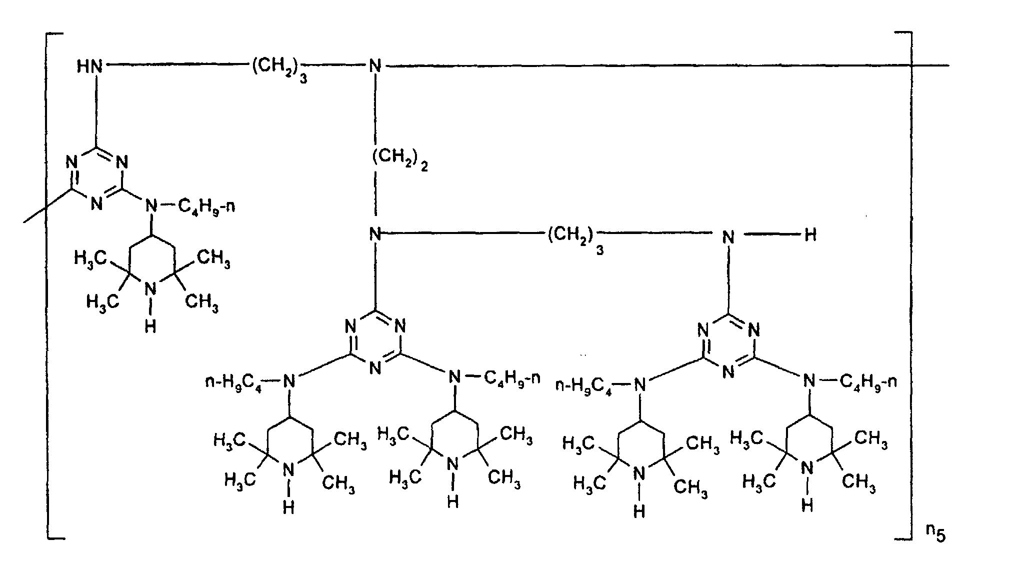

Eine bevorzugte Bedeutung der Formel B4-3 ist

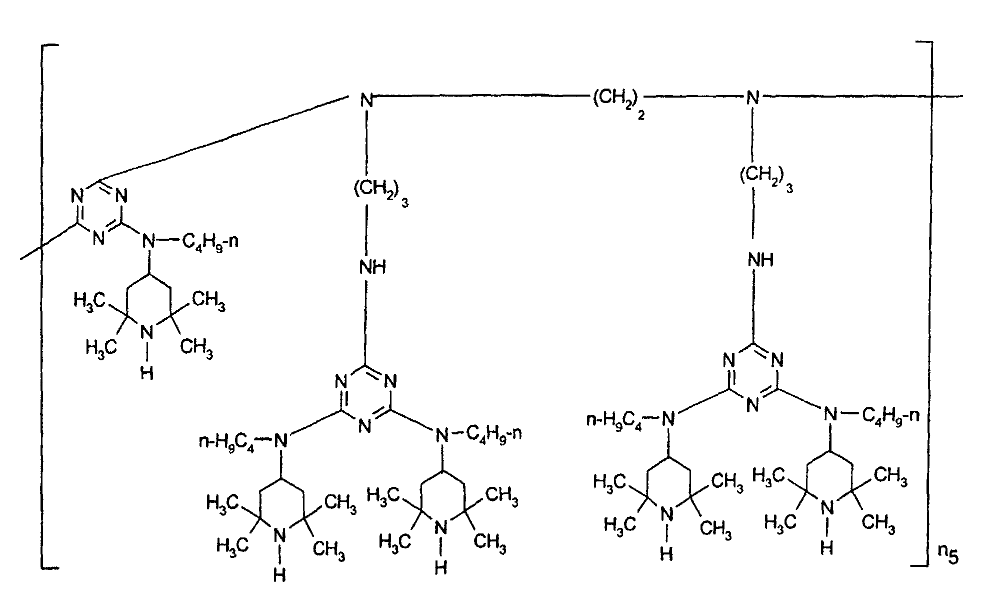

Bevorzugt sind Gemische von Verbindungen der Formel (III) mit Verbindungen der Formeln B1 bis B7, worin n und m unabhängig voneinander eine Zahl von 0 bis 10 sind, wobei n und m nicht gleichzeitig 0 sein können,
- R¹: Wasserstoff oder eine C₁-C₄-Alkylgruppe,
- R² und R³: unabhängig voneinander ein Wasserstoffatom, eine C₁-C₈- Alkylgruppe oder zusammen mit dem sie verbindenden C-Atom einen Ring der Ringgröße 6 bis 12, oder zusammen mit dem sie bindenden C-Atom eine Gruppe der Formel (IV),
- R⁴ und R⁵: unabhängig voneinander entweder Wasserstoff oder eine C₁-C₅-Alkylgruppe, ein Sauerstoffradikal O*, -OH, -NO, -CH₂CN, Benzyl, Allyl, eine C₁-C₁₀-Alkyloxygruppe, eine C₅-C₆-Cycloalkyloxygruppe, eine C₆-C₇-Aryloxygruppe, wobei der Arylrest zusätzlich noch substituiert sein kann, eine C₇-C₁₀-Arylalkyloxygruppe, wobei der Arylrest zusätzlich noch substituiert sein kann, eine C₃-C₆-Alkenylgruppe, eine C₃-C₆-Alkinylgruppe, eine C₁-C₄-Acylgruppe, Halogen, unsubstituiertes oder am Phenylring durch C₁-C₂-Alkyl substituiertes C₇-C₉-Phenylalkyl bedeuten,
- R¹³: Wasserstoff oder Methyl,
- R¹⁴: C₁-C₅-Alkylen,
- R¹⁷, R²¹: Wasserstoff oder C₁-C₄-Alkyl,
- R¹⁵, R¹⁸: eine direkte Bindung,
- R¹⁶, R²⁰: C₁-C₂₅-Alkyl, Phenyl,
- R¹⁹: Wasserstoff, C₁-C₁₂-Alkyl oder eine Gruppe der Formel B2a
- R²⁵, R²⁶, R²⁷, R²⁸ und R²⁹: unabhängig voneinander eine direkte Bindung oder C₁-C₅-Alkylen,
- R³⁰: Wasserstoff, C₁-C₄-Alkyl, C₅-C₆-Cycloalkyl, Phenyl,
- R³¹, R³³ und R³⁴: unabhängig voneinander Wasserstoff, C₁-C₁₀-Alkyl, C₅-C₆-Cycloalkyl oder eine Gruppe der Formel B5a bedeutet,
- R³²: C₂-C₁₀-Alkylen, C₅-C₆-Cycloalkylen,
- R³⁵ und R³⁶: unabhängig voneinander die Definition von R³⁴ , oder R³⁵ und R³⁶ zusammen mit dem N-Atom, an das sie gebunden sind einen 5 bis 7-gliedrigen heterocyclischen Ring bilden, wobei dieser noch ein oder mehrere Heteroatome, vorzugsweise ein O-Atom enthalten kann und mindestens einer der Reste R³¹,R³³,R³⁵ und/oder R³⁶ eine Gruppe der Formel B5a darstellt,
- R³⁷: C₁-C₅-Alkyl, C₅-C₆-Cycloalkyl oder Phenyl,
- R³⁸: C₃-C₅-Alkylen und
- n_{5'},n_{5"},n_{5"'}: 2 bis 4 bedeuten.

Ganz besonders bevorzugt sind Gemische, worin
- n und m: unabhängig voneinander eine Zahl von 0 bis 5 sind, wobei n und m nicht gleichzeitig 0 sein können,
- R¹: Methyl,
- R² und R³: zusammen mit dem sie verbindenden C-Atom einen Ring der Ringgröße 12, oder zusammen mit dem sie bindenden C-Atom eine Gruppe der Formel (IV),
- R⁴ und R⁵: unabhängig voneinander Wasserstoff, Acetyl, Methyl, Octyloxy oder Cyclohexyloxy,
- R¹³: Wasserstoff,
- R¹⁴: Ethylen,
- R¹⁷, R²¹: Wasserstoff oder Methyl,
- R¹⁵, R¹⁸: eine direkte Bindung,
- R¹⁶, R²⁰: C₁-C₂₅-Alkyl, Phenyl,
- R¹⁹: Hexadecyl oder eine Gruppe der Formel B2a,
- R²⁵, R²⁷: Methylen,
- R²⁶: eine direkte Bindung,
- R²⁸: 2,2-Dimethylethylen,
- R²⁹: 1,1-Dimethylethylen,
- R³⁰: n-Butyl,
- R³¹, R³³ und R³⁴: unabhängig voneinander Isooctyl, Cyclohexyl oder 2,2,6,6-Tetramethylpiperid-4-yl bedeuten, wobei mindestens einer der Reste R³¹, R³³ und R³⁴ 2,2,6,6-Tetramethylpiperid-4-yl bedeuten muß,
- R³²: Hexamethylen,
- R³⁵ und R³⁶: unabhängig voneinander die Definition von R³⁴, oder R³⁵ und R³⁶ zusammen mit dem N-Atom, an das sie gebunden sind einen 6-gliedrigen heterocyclischen Ring bilden, wobei dieser noch ein Sauerstoffatom enthält und folglich Morpholin ist, wobei mindestens einer der Reste R³¹,R³³,R³⁵ und/oder R³⁶ einen Rest 2,2,6,6-Tetramethylpiperid-4-yl bedeuten muß,
- R³⁷: Methyl,
- R³⁸: Trimethylen,
- n_{5'},n_{5"},n_{5"'}: 2 bis 4 bedeuten.

Ganz besonders bevorzugt sind Gemische, worin es sich bei den polymeren HALS-Verbindungen in Kombination mit Verbindungen der Formel (III) um die folgenden Substanzen handelt: 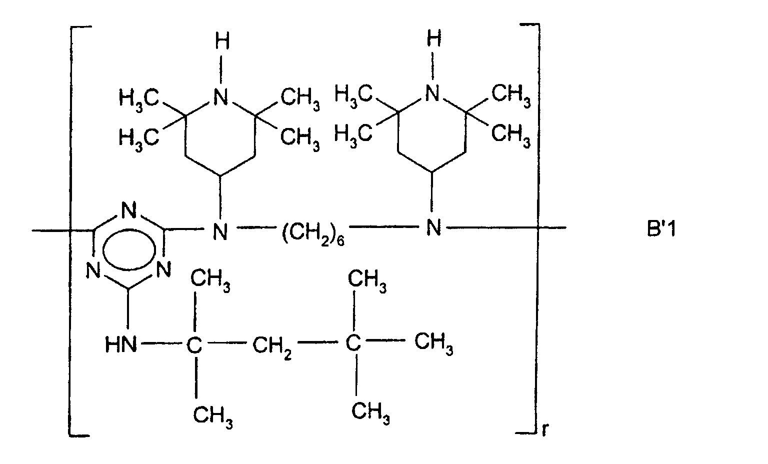 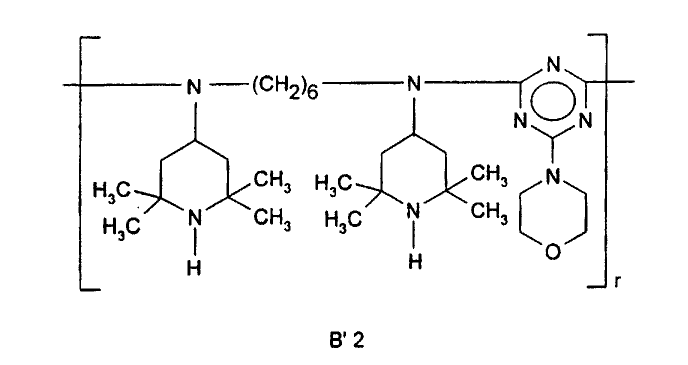 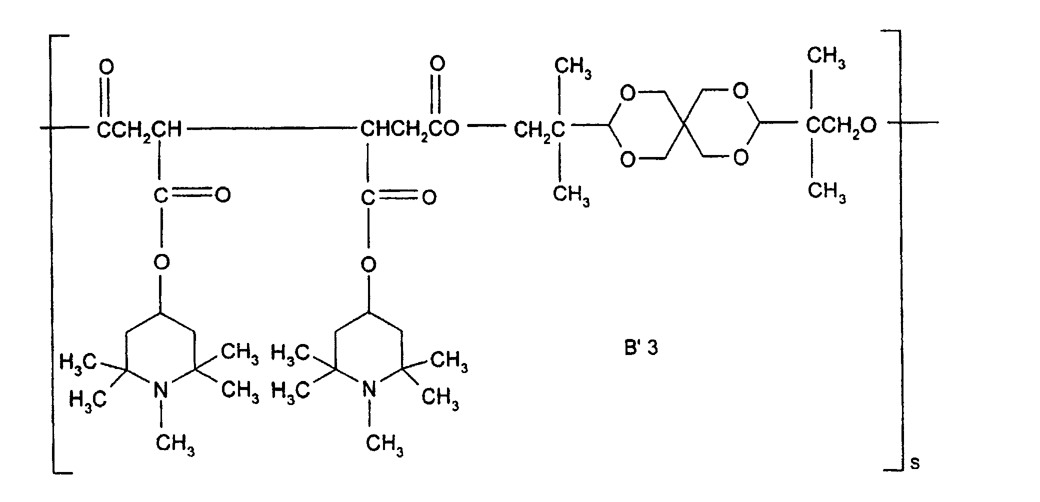 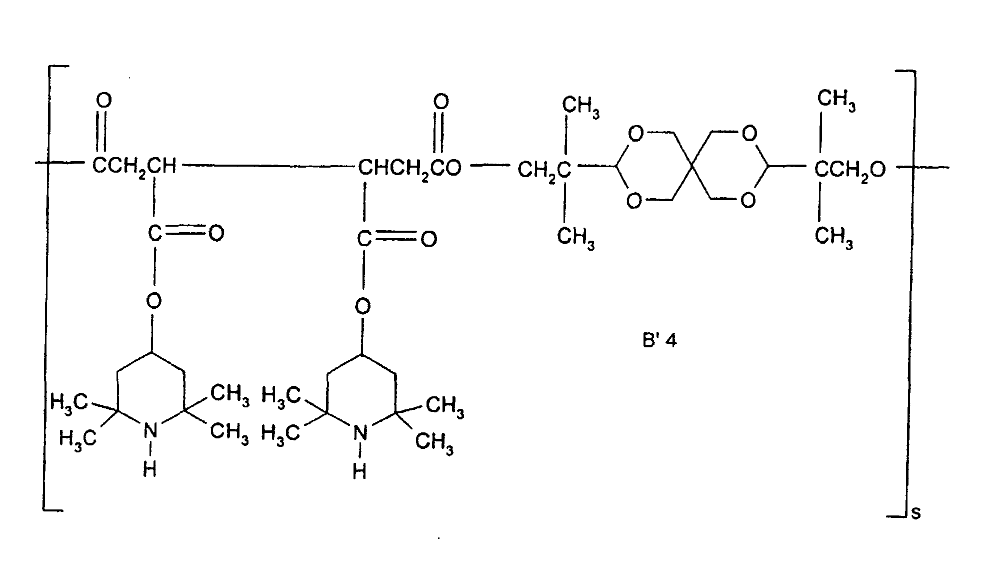 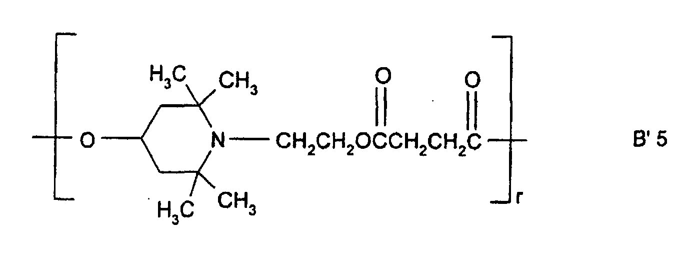 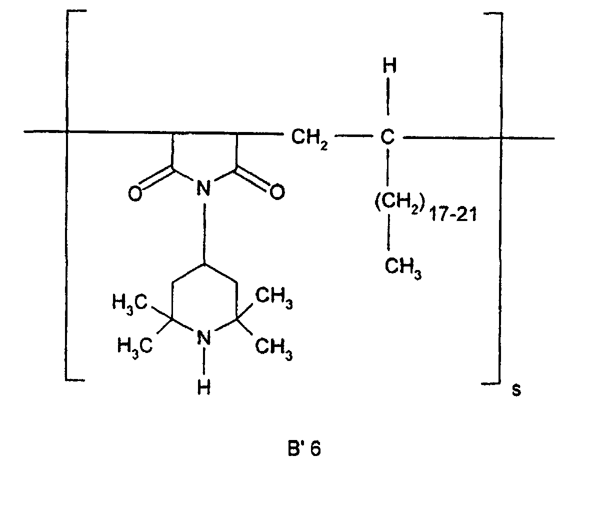 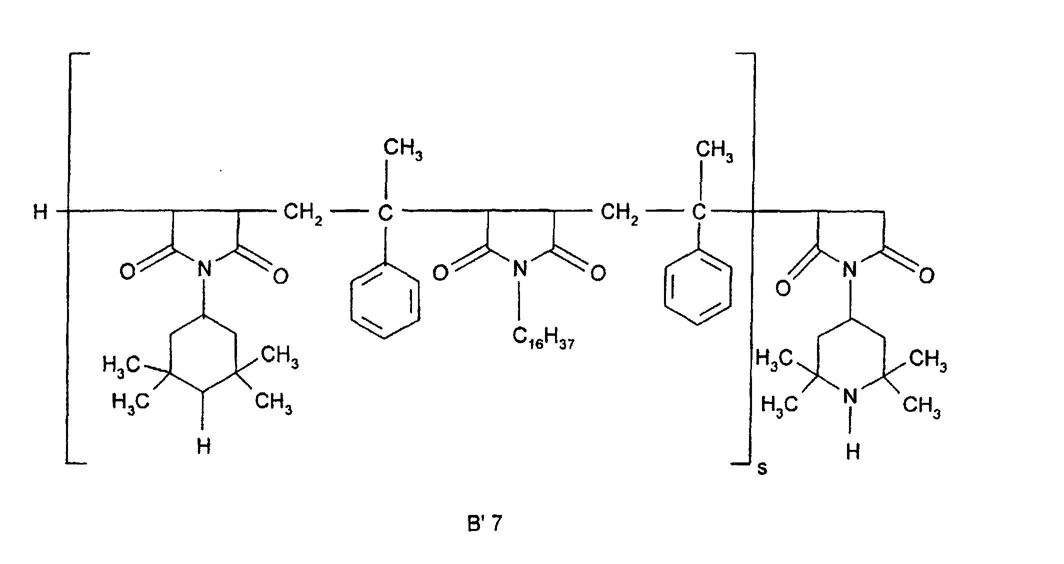 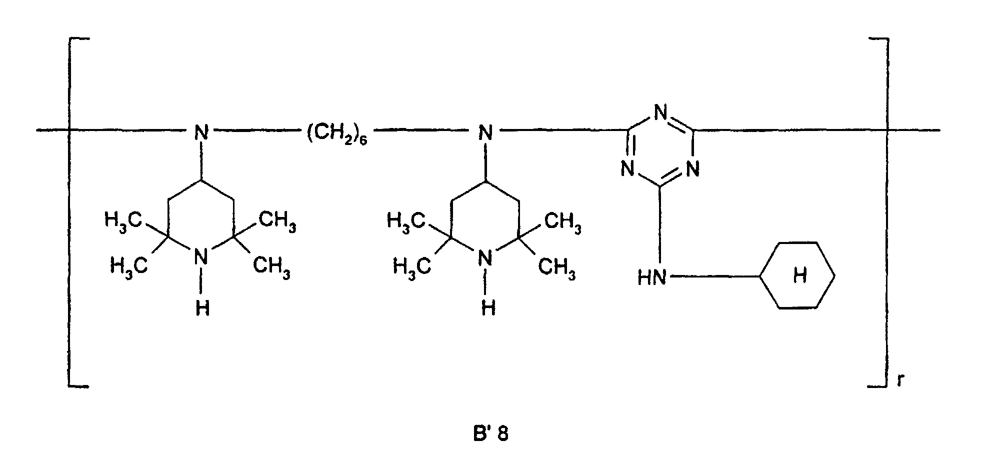 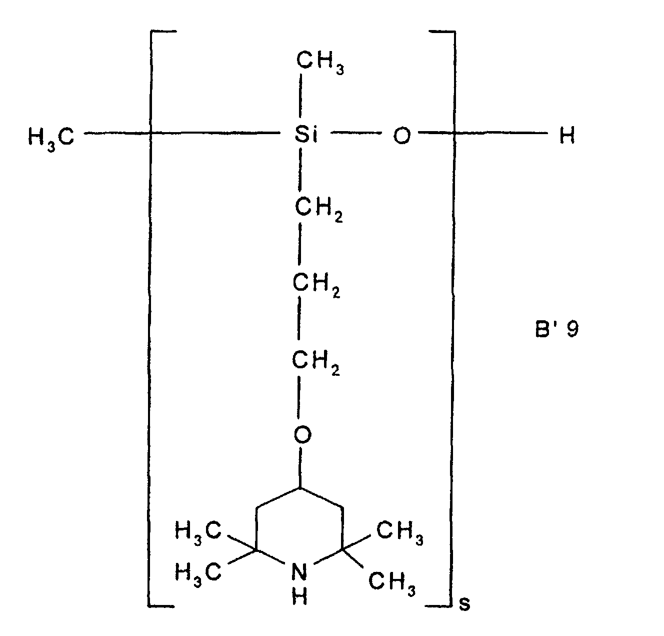 ein Produkt B'10 erhältlich durch Umsetzung eines durch Reaktion eines Polyamins der Formel B'10a:

H₂N-(CH₂)₃-NH-(CH₂)₂-NH-(CH₂)₃-NH₂ (B'10a)

mit Cyanursäurechlorid erhaltenen Produktes mit einer Verbindung der Formel (B'10b) 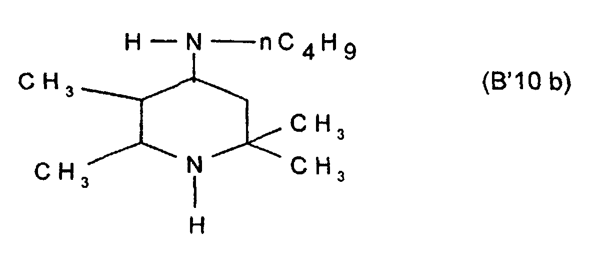 wobei B'10 eine Verbindung der Formel B4-1', B4-2', B4-3' 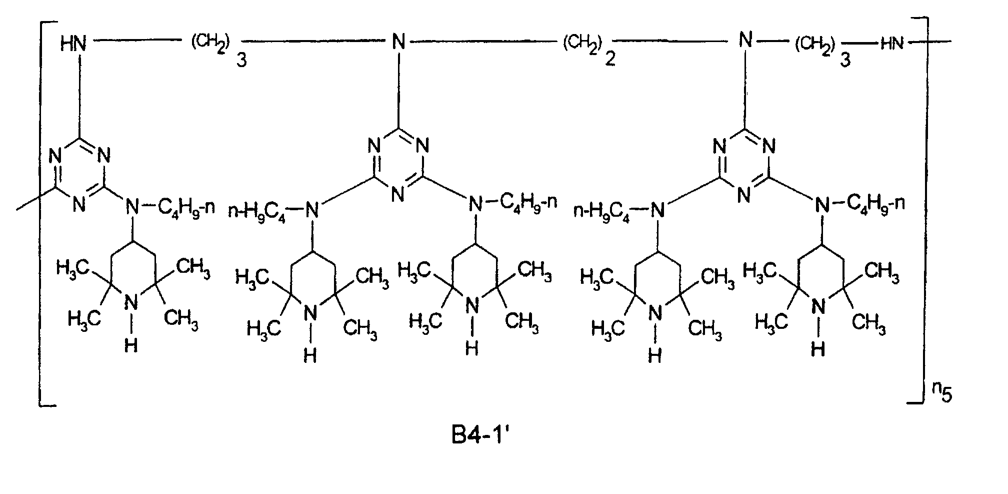 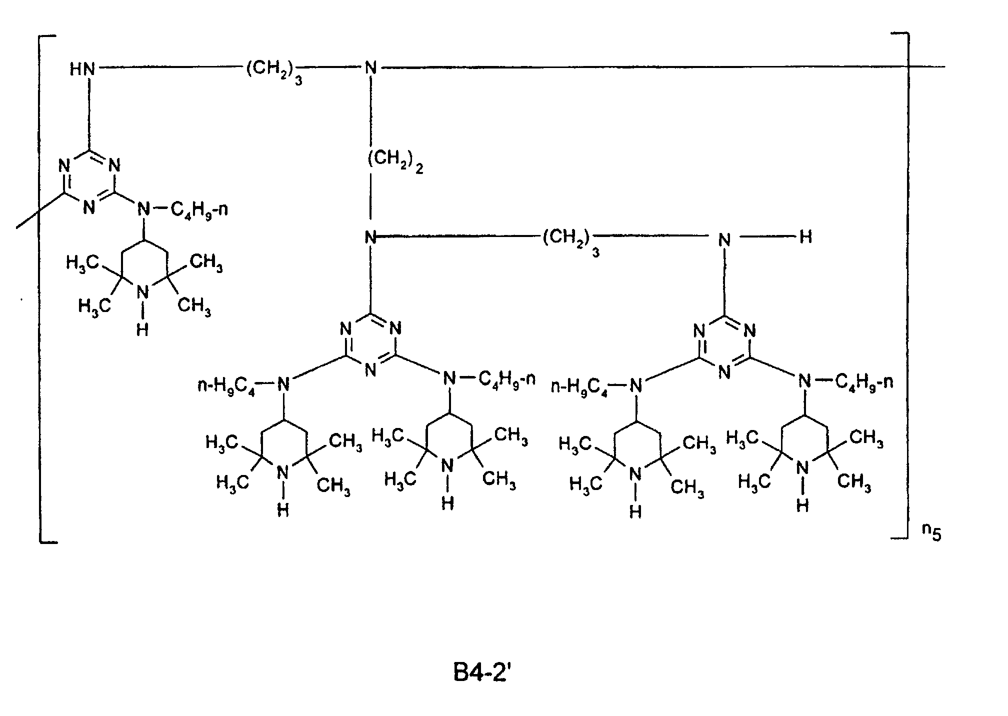 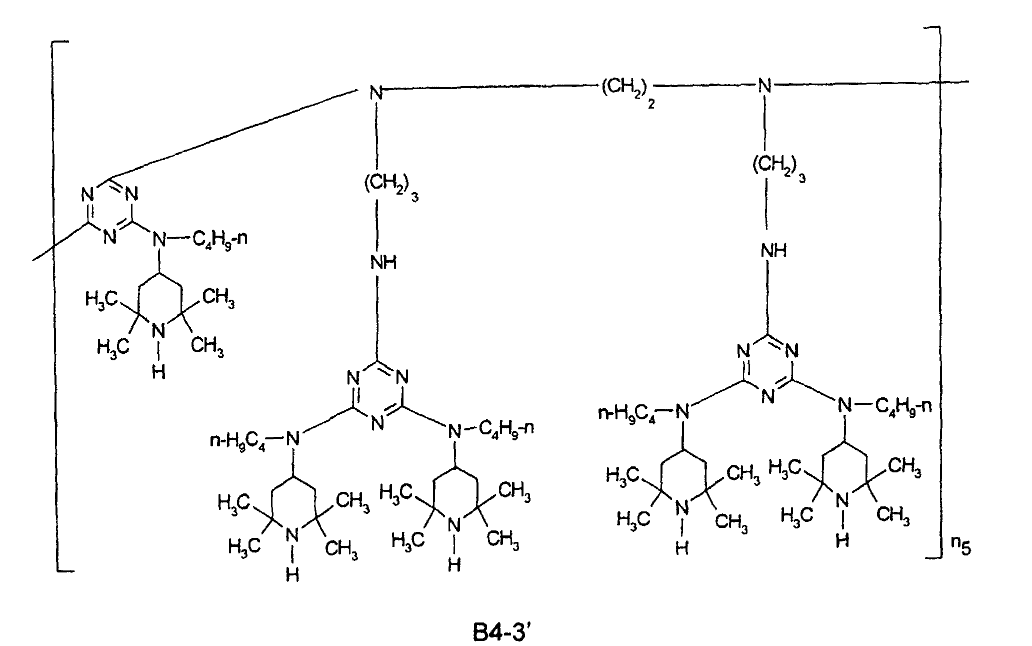 oder ein Gemisch daraus bedeutet, wobei n₅ 1 bis 20 bedeutet.

Besonders bevorzugt sind vorstehend beschriebene Gemische, bei denen es sich bei der (den) übrigen Mischungskomponente(n) um ®Chimassorb 944, ®Tinuvin 622, ®Dastib 1082, ®Uvasorb HA 88, ®Uvinul 5050, ®Lowilite 62, ®Uvasil 299, ®Cyasorb 3346, ®MARK LA 63, ®MARK LA 68 oder ®Luchem B 18 handelt.

Überraschenderweise konnte festgestellt werden, daß die gleichzeitige Verwendung der neuen Verbindungen (III) und der oben beschriebenen monomeren oder polymeren HALS-Stabilisatoren auffällige synergistische Effekte zeigt.

Besonders vorteilhaft ist auch die Kombination von Verbindung (III) mit Phosphiten in dem Sinne, daß der Stabilisator (III) die hydrolytische Zersetzung des Phosphits verringert oder unterdrückt, wie in EP-A-400454, EP-A-592364, EP-A-143464, EP-A-576833, EP-A-558040, EP-A-278578, EP-A-676405, DE-A-4418080. Besonders geeignet ist Verbindung (III) für die Stabilisierung von Phosphiten der Formeln C1 bis C7: 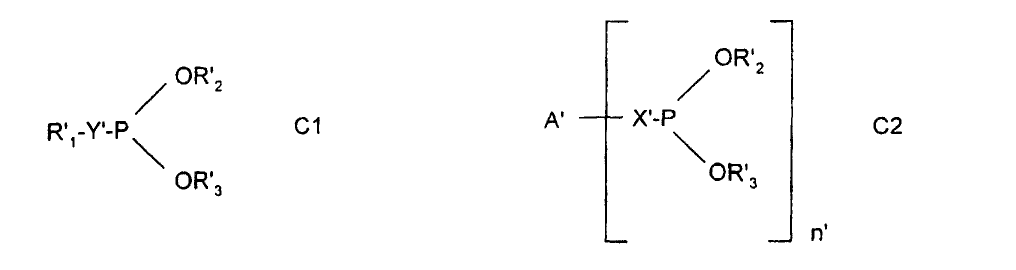 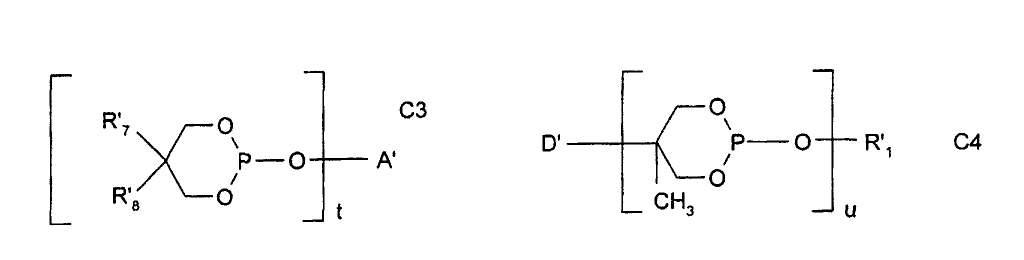 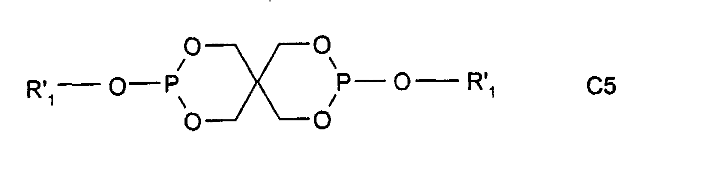 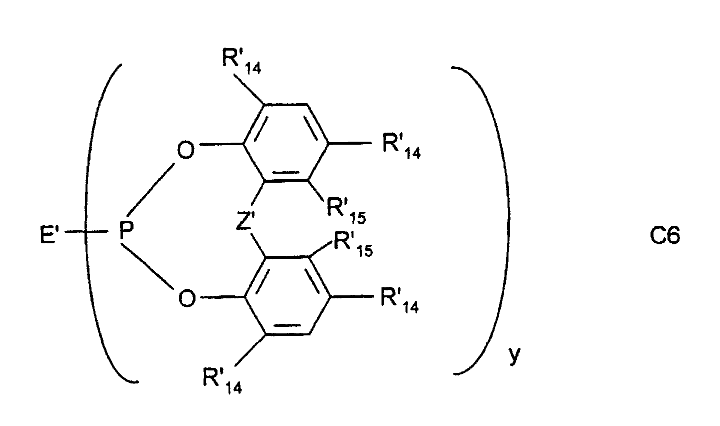 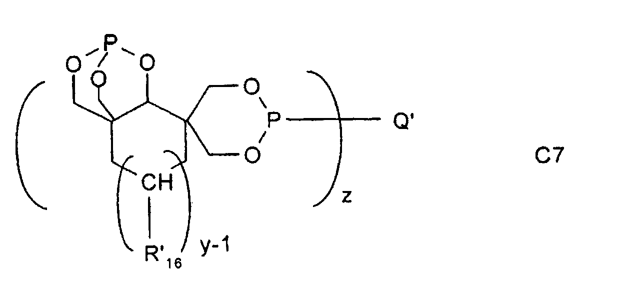 worin die Indices ganzzahlig sind und
n' für 2, 3 oder 4; u für 1 oder 2; t für 2 oder 3; y für 1, 2 oder 3; und z für 1 bis 6 steht; A', wenn n' 2 ist, Alkylen mit 2 bis 18 Kohlenstoffatomen; durch -S-, -O- oder - NR'₄- unterbrochenes Alkylen mit 2 bis 12 Kohlenstoffatomen; ein Rest einer der Formeln 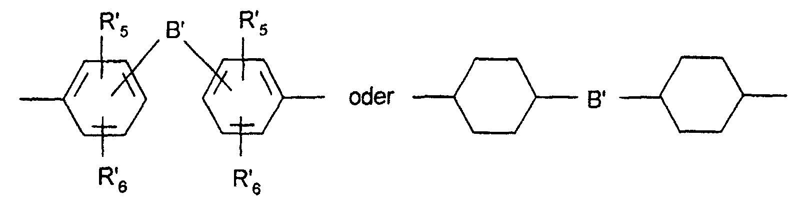 oder Phenylen ist;
A', wenn n' 3 ist, ein Rest der Formel -CᵣH₂ᵣ₋₁ ist;
A', wenn n' 4 ist, den Rest der Formel 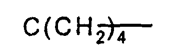 bedeutet;
A" die Bedeutung von A', wenn n'2 ist, hat;
B' einen Rest der Formel -CH₂-; -CHR'₄-; -CR',R'₄-; -S- oder eine direkte Bindung darstellt; oder C₅-C₇-Cycloalkyliden; oder mit 1 bis 4 C₁-C₄-Alkylresten in Position 3, 4 und/oder 5 substituierters Cyclohexyliden bedeutet,
D', wenn u 1 ist, Methyl und, wenn u 2 ist, -CH₂OCH₂- bedeutet;
E', wenn y 1 ist, Alkyl mit 1 bis 18 Kohlenstoffatomen, Phenyl, ein Rest der Formel -OR'₁ oder Halogen ist;
E', wenn y 2 ist, ein Rest der Formel -O-A"-O- ist;
E', wenn y 3 ist, ein Rest der Formel 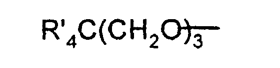 oder N(CH₂-CH₂-O-)₃ ist;
Q' für den Rest eines mindestens z-wertigen Alkohols oder Phenols steht, wobei dieser über das (die) alkoholische(n) bzw. phenolische(n) O-Atom(e) an das (die) P-Atom(e) gebunden ist;
R'₁, R'₂ und R'₃ unabhängig voneinander Alkyl mit 1 bis 30 Kohlenstoffatomen; mit Halogen, -COOR₄', -CN oder -CONR₄'R₄' substituiertes Alkyl mit 1 bis 18 Kohlenstoffatomen; durch -S-, -O- oder -NR'₄- unterbrochenes Alkyl mit 2 bis 18 Kohlenstoffatomen; Phenyl-C₁-C₄-alkyl; Cycloalkyl mit 5 bis 12 Kohlenstoffatomen; Phenyl oder Naphthyl; mit Halogen, 1 bis 3 Alkylresten oder Alkoxyresten mit insgesamt 1 bis 18 Kohlenstoffatomen oder mit Phenyl-C₁-C₄-Alkyl substituiertes Phenyl oder Naphthyl; oder ein Rest der Formel 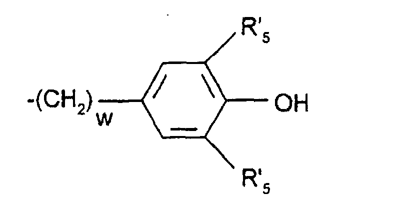 sind, worin w eine ganze Zahl aus dem Bereich 3 bis 6 bedeutet;
R'₄ beziehungsweise die Reste R'₄ unabhängig voneinander Wasserstoff, Alkyl mit 1 bis 18 Kohlenstoffatomen, Cycloalkyl mit 5 bis 12 Kohlenstoffatomen, oder Phenylalkyl mit 1 bis 4 Kohlenstoffatomen im Alkylteil;
R'₅ und R'₆ unabhängig voneinander Wasserstoff, Alkyl mit 1 bis 8 Kohlenstoffatomen oder Cycloalkyl mit 5 oder 6 Kohlenstoffatomen sind;
R'₇ und R'₈, im Fall t = 2 unabhängig voneinander C₁-C₄-Alkyl oder zusammen einen 2,3-Dehydropentamethylenrest darstellen; und
R'₇ und R'₈ im Fall t = 3, Methyl bedeuten;
die Substituenten R'₁₄ unabhängig voneinander Wasserstoff, Alkyl mit 1 bis 9 Kohlenstoffatomen oder Cyclohexyl sind;
die Substituenten R'₁₅ unabhängig voneinander Wasserstoff oder Methyl; und
R'₁₆ Wasserstoff oder C₁-C₄-Alkyl darstellt und im Fall, daß mehrere Reste R'₁₆ vorhanden sind, die Reste R'₁₆ gleich oder verschieden sind;
X' und Y' jeweils eine direkte Bindung oder -O- darstellen; und
Z eine direkte Bindung; -CH₂-; -C(R'₁₆)₂- oder -S- ist.

Besonders bevorzugt sind Phosphite oder Phosphonite der Formeln C1, C2, C5 oder C6, worin
n' für die Zahl 2 und y für die Zahl 1 oder 2 steht;
A' Alkylen mit 2 bis 18 Kohlenstoffatomen; p-Phenylen oder p-Biphenylen ist;
E' im Fall y = 1 C₁-C₁₈-Alkyl, -OR₁ oder Fluor; und im Fall y = 2 p-Biphenylen ist;
R'₁, R'₂ und R'₃ unabhängig voneinander Alkyl mit 1 bis 18 Kohlenstoffatomen; Phenyl-C₁-C₄-Alkyl; Cyclohexyl; Phenyl; mit 1 bis 3 Alkylresten mit insgesamt 1 bis 18 Kohlenstoffatomen substituiertes Phenyl bedeutet;
die Substituenten R'₁₄ unabhängig voneinander Wasserstoff oder Alkyl mit 1 bis 9 Kohlenstoffatomen sind;
R'₁₅ Wasserstoff oder Methyl ist;
X' eine direkte Bindung;
Y'-O-; und
Z' eine direkte Bindung oder -CH(R'₁₆)- ist.

Ganz besonders bevorzugt sind Phosphite oder Phosphonite einer der Formeln C1, C2, C5 oder C6 worin
n' für die Zahl 2 und y für die Zahl 1 steht;
A' p-Biphenylen ist;
E' C₁-C₁₈-Alkoxy ist;
R'₁, R'₂ und R'₃ unabhängig voneinander mit 2 oder 3 Alkylresten mit insgesamt 2 bis 12 Kohlenstoffatomen substitutiertes Phenyl bedeutet;
die Substituenten R'₁₄ unabhängig voneinander Methyl oder tert.-Butyl sind;
R'₁₅ Wasserstoff ist;
X' eine direkte Bindung;
Y' -O-; und
Z' eine direkte Bindung, -CH₂- oder -CH(CH₃)- ist.

Im Speziellen sind die konkreten Phosphorverbindungen der Formeln C'1 bis C'12 zu nennen:

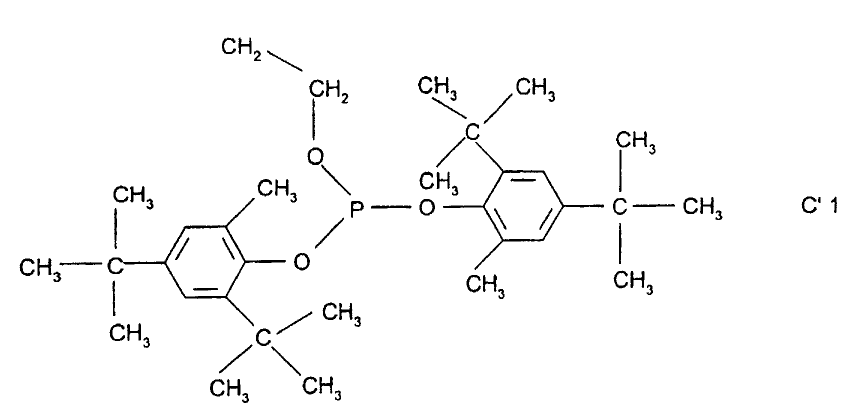

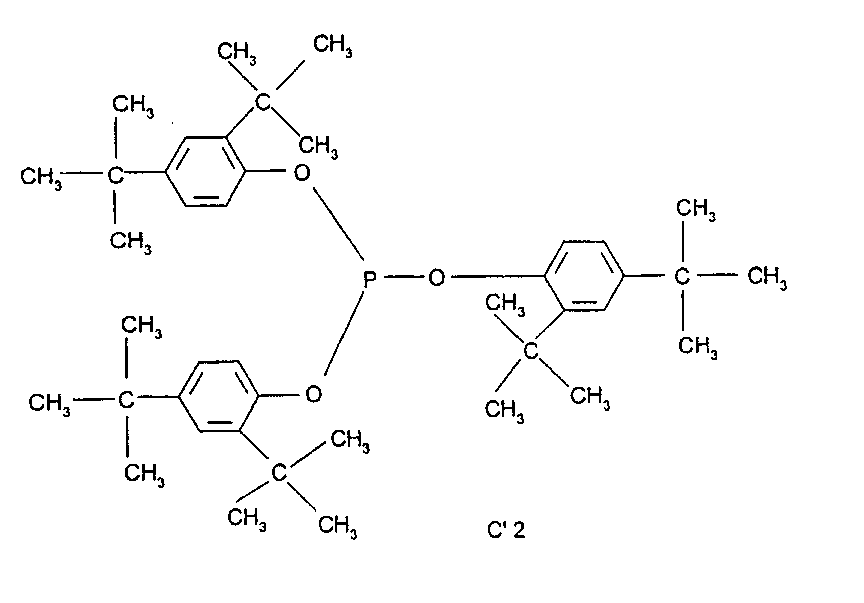

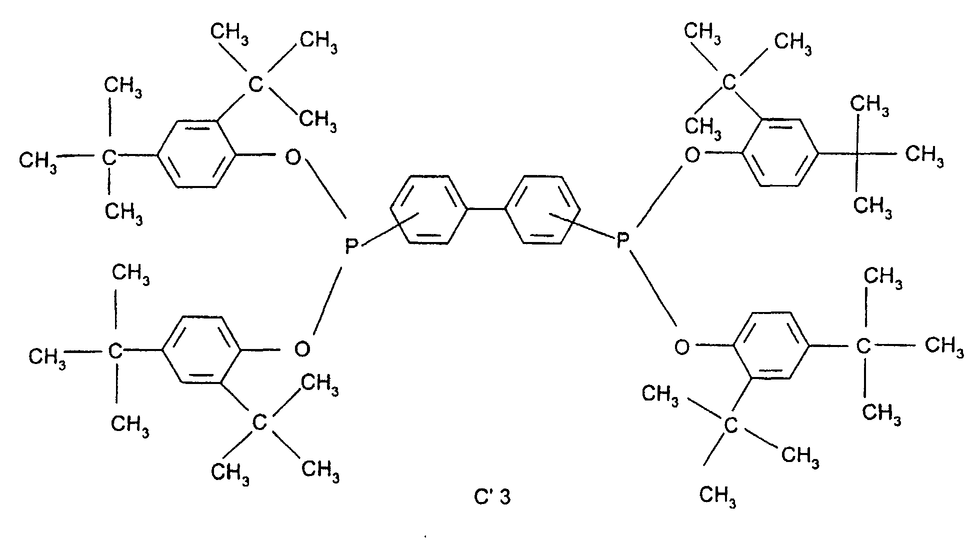

In der Formel C'3 befinden sich die beiden Phosphor-Substituenten hauptsächlich in 4- bzw. 4'-Stellung des Biphenylgrundgerüsts

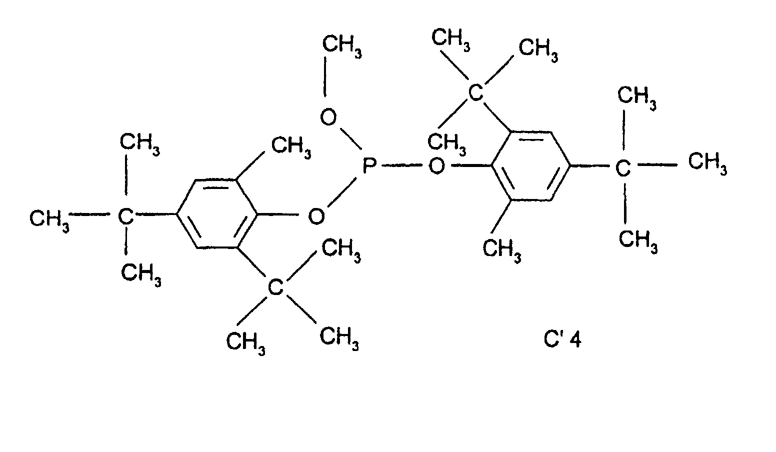

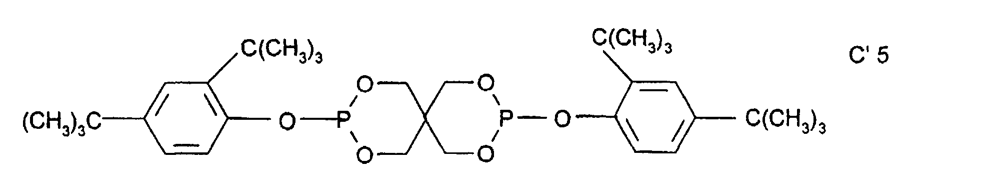

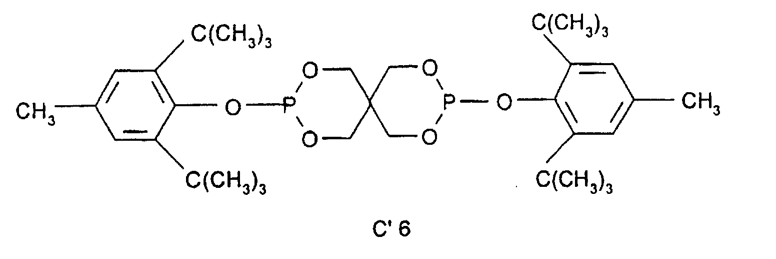

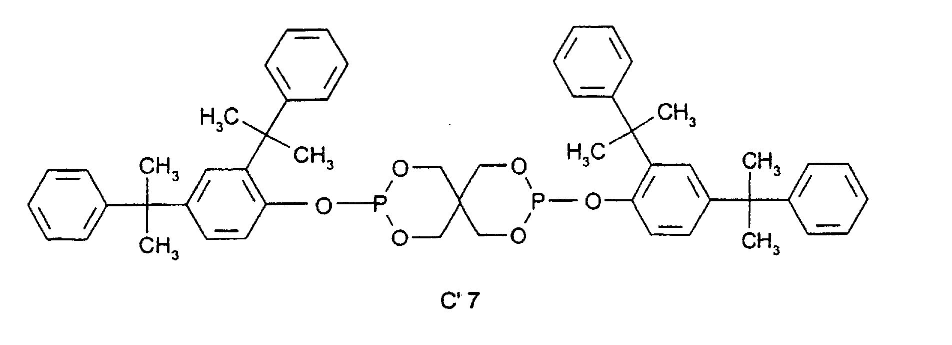

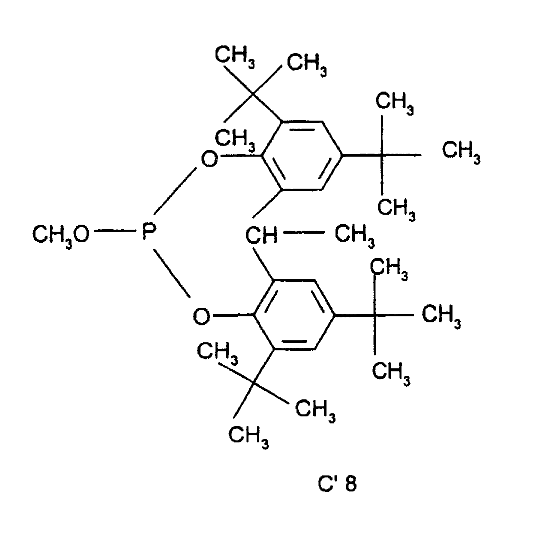

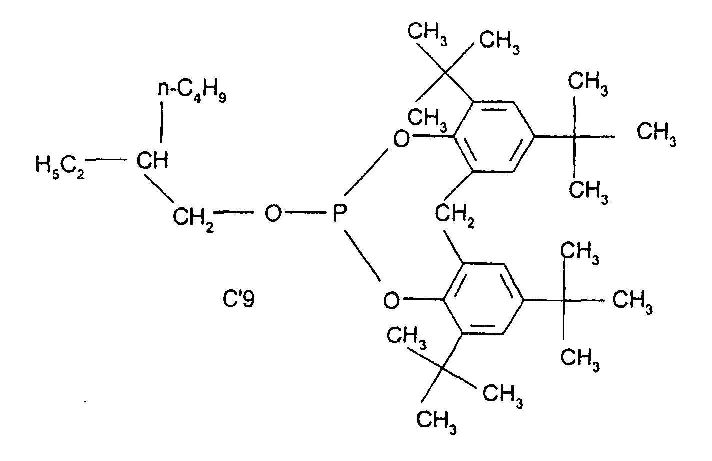

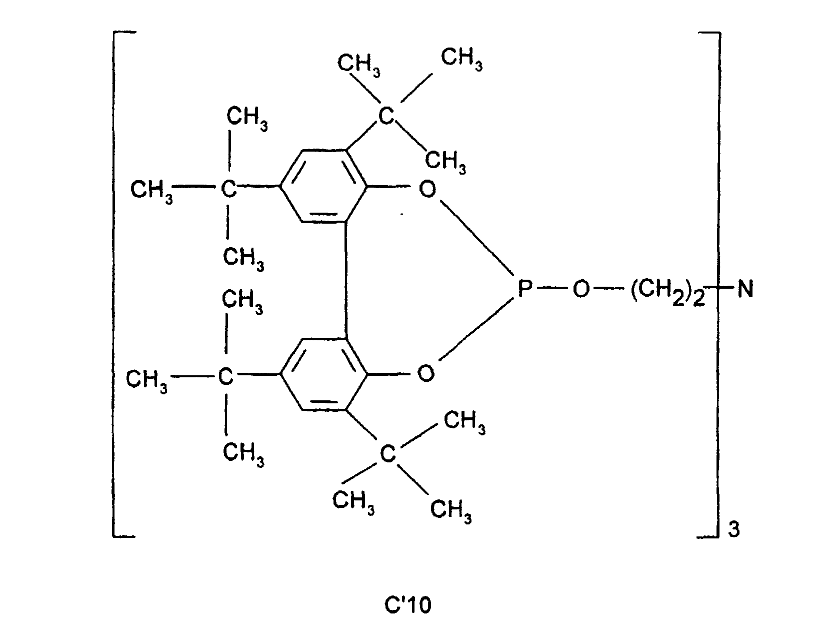

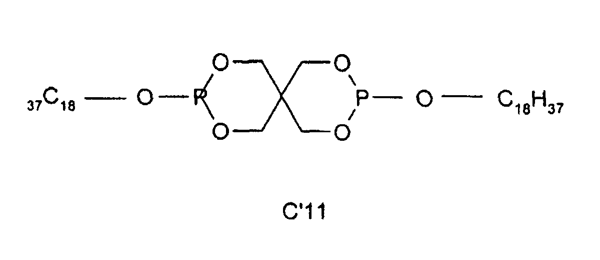

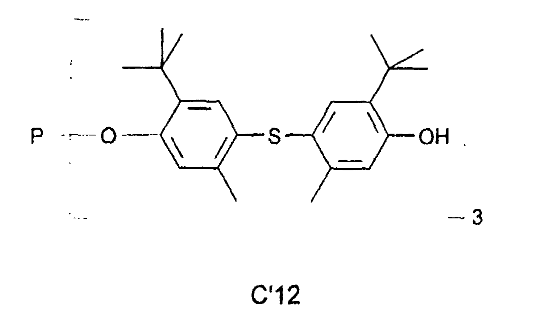

Die genannten Phosphite und Phosphonite sind bekannte Verbindungen, sie sind zum Teil kommerziell erhältlich.

Bei folgenden Stabilisatormischungen handelt es sich um besonders geeignete Ausführungsformen der Erfindung:
Verbindung III und ®Irgafos 38,
Verbindung III und ®Irgafos 12,
Verbindung III und ®Hostanox PAR 24,
Verbindung III und ®Hostanox OSP 1,
Verbindung III und ®Irgafos P-EPQ,
Verbindung III und ®Ultranox 626,
Verbindung III und ®Ultranox 618,
Verbindung III und ®Mark PEP-36 (von Asahi Denka),
Verbindung III und ®Mark HP10 (von Asahi Denka),
Verbindung III und ®Doverphos 9228

Die Kombination von Verbindung (III) mit Phosphiten eignet sich auch in hervorragender Weise in dem Sinne, daß das Phosphit die Wirkung der Verbindung (III) bei der Stabilisierung von organischem Material in einer synergistischen Art und Weise unterstützt. Synergistische Effekte dieser Art sind in EP-A-359276 und EP-A-567117 beschrieben. Besonders geeignet sind Mischungen der Verbindung (III) mit Phosphiten der Formeln C'1 bis C'12.

Die Verbindung (III) eignet sich auch in hervorragender Weise, mit Phosphit, und/oder einem sterisch gehinderten Phenol und/oder einem Säurefänger kombiniert zu werden. Besonders geeignet ist die Kombination der Verbindung (III) in Mischungen mit Phosphit, Phenol und Säurefänger, in einer Art und Weise, wie sie DE-A-19537140 beschreibt.

Die Verbindung (III) sowie die vorstehend beschriebenen Gemische eignen sich auch mit anderen Lichtschutzmitteln, wie z. B. aus der Klasse der UV-Absorber (2-Hydroxybenzophenone oder 2-Hydroxyphenylbenztriazole, Zimtsäurederivate, Oxanilide) und/oder Nickel-Quencher in einer synergistischen Art und Weise kombiniert zu werden.

Bei den vorstehend beschriebenen Gemischen kann der Anteil an Verbindungen der Formel (III) zwischen 1 und 99 Gew.-% liegen.

Die Verbindung (III) eignet sich auch zum Einsatz in Kombination mit Zeolithen oder Hydrotalciten, wie z.B. ®DHT4A analog EP-A-429731.

Die Verbindung (III) sowie die vorstehend beschriebenen Mischungen können auch mit einem oder mehreren N,N-dialkylsubstituierten Hydroxylaminen kombiniert werden, vorzugsweise mit N,N-Dioctadecylhydroxylamin.

Weiterhin kann die Verbindung (III) mit einem oder mehreren basischen oder sonstigen säurebindenden Costabilisatoren aus der Gruppe der Metallcarboxylate, -oxide, -hydroxide, -carbonate, und/oder Zeolithe, und/oder Hydrotalcite, kombiniert werden.

Bevorzugte Costabilisatoren sind Calciumstearat, und/oder Magnesiumstearat, und/oder Magnesiumoxid, und/oder Zinkoxid und/oder carbonathaltiges Zinkoxid und/oder Hydrotaicite.

Besonders bevorzugte Costabilisatoren sind ®Zinkoxid aktiv, ®Zinkoxid transparent und/oder eines der Hydrotalcite ®DHT 4A, ®DHT4 A2, ® Kyowaad 200, ®Kyowaad 300, ®Kyowaad 400, ®Kyowaad 500, ®Kyowaad 600, ®Kyowaad 700, ®Kyowaad 1000, ®Kyowaad 2000.

Die Verbindung (III) eignet sich allein oder in einer geeigneten Kombination mit einem oder mehreren weiteren Stabilisatoren in sehr guter Weise zur Stabilisierung von Pigmenten im Sinne von EP-A-241419, EP-A-612792, EP-A-612816 oder von Leder im Sinne von EP-A-665294 und DE-A-4411369.

Das Resultat dieser neuen Kombinationen sind Mischungen mit einem verbesserten Eigenschaftsprofil gegenüber den Einzelkomponenten, wie z. B. synergistische Effekte in der Lichtschutzwirkung. Kombinationen von Lichtschutzmitteln mit Säurefängern sind in US-5134181, US-4929652, US-5037870, US-5180762 beschrieben.

Weiterhin lassen sich mit den Verbindungen der Formel (III) oder den vorstehend beschriebenen Gemischen vorteilhafterweise kombinieren
- Synergisten vom Typ der 3-Pyrozalidinone
- Synergisten vom Typ der 3-Aryl-benzofuran-2-one
- Farbstoffe oder Pigmente auf organischer oder anorganischer Basis.

Von den 3-Arylbenzofuran-2-onen ist 3-(3,4-Dimethylphenyl)-5,7-di-tert.-butyl-3Hbenzofuran-2-on bevorzugt (Formel D)

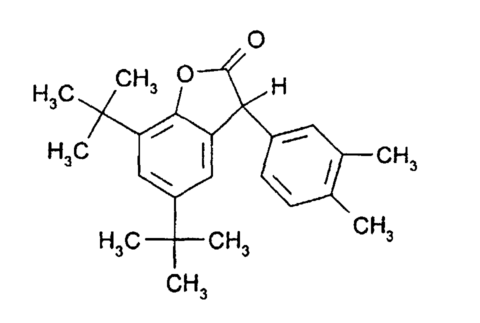

Kombinationen mit Synergisten vom Typ 3-Pyrazotidinon und 1,2,4-Triazolidin-3,5-dion sind in EP-A-517658 beschrieben.

Besonders eignet sich diese Kombination für Anwendungen in organischem Material, bevorzugt in polymerem Material, insbesonders Folien, Fasem und Bändchen bzw. hieraus hergestelltem Gewebe, welche in Kontakt mit aggressiven Chemikalien, insbesonders mit Pflanzenschutzmitteln stehen. Kombinationen dieser Art sind in EP-A-690094 beschrieben.

Gegenstand der vorliegenden Erfindung ist außerdem ein gegen die Einwirkung von Licht, Sauerstoff und Wärme stabilisiertes organisches Material, insbesondere Kunststoffe, Anstrichmittel, Lacke und Öle, welches Verbindungen der Formel (III) in den oben angegebenen Konzentrationen enthält.

Beispiele für derartige Materialien sind:
1. Polymere von Mono- und Diolefinen, beispielsweise Polypropylen, Polyisobutylen, Polybutylen, Polybuten-1, Poly-4-methylpenten-1, Polyisopropren oder Polybutadien sowie Polymerisate von Cycloolefinen wie z.B. von Cyclopenten oder Norbornen; ferner Polyethylen (das gegebenenfalls vernetzt sein kann); z.B. Polyethylen hoher Dichte (HDPE), Polyethylen hoher Dichte und hoher Molmasse (HDPE-HMW), Polyethylen hoher Dichte und ultrahoher Molmasse (HDPE-UHMW), Polyethylen mittlerer Dichte (MDPE), Polyethylen niederer Dichte (LDPE), lineares Polyethylen niederer Dichte (LLPE), verzweigtes Polyethylen niederer Dichte (VLDPE).
   Polyolefine, d.h. Polymere von Monoolefinen, wie sie beispielshaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur)
   b) mittels Katalysator, wobei der Katalysator gewöhnlich ein oder mehrere Metalle der Gruppe IVb, Vb, Vlb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder Π- oder σ-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkye, Metallhydride, Metallalkylhalogenide, Metallalkyloxide oder Metallalkyloxane, wobei die Metalle Elemente der Gruppen la, IIa und/oder IIIa sind. Die Aktivatoren können beispielsweise mit weiteren Ester-, Ether-, Amin- oder Silylether-Gruppen modifiziert sein. Diese Katalysatorsysteme werden gewöhnlich als Philipps, Standard Oil Indiana, Ziegler (-Natta), TNZ (DuPont), Metallocen oder Single Site Katalysatoren (SSC) bezeichnet.
2. Mischungen der unter 1) genannten Polymeren, z. B. Mischungen von Polypropylen mit Polyisobutylen, Polyethylen mit Polyisobutylen, Polypropylen mit Polyethylen (z.B. PP/HDPE/LDPE) und Mischungen verschiedener Polyethylentypen (z.B. LDPE/HDPE) untereinander.
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-lsobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere und deren Copolymere mit Kohlenstoffmonoxid, oder Ethylen-Acrylsäure-Copolymere und deren Salze (lonomere), sowie Terpolymere von Ethylen mit Propylen und einem Dien, wie Hexadien, Dicyclopentadien oder Ethylidenorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.
4. Kohlenwasserstoffharze (z.B. C₅-C₉) inklusive hydrierte Modifikationen davon (z.B. Klebrigmacherharze) und Mischungen von Polyalkylenen und Stärke.
5. Polystyrol, Poly(p-methylstyrol), Poly-(α-methylstyrol).
6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien; Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes und bromiertes Copolymer aus Isobutylen-Isopren (Halobutylkautschuk), chloriertes oder chlorsulfoniertes Polyethylen, Copolymere von Ethylen und chloriertem Ethylen, Epichlorhydrinhomo- und - copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivate ableiten, wie Polyacrylate und Polymethacrylate, mit Butylacrylate schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polypropylenoxyd oder deren Copolymere mit Bisglycidethern.
13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isopthalamid. Block-Copolymere der vorstehend genannten Polyamide mit Polyolefinen, Olefin-Copolymeren, lonomeren, oder chemisch gebundenen oder gepropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyharnstoffe, Polyimide, Polyamidimide, Polyetherimide, Polyesteramide, Polyhydantoine und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Potysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
24. Vernetzbare Acrylharze, die sich von substituierten Acrylsäurestern ableiten, wie z.B. von Epoxyacrylaten, Urethan-acrylaten oder Polyester-acrylaten.
25. Alkydharze, Polyesterharze und Acrylharze, die mit Melaminharzen, Harnstoffharzen, Isocyanaten, Isocyanuraten, Polyisocyanaten oder Epoxidharzen vernetzt sind.
26. Vernetzte Epoxidharze, die sich von aliphatischen, cycloaliphatischen, heterocyclischen oder aromatischen Glycidylverbindungen ableiten, z.B. Produkte von Bisphenol-A-diglycidylethern, Bisphenol-F-diglycidylethern, die mittels üblichen Härtern wie z.B. Anhydriden oder Aminen mit oder ohne Beschleunigern vernetzt werden.
27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBT/ABS, PC/ASA, PC/PBT, PVC/CPE, PVC/Acrylate, POM/thermoplastisches PUR, PC/thermoplastisches PUR, POM/Acrylat, POM/MBS, PPO/MBS, PPO/HIPS. PPO/PA 6.6 und Copolymere, PA/HDPE, PA/PP, PA/PPO, PBT/PC/ABS oder PBT/PET/PC.
29. Natürliche und synthetische organische Stoffe, die reine monomere Verbindungen oder Mischungen von solchen darstellen, beispielsweise Mineralöle, tierische oder pflanzliche Fette, Öle und Wachse, oder Öle, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.
30. Wässrige Emulsionen natürlicher oder synthetischer Kautschuke, wie z.B. Naturkautschuk-Latex oder Latices von carboxylierten Styrol-Butadien-Copolymeren.

Das durch die erfindungsgemäßen Verbindungen der Formel (III) oder durch eine geeignete Kombination mit dieser Verbindung stabilisierte organische Material kann gegebenenfalls noch weitere Additive enthatten, beispielsweise Antioxidantien, Lichtstabilisierungsmittel, Metalldesaktivatoren, antistatische Mittel, flammhemmende Mittel, Gleitmittel, Nukleierungsmittel, Säurefänger (basische Costabilisatoren), Pigmente und Füllstoffe. Antioxidantien und Lichtstabilisatoren, die neben den erfindungsgemäßen Verbindungen oder Kombinationen zugesetzt werden, sind beispielsweise Verbindungen auf der Basis sterisch gehinderter Amine oder sterisch gehinderter Phenole oder Schwefel oder Phosphor enthaltende Costabilisatoren. Als geeignete, zusätzlich in Kombination einsetzbare Additive sind beispielsweise Verbindungen verwendbar, wie sie nachfolgend aufgeführt sind:
1. Antioxidantien
   1.1 Alkylierte Monophenole, z.B. 2,6 Di-tert-butyl-4-methylphenol, 2-Butyl-4,6-dimethylphenol, 2,6-Di-tert-butyl-4-ethylphenol, 2,6-Di-tert-butyl-4-n-butylphenol, 2,6-Di-tert-butyl-4-isobutylphenol, 2,6-Di-cyclopentyl-4-methylphenol, 2-(α-Methylcyclohexyl)-4,6-dimethyl-phenol, 2,6-Di-octadecyl-4-methylphenol, 2,4,6-Tricyclohexylphenol, 2,6-Di-tert-butyl-4-methoxymethylphenol, lineare oder in der Seitenkette verzweigte Nonylphenole, wie z. B. 2,6-Dinonyl-4-methylphenol, 2,4-Dimethyl-6(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.
   1.2 Alkylthiomethylphenole, z.B. 2,4-Di-octylthiomethyl-6-tert-butylphenol, 2,4-Dioctyl-thiomethyl-6-methylphenol, 2,4-Di-octylthiomethyl-6-ethylphenol, 2,6-Didodecylthiomethyl-4-nonyl-phenol.
   1.3 Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxy-phenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenylstearat, Bis-(3,5-di-tert-butyl-4-hydroxy-phenyl)adipat.
   1.4 Hydroxylierte Thiodiphenylether, z.B. 2,2'-Thio-bis-(6-tert-butyl-4-methylphenol). 2,2'-Thio-bis-(4-octylphenol), 4,4'-Thio-bis-(6-tert-butyl-3-methylphenol), 4,4'-Thio-bis-(6-tert-butyl-2-methyl-phenol), 4,4'-Thio-bis-(3,6-di-sec.amylphenol), 4,4'-Bis-(2,6-dimethyl-4-hydroxyphenyl)-disulfid.
   1.5 Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methyl-cyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonyl-phenol], 2,2'-Methylen-bis-[6-(α,α-dimethylbenzyl)-4-nonylphenol], 4,4'-Methylen-bis-(2,6-di-tertbutylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methyl-phenol, 1,1,3-Tris-(5-tert-butyl-4-hydroxy-2-methyl-phenyl)-butan, 1,1-Bis-(5-tert-butyl-4-hydroxy-2-methylphenyl)-3-n-dodecylmercaptobutan, Bis-(3-tert-butyl-4-hydroxy-5-methylphenyl)-dicyclopentadien, Bis-[2-(3'-tert-butyl-2'-hydroxy-5'-methylbenzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methyl-phenyl)-pentan, Ethylenglycol-bis[3,3-bis(3'-tert-butyl-4'-hydroxyphenyl)butyrat].
   1.6 O-, N- und S-Benzylverbindungen, z.B. 3,5,3',5'-Tetra-tert-butyl-4,4'dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, lsooctyl-3,5-di-tert-butyl-4-hydroxybenzyl-mercaptoacetat, Tridecyl-4-hydroxy-3,5-di-tert-butylbenzyl-mercaptoacetat.
   1.7 Hydroxybenzylierte Malonate, z.B. Dioctadecyl-2,2-bis-(3,5-di-tert-butyl-2-hydroxy-benzyl)-malonat, Di-octadecyl-2-(3-tert-butyl-4-hydroxy-5-methylbenzyl)-malonat, Didodecylmercaptoethyl-2,2-bis-(3,5-di-tert-butyl-4-hydroxybenzyl)-malonat, Di-[4(1,1,3,3-tetramethylbutyl)-phenyl]-2,2-bis(3,5-di-tert-butyl-4-hydroxybenzyl)malonat.
   1.8 Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.
   1.9 Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-tert-butyl-4-hydroxybenzyl)-isocyanurat, 1,3,5-Tris-(4-tertbutyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.
   1.10 Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-di-tert-butyl-4-hydroxybenzylphosphonsäure-monoethylesters.
   1.11 Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.
   1.12 Ester der β-(3,5-Di-tert-butyl-4-hydroxyphenyl)-propionsäure mit ein- oder mehrwertigen Alkoholen, wie mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2,2,2]-octan.
   1.13 Ester der β-(5-tert-Butyl-4-hydroxy-3-methylphenyl)-propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodietyhlenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2,2,2]-octan.
   1.14 Ester der β-(3,5-Dicyclohexyl-4-hydroxyphenyl)-propionsäure mit ein- oder mehrwertigen Alkoholen, wie z.B. Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-(2,2,2)-octan.
   1.15 Ester der 3,5-Di-tert-butyl-4-hydroxyphenylessigsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2,2,2]-octan.
   1.16 Ester der 3,3-Bis-(3'-tert-butyl-4'-hydroxyphenyl)-buttersäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2,2,2]-octan.
   1.17 Amide der β-(3,5-Di-tert-butyl-4-hydroxyphenyl)-propionsäure, wie z.B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.
   1.18. Tocopherol, wie z. B. α-Tocopherol, β-Tocopherol, γ-Tocopherol, δ-Tocopherol und Mischungen davon (Vitamin E).
   1.19. Ascorbinsäure (Vitamin C).
   1.20. Aminische Antioxidantien, wie z. B. N, N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di(naphthyl-2-)-p-phenylendiamin, N-Isopropyl,N'-phenyl-p- phenylendiamin, N-(1,3-Dimethyl-butyl)-N'-phenyl-p-phenylendiamin, N-(1-Methyl-heptyl)-N'-phenyl p- phenylendiamin, N-Cyclohexyl-N'-phenyl-p-phenylendiamin, 4-(p-Toluolsulfonamido)-diphenylamin, N,N'-Dimethyl-N.N'-di-sec-butyl-p-phenylendiamin, Diphenylamin, N-Allyldiphenylamin, 4-lsopropoxy-diphenylamin, N-Phenyl-1-naphthylamin, N-(4-tert-octylphenyl)-1-naphthylamin, N-Phenyl-2-naphthylamin, octyliertes Diphenylamin, z. B. p,p'-Di-tert-octyldiphenylamin, 4-n-Butylaminophenol, 4-Butyrylaminophenol, 4-Nonanoylaminophenol, 4-Dodecanoylaminophenol, 4-Octadecanoylaminophenol, Di(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'diamino-diphenylmethan, 1,2-Di-[(2-methyl-phenyl)-amino]-ethan, 1,2-Di-(phenylamino)-propan, (o-Tolyl)-biguanid, Di-[4-(1',3'-di-methyl-butyl)-phenyl]amin, tert-octyliertes N-Phenyl-1-naphthylamin, Gemisch aus mono- und dialkylierten tert Butyl-/tert-Octyldiphenylaminen, Gemisch aus mono- und dialkylierten Nonyldiphenylaminen, Gemisch aus mono- und dialkylierten Dodecyldiphenylaminen, Gemisch aus mono- und dialkylierten Isopropyl/Isohexyl-diphenylaminen, Gemisch aus mono- und dialkylierten tert-Butyldiphenylaminen, 2,3-Dihydro-3,3-dimethyl-4H-1,4-benzothiazin, Phenothiazin, Gemisch aus mono- und dialkylierten tert-Butyl/tert-Octyl-phenothiazinen, Gemisch aus mono- und dialkylierten tert-Octyl-phenothiazinen, N-Allylphenothiazin, N,N,N',N'-Tetra-phenyl-1,4-diaminobut-2-en, N,N-Bis(2,2,6,6-tetramethylpiperidin-4-yl)-hexamethylendiamin, Bis-(2,2,6,6-tetramethylpiperidin-4-yl)-sebacat, 2,2,6,6-Tetramethylpiperidin-4-on, 2,2,6,6-Tetramethylpiperidin-4-ol.
2. UV-Absorber und Lichtschutzmittel
   2.1 2-(2'-Hydroxyphenyl)-benztriazole, wie z.B. 2-(2'-Hydroxy-5'-methylphenyl)-benztriazol, 2-(3',5'-Di-tert-butyl-2'-hydroxyphenyl)-benztriazol, 2-(5'-tert-Butyl-2'-hydroxyphenyl)-benztriazol, 2-[2'-Hydroxy-5'-(1,1,3,3-tetramethylbutyl)phenyl]-benztriazol, 2-(3',5'-Di-tert-butyl-2'-hydroxy-phenyl)-5-chlor-benztriazol,2-(3' -tert-Butyl-2'-hydroxy-5'-methylphenyl)-5-chlor-benztriazol, 2-(3'-sec-Butyl-5'-tert-butyl-2'-hydroxyphenyl)-benztriazol, 2-(2'-Hydroxy-4'-octoxyphenyl)-benztriazol, 2-(3',5'-Ditert-amyl-2'-hydroxyphenyl)-benztriazol, 2-(3',5'-Bis-(α,α-dimethylbenzyl)-2'-hydroxyphenyl)-benztriazol, Mischung aus 2-(3'-tert-Butyl-2'-hydroxy-5'-(2-octyloxycarbonylethyl)-phenyl)-5-chlor-benztriazol, 2-(3'-tert-Butyl-5'-[2-(2-ethylhexyloxy)-carbonylethyl]-2'-hydroxyphenyl)-5-chlor-benztriazol, 2-(3'-tert-Butyl-2'-hydroxy-5'-(2-methoxycarbonylethyl)phenyl)-5-chlor-benztriazol, 2-(3'-tert-Butyl-2'-hydroxy-5'-(2-methoxycarbonylethyl)phenyl)-benztriazol, 2-(3'-tert-Butyl-2'-hydroxy-5'-(2-octyloxycarbonylethyl)phenyl)-benztriazol, 2-(3'-tert-Butyl-5'-[2-(2-ethylhexyloxy)carbonylethyl]-2'-hydroxyphenyl)-benztriazol, 2-(3'-Dodecyl-2'-hydroxy-5'-methylphenyl)-benztriazol, und 2-(3'-tert-Butyl-2'-hydroxy-5'-(2-isooctylocycarbonylethyl)phenyl-benztriazol, 2,2'-Methylen-bis-[4-(1,1,3,3-tetramethylbutyl)-6-benztriazol-2-yl-phenol]; Umesterungsprodukt von 2-[3'-tert-Butyl-5'(2-methoxycarbonylethyl)-2'-hydroxyphenyl]-benztriazol mit Polyethylenglycol 300;
      [R-CH₂CH₂-COO(CH₂)₃]-₂ mit R = 3'-tert-Butyl-4'-hydroxy-5'-2H-benztriazol-2-yl-phenyl.
   2.2 2-Hydroxybenzophenone, wie z.B. das 4-Hydroxy-, 4-Methoxy-, 4-Octoxy, 4-Decyloxy, 4-Dodecyloxy-, 4-Benzyloxy-, 4,2',4'-Trihydroxy-, 2'-Hydroxy-4,4'-dimethoxy-Derivat.
   2.3 Ester von gegebenenfalls substituierten Benzoesäuren, wie z.B. 4-tert-Butylphenylsalicylat, Phenylsalicylat, Octylphenylsalicylat, Dibenzoylresorcin, Bis-(4-tert-butylbenzoyl)-resorcin, Benzoyiresorcin, 3,5-Di-tert-butyl-4-hydroxybenzoesäure-2,4-di-tert-butylphenylester, 3,5-Di-tert-butyl-4-hydroxybenzosäurehexadecylester, 3,5-Di-tert-butyl-4-hydroxy-benzoesäureoctadecylester, 3,5-Di-tert-butyl-4-hydroxybenzoesäure-2-methyl-4,6-di-tert-butylphenylester.
   2.4 Acrylate wie z.B. α-Cyan-β,β-diphenylacrylsäureethylester bzw. -isooctylester, α-Carbomethoxyzimtsäuremethylester, α-Cyano-β-methyl-p-methoxyzimtsäuremethyl-ester bzw. -butylester, α-Carbomethoxy-p-methoxyzimtsäuremethylester, N-(β-Carbo-methoxy-β-cyanovinyl)-2-methylindolin.
   2.5 Nickelverbindungen wie z.B. Nickelkomplexe des 2,2'-Thio-bis-[4-(1,1,3,3 tetramethylbutyl)-phenols] wie der 1:1 oder der 1:2 Komplex, gegebenenfalls mit zusätzlichen Liganden, wie n-Butylamin, Triethanolamin oder N-Cyclohexyldiethanolamin, Nickeldibutyldithiocarbamat, Nickelsalze von 4-Hydroxy-3,5-di-tertbutyl-benzylphosphonsäuremonoalkylestern, wie vom Methyl- oder Ethylester, Nickelkomplexe von Ketoximen, wie von 2-Hydroxy-4-methyl-phenylundecylketoxim, Nickelkomplexe des 1-Phenyl-4-lauroyl-5-hydroxy-pyrazols, gegebenenfalls mit zusätzlichen Liganden.
   2.6 Sterisch gehinderte Amine, wie z.B. Bis-(2,2,6,6-tetramethyl-piperidin-4-yl)-sebacat, Bis-(2,2,6,6-tetramethylpiperidin-4-yl)-glutarat, Bis-(2,2,6,6-tetramethylpiperidin-4-yl)-succinat, Bis-(1,2,2,6,6-pentamethylpiperidin-4-yl)-sebacat, Bis-(1,2,2,6,6-pentamethylpiperidin-4-yl)-glutarat, n-Butyl-3,5-di-tert-butyl-4-hydroxybenzylmalonsäure-bis(1,2,2,6,6-pentamethylpiperidyl)-ester, 2,2,6,6-Tetramethylpiperidylbehenat, 1,2,2,6,6-Pentamethylpiperidylbehenat, Kondensations-produkt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3.4-butantetraoat, 1,1'-(1,2-Ethandiyl)-bis-(3,3,5,5-tetramethylpiperazinon), 4-Benzoyl-2,2,6,6-tetramethylpiperidin, 4-Stearyloxy-2,2,6,6-tetramethylpiperidin, 4-Stearoyloxy-2,2,6,6-tetramethylpiperidin, 4-Stearyloxy-1,2,2,6,6-pentamethylpiperidin, 4-Stearoyloxy-1,2,2,6,6-pentamethylpiperidin, Bis-(1,2,2,6,6-pentamethylpiperidyl)-2-n-butyl-2-(2-hydroxy-3,5-di-tert-butylbenzyl)-malonat, Bis-(1,2,2,6,6-pentamethylpiperidyl)-2-n-butyl-2-(4-hydroxy-3,5-di-tert-butylbenzyl)-malonat, 3-n-Octyl-7,7,9,9-tetramethyl-1,3,8-triazaspiro [4,5] decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus N,N'-Bis(2,2,6,6-tetramethyl-4-piperidyl)-hexamethylendiamin und 4-Cyclohexylamino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)ethan, Kondensationsprodukt aus 2-Chlor-4,6-di-(4-methoxypropylamino-2,2,6,6-tetramethylpiperidyl)-1,3,5-triazin und 1,2-Bis-(3-aminopropylamino)ethan, Kondensationsprodukt aus 2-Chlor-4,6-di-(4-methoxypropylamino-1,2,2,6,6-pentamethylpiperidyl)-1,3,5-triazin und 1,2-Bis-(3-aminopropylamino)ethan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)ethan, Umsetzungsprodukte von 2-Chlor-4,6-di-(4-n-butylamino-2,2,6,6-tetramethylpiperidyl)-1,3,5-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)äthan, Umsetzungsprodukte von 2-Chlor-4,6-di-(4-n-butylamino-1,2,2,6,6-pentamethylpiperidyl)-1,3,5-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)ethan, Umsetzungsprodukte von 2-Chlor-4,6-di-(4-n-butylamino-2,2,6,6-tetramethyl-piperidyl)-1,3,5-triazin und 4-tert-Octylamino-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)ethan, Umsetzungsprodukte von 2-Chlor-4,6-di-(4-n-butylamino-1,2,2,6,6-pentamethylpiperidyl)-1,3,5-triazin und 4-tert-Octylamino-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)ethan, Umsetzungsprodukte von 2-Chlor-4,6-di-(4-n-butylamino-2,2,6,6-tetramethylpiperidyl)-1,3,5-triazin und 4-(4-n-Butylamino-2,2,6,6-tetramethylpiperidyl)-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)-ethan, Umsetzungsprodukte von 2-Chlor-4,6-di-(4-n-butylamino-1,2,2,6,6-pentamethylpiperidyl)-1,3,5-triazin und 4-(4-n-Butylamino-2,2,6,6-tetramethylpiperidyl)-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)ethan, Kondensationprodukt aus 1,2-Bis(3-aminopropylamino)-ethan und 2,4,6-Trichlor-1,3,5-triazin sowie 4-Butylamino-2,2,6,6-tetramethylpiperidin, N-(2,2,6,6-tetramethyl-4-piperidyl)-n-dodecylsuccinimid, N-(1,2,2,6,6-pentamethyl-4-piperidyl)-n-dodecylsuccinimid, 8-Acetyl-3-Dodecyl-7,7,9,9-tetramethyl-1,3,8-triazaspiro[4,5]decan-2,4-dion, Oligomerisiertes 2,2,4,4-Tetramethyl-20-(oxiranylmethyl)-7-oxa-3,20-diazadispiro[5.1.11.2]heneicosan-21-on, Oligomerisiertes 1,2,2,4,4-Pentamethyl-20-(oxiranylmethyl)-7-oxa-3,20-diazadispiro-[5.1.11.2]heneicosan-21-on, Oligomerisiertes 1-Acetyl-2,2,4,4-tetramethyl-20-(oxiranyl-methyl)-7-oxa-3,20-diazadispiro[5.1.11.2]heneicosan-21-on, 3-Dodecyl-1-(2,2,6,6-tetra-methyl-4-piperidyl)pyrrolidin-2,5-dion, 3-Dodecyl-1-(1,2,2,6,6-pentamethyl-4-piperidyl)-pyrrolidin-2,5-dion, 2,2,4,4-Tetramethyl-7-oxa-3,20-diazadispiro-[5.1.11.2]-heneicosan-21-on, 2,2,4,4-Tetramethyl-7-oxa-21-oxo-3,20-diazadispiro-[5.1.11.2]-heneicosan-3-propansäure-dodecylester, 2,2,4,4-Tetramethyl-7-oxa-21-oxo-3,20-diazadispiro-[5.1.11.2]-heneicosan-3-propansäuretetradecylester, 2,2,3,4,4-Pentamethyl-7-oxa-3,20-diazadispiro-[5.1.11.2]heneicosan-21-on, 2,2,3,4,4-Pentamethyl-7-oxa-21-oxo-3,20-diaza-dispiro-[5.1.11.2]heneicosan-3-propansäuredodecylester, 2,2,3,4,4-Pentamethyl-7-oxa-21-oxo-3,20-diazadispiro-[5.1.11.2]-heneicosan-3-propansäuretetradecylester, 3-Acetyl-2,2,4,4-tetramethyl-7-oxa-3,20-diazadispiro-[5.1.11.2]heneicosan-21-on 3-Acetyl-2,2,4,4-tetramethyl-7-oxa-21-oxo-3,20-diaza-dispiro-[5.1.11.2]heneicosan-3-propansäure-dodecylester, 3-Acetyl-2,2,4,4-tetramethyl-7-oxa-21-oxo-3,20-diazadispiro-[5.1.11.2]heneicosan-3-propansäuretetradecylester, 1,1',3,3',5,5'-Hexahydro-2,2',4,4',6,6'-hexaaza-2,2',6,6'bismethano-7,8-dioxo-4,4'-bis-(1,2,2,6,6-pentamethyl-4-piperidyl)-biphenyl, Poly-N,N'-bis-(2,2,6,6-tetramethyl-4-piperidyl)-1,8-diazadecyclen, Additionsverbindung von 2,2,6,6-Tetramethyl-4-allyloxy-piperidin und Polymethylhydrogensitoxan (Molmasse bis 4000), Additionsverbindung von 1,2,2,6,6-Pentamethyl-4-allyloxypiperidin und Polymethylhydrogensiloxan (Molmasse bis 4000), N,N'-Diformyl-N,N'-bis-(2,2,6,6-tetramethyl-4-piperidinyl)-hexamethylendiamin, N,N'-Diformyl-N,N'-bis-(1,2,2,6,6-pentamethyl-4-piperidinyl)-hexamethylendiamin, 5,11-Bis-(2,2,6,6-tetramethyl-4-piperidinyl)-3,5,7,9,11,13-hexaaza-tetracyclo-[7.4.0.0^{2,7}.1^{3,13}]-tetradecan-8,14-dion, 5,11-Bis-(1,2,2,6,6-pentamethyl-4-piperidinyl)-3,5,7,9,11,13-hexaazatetracyclo-[7.4.0.0^{2,7}.1^{3,13}]-tetradecan-8,14-dion, [(4-Methoxyphenyl)-methylen]-propandisäure-bis-(2,2,6,6-tetramethyl-4-piperidinyl)-ester, [(4-Methoxyphenyl)-methylen]-propandisäure-bis-(1,2,2,6,6-pentamethyl-4-piperidinyl)-ester, 2,4,6-Tris-(N-cyclohexyl-N-[2-(3,3,4,5,5-pentamethyl-piperazinon-1-yl)-ethyl]-amino)-1,3,5-triazin, Copolymerisat aus Styrol mit Methylstyrol und Maleinsäureanhydrid umgesetzt mit 4-Amino-2,2,6,6-tetramethylpiperidin und Octadecylamin, Copolymerisat aus Styrol mit α-Methylstyrol und Maleinsäureanhydrid umgesetzt mit 4-Amino-1,2,2,6,6-pentamethylpiperidin und Octadecylamin, Polycarbonat mit 2,2'-[(2,2,6,6-Tetramethyl-4-piperidinyl)-imino]-bis-[ethanol] als Diolkomponente, Polycarbonat aus 2,2'-(1,2,2,6,6-Pentamethyl-4-piperidinyl)-imino]-bis-[ethanol] als Diolkomponente, Copolymer aus Maleinsäureanhydrid und einem α-Olefin bis C₃₀ umgesetzt mit 4-Amino-2,2,6,6-tetramethylpiperidin, Copolymer aus Maleinsäureanhydrid und einem α-Olefin bis C₃₀ umgesetzt mit 1-Acetyl-4-amino-2,2,6,6-tetramethylpiperidin, Copolymer aus Maleinsäureanhydrid und einem α-Olefin bis C₃₀ umgesetzt mit 4-Amino-1,2,2,6,6-pentamethylpiperidin, sowie die N-Alkyl- und N-Aryl-oxyderivate der oben genannten Verbindungen mit freien NH-Gruppen am Piperidin, speziell α-Methylbenzyloxy- und Alkyloxy- von C₁ bis C₁₈.
   2.7 Oxalsäurediamide, wie z.B. 4,4'-Di-octyloxyoxanilid, 2,2'-Diethoxyoxanilid, 2,2'-Di-octyloxy-5,5'-di-tert-butyl-oxanilid, 2,2'-Di-dodecyloxy-5,5'-di-tert-butyl-oxanilid, 2-Ethoxy-2'-ethyloxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-disubstituierten Oxaniliden.
   2.8. 2-(2-Hydroxyphenyl)-1,3,5-triazine, wie z.B. 2,4,6-Tris-(2-hydroxy-4-octyloxyphenyl)-1,3,5-triazin, 2-(2-Hydroxy-4-octyloxyphenyl)-4',6-bis-(2',4-dimethylphenyl)-1,3,5-triazin, 2-(2,4-Dihydroxyphenyl)-4,6-bis-(2,4-dimethylphenyl)-1,3,5-triazin, 2-(2,4-Dihydroxyphenyl)-4,6-bis-(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-di-methylphenyl)-1,3,5-triazin, 2-[2-Hydroxy-4-(2-hydroxy-3-butyloxypropyloxy)phenyl]-4,6-bis(2,4-dimethylphenyl)-1,3,5-triazin, 2-[2-hydroxy-4-(2-hydroxy-3-octyloxypropyloxy)phenyl]-4,6-bis(2,4-dimethylphenyl)-1,3,5-triazin, 2-(2-Hydroxy-4-tridecyloxyphenyl)-4,6-bis-(2,4-dimethylphenyl)-1,3,5-triazin, 2-[4-dodecyloxy/tridecyloxy-2-hydroxypropoxy)-2-hydroxy-phenyl]-4,6-bis(2,4-dimethylphenyl)-1,3,5-triazin, 2-[2-Hydroxy-4-(2-hydroxy-3-dodecyloxypropoxy)phenyl]-4,6-bis(2,4-dimethylphenyl)-1,3,5-triazin, 2-(2-Hydroxy-4-hexyloxy)phenyl-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxypropoxy)-phenyl]-1,3,5-triazin, 2-(2-Hydroxyphenyl)-4-(4-methoxyphenyl)-6-phenyl-1,3,5-triazin.
3. Metalldesaktivatoren, wie z.B. N,N'-Diphenyloxalsäurediamid, N-Salicylal-N'salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäuredihydrazid, Sebacinsäurebisphenylhydrazid, N,N'-Diacetyladipinsäuredihydrazid, N,N'-Bis-salicyloyloxalsäuredihydrazid, N,N'-Bis-salicyloylthiopropionsäuredihydrazid.
4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearylpentaerythritdiphosphit, Tris-(2,4-di-tert-butylphenyl)-phosphit, Diisodecylpentaerythritdiphosphit, Bis-(2,4-di-tert-butylphenyl)-pentaerythritdiphosphit, Bis-(2,6,-di-tert-butyl-4-methylphenyl)-pentaerythritdiphosphit, Bis-isodecyloxy-penta-erythritdiphosphit, Bis-(2,4-di-tert-butyl-6-methylphenyl)-pentaerythritdiphosphit, Bis-(2,4,6-tri-tert-butylphenyl)-pentaerytritdiphosphit, Tristearyl-sorbit-triphosphit, Tetrakis-(2,4-di-tert-butylphenyl)-4,4'-biphenylendiphosphonit, 6-lsooctyloxy-2,4,8,10-tetra-tert-butyl-12H-di-benz[d,g]-1,3,2-dioxaphosphocin, 6-Fluor-2,4,8,10-tetra-tert-butyl-12-methyl-dibenz[d,g]-1,3,2-dioxaphophocin, Bis-(2,4-di-tert-butyl-6-methylphenyl)-methylphosphit, Bis-(2,4-di-tert-butyl-6-methylphenyl)-ethylphosphit, Tris(2-tert-butyl-4-thio(2'-methyl-4'-hydroxy-5'-tert-butyl)-phenyl-5-methyl)-phenylphosphit, 2,2',2"-Nitrilo[triethyl-tris(3,3',5,5'-tetra-tert-butyl-1,1'-biphenyl-2,2'-diyl)phosphit], Bis[2-methyl-4,6-bis(1,1-dimethylethyl)phenol]phosphorigsäureethylester.
5. Hydroxylamine, wie z. B. N,N-Dibenzylhydroxylamin, N,N-Diethylhydroxylamin, N,N-Dioctylhydroxylamin, N,N-Dilaurylhydroxylamin, N,N-Ditetradecylhydroxylamin, N,N-Dihexadecylhydroxylamin, N,N-Dioctadecylhydroxylamin, N-Hexadecyl-N-octadecylhydroxylamin, N-Heptadecyl-N-octadecylhydroxylamin, N,N-Dialkylhydroxylaminen hergestellt aus hydriertem Talgfettamin.
6. Nitrone, wie z. B. N-Benzyl-alpha-phenylnitron, N-Ethyl-alpha-methylnitron, N-Octyl-alphaheptyl-nitron, N-Lauryl-alphaundecylnitron, N-Tetradecyl-alpha-tridecylnitron, N-Hexadecyl-alpha-pentadecylnitron, N-Octadecyl-alpha-heptadecylnitron, N-Hexadecyl-alpha-heptadecylnitron, N-Octadecyl-alpha-pentadecylnitron, N-Heptadecyl-alpha-heptadecylnitron, N-Octa-decyl-alpha-hexadecylnitron, Nitrone abgeleitet von N,N-Dialkylhydroxylaminen, hergestellt aus hydrierten Talgfettaminen.
7. Zeolithe und Hydrotalcite, wie z. B. ®DHT 4A. Solche Hydrotalcite können nach der allgemeinen Formel

   [(M²⁺)₁₋ₓ (M³⁺)ₓ (OH)₂ (Aⁿ⁻)_{x/n} yH₂O],

   beschrieben werden, wobei
   - (M²⁺): bedeutet Mg, Ca, Sr, Ba, Zn, Pb, Sn, Ni
   - (M³⁺): bedeutet Al, B, Bi
   - Aⁿ: bedeutet ein Anion der Wertigkeit n
   - n: bedeutet eine ganze Zahl von 1 - 4
   - x: bedeutet einen Wert zwischen 0 und 0,5
   - y: bedeutet einen Wert zwischen 0 und 2

   A bedeutet OH⁻, Cl⁻, Br⁻, I⁻, ClO₄-, CH₃COO⁻, C₆H₅COO⁻, CO₃²⁻, SO₄²⁻, (OOC-COO)²⁻, (CHOHCOO)₂²⁻, HO(CHOH)₄CH₂COO⁻, C₂H₄(COO)₂²⁻, (CH₂COO)₂²⁻, CH₃CHOHCOO⁻, SiO₃²⁻, SiO₄⁴⁻, Fe(CN)₆³⁻, Fe(CN)₆⁴⁻, BO₃³⁻, PO₃³⁻, HPO₄²⁻.
   Bevorzugt eingesetzt werden Hydrotaicite, in welchen (M²⁺) für (Ca²⁺), (Mg²⁺) oder eine Mischung aus (Mg²⁺) und (Zn²⁺); (Aⁿ⁻) für CO₃²⁻, BO₃³⁻, PO₃³⁻ stehen. x hat einen Wert von 0 bis 0,5 und y hat einen Wert von 0 bis 2. Weiterhin können auch Hydrotalcite, die mit der Formel

   [(M²⁺)ₓ (Al³⁺)₂ (OH)_{2x+6nz} (Aⁿ⁻)₂ yH₂O]

   beschrieben werden können, eingesetzt werden. Hier steht (M²⁺) für Mg²⁺, Zn²⁺, bevorzugterweise jedoch Mg²⁺. (Aⁿ⁻) steht für ein Anion, besonders aus der Gruppe CO₃²⁻, (OOC-COO)²⁻, OH- und S²⁻, wobei n die Wertigkeit des lons beschreibt.
   y steht für eine positive Zahl, bevorzugterweise zwischen 0 und 5, ganz besonders zwischen 0,5 und 5. x und z haben positive Werte, die bei x bevorzugt zwischen 2 und 6 liegen und bei z kleiner als 2 sein sollten. Besonders bevorzugt sind die Hydrotalcite der folgenden Formeln anzusehen:

   Al₂O₃ × 6MgO × CO₂ × 12H₂O,

   Mg_{4.5}Al₂(OH)₁₃ × CO₃ × 3.5H₂O,

   4MgO × Al₂O₃ × CO₂ × 9H₂O,

   4MgO × Al₂O₃ × CO₂ × 6H₂O,

   ZnO × 3MgO × Al₂O₃ × CO₂ × 8-9H₂O,

   ZnO × 3MgO × Al₂O₃ × CO₂ × 5-6H₂O,

   Mg_{4.5}Al₂(OH)₁₃ × CO₃.

   Hydrotalcite werden im Polymer bevorzugt in einer Konzentration von 0.01 bis 5 Gew.%, besonders von 0,2 bis 3 Gew.%, bezogen auf die gesamte Polymerzubereitung, eingesetzt.
8. Thiosynergisten, wie z. B. Thiodipropionsäuredilaurylester oder Thiodipropionsäuredistearylester.
9. Peroxidzerstörende Verbindungen wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-alkyl-dithiocarbamate, Zinkdibutyl-dithiocarbamat, Dioctadecylmonosulfid, Dioctadecyldisutfid, Pentaerythrit-tetrakis-(β-dodecyl-mercapto)-propionat.
10. Polyamidstabilisatoren, wie z.B. Kupfersalze in Kombination mit Jodiden und/oder Phosphorverbindungen und Salze des zweiwertigen Mangans.
11. Basische Co-Stabilisatoren wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat, Alkali- und Erdalkalisalze sowie das Zinksalz oder das Aluminiumsalz von Milchsäure.
12. Nukleierungsmittel, wie z.B. anorganische Stoffe, wie z. B. Talk, Metalloxide, wie z. B. Titandioxid oder Magnesiumoxid, Phosphate, Carbonate oder Sulfate von vorzugsweise Erdalkalimetallen, organische Verbindungen, wie Mono- oder Polycarbonsäuren sowie auch ihrer Salze, wie z. B. 4-tert-Butylbenzoesäure, Adipinsäure; Diphenylessigsäure; Natriumsuccinat oder Natriumbenzoat; Acetale von aromatischen Aldehyden und polyfunktionellen Alkoholen wie z. B. Sorbitol, wie z.B. 1,3-2,4-Di(benziliden)-D-sorbitol, 1,3-2,4-Di(4-tolyliden)-D-sorbitol, 1,3-2,4-Di(4-ethylbenziliden)-D-sorbitol, polymere Verbindungen, wie z. B. ionische Copolymerisate ("Ionomere").
13. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit, Holzmehl und andere Mehle oder Fasern anderer Naturprodukte, synthetische Fasern.
14. Sonstige Zusätze, wie z.B. Weichmacher, Gleitmittel, Emulgatoren, Pigmente, Rheologieadditive, Katalysatoren, Verlaufshilfsmittel, Optische Aufheller, Flammschutzmittel, Antistatika, Treibmittel.
15. Benzofuranone bzw. Indoline, wie z. B. in US-4325863, US-4338244, US-5175312; US-5216052; US-5252643, DE-A-4316611, DE-A-4316622, DE-A-4316876, EP-A-0589839 oder EP-A-0591102 beschrieben oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuranon-2-on, 3,3'-Bis[5,7-di-tert-butyl-3-(4-[2-hydroxyethoxy]-phenyl)-benzofuran-2-on, 5,7-Di-tert-butyl-3-(4-ethoxyphenyl)-benzofuran-2-on, 3-(4-Acetoxy-3,5-dimethylphenyl)-5,7-di-tert-butyl-benzofuran-2-on, 3-(3,5-Diethyl-4-pivaloyloxy-phenyl)-5,7-di-tert-butyl-benzofuran-2-on.

Die Additive der allgemeinen Formel (III) oder die beschriebenen Kombinationen werden nach den allgemein üblichen Methoden in das organische Material, vorzugsweise in das Polymere eingearbeitet. Die Einarbeitung kann beispielsweise durch Einmischen oder Aufbringen der Verbindungen und gegebenenfalls weiterer Additive in oder auf das Polymere unmittelbar vor, während oder nach der Polymerisation oder in die Polymerschmelze vor oder während der Formgebung erfolgen. Auch durch Aufbringen der gelösten oder dispergierten Verbindungen auf das Polymere direkt oder Einmischen in eine Lösung, Suspension oder Emulsion des Polymeren, gegebenenfalls unter nachträglicher Verdunstung des Lösemittels, kann die Einarbeitung erfolgen. Die Verbindungen sind auch wirksam, wenn sie in ein bereits granuliertes Polymer nachträglich in einem gesonderten Verarbeitungsschritt eingebracht werden. Die erfindungsgemäßen Verbindungen der Formel (III) können auch in Form eines Masterbatches, der diese Verbindungen beispielsweise in einer Konzentration von 1 bis 75, vorzugsweise 2,5 bis 30 Gew.-% enthält, den zu stabilisierenden Polymeren zugesetzt werden.

Die nachfolgenden Beispiele sollen die Erfindung näher erläutern, ohne diese in irgendeiner Weise näher einzuschränken. Alle hergestellten erfindungsgemäßen Verbindungen der Formel (III) konnten durch ihre ¹³C NMR-Spektren charakterisiert werden; die Schmelzbereiche der hergestellten Oligomere sind im Text und in Tabelle 1 angegeben.

### Beispiel 1:

47.0 g (0.06 mol) 20,20'-(2-Hydroxy-1,3-propandiyl)-bis-[2,2,4,4-tetramethyl-7-oxa-3,20-diazadispiro-[5.1.11.2]-heneicosan-21-on] (II') und 151.2 g (0.36 mol) 2,2,4,4-Tetramethyl-20-(oxiranylmethyl)-7-oxa-3,20-diazadispiro[5.1.11.2]heneicosan-21-on (I') werden im Vakuum 6 h bei 200/C polymerisiert. Dabei schmelzen die Festsubstanzen auf und es entsteht eine farblose, zähe Schmelze. Nach dem Abkühlen des Reaktionsguts wird die spröde Mischung mit flüssigem Stickstoff aus dem Kolben gesprengt und pulverisiert. Der Schmelzbereich des Oligomers beträgt 188 bis 230°C.

**Tabelle. 1:**

| Mischungsverhältnisse der Edukte (I') und (II') und Schmelzbereiche der hergestellten Oligomere | | | |
|---|---|---|---|
| Beispiel: | (I') [mol] | (II') [mol] | Schmelzbereich des Oligomers [°C] |
| 2 | 0.24 | 0.12 | 162-212 |
| 3 | 0.30 | 0.10 | 171-219 |
| 4 | 0.32 | 0.08 | 179-220 |

| Beispiel: | (I') [mol] | (II') [mol] | Schmelzbereich des Oligomers [°C] |
|---|---|---|---|
| 5 | 0.55 | 0.11 | 180-221 |

### Beispiel 6: Lichtstabilisierende Wirkung in Polyethylen mit und ohne Chemikalienkontakt

100 Gewichtsteile unstabilisiertes Polyethylen (®Polyethylen LE 4510 der Fa. Borealis) wurden zusammen mit 0.1 Gewichtsteilen Pentaerythrityl-tetrakis-[3-(3,5-di- tert.butyl-4-hydroxyphenyl)-propionat] (®Hostanox O 10), 0.05 Gewichtsteilen Tris(2,4-di-tert.-butylphenyl)phosphit (®Hostanox PAR 24) vermischt. Diese Grundformulierung wird mit 0.2 Gewichtsteilen des Stabilisators aus Beispiel 1 allein bzw. in Kombination mit 0.1 Gewichtsteilen des Hydrotalcits ®DHT 4A oder einer Mischung aus 0.05 Gewichtsteilen Zinkoxid und 0.05 Gewichtsteilen Calciumstearat mit einem Brabender-Extruder mit Einfach-Schnecke, 125 Upm, Heizzonen 150/180/220 °C, zweimal granuliert und auf diese Weise intensiv homogenisiert. Diese Grundmischung wird in einem Zwei-schneckenextruder aufgeschmolzen und dann granuliert und daraus Blasfolien der Dicke 200 µm hergestellt. Die auf diese Weise erhaltenen Prüfkörper werden in einem Schnellbewitterungsgerät (®Xenotest 1200) belichtet. Als Kriterium der Stabilität der Folie wurde die Veränderung des Carbonylindexes während der Belichtungszeit herangezogen, welcher in regelmäßigen Abständen (alle 200 h) gemessen wird. Der Carbonylindex CO wurde hierbei gemäß der Formel CO = E₁₇₂₀/E₂₀₂₀ bestimmt (E = Extinktion); der Endpunkt der Belichtung wird dabei durch die Zunahme des Carbonylindex um eine Einheit festgelegt. Zu Vergleichszwecken wurde eine Folie unter den gleichen Bedingungen, jedoch ohne den Zusatz des erfindungsgemäßen Stabilisators aus Beispiel 1 getestet. Die Versuchsergebnisse sind in Tabelle 1' zusammengefaßt.

**Tabelle 1':**

| Abbau von stabilisiertem Polyethylen | | |
|---|---|---|
| **Zusätzlich zur Grundstabilisierung eingesetzter Stabilisator** | **Konzentration in Gew.-%** | **Stunden, bis ΔCO>1 erreicht ist** |
| a) keiner | ---------------- | 310 |
| b) Stabilisator aus Beispiel 1 | 0,2 | 1010 |
| c) Zinkoxid und Calciumstearat | 0,05/0,05 | 580 |
| d) Hydrotalcit (DHT 4a) | 0,1 | 520 |
| e) Stabilisator aus Beispiel 1 Zinkoxid und Calciumstearat | 0,2 0,05/0,05 | 2240 |
| f) Stabilisator aus Beispiel 1 Hydrotalcit (DHT 4a) | 0,2 0,1 | 1610 |

Die grundstabilisierte Folie erreicht das Kriterium ΔCO>1, welches den Polymerabbau beschreibt, nach 310 h. Die stabilisierende Wirkung der zusätzlich eingesetzten Stabilisatoren ergibt sich aus der Stundenzahl, die über die 310h hinausgehen. So leisten z.B. 0,2 Gew.-% an Stabilisator aus Beispiel 1 einen stabilisierenden Beitrag von (1010-310)=700 Stunden. Der stabilisierende Beitrag von 0,1 Gew.-% Hydrotalcit liegt bei (520-310) = 210 Stunden. Ein additiver stabilisierender Effekt würde bei einer Kombination vom Stabilisator aus Beispiel 1 mit Hydrotalcit (DHT 4a) zu einer Gesamtstabilisierung von (310 h + 700 h + 210 h) = 1220 h führen. Tatsächlich aber führt die Kombination vom Stabilisator aus Beispiel 1 mit Hydrotalcit (DHT 4a) zu einer Gesamtstabilisierung von 1610 h; die Kombination ist also synergistisch. Im gleichen Sinne wird ein Synergismus bei Kombination e) beobachtet.

### Beispiel 7: Lichtstabilisierende Wirkung in Polypropylen-Platten

100 Gewichtsteile unstabilisiertes Polypropylen ®Hostalen PPK 0160 wurden zusammen mit 0.1 Gewichtsteilen Pentaerythrityl-tetrakis-[3-(3,5-di- tert.butyl-4-hydroxyphenyl)-propionat (®Hostanox O 10) und 0,1 Gewichtsteilen Calciumstearat (Firma Greven) vermischt. Diese Grundformulierung wird mit 0.1 Gewichtsteilen des Stabilisators aus Beispiel 1 bzw. einer Kombination des Stabilisators aus Beispiel 1 (0.05 Gew. %) und den in Tab. 2 angegebenen monomeren HALS-Stabilisatoren (0.05 Gew. %) zweimal mit einem Leistritz-Doppelschneckenextruder granuliert (gegenläufige Schneckenführung, Heizzonen 210 °C, 220 °C, 230 °C, 240 °C). Anschließend wird das Granulat zu Spritzplatten der Dicke 2 mm verarbeitet (Toshiba Doppelschneckenextruder, gegenläufige Schneckenführung, Heizzonen 210 °C, 220 °C, 230 °C, 240 °C). Die auf diese Weise erhaltenen Prüfkörper werden in einem Schnellbewitterungsgerät (®Xenotest 1200) belichtet. Als Kriterium der Stabilität der Prüfkörper wurde die Zunahme der Verfärbung herangezogen, welche in regelmäßigen Abständen (alle 150 h) gemessen wurde. Zu Vergleichszwecken wurde ein Prüfkörper unter den gleichen Bedingungen, jedoch ohne den Zusatz des erfindungsgemäßen Stabilisators aus Beispiel 1 getestet.

**Tabelle 2:**

| Verfärbung von stabilisiertem Polypropylen (Beurteilung erfolgt visuell mit Mikroskop, die Beurteilungsstufen bedeuten: 0=keine, 1=spur, 2=wenig, 3=etwas, 4=merklich, 5=wesentlich, 6=stark). | | | | |
|---|---|---|---|---|
| **Zusätzlich zur Grundstabilisierung eingesetzter Stabilisator** | **Konzentration in Gew.-%** | **290 h** | **508 h** | **674 h** |
| a) keiner | ---------------- | 0 | 0 | 6 |
| b) Stabilisator aus Beispiel 1 | 0,1 | 0 | 0 | 2 |
| c) Stabilisator A* | 0,1 | 0 | 1 | 3 |
| d) Stabilisator B** | 0,1 | 0 | 0 | 1 |
| e) Stabilisator aus Beispiel 1 Stabilisator A* | 0,05 0,05 | 0 | 0 | 0 |
| f) Stabilisator aus Beispiel 1 Stabilisator B* | 0,05 0,05 | 0 | 0 | 0 |

| | | | | |
|---|---|---|---|---|
| * Bis-(2,2,6,6-tetramethyl-4-piperidyl)sebacat (®Tinuvin 770, CIBA Specialty Chem.) | | | | |
| ** 2,2,4,4-Tetramethyl-7-oxa-3,20-diazadispiro-[5.1.11.2]-heneicosan-21-on (®Hostavin N 20, Clariant GmbH) | | | | |

Die Tabelle belegt, daß die stabilisierende Wirkung einer Kombination aus Stabilisator aus Beispiel 1 mit monomeren Lichtschutzmitteln auf Basis von sterisch gehinderten Aminen besser ist als von der Summe der Einzelkomponenten her hätte erwartet werden können.

### Beispiel 8: Lichtstabilisierende Wirkung in Polypropylen-Preßfolien

100 Gewichtsteile unstabilisiertes Polypropylen ®Hostalen PPK 0160 wurden zusammen mit 0.1 Gewichtsteilen Pentaerythrityl-tetrakis-[3-(3,5-di-tert.butyl-4-hydroxyphenyl)-propionat (®Hostanox O 10) und 0.1 Gewichtsteilen Calciumstearat vermischt. Diese Grundformulierung wird mit 0.1 Gewichtsteilen des Stabilisators aus Beispiel 1 bzw. einer Kombination des Stabilisators aus Beispiel 1 (0.05 Gew. %) und dem in Tab. 3 angegebenen polymeren HALS-Stabilisator (0.05 Gew. %) in einem Brabender-Mischer 10 min bei 200°C und 20 U/min geknetet und auf diese Weise intensiv homogenisiert. Aus dieser Grundmischung wurden Preßfolien der Dicke 100 µm hergestellt. Die auf diese Weise erhaltenen Prüfkörper werden in einem Schnellbewitterungsgerät (®Xenotest 1200) belichtet. Als Kriterium der Stabilität der Folie wurde die Veränderung des Carbonylindexes während der Belichtung herangezogen, welcher in regelmäßigen Abständen (alle 150 h) gemessen wird. Der Carbonylindex CO wurde hierbei gemäß der Formel CO = E₁₇₂₀/E₂₀₂₀ bestimmt (E = Extinktion); der Endpunkt der Belichtung wird dabei durch die Zunahme des Carbonylindex um eine Einheit festgelegt. Zu Vergleichszwecken wurde ein Prüfkörper unter den gleichen Bedingungen, jedoch ohne den Zusatz des erfindungsgemäßen Stabilisators aus Beispiel 1 getestet.

**Tabelle 3:**

| Abbau von stabilisiertem Polypropylen | | |
|---|---|---|
| **Zusätzlich zur Grundstabilisierung eingesetzter Stabilisator** | **Konzentration in Gew.-%** | **Stunden, bis ΔCO > 1 erreicht ist** |
| a) keiner | ---------------- | 350 |
| b) Stabilisator aus Beispiel 1 | 0,1 | 900 |
| c) Stabilisator C* | 0,1 | 700 |
| d) Stabilisator aus Beispiel 1 Stabilisator C | 0,05 0,05 | 896 |

| | | |
|---|---|---|
| * Polymer aus Butandisäure mit 4-Hydroxy-2,2,6,6-tetramethyl-1-(2-hydroxyethyl)-piperidin (®Tinuvin 622, CIBA Specialty Chem.) | | |

Die grundstabilisierte Folie erreicht das Kriterium ΔCO > 1, welches den Polymerabbau beschreibt, nach 350 h. Die stabilisierende Wirkung der zusätzlich eingesetzten Stabilisatoren ergibt sich aus der Stundenzahl, die über die 350 h hinausgehen. So leisten 0.1 Gew.-% an Stabilisator aus Beispiel 1 einen stabilisierenden Beitrag von (900 h - 350 h) = 550 h. Der stabilisierende Beitrag vom Stabilisator C liegt bei (700 h - 350 h) = 350 h. Bei der Bewertung der Ergebnisse ist zu berücksichtigen, daß die Konzentrationen der Stabilisatoren in Beispiel d auf die Hälfte reduziert wurden. Ein additiver stabilisierender Effekt würde bei einer Kombination vom Stabilisator aus Beispiel 1 mit Stabilisator C zu einer Gesamtstabilisierung von (350 h + 550/2 h + 350/2 h) = 800 h führen. Tatsächlich aber führt die Kombination vom Stabilisator aus Beispiel 1 mit Stabilisator C zu einer Gesamtstabilisierung von 896 h; die Kombination ist also synergistisch.

### Beispiel 9: Lichtstabilisierende Wirkung in Polypropylen-Preßfolien

100 Gewichtsteile unstabilisiertes Polypropylen ®Hostalen PPK 0160 wurden zusammen mit 0.1 Gewichtsteilen Pentaerythrityl-tetrakis-[3-(3,5-di- tert.butyl-4-hydroxyphenyl)-propionat (®Hostanox O 10) und 0.1 Gewichtsteilen Calciumstearat vermischt. Diese Grundformulierung wird mit 0.1 Gewichtsteilen des Stabilisators aus Beispiel 1 bzw. einer Kombination des Stabilisators aus Beispiel 1 (0.05 Gew. %) und dem in Tab. 3 angegebenen UV-Absorber (0.05 Gew.-%) in einem Brabender-Mischer 10 min bei 200°C und 20 U/min geknetet und auf diese Weise intensiv homogenisiert. Aus dieser Grundmischung wurden Preßfolien der Dicke 100 µm hergestellt. Die auf diese Weise erhaltenen Prüfkörper werden in einem Schnellbewitterungsgerät (®Xenotest 1200) belichtet. Als Kriterium der Stabilität der Folie wurde die Veränderung des Carbonylindexes während der Belichtung herangezogen, welcher in regelmäßigen Abständen (alle 150 h) gemessen wird. Der Carbonylindex CO wurde hierbei gemäß der Formel CO = E₁₇₂₀/E₂₀₂₀ bestimmt (E = Extinktion); der Endpunkt der Belichtung wird dabei durch die Zunahme des Carbonylindex um eine Einheit festgelegt. Zu Vergleichszwecken wurde ein Prüfkörper unter den gleichen Bedingungen, jedoch ohne den Zusatz des erfindungsgemäßen Stabilisators aus Beispiel 1 getestet.

**Tabelle 4:**

| Abbau von stabilisiertem Polypropylen | | |
|---|---|---|
| **Zusätzlich zur Grundstabilisierung eingesetzter Stabilisator** | **Konzentration in Gew.-%** | **Stunden, bis ΔCO > 1 erreicht ist** |
| a) keiner | ---------------- | 350 |
| b) Stabilisator aus Beispiel 1 | 0,1 | 900 |
| c) Stabilisator D* | 0,1 | 458 |
| d) Stabilisator aus Beispiel 1 Stabilisator D* | 0,05 0,05 | 710 |

| | | |
|---|---|---|
| * Benzotriazol-Stabilisator (®Tinuvin P, CIBA Specialty Chem.) | | |

Die grundstabilisierte Folie erreicht das Kriterium ΔCO > 1, welches den Polymerabbau beschreibt, nach 350 h. Die stabilisierende Wirkung der zusätzlich eingesetzten Stabilisatoren ergibt sich aus der Stundenzahl, die über die 350 h hinausgehen. So leisten 0.1 Gew.-% an Stabilisator aus Beispiel 1 einen stabilisierenden Beitrag von (900 h- 350 h) = 550 h. Der stabilisierende Beitrag vom Stabilisator D liegt bei (458 h- 350 h) = 108 h. Bei der Bewertung der Ergebnisse ist zu berücksichtigen, daß die Konzentrationen der Stabilisatoren in Beispiel d auf die Hälfte reduziert wurden. Ein additiver stabilisierender Effekt würde bei einer Kombination vom Stabilisator aus Beispiel 1 mit Stabilisator C zu einer Gesamtstabilisierung von (350 h + 550/2 h + 108/2 h) = 679 h führen. Tatsächlich aber führt die Kombination vom Stabilisator aus Beispiel 1 mit Stabilisator C zu einer Gesamtstabilisierung von 710 h; die Kombination ist also synergistisch.

### Beispiel 10: Lichtstabilisierende Wirkung in Polypropylen-Preßfolien

100 Gewichtsteile unstabilisiertes Polypropylen ®Hostalen PPK 0160 wurden zusammen mit 0.1 Gewichtsteilen Pentaerythrityl-tetrakis-[3-(3,5-di- tert.butyl-4-hydroxyphenyl)-propionat (®Hostanox O 10) und 0.1 Gewichtsteilen Calciumstearat vermischt. Diese Grundformulierung wird mit 0.1 Gewichtsteilen des Stabilisators aus Beispiel 1 bzw. einer Kombination des Stabilisators aus Beispiel 1 (0.05 Gew. %) und den in Tab. 5 angegebenen sterisch gehinderten Phosphiten (0.05 Gew. %) in einem Brabender-Mischer 10 min bei 200°C und 20 U/min geknetet und auf diese Weise intensiv homogenisiert. Aus dieser Grundmischung wurden Preßfolien der Dicke 100 µm hergestellt. Die auf diese Weise erhaltenen Prüfkörper werden in einem Bewitterungsgerät (®Xenotest 1200) belichtet. Als Kriterium der Stabilität der Folie wurde die Veränderung des Carbonylindexes während der Belichtung herangezogen, welcher in regelmäßigen Abständen (alle 150 h) gemessen wird. Der Carbonylindex CO wurde hierbei gemäß der Formel CO = E₁₇₂₀/E₂₀₂₀ bestimmt (E = Extinktion); der Endpunkt der Belichtung wird dabei durch die Zunahme des Carbonylindex um eine Einheit festgelegt. Zu Vergleichszwecken wurde ein Prüfkörper unter den gleichen Bedingungen, jedoch ohne den Zusatz des erfindungsgemäßen Stabilisators aus Beispiel 1 getestet.

**Tabelle 5:**

| Abbau von stabilisiertem Polypropylen | | |
|---|---|---|
| **Zusätzlich zur Grundstabilisierung eingesetzter Stabilisator** | **Konzentration in Gew.-%** | **Stunden, bis ΔCO > 1 erreicht ist** |
| a) Keiner | -- | 300 |
| b) Stabilisator aus Beispiel 1 | 0.1 | 900 |
| c) Stabilisator E* | 0.1 | 460 |
| d) Stabilisator F** | 0.1 | 400 |
| e) Stabilisator aus Beispiel 1 Stabilisator E* | 0.05 0.05 | 820 |
| f) Stabilisator aus Beispiel 1 Stabilisator F** | 0.05 0.05 | 700 |

| | | |
|---|---|---|
| * Stabilisator E: Bis-(2,4-di-tert.-butylphenyl)-pentaerythritol-diphosphit (®Weston 626, Borg Warner) | | |
| ** Stabilisator F: Tris-(2,4-di-tert.-butylphenyl)-phosphit (Hostanox PAR 24, Clariant GmbH) | | |

Die grundstabilisierte Folie erreicht das Kriterium ΔCO > 1, welches den Polymerabbau beschreibt, nach 300 h. Die stabilisierende Wirkung der zusätzlich eingesetzten Stabilisatoren ergibt sich aus der Stundenzahl, die über die 300 h hinausgehen. So leisten z. B. 0.1 Gew.-% an Stabilisator aus Beispiel 1 einen stabilisierenden Beitrag von (900 h - 300 h) = 600 h. Der stabilisierende Beitrag vom Stabilisator E liegt bei (460 h - 300 h) = 160 h. Bei der Bewertung der Ergebnisse ist zu berücksichtigen, daß die Konzentrationen der Stabilisatoren in Beispiel e und f auf die Hälfte reduziert wurden. Ein additiver stabilisierender Effekt würde bei einer Kombination vom Stabilisator aus Beispiel 1 mit Stabilisator E zu einer Gesamtstabilisierung von (300 h + 600/2 h + 160/2 h) = 680 h führen. Tatsächlich aber führt die Kombination vom Stabilisator aus Beispiel 1 mit Stabilisator E zu einer Gesamtstabilisierung von 820 h; die Kombination ist also synergistisch.

### Beispiel 11: Hydrolyse von Phosphiten (Gewichtszunahme) in Abhängigkeit von der Zeit

Bei der Hydrolyse von Phosphiten handelt es sich um eine Aufnahme von Wasser (z. B. aus der Luftfeuchtigkeit). So nehmen Phosphite einerseits bei der Lagerung, andererseits aber auch in eingearbeiteter Form im Polymer Wasser auf und werden mit zunehmender Hydrolyse unwirksam. Diese Hydrolyse kann sichtbar gemacht werden, indem die Wasseraufnahme eines Phosphits über die Gewichtszunahme beobachtet wird. Hierzu wird eine Menge von 125 g Bis-(2,4-di-tert.-butylphenyl)-pentaerythritol-diphosphit (®Weston 626, Borg Warner) offen in einem Klimaraum bei 23 °C und einer relativen Luftfeuchtigkeit von 50 % gelagert. Mit dem Zweck, die Abnahme der Hydrolyseneigung dieses Phosphits durch den neuen Stabilisator aus Beispiel 1 nachzuweisen, wird eine zweite Probe (diesmal eine homogene Mischung aus 125 g Bis-(2,4-di-tert.-butylphenyl)-pentaerythritol-diphosphit und 5 g des Stabilisators aus Beispiel 1) ebenfalls offen in einem Klimaraum bei 23 °C und einer relativen Luftfeuchtigkeit von 50 % gelagert. Beide Proben lagen in Form von Kristallpulver vor.
Bis-(2,4-di-tert.-butylphenyl)-pentaerythritol-diphosphit nimmt im Laufe von 21 Tagen eine Menge von 3 g Wasser auf; das Gemisch aus Bis-(2,4-di-tert.-butylphenyl)-pentaerythritol-diphosphit und dem Stabilisator aus Beispiel 1 nimmt jedoch nur 2 g Wasser auf.
Damit ist nachgewiesen, daß der Stabilisator aus Beispiel 1 die Hydrolyseneigung von Phosphiten deutlich senkt.

Fazit: Die erfindungsgemäßen Verbindungen der Formel (III) wirken in hervorragender Weise als Stabilisator für polymeres Material. Mischungen der erfindungsgemäßen Verbindungen der Formel (III) mit Monomer-, Polymer-HALS und/oder Phosphiten und/oder Säurefängern wirken stabilisierend auf organisches Material und zeigen ein verbessertes Eigenschaftsprofil gegenüber den Einzelkomponenten, wie z. B. synergistische Effekte in der Lichtschutzwirkung.

## Patentansprüche

1. Verbindungen der allgemeinen Formel (III), 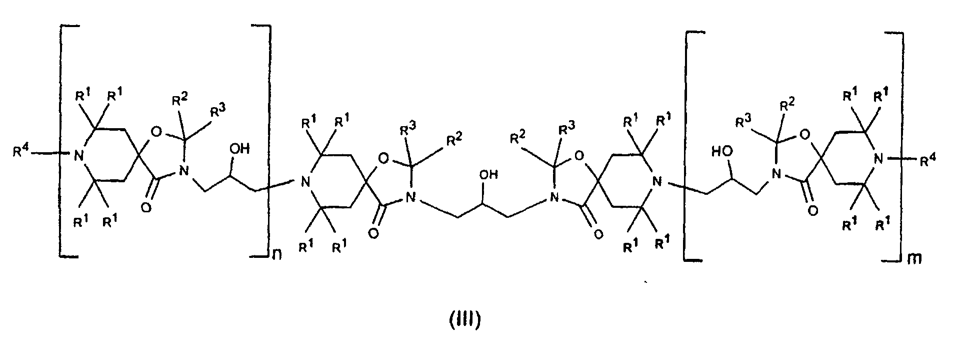 worin
n und m unabhängig voneinander eine Zahl von 0 bis 100 sind, wobei n und m nicht gleichzeitig 0 sein können,
R¹ Wasserstoff, C₅-C₇-Cycloalkyl, oder eine C₁-C₁₂ -Alkylgruppe,
R² und R³ unabhängig voneinander ein Wasserstoffatom, eine C₁-C₁₈ -Alkylgruppe oder zusammen mit dem sie verbindenden C-Atom einen Ring der Ringgröße 5 bis 13 oder zusammen mit dem sie bindenden C-Atom eine Gruppe der Formel (IV), 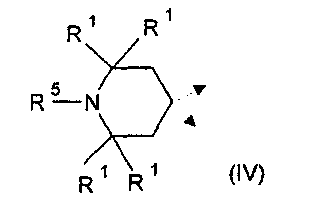
R⁴ und R⁵ unabhängig voneinander entweder Wasserstoff oder eine C₁-C₂₂ - Alkylgruppe, ein Sauerstoffradikal O*, -OH, -NO, -CH₂CN, Benzyl, Allyl, eine C₁-C₃₀-Alkyloxygruppe, eine C₅-C₁₂-Cycloalkyloxygruppe, eine C₆-C₁₀-Aryloxygruppe, wobei der Arylrest zusätzlich noch substituiert sein kann, eine C₇-C₂₀-Arylalkyloxygruppe, wobei der Arylrest zusätzlich noch substituiert sein kann, eine C₃-C₁₀- Alkenylgruppe, eine C₃-C₆-Alkinylgruppe, eine C₁-C₁₀-Acylgruppe, Halogen, unsubstituiertes oder am Phenylring durch C₁-C₄ substituiertes C₇-C₉-Phenylalkyl bedeuten.

2. Verbindungen gemäß Anspruch 1, **dadurch gekennzeichnet, daß**
n und m unabhängig voneinander eine Zahl von 0 bis 10 sind, wobei n und m nicht gleichzeitig 0 sein können,
R¹ Wasserstoff, C₆-Cycloalkyl, oder eine C₁-C₄-Alkylgruppe,
R² und R³ unabhängig voneinander ein Wasserstoffatom, eine C₁-C₈- Alkylgruppe oder zusammen mit dem sie verbindenden C-Atom einen Ring der Ringgröße 6 bis 12, oder zusammen mit dem sie bindenden C-Atom eine Gruppe der Formel (IV),
R⁴ und R⁵ unabhängig voneinander entweder Wasserstoff oder eine C₁-C₅-Alkylgruppe, ein Sauerstoffradikal O*, -OH, -NO, -CH₂CN, Benzyl, Allyl, eine C₁-C₁₀-Alkyloxygruppe, eine C₅-C₆-Cycloalkyloxygruppe, eine C₆-C₇-Aryloxygruppe, wobei der Arylrest zusätzlich noch substituiert sein kann, eine C₇-C₁₀-Arylalkyloxygruppe, wobei der Arylrest zusätzlich noch substituiert sein kann, eine C₃-C₆-Alkenylgruppe, eine C₃-C₆-Alkinylgruppe, eine C₁-C₄-Acylgruppe, Halogen, unsubstituiertes oder am Phenylring durch C₁-C₂-Alkyl substituiertes C₇-C₉-Phenylalkyl bedeuten.

3. Verbindungen gemäß Anspruch 1, **dadurch gekennzeichnet, daß**
n und m unabhängig voneinander eine Zahl von 0 bis 5 sind, wobei n und m nicht gleichzeitig 0 sein können,
R¹ Methyl,
R² und R³ zusammen mit dem sie verbindenden C-Atom einen Ring der Ringgröße 12, oder zusammen mit dem sie verbindenden C-Atom eine Gruppe der Formel (IV)
R⁴ und R⁵ xunabhängig voneinander Wasserstoff, Methyl, Acetyl, Octyloxy oder Cyclohexyloxy, bedeuten.

4. Verbindungen gemäß Anspruch 1 bis 3, **dadurch gekennzeichnet, daß** es sich um Stabilisatoren gegen den schädigenden Einfluß von Sauerstoff, Licht und Wärme handelt .

5. Verfahren zur Herstellung von Verbindungen der Formel (III) gemäß Anspruch 1, **dadurch gekennzeichnet, daß** man Verbindungen der Formel (I) mit Verbindungen der Formel (II) 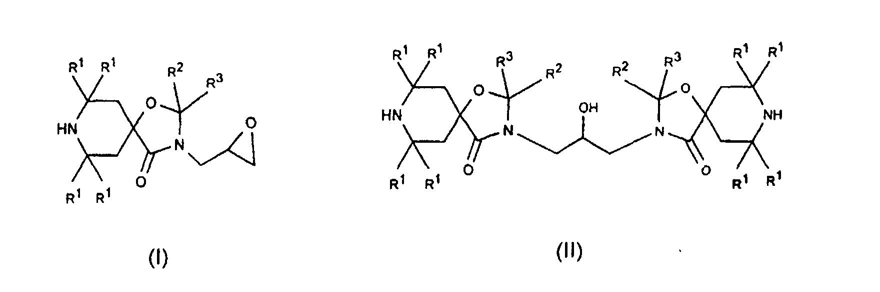 worin R¹ bis R⁴ die in Anspruch 1 angegebenen Bedeutungen besitzen, mit oder ohne Lösemittel und mit oder ohne Katalysator im Molverhältnis 1:1 bis 100:1 und bei 100 bis 300°C umsetzt.

6. Verfahren gemäß Anspruch 5, **dadurch gekennzeichnet, daß** die Umsetzung ohne Lösemittel und ohne Katalysator im Vakuum bei 120 bis 250°C stattfindet.

7. Verwendung von Verbindungen gemäß den Ansprüchen 1 bis 4 zum Stabilisieren von organischem Material gegen die Schädigung durch Licht und Wärme.

8. Gemisch aus einer Verbindung der allgemeinen Formel (III) gemäß Anspruch 1 mit einer oder mehreren Stabilisatoren auf Basis sterisch gehinderter Amine der Formeln A1 bis A10, 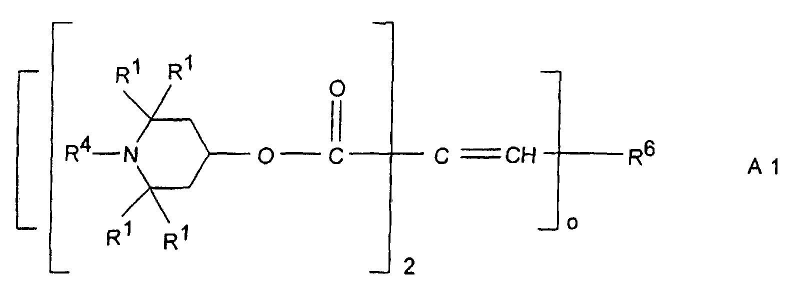 worin
R¹ und R⁴ die in Anspruch 1 angeführten Bedeutungen haben,
R⁶ einen ein oder mehrfach durch Wasserstoff, C₁-C₄-Alkyl, C₁-C₄- Alkoxy, Halogen, Cyan, Carboxy, Nitro, Amino, C₁-C₄-Alkylamino, C₁-C₄-Dialkylamino, oder Acyl substituierten aromatischen Rest,
o 1 oder 2 bedeutet, 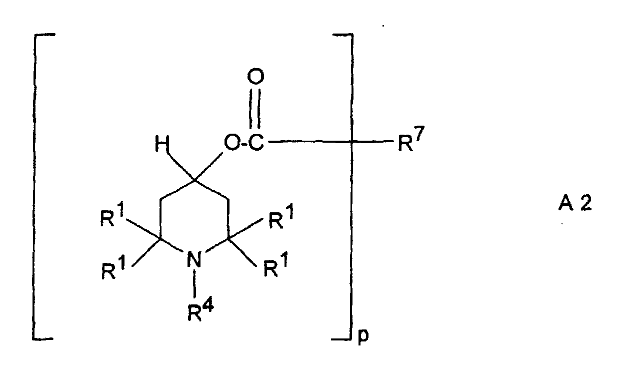
worin
R¹ und R⁴ die in Anspruch 1 angeführten Bedeutungen haben,
p = 1 oder 2 und
für p = 1
R⁷ C₁-C₂₂-Alkyl, C₂-C₁₈-Oxaalkyl, C₂-C₁₈-Thiaalkyl, C₂-C₁₈-Azaalkyl oder C₂-C₈-Alkenyl,
für p = 2
R⁷ C₁-C₂₂-Alkylen, C₂-C₁₈-Oxaalkylen, C₂-C₁₈-Thiaalkylen, C₂-C₁₈-Azaalkylen oder C₂-C₈-Alkenylen, 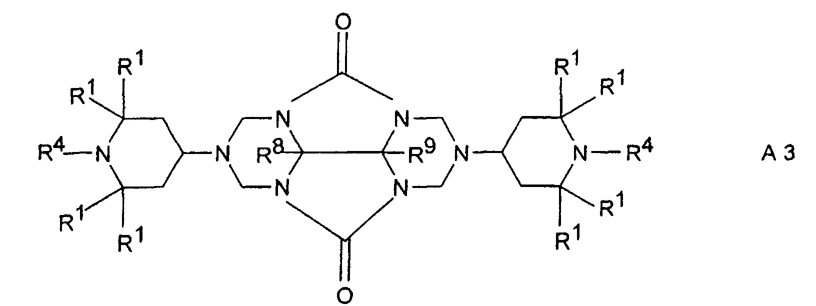 worin
R¹ und R⁴ die in Anspruch 1 angeführten Bedeutungen haben,
R⁸ und R⁹ unabhängig voneinander Wasserstoff, C₁-C₆-Alkyl, C₇-C₁₂-Aralkyl, -Aryl oder Carbonester,
R⁸ und R⁹ zusammen eine Tetra- oder Pentamethylgruppe bedeuten, 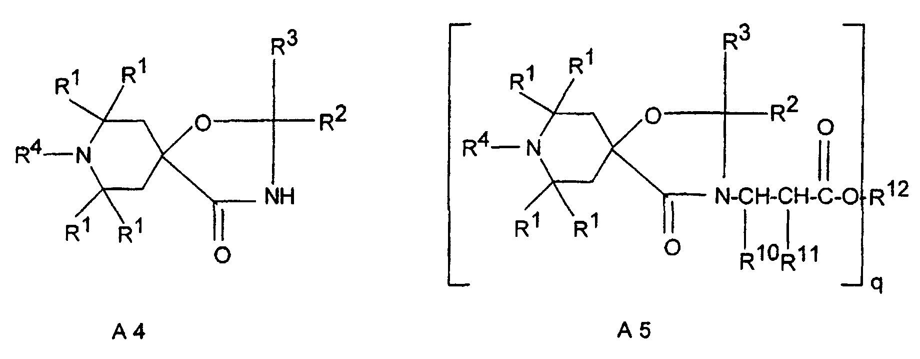
worin
R¹, R², R³ und R⁴ die in Anspruch 1 angeführten Bedeutungen haben,
q eine Zahl von 1 oder 2,
R¹⁰ Wasserstoff, Methyl, Phenyl oder Carb-C₁-C₂₁-Alkoxy,
R¹¹ Wasserstoff oder Methyl,
R¹² für q = 1, Wasserstoff, C₁-C₂₁-Alkyl, C₂-C₂₂-Alkenyl, C₅-C₁₂-Cycloalkyl, ein Radikal der Formel 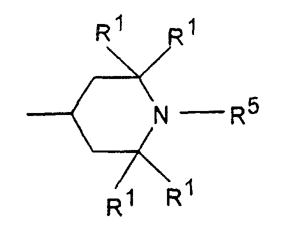
bedeutet,
wobei
R¹ und R⁵ die in Anspruch 1 angeführte Bedeutung haben, und
R¹² für q = 2, C₁-C₁₈-Alkylen, C₅-C₉-Cycloalkylen oder Arylen bedeutet, 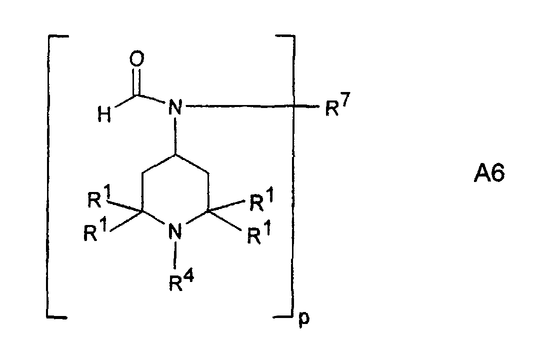
wobei R¹, R⁴, R⁷ und p die oben genannten Definitionen besitzen, 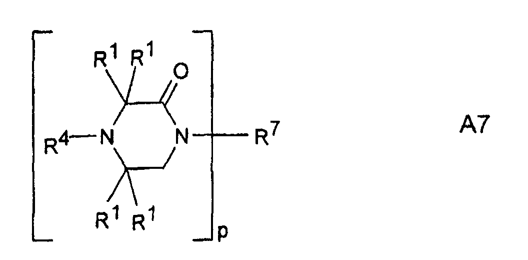 wobei R¹, R⁴, R⁷ und p die oben genannten Definitionen besitzen, 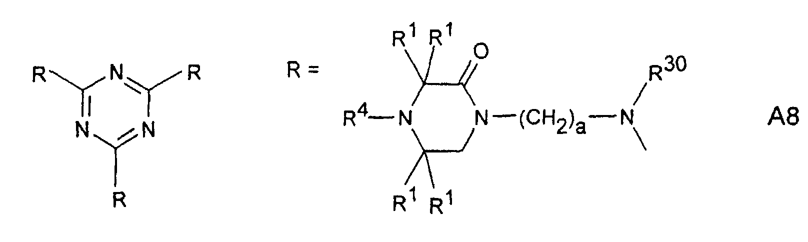 wobei R¹, R⁴ die oben genannten Definitionen besitzen,
R³⁰ Wasserstoff, C₁-C₁₂-Alkyl, C₅-C₁₂-Cycloalkyl, Phenyl oder C₇-C₉-Phenylalkyl, und
a eine Zahl von 1 bis 10 bedeutet, 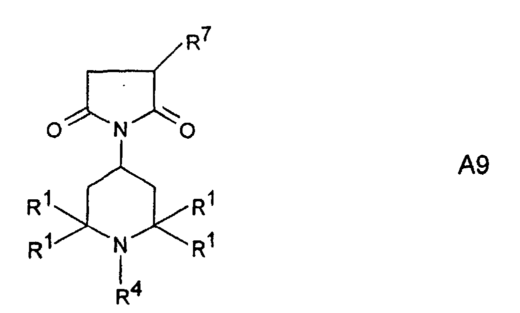
wobei R¹ und R⁴ die obige Bedeutung hat und R⁷ die Bedeutung für R⁷ und p = 1 in der Formel A2 ist;
ein Produkt A10 erhältlich durch Umsetzung eines Polyamins der Formel A10a mit Formel A10b: 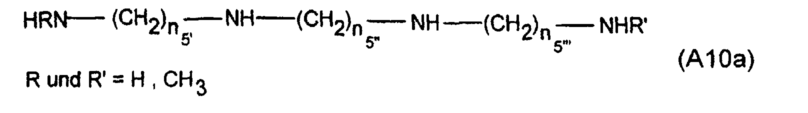 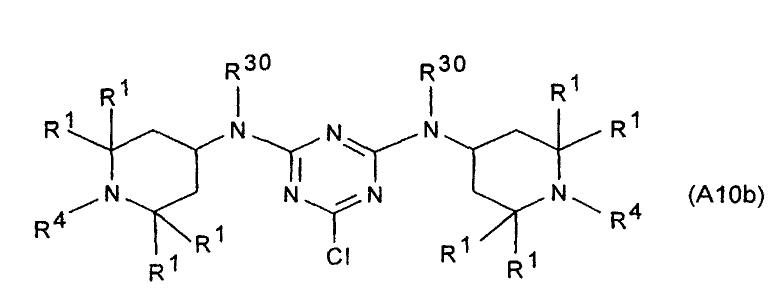 wobei
R¹,R⁴ und R³⁰ die oben angegebene Bedeutung haben,
n_{5'}, n_{5"} und n_{5'''} unabhängig voneinander eine Zahl von 2 bis 12 ist.

9. Gemisch gemäß Anspruch 8, **dadurch gekennzeichnet, daß**
n und m unabhängig voneinander eine Zahl von 0 bis 10 sind, wobei n und m nicht gleichzeitig 0 sein können,
R¹ Wasserstoff oder eine eine C₁-C₄-Alkylgruppe,
R² und R³ unabhängig voneinander ein Wasserstoffatom, eine C₁-C₈-Alkylgruppe oder zusammen mit dem sie verbindenden C-Atom einen Ring der Ringgröße 6 bis 12, oder zusammen mit dem sie bindenden C-Atom eine Gruppe der Formel (IV),
R⁴ und R⁵ unabhängig voneinander entweder Wasserstoff oder eine C₁-C₅-Alkylgruppe. ein Sauerstoffradikal O*, -OH, -NO, -CH₂CN, Benzyl, Allyl, eine C₁-C₁₀-Alkyloxygruppe, eine C₅-C₆-Cycfoalkyloxygruppe, eine C₆-C₇-Aryloxygruppe, wobei der Arylrest zusätzlich noch substituiert sein kann; eine C₇-C₁₀-Arylalkyloxygruppe, wobei der Arylrest zusätzlich noch substituiert sein kann, eine C₃-C₆-Alkenylgruppe, eine C₃-C₆-Alkinylgruppe, eine C₁-C₄-Acylgruppe, Halogen, unsubstituiertes oder am Phenylring durch C₁-C₂-Alkyl substituiertes C₇-C₉-Phenylalkyl,
R⁷ ein geradkettiges C₁-C₁₀-Alkylen (für p = 2); C₁-C₁₂-Alkyl (für p = 1)
R⁸ und R⁹ unabhängig voneinander Wasserstoff, C₁-C₂-Alkyl, C₇-C₈-Arylalkyl, Aryl- oder Carbonsäureester,
R¹⁰ Wasserstoff. Methyl, Phenyl oder C₁-C₂-Alkoxy
R¹¹ Wasserstoff oder Methyl,
R¹² für q = 1 Wasserstoff, C₁-C₁₆-Alkyl, C₂-C₁₆-Alkenyl, C₅-C₆-Cycloalkyl, ein Radikal der Formel 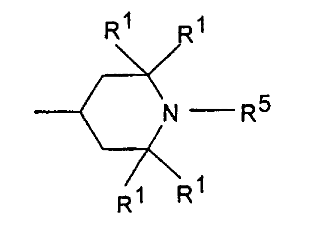
R¹² für q = 2 , C₁-C₁₆-Alkylen, C₅-C₆-Cycloalkylen oder Arylen bedeutet,
R³⁰ Wasserstoff, C₁-C₈-Alkyl, C₅-C₇ - Cycloalkyl, Phenyl oder C₇-C₈-Phenylalkyl,
a 1 bis 5,
o 1 und
p 2 bis 5 bedeutet.

10. Gemisch gemäß Anspruch 8, **dadurch gekennzeichnet, daß**
n und m unabhängig voneinander eine Zahl von 0-5 ist, wobei n und m nicht gleichzeitig 0 sein können,
R¹ Methyl,
R² und R³ zusammen mit dem sie verbindenden C-Atom einen Ring der Ringgröße 12, oder zusammen mit dem sie bindenden C-Atom eine Gruppe der Formel (IV),
R⁴ und R⁵ unabhängig voneinander Wasserstoff, Methyl, Acetyl, Octyloxy oder Cyclohexyloxy,
R⁶ p-Methoxyphenyl,
R⁷ Octamethylen, Hexamethylen oder Ethylen (für p = 2), Dodecyl (für p =1),
R⁸ und R⁹ Wasserstoff,
R¹⁰ Wasserstoff,
R¹¹ Wasserstoff,
R¹² Dodecamethylen oder Tetradecamethylen,
R³⁰ Cyclohexyl oder n-Butyl,
a gleich 2,
o gleich 1,
p gleich 2
und q gleich 1 ist.

11. Gemisch gemäß Anspruch 8, **dadurch gekennzeichnet, daß** es sich bei den HALS-Verbindungen der Mischung mit Verbindungen der Formel (III) um die folgenden Substanzen handelt: 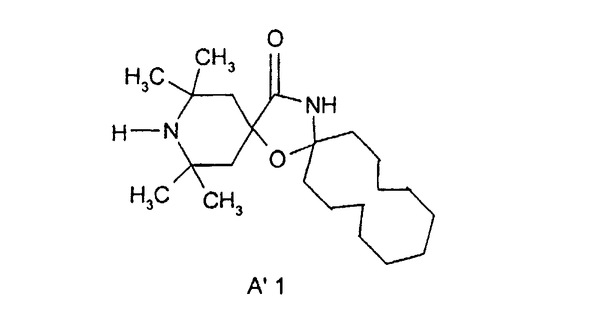 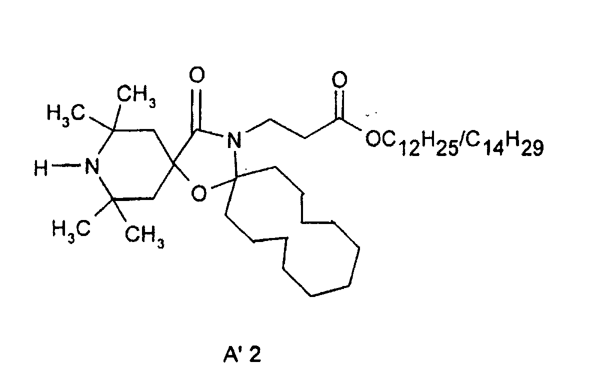 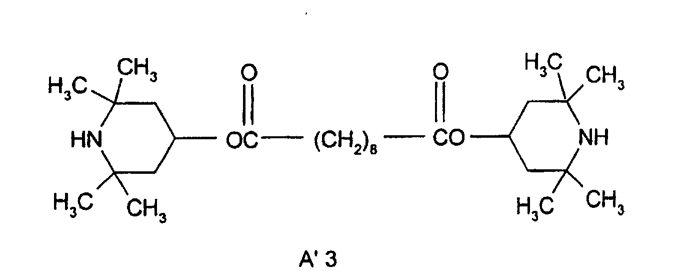 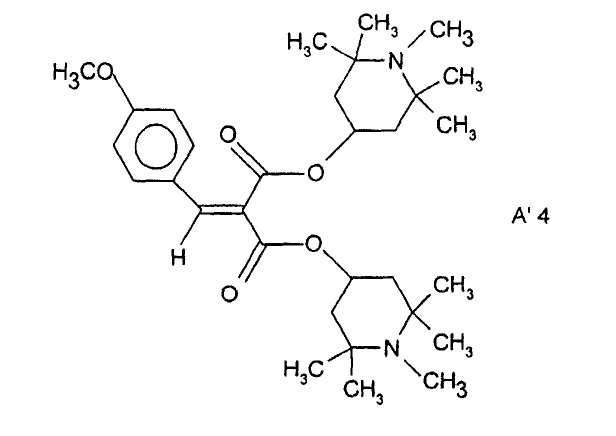 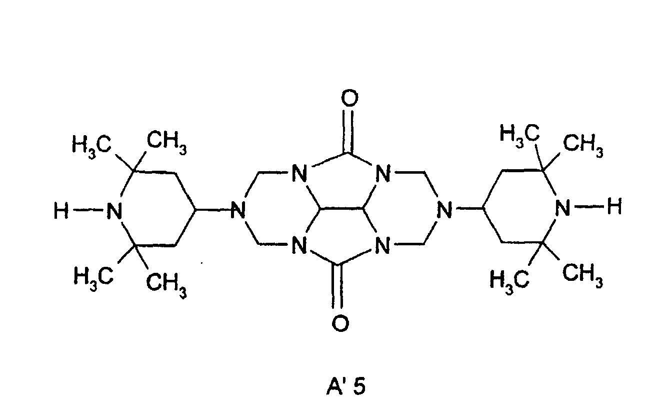 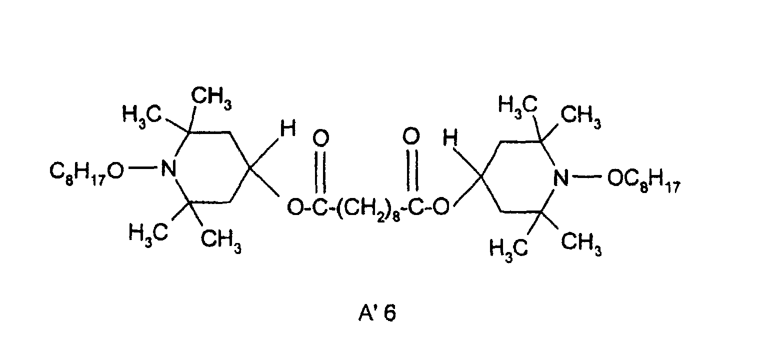 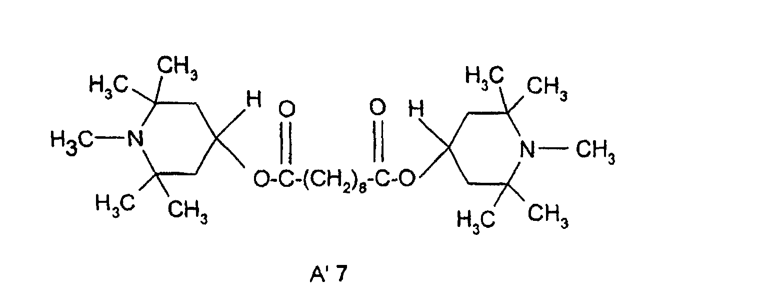 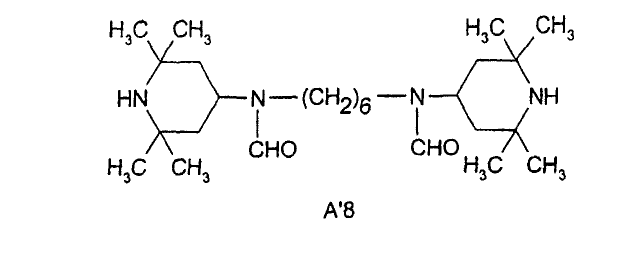 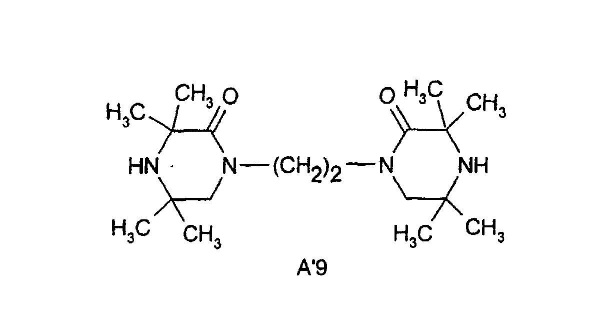 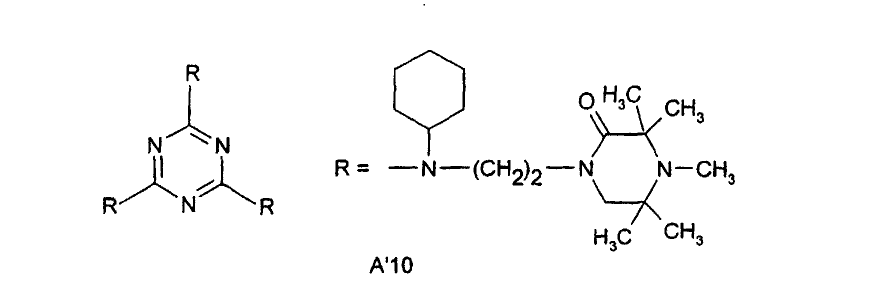 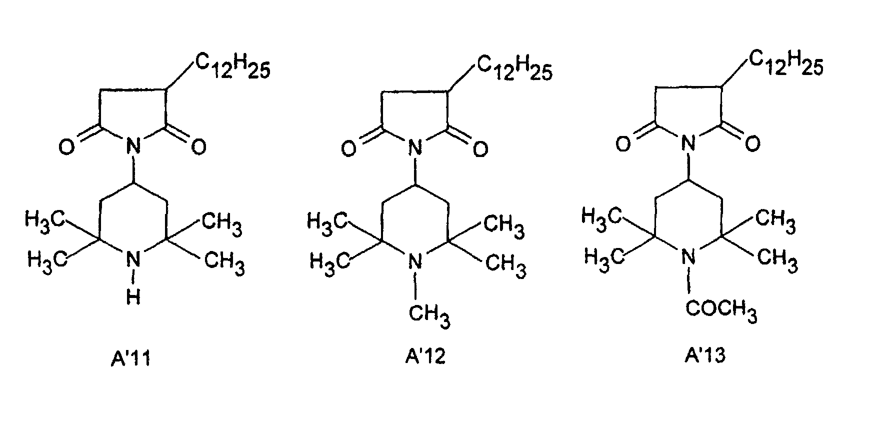 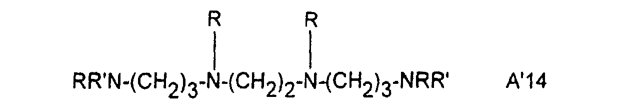 wobei 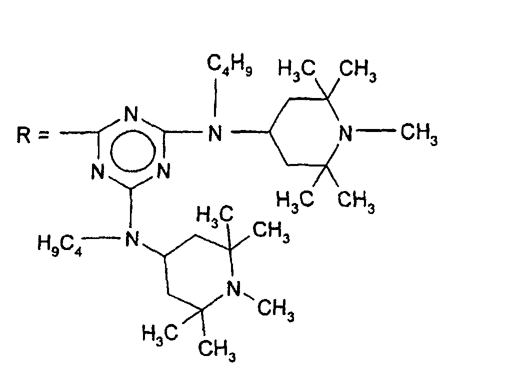 und
R' = H, CH₃

12. Gemisch gemäß Anspruch 8 **dadurch gekennzeichnet, daß** es sich bei der (den) Mischungskomponente(n) um ®Tinuvin 770, ®Tinuvin 765, ®Tinuvin 123, ®Hostavin N 20, ®Hostavin N 24, ®Uvinul 4049, ®Sanduvor PR 31, ®Uvinul 4050, ®Good-rite UV 3034 oder ®Good-rite 3150, ®Sanduvor 3055, ®Sanduvor 3056, ®Sanduvor 3058, ®Chimassorb 119 und ®Chimassorb 905 handelt.

13. Gemisch aus einer Verbindung der allgemeinen Formel (III) gemäß Anspruch 1 mit einer oder mehrerer polymerer HALS-Verbindungen der Formeln B1 bis B7 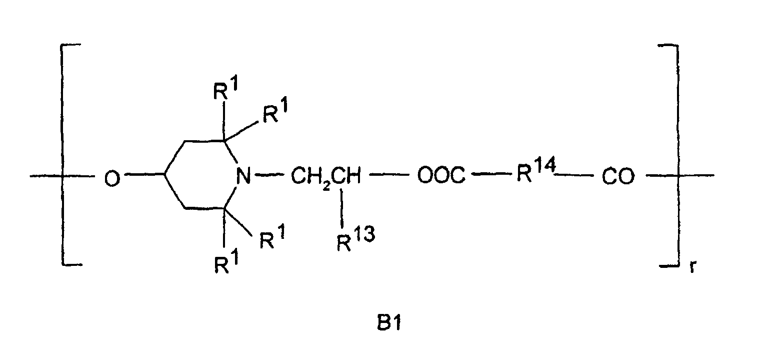 worin
R¹ die in Anspruch 1 angegebene Bedeutung hat,
R¹³ Wasserstoff oder Methyl,
R¹⁴ eine direkte Bindung oder C₁-C₁₀-Alkylen und
r eine Zahl von 2 bis 50 bedeutet, 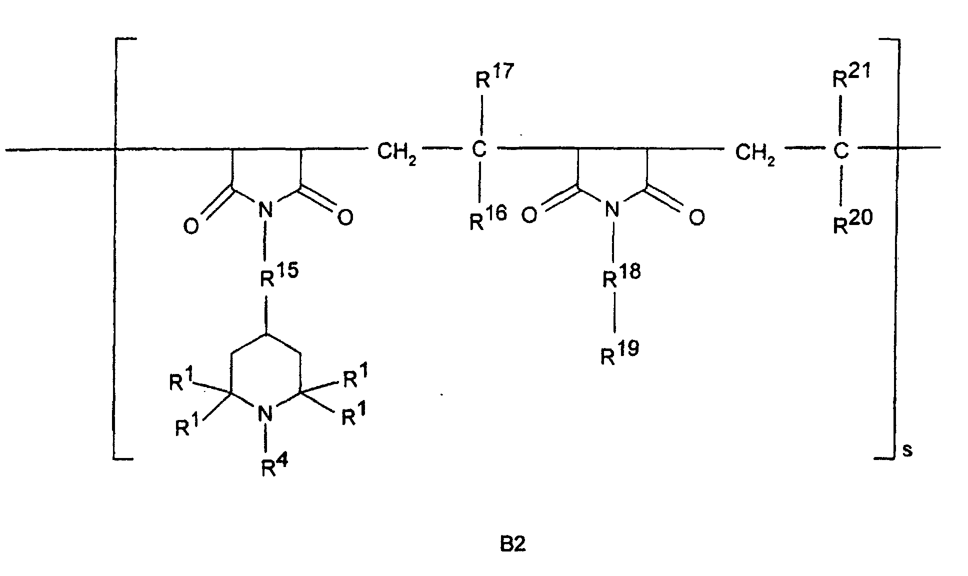
wobei
R¹ und R⁴ die in Anspruch 1 angegebenen Bedeutungen haben,
R¹⁵ und R¹⁸ unabhängig voneinander eine direkte Bindung oder eine Gruppe -N(R²²)-CO- R²³-CO-N(R²⁴)-,
R²² und R²⁴ unabhängig voneinander Wasserstoff, C₁-C₈-Alkyl, C₅-C₁₂-Cyclolalkyl, Phenyl, C₇-C₉-Phenylalkyl, oder eine Gruppe der Formel 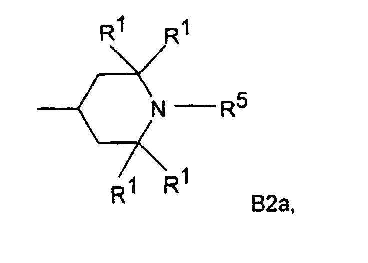
R²³ eine direkte Bindung oder C₁-C₄-Alkylen,
R¹⁶, R¹⁷, R²⁰, R²¹ unabhängig voneinander Wasserstoff, C₁-C₃₀-Alkyl, C₅-C₁₂-Cycloalkyl, Phenyl, oder eine Gruppe der Formel B2a,
R¹⁹ Wasserstoff, C₁-C₃₀-Alkyl, C₅-C₁₂-Cycloalkyl, C₇-C₉-Phenylalkyl, Phenyl oder eine Gruppe der Formel B2a bedeuten und
s eine Zahl von 1 bis 50 ist, 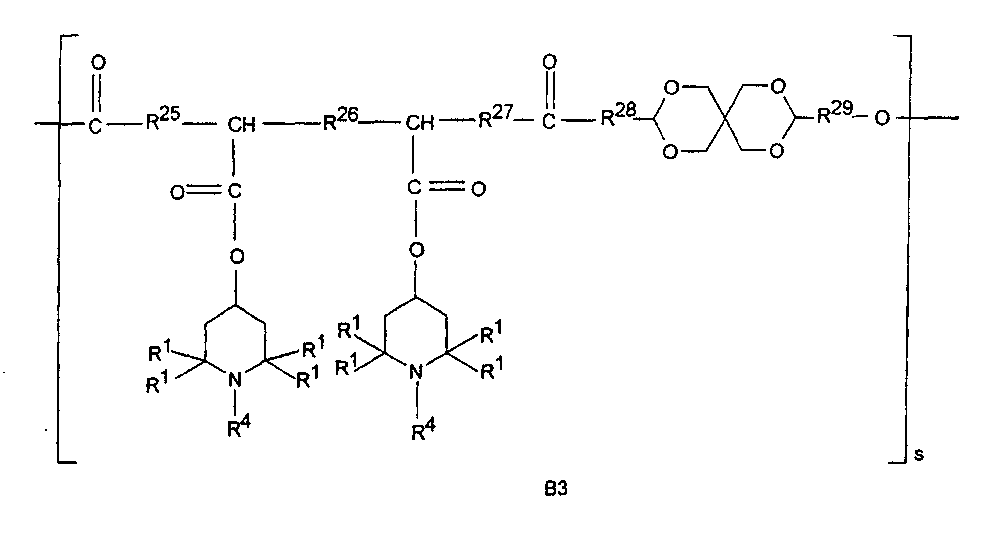
wobei
R¹, R⁴ und s die oben angegebenen Bedeutungen haben,
R²⁵, R²⁶, R²⁷, R²⁸ und R²⁹ unabhängig voneinander eine direkte Bindung oder C₁-C₁₀-Alkylen sind,
ein Produkt B4 erhältlich durch Umsetzung eines durch Reaktion eines Polyamins der Formel B4a mit Cyanursäurechlorid erhaltenen Produktes mit einer Verbindung der Formel B4b, 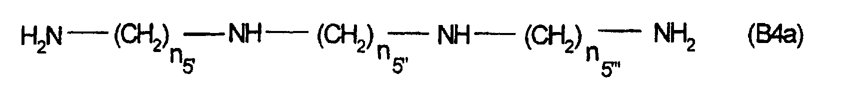 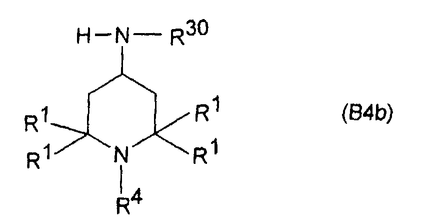 wobei
R¹ und R⁴ die in Anspruch 1 angegebenen Bedeutungen haben,
n_{5'}, n_{5"} und n_{5"'} unabhängig voneinander eine Zahl von 2 bis 12 ist,
R³⁰ Wasserstoff, C₁-C₁₂-Alkyl, C₅-C₁₂-Cycloalkyl, Phenyl oder C₇-C₉-Phenylalkyl ist; wobei B4 eine Verbindung der Formel B4-1, B4-2, B4-3 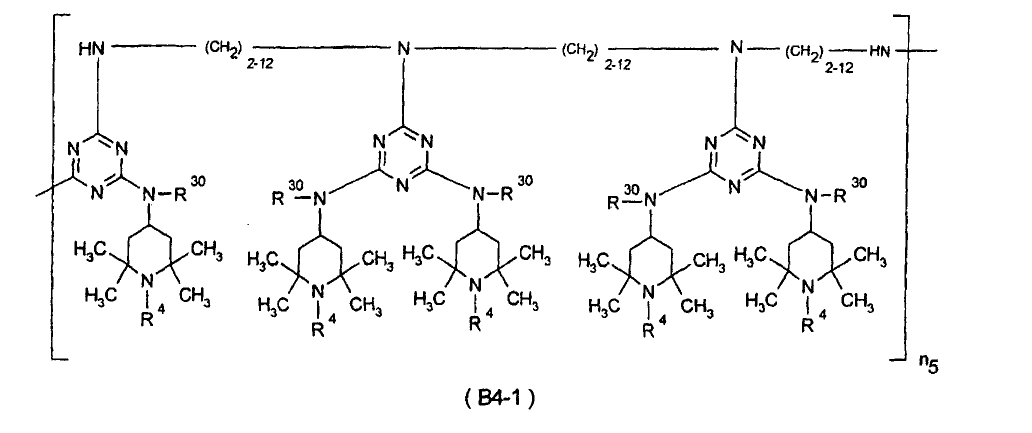 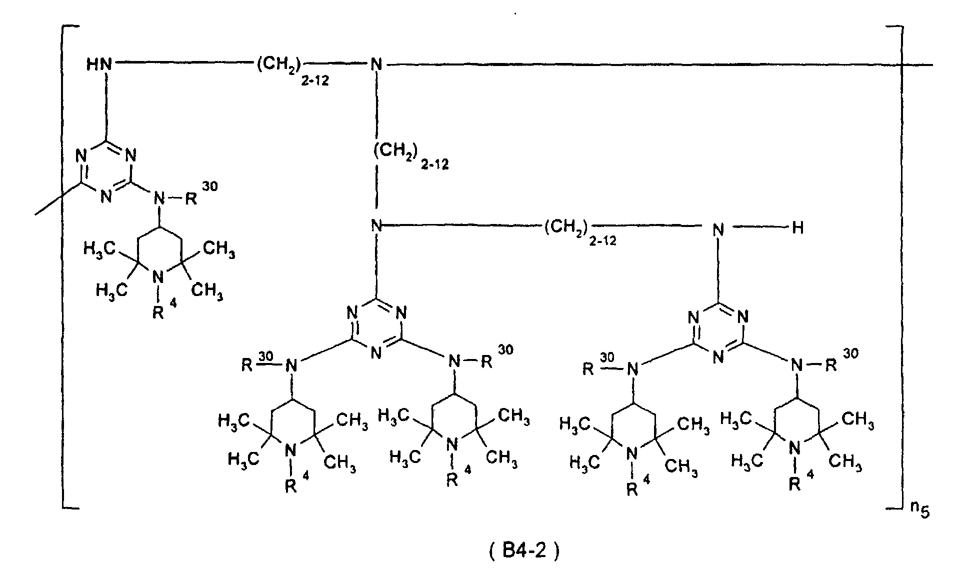 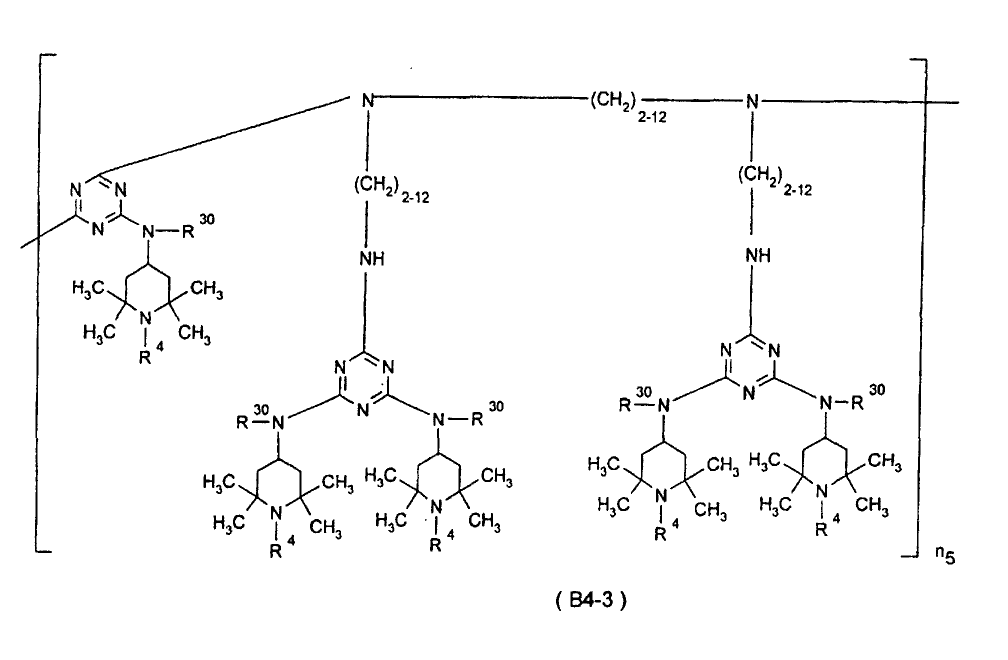
oder ein Gemisch daraus darstellt, worin
n₅ 1 bis 20,
R⁴ und R³⁰ die oben angegebene Bedeutung haben, 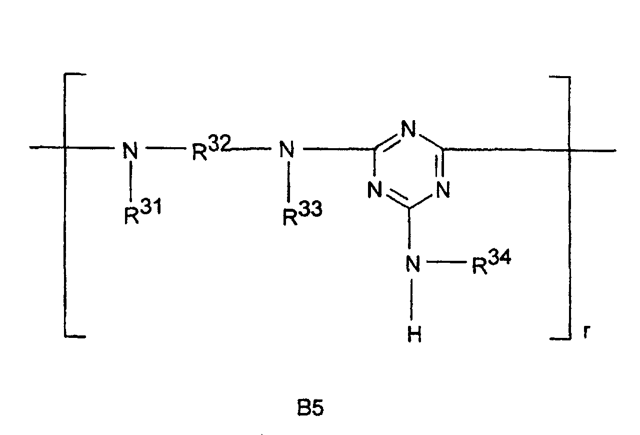
wobei
r die bei Formel B1 angegebene Bedeutung hat,
R³¹, R³³ und R³⁴ unabhängig voneinander Wasserstoff, C₁-C₁₂-Alkyl, C₅-C₁₂-Cycloalkyl, durch C₁-C₄-Alkyl substituiertes C₅-C₁₂-Cycloalkyl, Phenyl, durch-OH und/oder C₁-C₁₀-Alkyl substituiertes Phenyl, C₇-C₉-Phenylalkyl, am Phenylrest durch -OH und/oder C₁-C₁₀-Alkyl substituiertes C₇-C₉-Phenylkalkyl oder eine Gruppe der Formel B5a bedeuten, 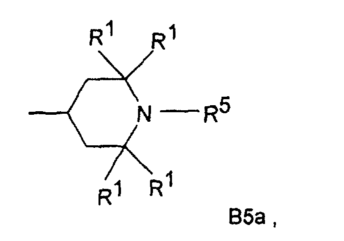
wobei
R¹ und R⁵ die in Anspruch 1 angegebenen Bedeutungen haben, und
R³¹ und R³³ zusätzlich unabhängig voneinander Wasserstoff darstellen,
R³² C₂-C₁₈-Alkylen, C₅-C₇-Cycloalkylen oder C₁-C₄-Alkylendi(C₅-C₇-Cyloalkylen) ist oder die Reste R³¹, R³² und R³³ zusammen mit den Stickstoffatomen, an die sie gebunden sind, einen 5- bis 10-gliedrigen heterocyclischen Ring bilden, und wobei mindestens einer der Reste R³¹, R³³ und R³⁴ eine Gruppe der Formel B5a darstellt; 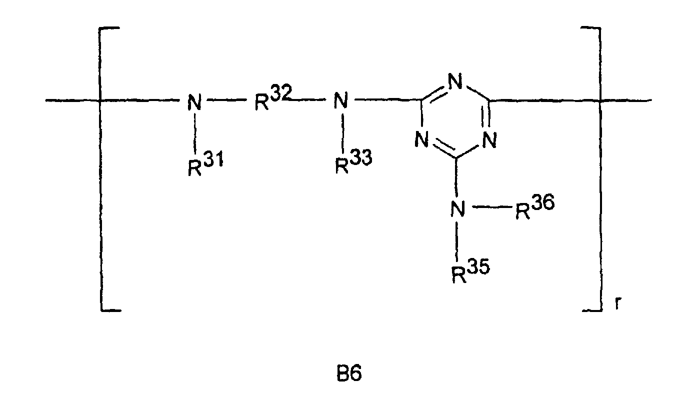
worin
R³¹, R³², R³³ und r die oben angegebenen Bedeutungen haben,
R³⁵ und R³⁶ unabhängig voneinander die Definition von R³⁴ haben kann, oder R³⁵ und R³⁶ zusammen mit dem Stickstoffatom, an das sie gebunden sind, einen 5- bis 10-gliedrigen heterozyklischen Ring bilden, wobei dieser zusätzlich zum Stickstoff-Heteroatom noch ein oder mehrere Heteroatome, vorzugsweise ein Sauerstoffatom, enthalten kann und mindestens einer der Reste R³¹, R³³, R³⁵ und/oder R³⁶ eine Gruppe der Formel (B5a) darstellt, 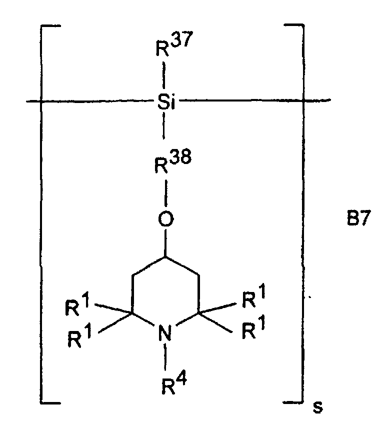
wobei
R¹ und R⁴ die in Anspruch 1 angegebene Bedeutung haben,
s die in Formet B3 angegebenen Bedeutung hat,
R³⁷ C₁-C₁₀-Alkyl, C₅-C₁₂-Cycloalkyl, durch C₁-C₄-Alkyl substituiertes C₅-C₁₂-Cycloalkyl, Phenyl oder durch C₁-C₁₀-Alkyl substituiertes Phenyl ist, und
R³⁸ C₃-C₁₀-Alkylen darstellt.

14. Gemisch gemäß Anspruch 13, **dadurch gekennzeichnet, daß**
n und m unabhängig voneinander eine Zahl von 0 bis 10 sind, wobei n und m nicht gleichzeitig 0 sein können,
R¹ Wasserstoff oder eine C₁-C₄-Alkylgruppe,
R² und R³ unabhängig voneinander ein Wasserstoffatom, eine C₁-C₈-Alkylgruppe oder zusammen mit dem sie verbindenden C-Atom einen Ring der Ringgröße 6 bis 12, oder zusammen mit dem sie bindenden C-Atom eine Gruppe der Formel (IV),
R⁴ und R⁵ unabhängig voneinander entweder Wasserstoff oder eine C₁-C₅-Alkylgruppe, ein Sauerstoffradikal O*, -OH, -NO, -CH₂CN, Benzyl, Allyl, eine C₁-C₁₀-Alkyloxygruppe, eine C₅-C₆-Cycloalkyloxygruppe, eine C₆-C₇-Aryloxygruppe, wobei der Arylrest zusätzlich noch substituiert sein kann, eine C₇-C₁₀-Arylalkyloxygruppe, wobei der Arylrest zusätzlich noch substituiert sein kann, eine C₃-C₆-Alkenylgruppe, eine C₃-C₆-Alkinylgruppe, eine C₁-C₄-Acylgruppe, Halogen, unsubstituiertes oder am Phenylring durch C₁-C₂-Alkyl substituiertes C₇-C₉-Phenylalkyl bedeuten,
R¹³ Wasserstoff oder Methyl,
R¹⁴ C₁-C₅-Alkylen,
R¹⁷, R²¹ Wasserstoff oder C₁-C₄-Alkyl,
R¹⁵, R¹⁸ eine direkte Bindung,
R¹⁶, R²⁰ C₁-C₂₅-Alkyl, Phenyl,
R¹⁹ Wasserstoff, C₁-C₁₂-Alkyl oder eine Gruppe der Formel B2a
R²⁵, R²⁶, R²⁷, R²⁸ und R²⁹ unabhängig voneinander eine direkte Bindung oder C₁-C₅-Alkylen,
R³⁰ Wasserstoff, C₁-C₄-Alkyl, C₅-C₆-Cycloalkyl, Phenyl,
R³¹, R³³ und R³⁴ unabhängig voneinander Wasserstoff, C₁-C₁₀-Alkyl, C₅-C₆-Cycloalkyl oder eine Gruppe der Formel B5a bedeutet,
R³² C₂-C₁₀-Alkylen, C₅-C₆-Cycloalkylen
R³⁵ und R³⁶ unabhängig voneinander die Definition von R³⁴, oder R³⁵ und R³⁶ zusammen mit dem N-Atom, an das sie gebunden sind einen 5 bis 7-gliedrigen heterocyclischen Ring bilden, wobei dieser noch ein- oder mehrere Heteroatome, vorzugsweise ein O-Atom enthalten kann und mindestens einer der Reste R³¹,R³³,R³⁵ und/oder R³⁶ eine Gruppe der Formel B5a darstellt
R³⁷ C₁-C₅-Alkyl, C₅-C₆-Cycloalkyl oder Phenyl
R³⁸ C₃-C₅-Alkylen und
n_{5'},n_{5''},n_{5'''} 2 bis 4 bedeuten.

15. Gemisch gemäß Anspruch 13, **dadurch gekennzeichnet, daß** n und m unabhängig voneinander eine Zahl von 0 bis 5 sind, wobei n und m nicht gleichzeitig 0 sein können,
R¹ Methyl,
R² und R³ zusammen mit dem sie verbindenden C-Atom einen Ring der Ringgröße 12, oder zusammen mit dem sie bindenden C-Atom eine Gruppe der Formel (IV),
R⁴ und R⁵ unabhängig voneinander Wasserstoff, Acetyl, Methyl, Octyloxy oder Cyclohexyloxy,
R¹³ Wasserstoff,
R¹⁴ Ethylen,
R¹⁷, R²¹ Wasserstoff oder Methyl,
R¹⁵, R¹⁸ eine direkte Bindung,
R¹⁶, R²⁰ C₁-C₂₅-Alkyl, Phenyl,
R¹⁹ Hexadecyl oder eine Gruppe der Formel B2a,
R²⁵, R²⁷ Methylen,
R²⁶ eine direkte Bindung,
R²⁸ 2,2-Dimethylethylen,
R²⁹ 1,1-Dimethylethylen,
R³⁰ n-Butyl,
R³¹, R³³ und R³⁴ unabhängig voneinander Isooctyl, Cyclohexyl oder 2,2,6,6-Tetramethylpiperid-4-yl bedeuten, wobei mindestens einer der Reste R³¹, R³³ und R³⁴ 2,2,6,6-Tetramethylpiperid-4-yl bedeuten muß,
R³² Hexamethylen
R³⁵ und R³⁶ unabhängig voneinander die Definition von R³⁴, oder R³⁵ und R³⁶ zusammen mit dem N-Atom, an das sie gebunden sind einen 6-gliedrigen heterocyclischen Ring bilden, wobei dieser noch ein Sauerstoffatom enthält und folglich Morpholin ist, wobei mindestens einer der Reste R³¹,R³³,R³⁵ und/oder R³⁶ einen Rest 2,2,6,6-Tetramethylpiperid-4-yl bedeuten muß,
R³⁷ Methyl,
R³⁸ Trimethylen,
n_{5'},n_{5"},n_{5"'} 2 bis 4 bedeuten.

16. Gemisch gemäß Anspruch 13, **dadurch gekennzeichnet, daß** es sich bei den polymeren HALS-Verbindungen in Kombination mit Verbindungen der Formel (III) um die folgenden Substanzen handelt: 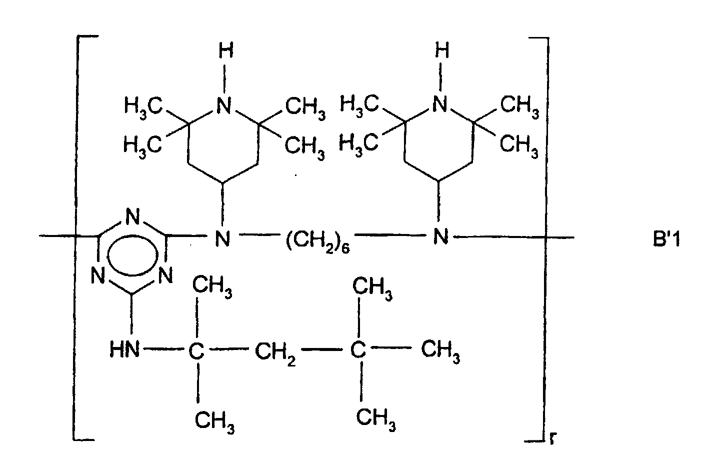 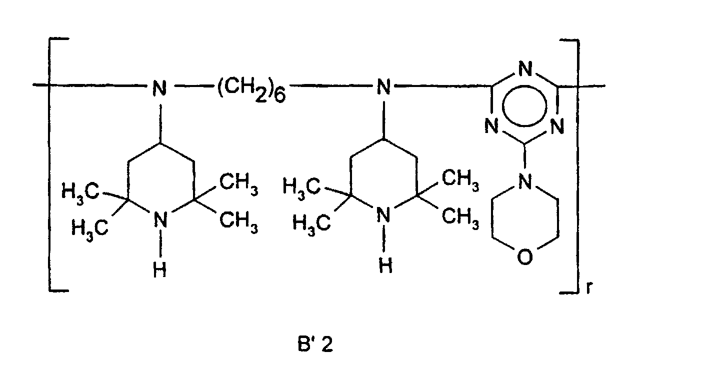 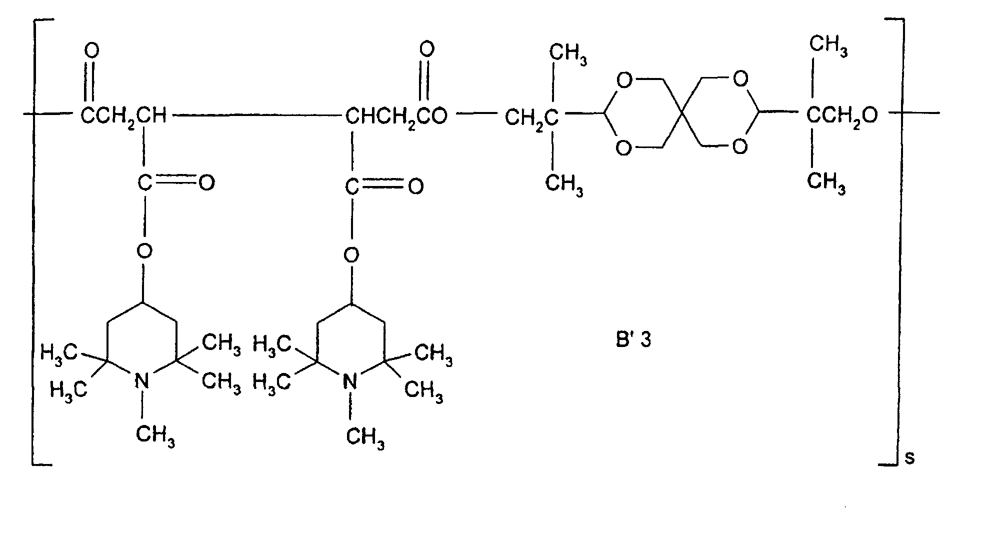 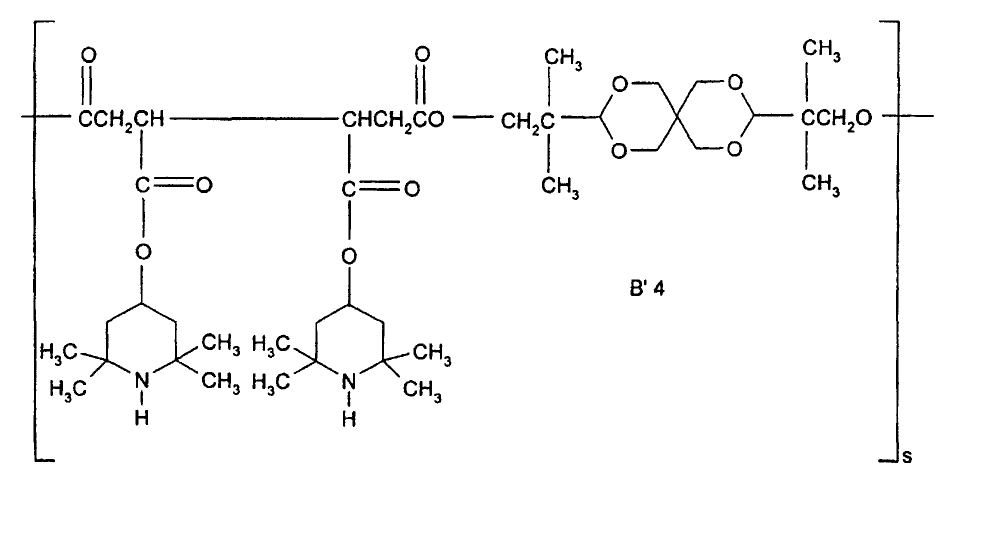 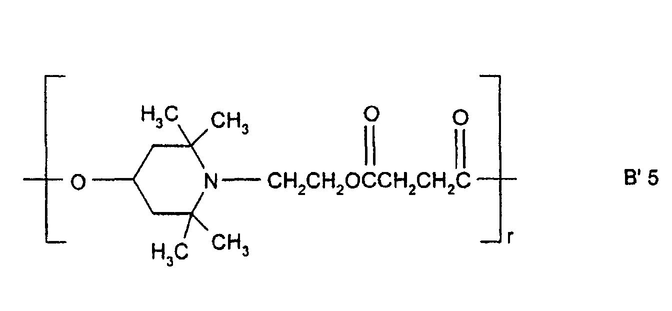 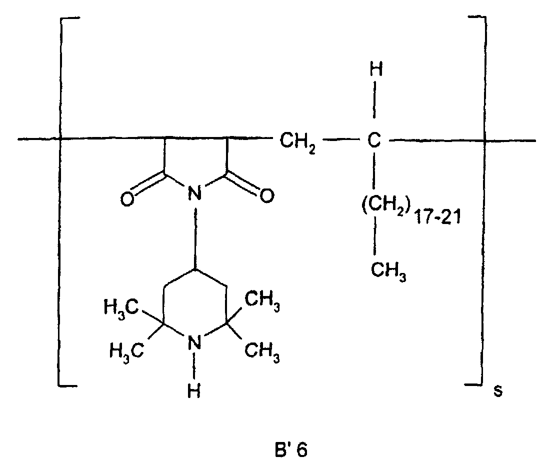 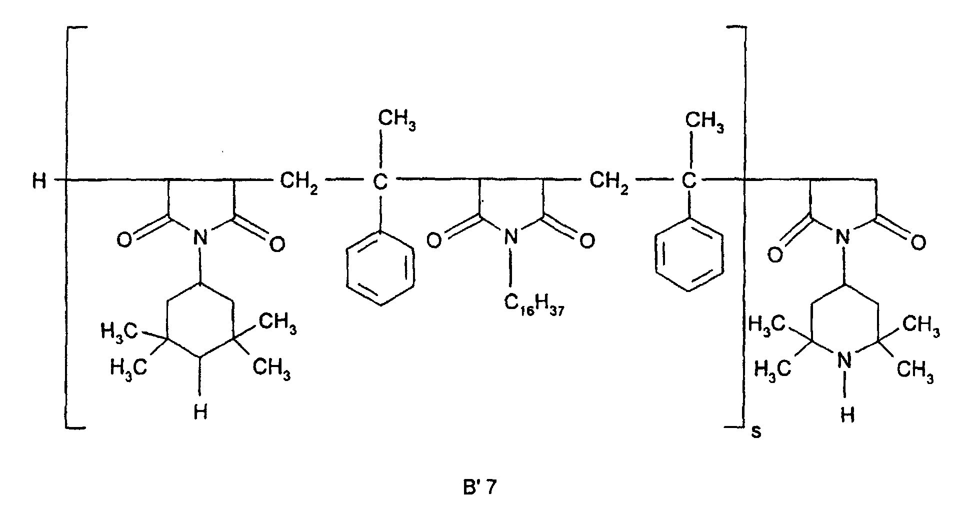 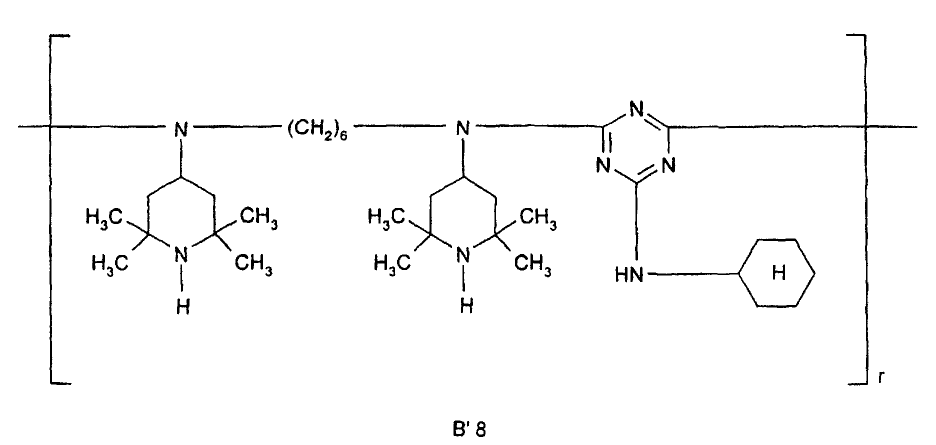 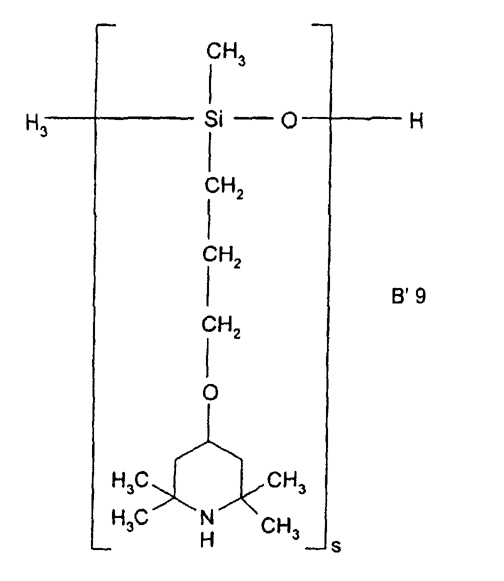 ein Produkt B'10 erhältlich durch Umsetzung eines durch Reaktion eines Polyamins der Formel B'10a:
H₂N-(CH₂)₃-NH-(CH₂)₂-NH-(CH₂)₃-NH₂ (B'10a)
mit Cyanursäurechlorid erhaltenen Produktes mit einer Verbindung der Formel (B'10b) 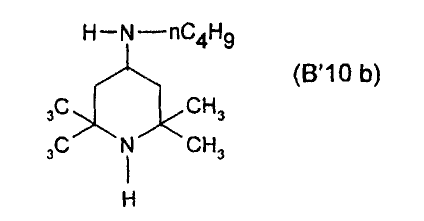 wobei B'10 eine Verbindung der Formel B4-1', B4-2', B4-3' 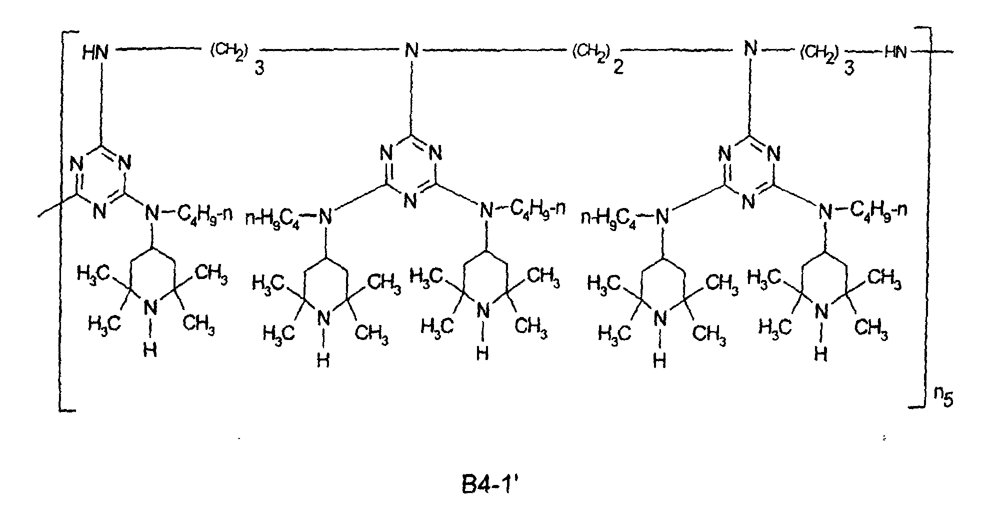 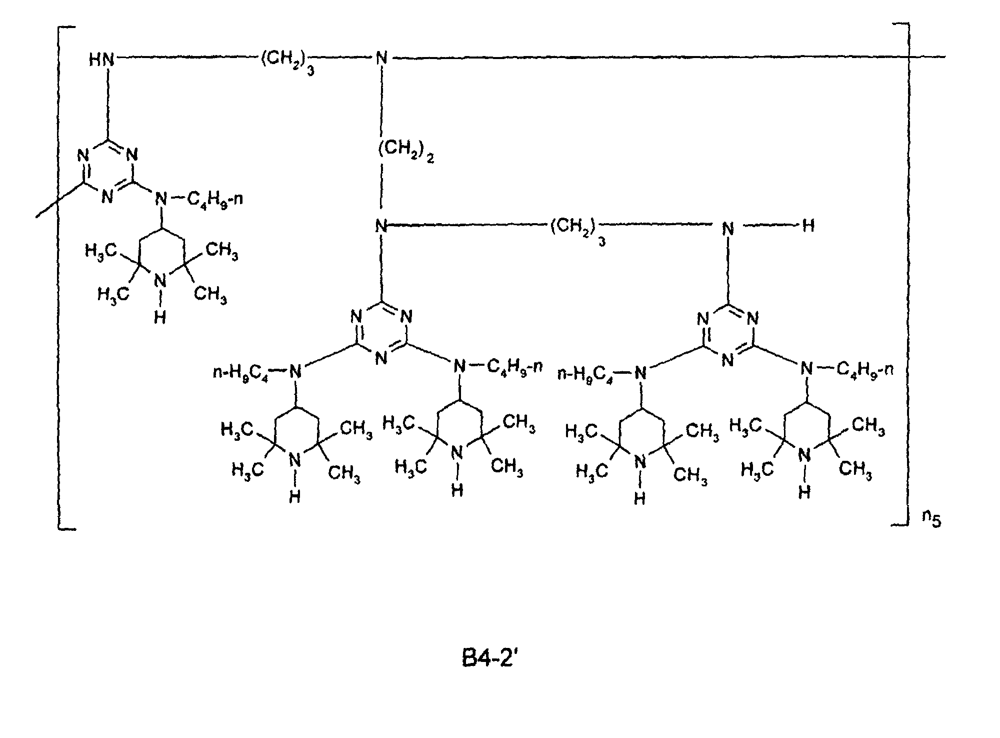 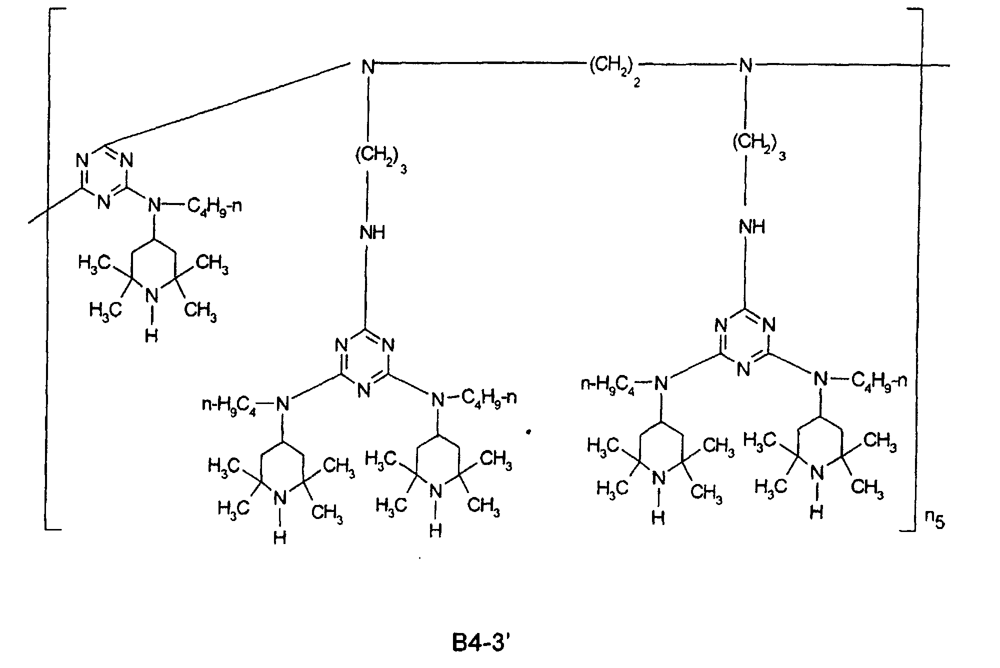 oder ein Gemisch daraus bedeutet, wobei n₅ 1 bis 20 bedeutet.

17. Gemisch gemäß Anspruch 13, **dadurch gekennzeichnet, daß** es sich in Mischungen mit Verbindungen der Formel (III) bei der (den) übrigen Mischungskomponente(n) um ®Chimassorb 944, ®Tinuvin 622, ®Dastib 1082, ®Uvasorb HA 88, ®Uvinul 5050, ®Lowilite 62, ®Uvasil 299, ®Cyasorb 3346, ®MARK LA 63, ®MARK LA 68 oder ®Luchem B 18 handelt.

18. Gemisch aus der allgemeinen Formel (III) gemäß Anspruch 1 und einem oder mehreren Phosphor-Stabilisatoren der allgemeinen Formeln C1 bis C7 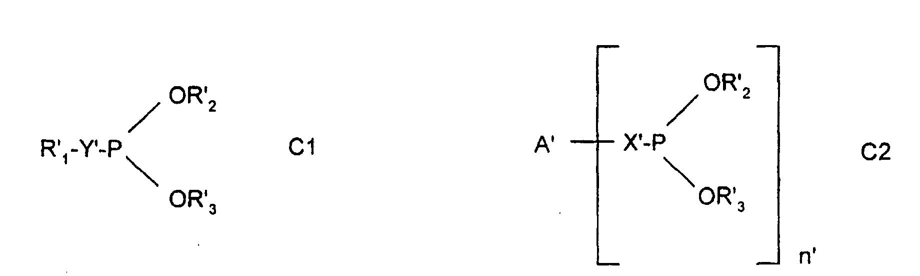 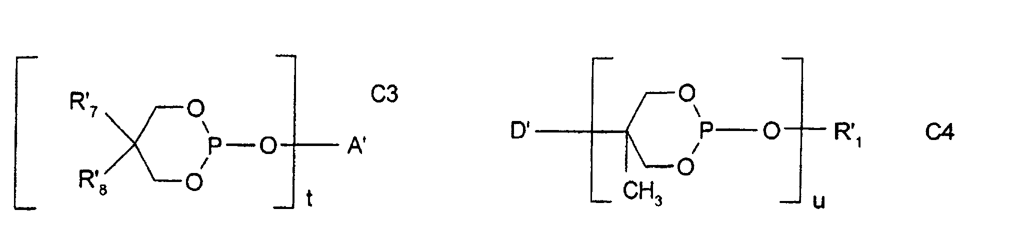 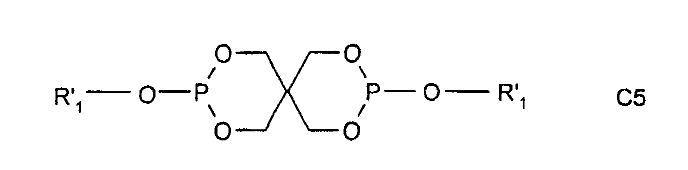 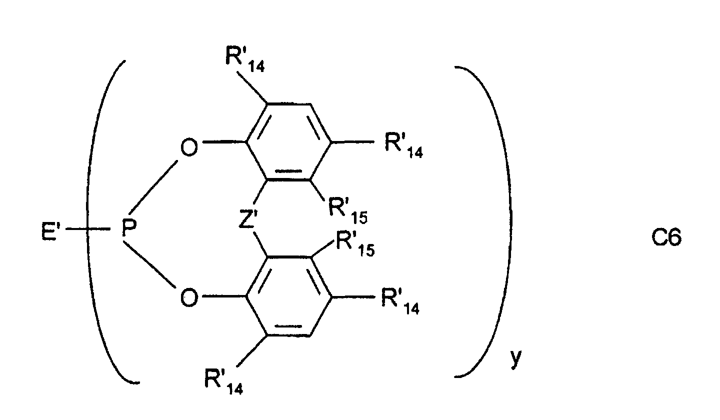 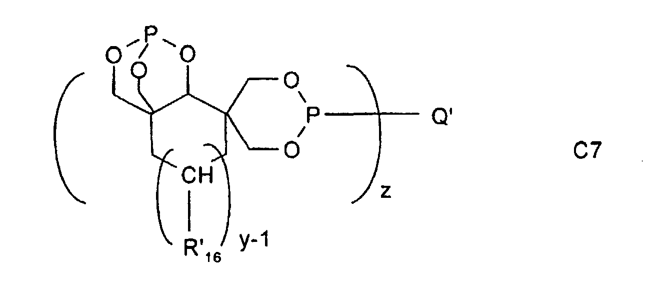 worin die Indices ganzzahlig sind und
n' 2, 3 oder 4;
u 1 oder 2;
t 2 oder 3;
y 1, 2 oder 3; und
z 1 bis 6 bedeutet;
A', wenn n' 2 ist, Alkylen mit 2 bis 18 Kohlenstoffatomen; durch -S-, -O- oder -NR'₄-unterbrochenes Alkylen mit 2 bis 12 Kohlenstoffatomen; ein Rest einer der Formeln 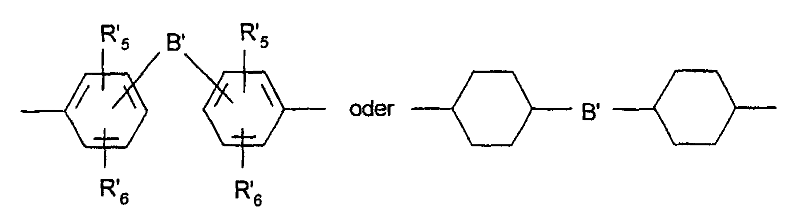 oder Phenylen ist,
A', wenn n' 3 ist, ein Rest der Formel -CᵣH₂ᵣ₋₁ ist;
A', wenn n' 4 ist, den Rest der Formel 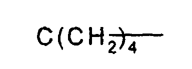 bedeutet;
A" die Bedeutung von A', wenn n' 2 ist, hat,
B' einen Rest der Formel -CH₂-, -CHR'₄-, -CR'₁R'₄-, -S- oder eine direkte Bindung oder C₅-C₇-Cycloalkyliden; oder mit 1 bis 4 C₁-C₄-Alkylresten in Position 3, 4 und/oder 5 substituiertes Cyclohexyliden bedeutet,
D', wenn u 1 ist, Methyl und, wenn u 2 ist, -CH₂OCH₂- bedeutet;
E', wenn y 1 ist, Alkyl mit 1 bis 18 Kohlenstoffatomen, Phenyl, ein Rest der Formel -OR'₁ oder Halogen ist,
E', wenn y 2 ist, ein Rest der Formel -O-A"-O- ist,
E', wenn y 3 ist, ein Rest der Formel 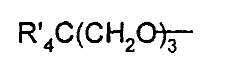 oder
N(CH₂-CH₂-O-)₃
ist,
Q' für den Rest eines mindestens z-wertigen Alkohols oder Phenols steht, wobei dieser über das (die) alkoholische(n) bzw. phenolische(n) O-Atom(e) an das (die) P-Atom(e) gebunden ist;
R'₁, R'₂ und R'₃ unabhängig voneinander C₁-C₃₀-Alkyl, mit Halogen, -COOR₄', -CN oder -CONR₄'R₄' substituiertes C₁-C₁₈-Alkyl, durch -S-, -O- oder -NR'₄-unterbrochenes C₂-C₁₈-Alkyl, Phenyl-C₁-C₄-alkyl, C₅-C₁₂-Cycloalkyl, Phenyl oder Naphthyl, mit Halogen, 1 bis 3 Alkylresten oder Alkoxyresten mit insgesamt 1 bis 18 Kohlenstoffatomen oder mit Phenyl-C₁-C₄-Alkyl substituiertes Phenyl oder Naphthyl; oder ein Rest der Formel 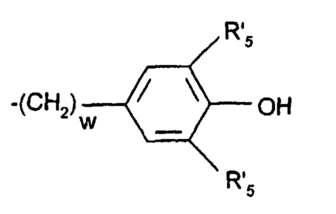 sind, worin w eine ganze Zahl aus dem Bereich 3 bis 6 bedeutet;
R'₄ beziehungsweise die Reste R'₄ unabhängig voneinander Wasserstoff, C₁-C₁₈-Alkyl, C₅-C₁₂-Cycloalkyl oder Phenylalkyl mit 1 bis 4 Kohlenstoffatomen im Alkylteil;
R'₅ und R'₆ unabhängig voneinander Wasserstoff, C₁-C₈-Alkyl oder C₅-C₆-Cycloalkyl,
R'₇ und R'₈, im Fall t = 2 unabhängig voneinander C₁-C₄-Alkyl oder zusammen einen 2,3-Dehydropentamethylenrest darstellen; und
R'₇ und R'₈ im Fall t = 3, Methyl bedeuten;
R'₁₄ unabhängig voneinander Wasserstoff, C₁-C₉-Alkyl oder Cyclohexyl sind;
R'₁₅ unabhängig voneinander Wasserstoff oder Methyl; und
R'₁₆ Wasserstoff oder C₁-C₄-Alkyl darstellt und im Fall, daß mehrere Reste R'₁₆ vorhanden sind, die Reste R'₁₆ gleich oder verschieden sind;
X' und Y' jeweils eine direkte Bindung oder -O- darstellen; und
Z' eine direkte Bindung; -CH₂-; -C(R'₁₆)₂- oder -S- ist.

19. Stabilisatorgemisch gemäß Anspruch 18 mit einer oder mehreren Phosphor-Verbindungen der Formeln C'1 bis C'12

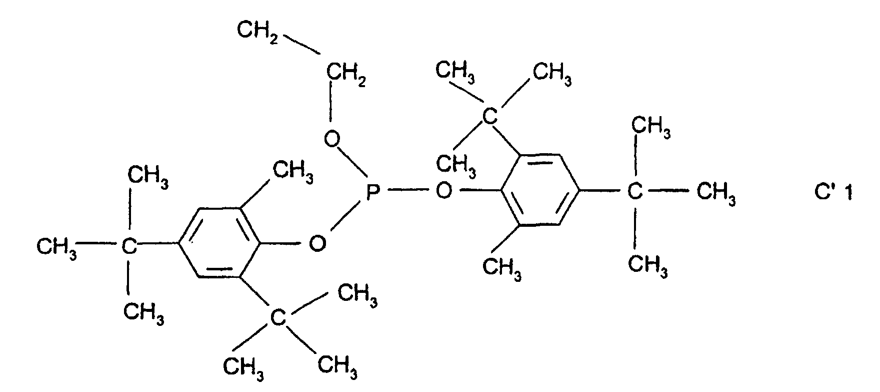

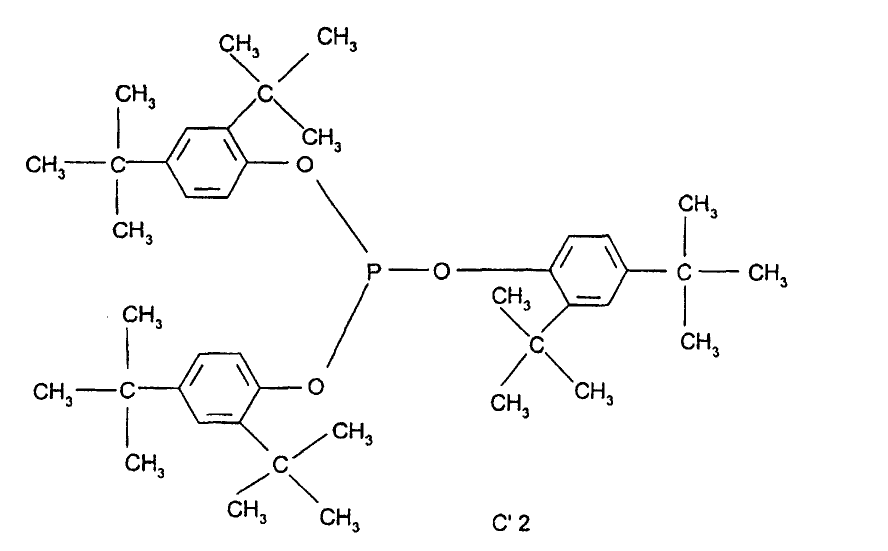

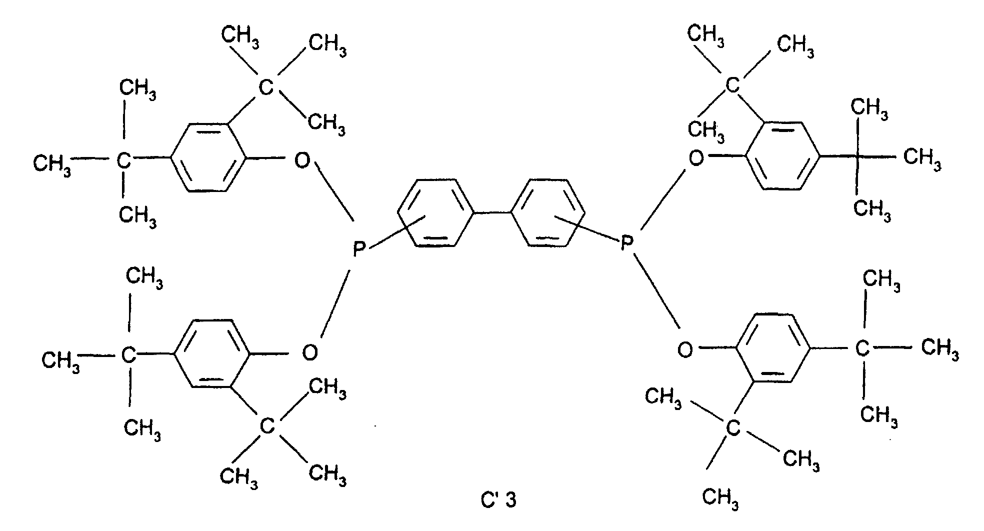

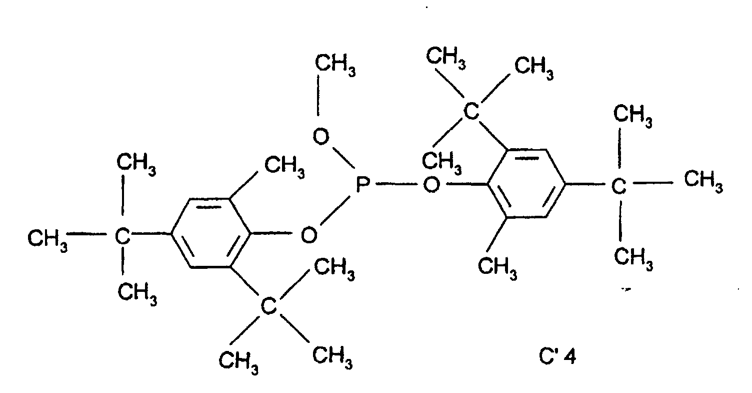

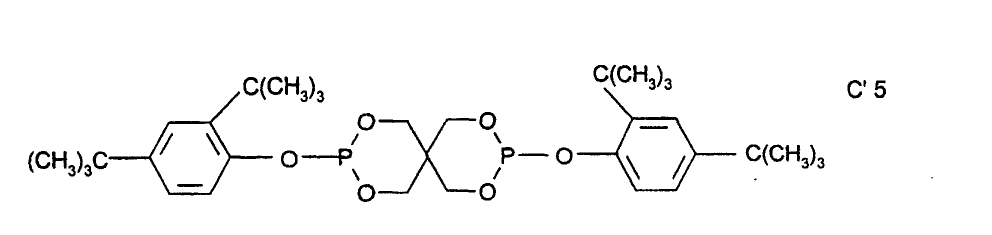

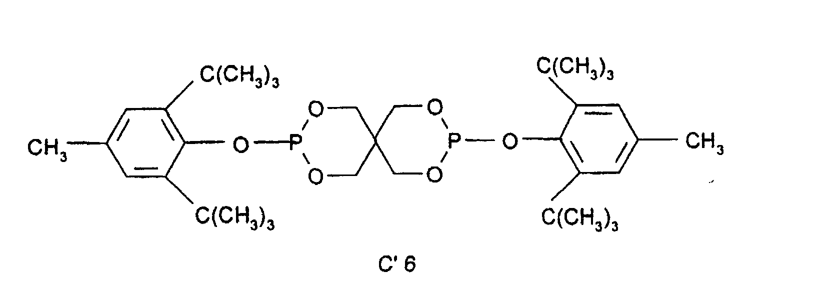

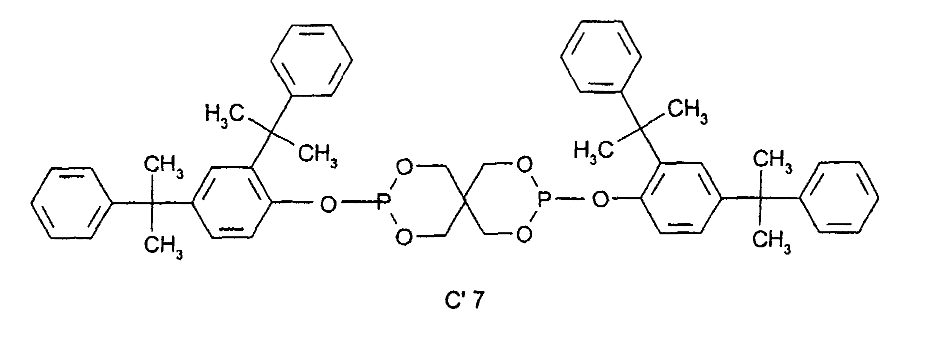

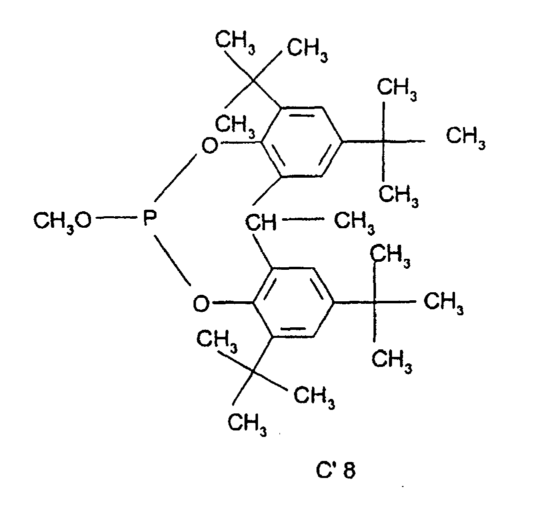

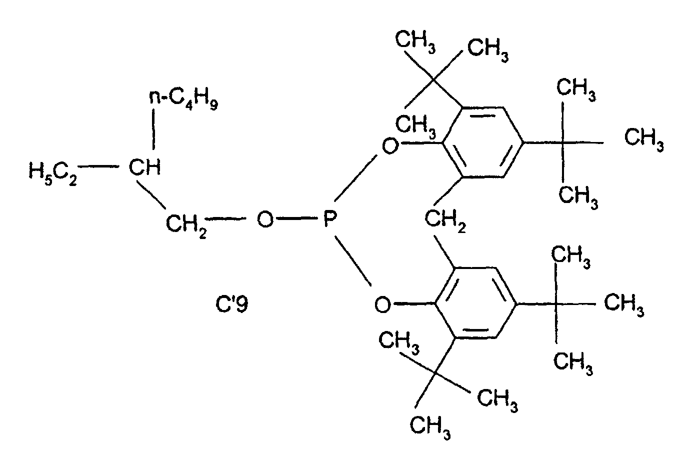

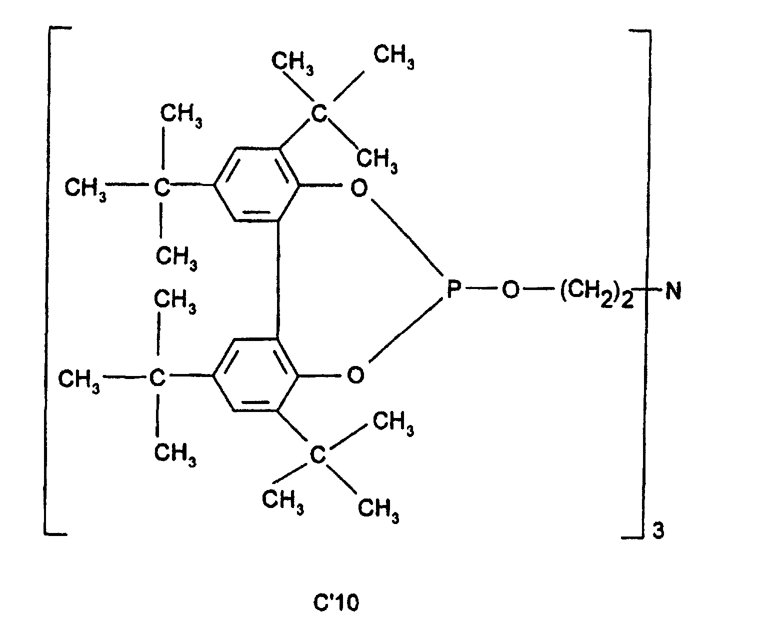

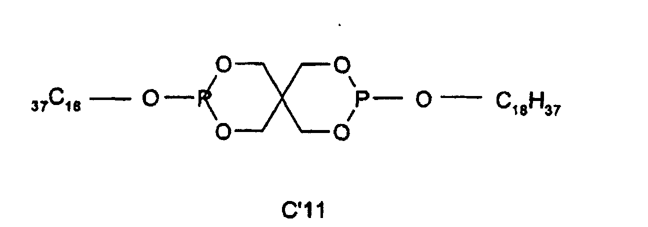

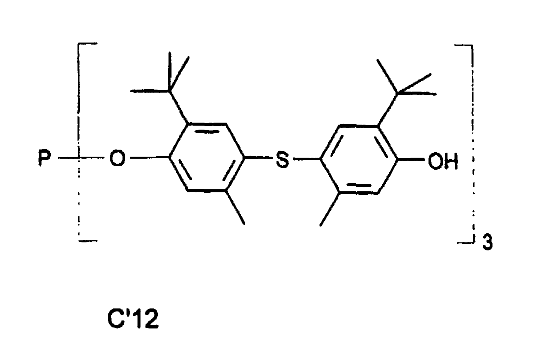

20. Gemisch gemäß Anspruch 18, **dadurch gekennzeichnet, daß** es sich bei den Phosphor-Verbindungen um ®Irgafos 38, ®Irgafos 12, ®Hostanox PAR 24, ®Hostanox OSP 1, ®Irgafos P-EPQ, ®Ultranox 626, ®Ultranox 618, ®Mark PEP-36 , ®Mark HP10, ®Doverphos 9228 handelt.

21. Gemisch aus der allgemeinen Formel (III) gemäß Anspruch 1 und UV-Absorber, wobei diese UV-Absorber Verbindungen aus der Klasse der 2-Hydroxybenzo-phenone, der 2-Hydroxyphenylbenzotriazole, der Zimtsäurederivate, der Oxanilide sein können.

22. Gemisch gemäß den Ansprüchen 8 bis 21, **dadurch gekennzeichnet, daß** der Anteil an Verbindung der Formel (III) zwischen 1 und 99 Gew.% liegen kann.

23. Mischung aus einem Gemisch gemäß den Ansprüchen 8 bis 21 und Lichtschutzmitteln aus der Klasse der UV-Absorber.

24. Mischung aus Verbindungen der Formel (III) gemäß Anspruch 1 allein oder in Mischungen gemäß den Ansprüchen 8 bis 21 und einem oder mehreren N,N-dialkylsubstituierten Hydroxylaminen.

25. Mischung gemäß Anspruch 24, **dadurch gekennzeichnet, daß** es sich bei dem N,N-dialkylsubstituierten Hydroxylamin um N,N-Dioctadecylhydroxylamin handelt.

26. Gemisch der Verbindungen der allgemeinen Formel (III) gemäß Anspruch 1 mit einem oder mehreren basischen oder sonstigen säurebindenden Costabilisatoren, **dadurch gekennzeichnet, daß** letztere ausgewählt sind aus der Gruppe der Metall-carboxylate, -oxide, -hydroxide, -carbonate, und/oder Zeolithe, und/oder Hydrotalcite.

27. Gemisch gemäß Anspruch 26, **dadurch gekennzeichnet, daß** es sich bei den Costabilisatoren um Calciumstearat, und/oder Magnesiumstearat, und/oder Magnesiumoxid, und/oder Zinkoxid und/oder carbonathaltiges Zinkoxid und/oder Hydrotalcite handelt.

28. Gemisch gemäß Anspruch 26, **dadurch gekennzeichnet, daß** es sich bei den Costabilisatoren um ®Zinkoxid aktiv, ®Zinkoxid transparent und/oder eines der Hydrotalcite ®DHT 4A, ®DHT4 A2, ® Kyowaad 200, ®Kyowaad 300, ®Kyowaad 400, ®Kyowaad 500, ®Kyowaad 600, ®Kyowaad 700, ®Kyowaad 1000, ®Kyowaad 2000, handelt.

29. Gemisch der Verbindungen der allgemeinen Formel (III) gemäß Anspruch 1 mit sterisch gehinderten, phenolischen Antioxidantien.

30. Mischung aus einem Gemisch gemäß den Ansprüchen 8 bis 21 mit sterisch gehinderten, phenolischen Antioxidantien.

31. Mischung aus einem Gemisch gemäß Anspruch 30 mit Synergisten vom Typ der 3-Pyrazolidinone.

32. Mischung aus einem Gemisch gemäß Anspruch 30 mit Synergisten vom Typ der 1,2,4-Triazolidin-3,5-dione.

33. Mischung aus einem Gemisch gemäß Anspruch 30 mit Synergisten vom Typ der 3-Aryl-benzofuran-2-one.

34. Mischung aus einem Gemisch gemäß Anspruch 30, **dadurch gekennzeichnet, daß** es sich bei dem 3-Aryl-benzofuran-2-on um 3-(3,4-Dimethylphenyl)-5,7-di-tert.-butyl-3H-benzofuran-2-on handelt (Formel D).

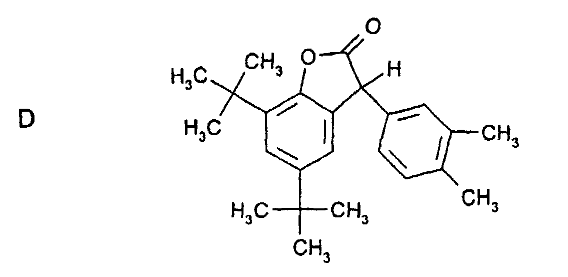

35. Mischung aus einem Gemisch gemäß Anspruch 30 und basischen oder sonstigen säurebindenden Costabilisatoren.

36. Kombination einer Verbindung der Formel (III) gemäß Anspruch 1 und Farbstoffen oder Pigmenten auf organischer oder anorganischer Basis.

37. Verwendung von Gemischen gemäß den Ansprüchen 8 bis 36 zum Stabilisieren von organischem Material.

38. Stabilisiertes organisches Material, enthaltend Gemische gemäß den Ansprüchen 8 bis 36.

39. Stabilisiertes organisches Material gemäß Anspruch 38, **dadurch gekennzeichnet, daß** es sich um ein Polymer handelt.

40. Stabilisiertes organisches Material gemäß Anspruch 38, **dadurch gekennzeichnet, daß** es die erfindungsgemäße Verbindung (III) gemäß Anspruch 1 in einer Konzentration von 0.001 bis 5 Gew.-%, bezogen auf das stabilisierte organische Material, enthält.

## Claims

1. A compound of the formula (III), 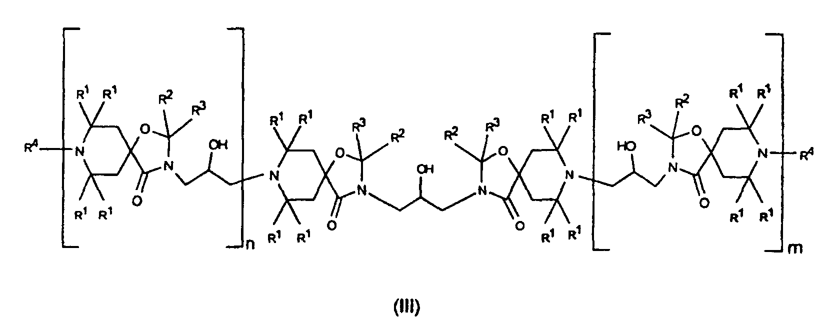 in which
n and m independently of one another are a number from 0 to 100, but n and m cannot both be 0,
R' is hydrogen, C₅-C₇-cycloalkyl, or a C₁-C₁₂-alkyl group,
R² and R³ independently of one another are a hydrogen atom or a C₁-C₁₈-alkyl group or, together with the carbon atom connecting them, are a 5- to 13-membered ring or, together with the carbon atom connecting them, are a group of the formula (IV) 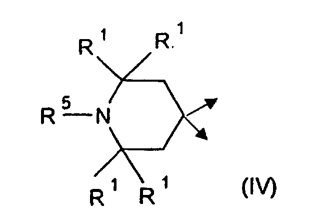 R⁴ and R⁵ independently of one another are either hydrogen or a C₁-C₂₂-alkyl group, an oxygen radical O*, -OH, -NO, -CH₂CN, benzyl, allyl, a C₁-C₃₀-alkyloxy group, a C₅-C₁₂-cycloalkyloxy group, a C₆-C₁₀-aryloxy group in which additionally the aryl radical may also be substituted, a C₇-C₂₀-arylalkyloxy group in which additionally the aryl radical may also be substituted, a C₃-C₁₀- alkenyl group, a C₃-C₆-alkynyl group, a C₁-C₁₀-acyl group, halogen or C₇-C₉-phenylalkyl which is unsubstituted or substituted on the phenyl ring by C₁-C₄-alkyl.

2. A compound as claimed in claim 1, wherein n and m independently of one another are a number from 0 to 10, but n and m cannot both be 0,
R¹ is hydrogen, C₆-cycloalkyl, or a C₁-C₄-alkyl group,
R² and R³ independently of one another are a hydrogen atom or a C₁-C₈- alkyl group, or, together with the carbon atom connecting them, are a 6- to 12-membered ring or, together with the carbon atom connecting them, are a group of the formula (IV),
R⁴ and R⁵ independently of one another are either hydrogen or a C₁-C₅-alkyl group, an oxygen radical O*, -OH, -NO, -CH₂CN, benzyl, allyl, a C₁-C₁₀-alkyloxy group, a C₅-C₆-cycloalkyloxy group, a C₆-C₇-aryloxy group in which additionally the aryl radical can also be substituted, a C₇-C₁₀-arylalkyloxy group in which additionally the aryl radical can also be substituted, a C₃-C₆-alkenyl group, a C₃-C₆-alkynyl group, a C₁-C₄-acyl group, halogen or C₇-C₉-phenylalkyl which is unsubstituted or substituted on the phenyl ring by C₁-C₂-alkyl.

3. A compound as claimed in claim 1, wherein n and m independently of one another are a number from 0 to 5, but n and m cannot both be 0,
R¹ is methyl,
R² and R³ together with the carbon atom connecting them, are a 12-membered ring or, together with the carbon atom connecting them, are a group of the formula (IV).
R⁴ and R⁵ independently of one another are hydrogen, methyl, acetyl, octyloxy or cyclohexyloxy.

4. A compound as claimed in claim 1 to 3, which is a stabilizer against the damaging effect of oxygen, light and heat.

5. A process for preparing a compound of the formul a (III) as claimed in claim 1, which comprises reacting a compound of the formula (I) with a compound of the formula (II) 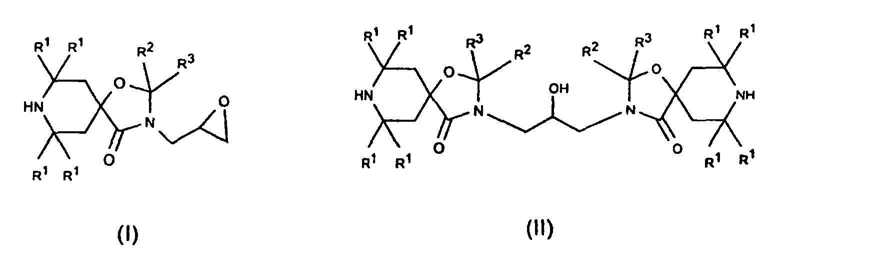 in which R¹ to R⁴ are as defined in claim 1 with or without solvent and with or without catalyst in a molar ratio of from 1:1 to 100:1 and at from 100 to 300°C.

6. The process as claimed in claim 5, wherein the reaction takes place without solvent and without catalyst in vacuo at from 120 to 250°C.

7. The use of a compound as claimed in claims 1 to 4 for stabilizing organic material against damage by light and heat.

8. A mixture of a compound of the formula (III) as claimed in claim 1 with one or more stabilizers based on sterically hindered amines of the formulae A1 to A10 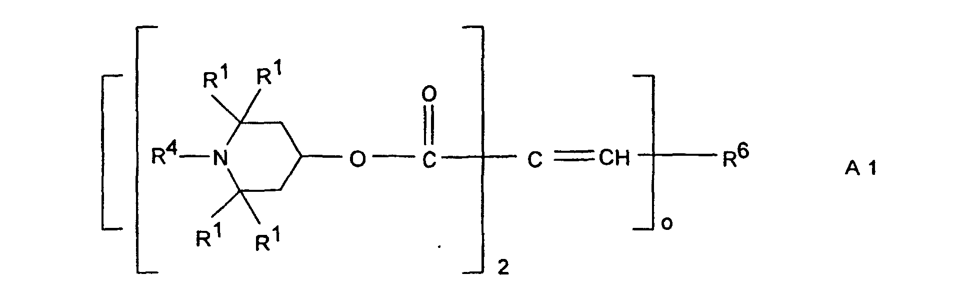 in which R¹ and R⁴ are as defined in claim 1
R⁶ is an aromatic radical substituted one or more times by hydrogen, C₁-C₄- alkyl, C₁₋C₄-alkoxy, halogen, cyano, carboxyl, nitro, amino, C₁-C₄-alkylamino, C₁-C₄₋ dialkylamino, or acyl,
o is 1 or 2, 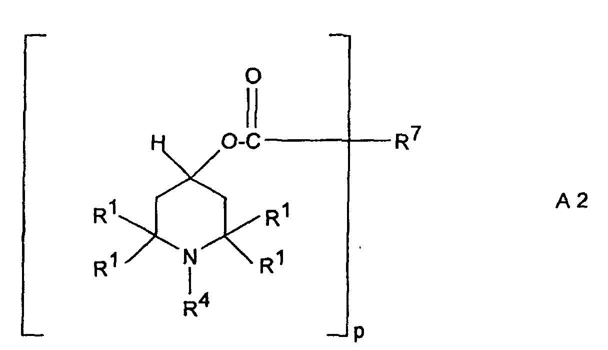 in which R¹ and R⁴ are as defined in claim 1,
p is 1 or 2 and if p = 1 R⁷ is C₁-C₂₂-alkyl, C₂-C₁₈-oxaalkyl, C₂-C₁₈-thiaalkyl, C₂-C₁₈-azaalkyl or C₂-C₈-alkenyl; if p = 2 R⁷ is C₁-C₂₂-alkylene, C₂-C₁₈-oxaalkylene, C₂-C₁₈-thiaalkylene, C₂-C₁₈-azaalkylene or C₂-C₈-alkenylene; 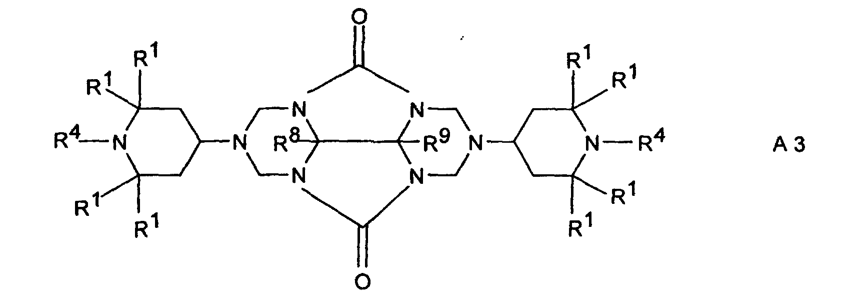 in which R¹ and R⁴ are as defined in claim 1,
R⁸ and R⁹ independently of one another are hydrogen, C₁-C₆-alkyl, C₇-C₁₂-aralkyl, -aryl or carboxylic ester,
R⁸ and R⁹ together are a tetra- or pentamethyl group; 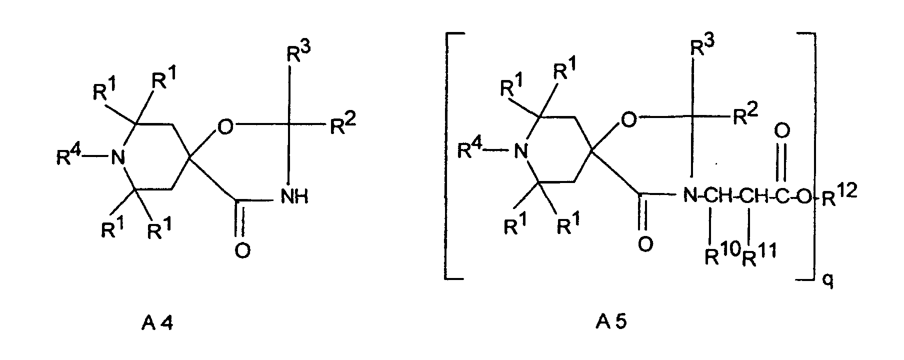 in which R¹, R², R³ and R⁴ are as defined in claim 1,
q is a number 1 or 2,
R¹⁰ is hydrogen, methyl, phenyl or carb-C₁-C₂₁-alkoxy,
R¹¹ is hydrogen or methyl,
R¹² if q = 1, is hydrogen, C₁-C₂₁-alkyl, C₂-C₂₂-alkenyl, C₅-C₁₂-cycloalkyl, a radical of the formula 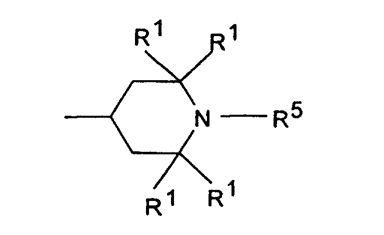 where R¹ and R⁵ are as defined in claim 1, and
R¹² if q = 2, is C₁-C₁₈-alkylene, C₅-C₉-cycloalkylene or arylene; 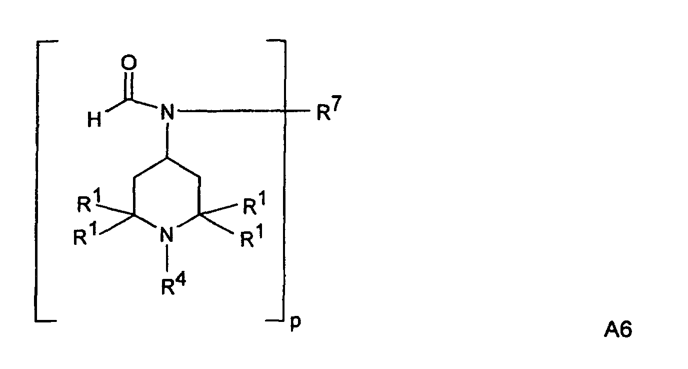 where R¹, R⁴, R⁷ and p are as defined above; 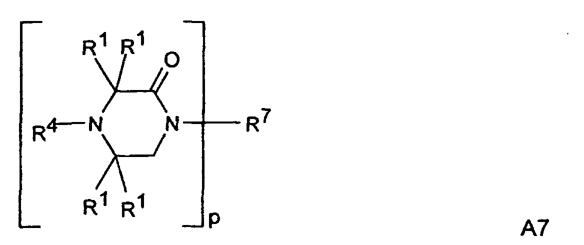 where R¹, R⁴, R⁷ and p are as defined above; 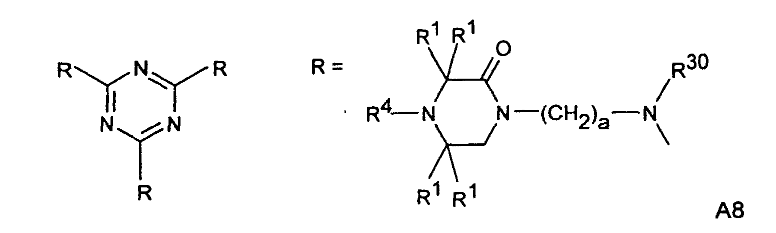 where R¹, R⁴ are as defined above,
R³⁰ is hydrogen, C₁-C₁₂-alkyl, C₅-C₁₂-cycloalkyl, phenyl or C₇-C₉ -phenylalkyl, and
a is a number from 1 to 10; 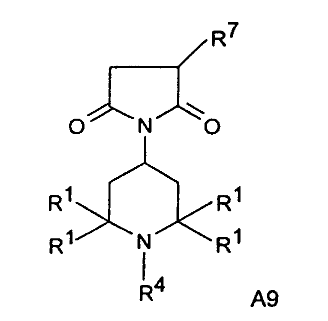 where R¹ and R⁴ are as defined above and R⁷ is as defined for p = 1 in the formula A2;
a product A10 obtainable by reacting a polyamine of the formula A10a with formula A10b 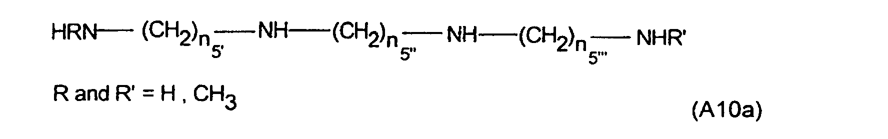 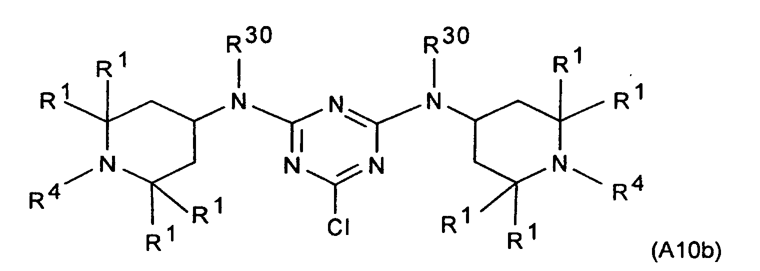 where R¹,R⁴ and R³⁰ are as defined above,
n_{5'}, n_{5"} and n_{5"'} independently of one another are a number from 2 to 12.

9. A mixture as claimed in claim 8, wherein n and m independently of one another are a number from 0 to 10, but n and m cannot both be 0,
R¹ is hydrogen or a C₁-C₄-alkyl group,
R² and R³ independently of one another are a hydrogen atom or a C₁-C₈-alkyl group or, together with the carbon atom connecting them, are a 6- to 12-membered ring or, together with the carbon atom connecting them, are a group of the formula (IV),
R⁴ and R⁵ independently of one another are either hydrogen or a C₁-C₅-alkyl group, an oxygen radical O*, -OH, -NO, -CH₂CN, benzyl, allyl, a C₁-C₁₀-alkyloxy group, a C₅-C₆-cycloalkyloxy group, a C₆-C₇-aryloxy group in which additionally the aryl radical may also be substituted; a C₇-C₁₀-arylalkyloxy group in which additionally the aryl radical may also be substituted, a C₃-C₆-alkenyl group, a C₃-C₆-alkynyl group, a C₁-C₄-acyl group, halogen or C₇-C₉-phenylalkyl which is unsubstituted or substituted on the phenyl ring by C₁-C₂-alkyl,
R⁷ is a straight-chain C₁-C₁₀-alkylene (if p = 2); C₁-C₁₂-alkyl (if p = 1)
R⁸ and R⁹ independently of one another are hydrogen, C₁-C₂-alkyl, C₇-C₈-arylalkyl, aryl- or carboxylic ester,
R¹⁰ is hydrogen, methyl, phenyl or C₁-C₂-alkoxy
R¹¹ is hydrogen or methyl,
R¹² if q = 1, is hydrogen, C₁-C₁₆-alkyl, C₂-C₁₆-alkenyl, C₅-C₆-cycloalkyl, a radical of the formula 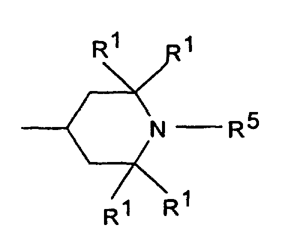 R¹² if q = 2, is C₁-C₁₆-alkylene, C₅-C₆-cycloalkylene or arylene,
R³⁰ is hydrogen, C₁-C₈-alkyl, C₅-C₇-cycloalkyl, phenyl or C₇-C₈-phenylalkyl,
a is 1 to 5,
o 1 and
p 2 to 5.

10. A mixture as claimed in claim 8, wherein n and m independently of one another are a number 0-5, but n and m cannot both be 0,
R¹ is methyl,
R² and R³ together with the carbon atom connecting them, are a 12-membered ring or, together with the carbon atom connecting them, are a group of the formula (IV)
R⁴ and R⁵ independently of one another are hydrogen, methyl, acetyl, octyloxy or cyclohexyloxy,
R⁶ is p-methoxyphenyl,
R⁷ is octamethylene, hexamethylene or ethylene (if p = 2), dodecyl (if p =1),
R⁸ and R⁹ are hydrogen,
R¹⁰ is hydrogen,
R¹¹ is hydrogen,
R¹² is dodecamethylene or tetradecamethylene,
R³⁰ is cyclohexyl or n-butyl,
a is 2,
0 is 1,
p is 2 and
q is 1.

11. A mixture as claimed in claim 8, wherein the HALS compounds of the mixture comprising compounds of the formula (III) are the following substances: 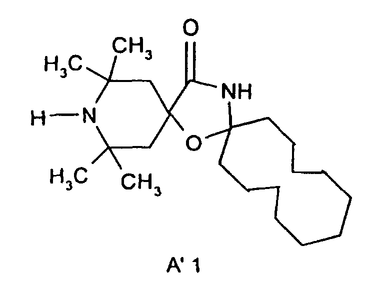 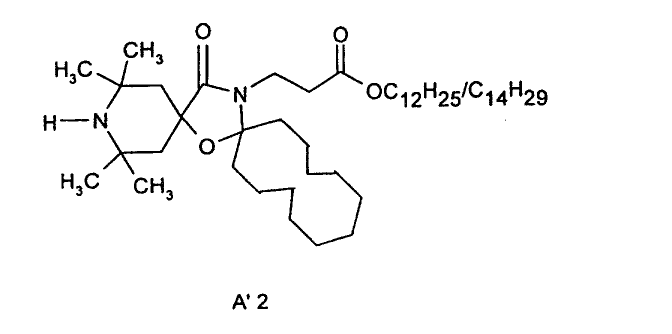 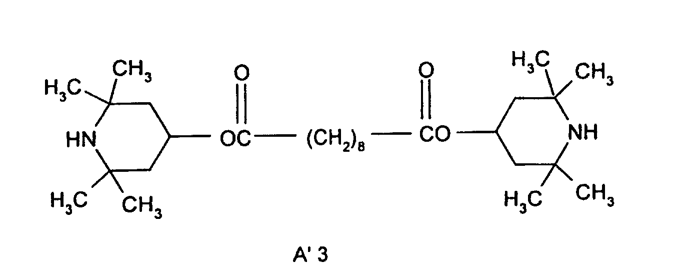 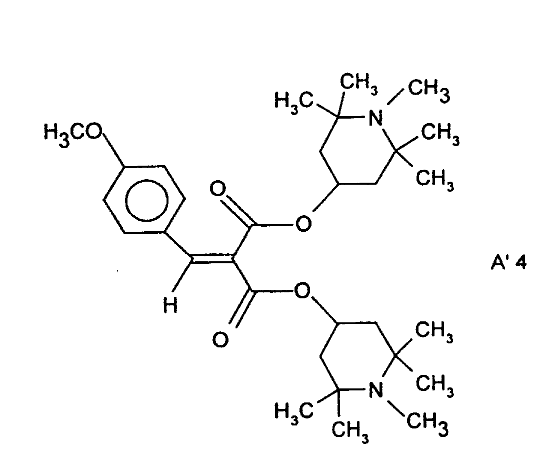 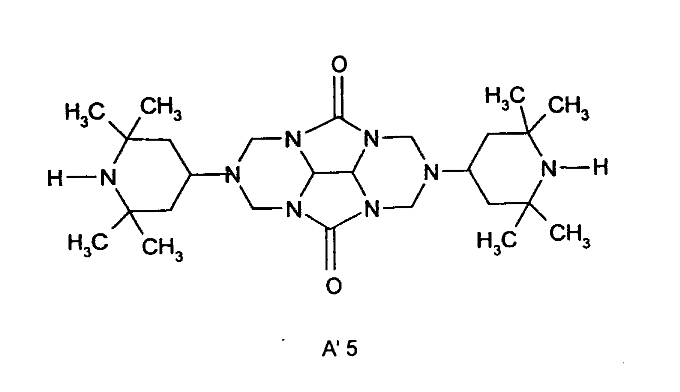 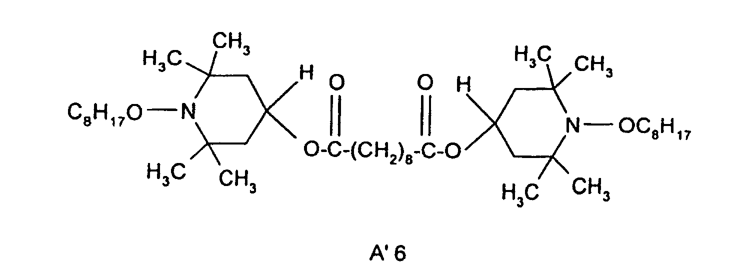 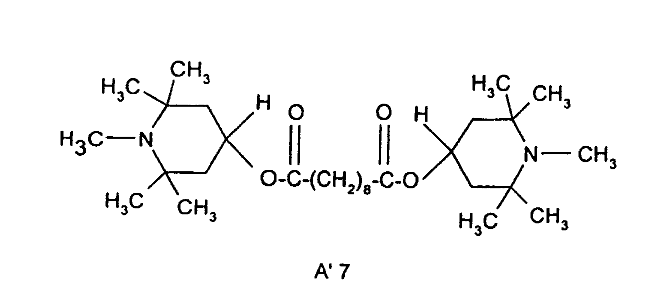 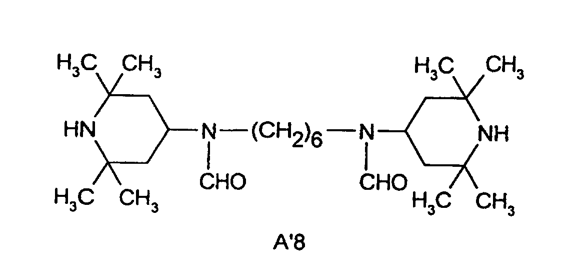 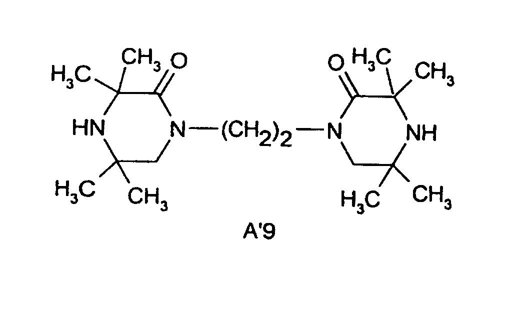 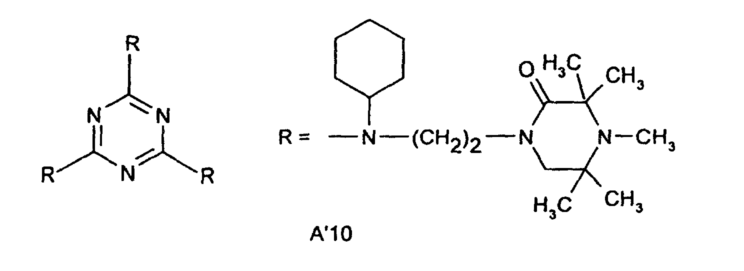 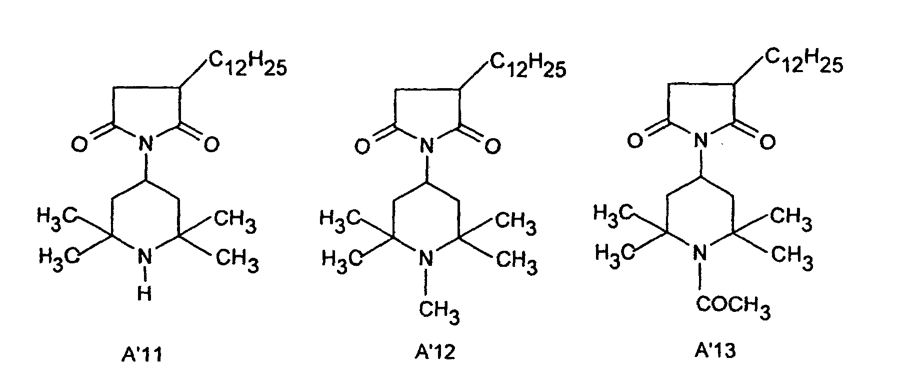 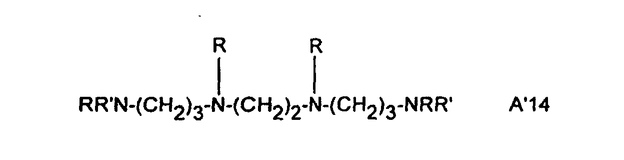 where 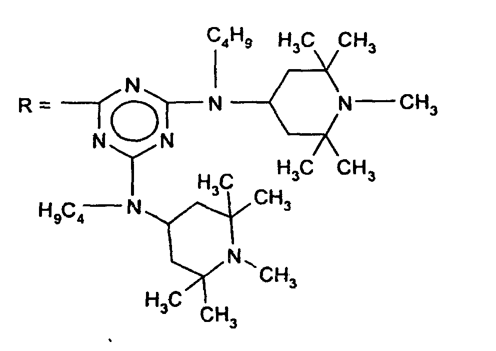 and
R' = H, CH₃

12. A mixture as claimed in claim 8, wherein the co-component(s) is (are) ®Tinuvin 770, ®Tinuvin 765, ®Tinuvin 123, ®Hostavin N 20, ®Hostavin N 24, ®Uvinul 4049, ®Sanduvor PR 31, ®Uvinul 4050, ®Good-rite UV 3034 or ®Good-rite 3150, ®Sanduvor 3055, ®Sanduvor 3056, ®Sanduvor 3058, ®Chimassorb 119 and ®Chimassorb 905.

13. A mixture of a compound of the formula (III) as claimed in claim 1 with one or more polymeric HALS compounds of the formulae B1 to B7 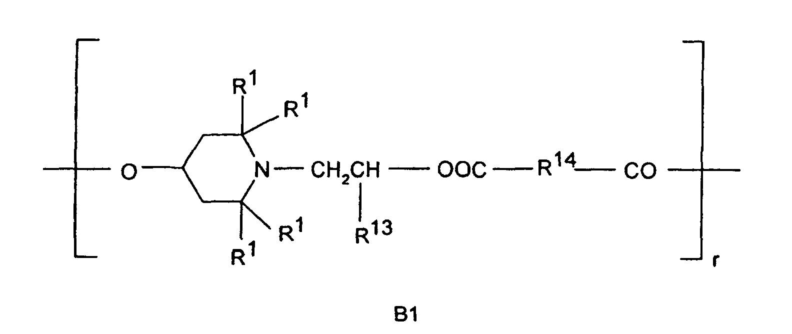 in which R¹ is as defined in claim 1,
R¹³ is hydrogen or methyl,
R¹⁴ is a direct bond or C₁-C₁₀-alkylene and
r is a number from 2 to 50; 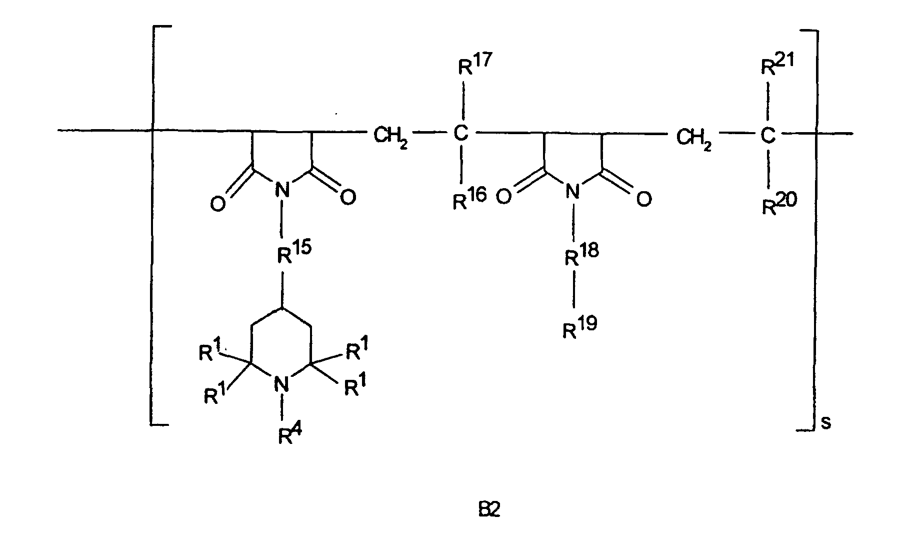 where R¹ and R⁴ are as defined in claim 1,
R¹⁵ and R¹⁸ independently of one another are a direct bond or a group -N(R²²)-CO- R²³-CO-N(R²⁴)-,
R²² and R²⁴ independently of one another are hydrogen, C₁-C₈-alkyl, C₅₋C₁₂-cycloalkyl, phenyl, C₇-C₉-phenylalkyl, or a group of the formula 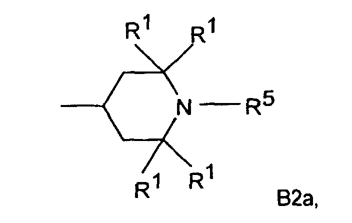 R²³ is a direct bond or C₁-C₄-alkylene,
R¹⁶, R¹⁷, R²⁰, R²¹ independently of one another are hydrogen, C₁-C₃₀-alkyl, C₅-C₁₂-cycloalkyl, phenyl, or a group of the formula B2a,
R¹⁹ is hydrogen, C₁-C₃₀-alkyl, C₅-C₁₂-cycloalkyl, C₇-C₉-phenylalkyl, phenyl or a group of the formula B2a and
s is a number from 1 to 50; 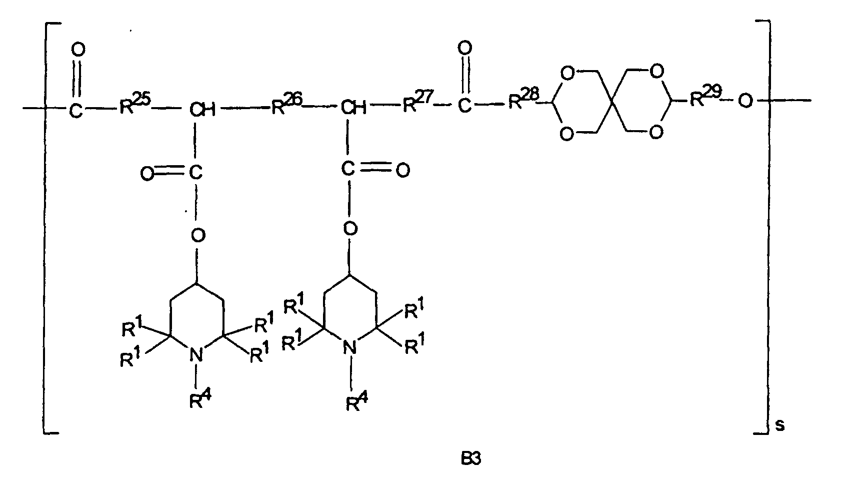 where R¹, R⁴ and s are as defined above,
R²⁵, R²⁶, R²⁷, R²⁸ and R²⁹ independently of one another are a direct bond or C₁-C₁₀-alkylene;
a product B4 obtainable by reacting a polyamine of the formula B4a with cyanuric chloride and then reacting the resulting product with a compound of the formula B4b, 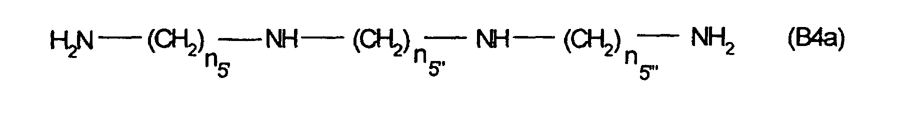 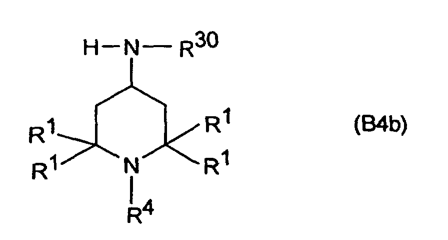 where R¹ and R⁴ are as defined in claim 1,
n_{5'}, n_{5"} and n_{5"'} independently of one another are a number from 2 to 12,
R³⁰ is hydrogen, C₁-C₁₂-alkyl, C₅-C₁₂-cycloalkyl, phenyl or C₇-C₉-phenylalkyl; where B4 is a compound of the formula B4-1, B4-2, B4-3 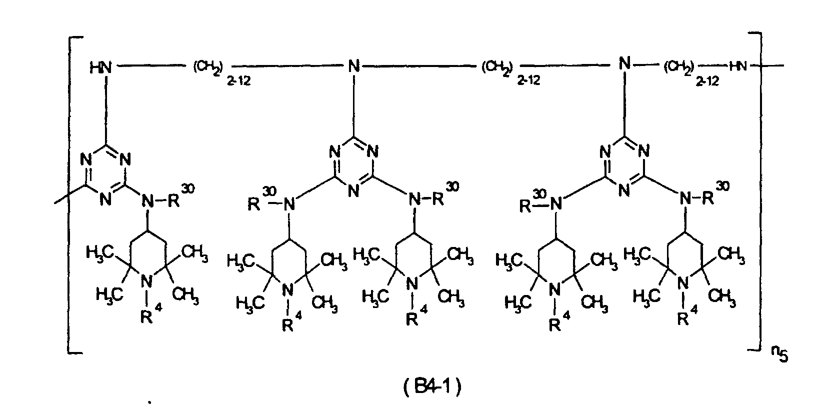 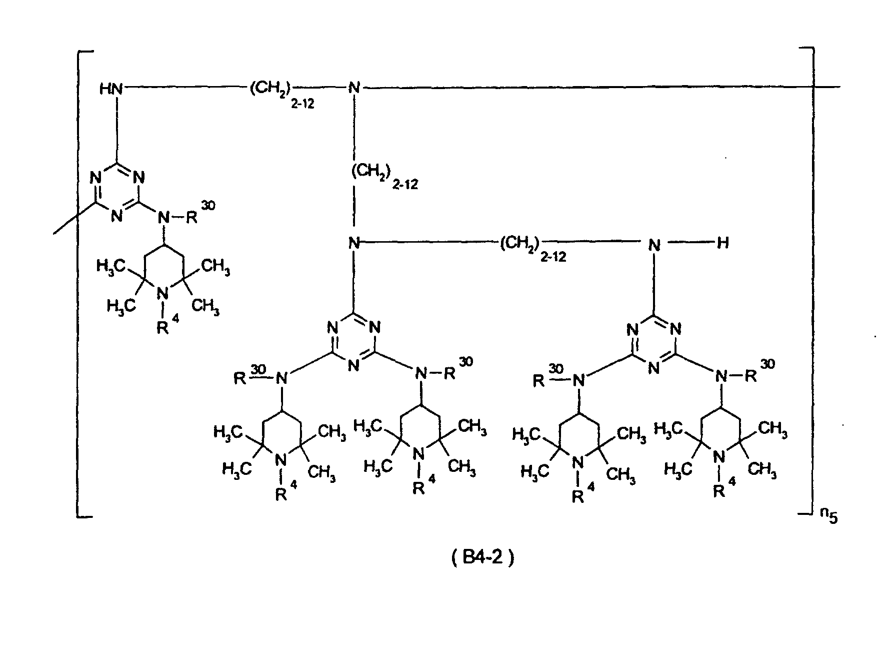 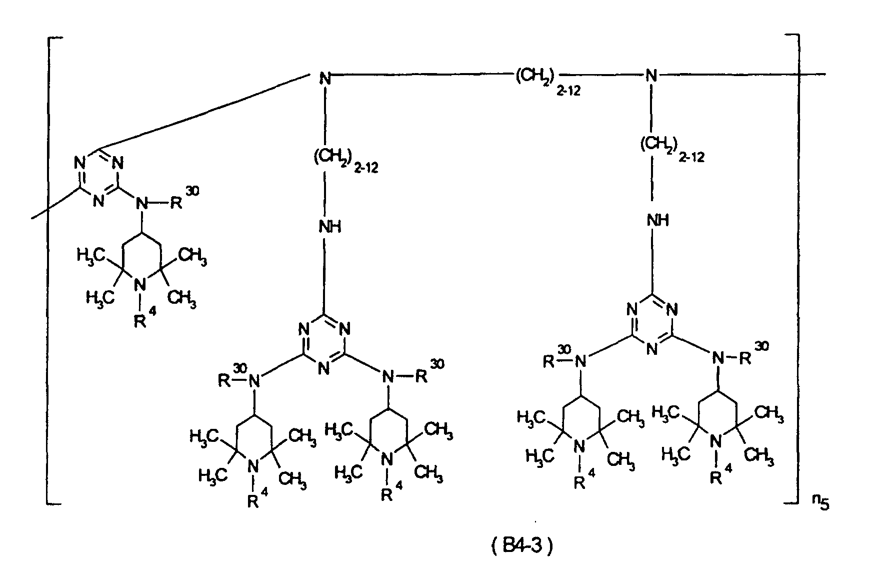 or a mixture thereof, in which n₅ is 1 to 20,
R⁴ and R³⁰ are as defined above; 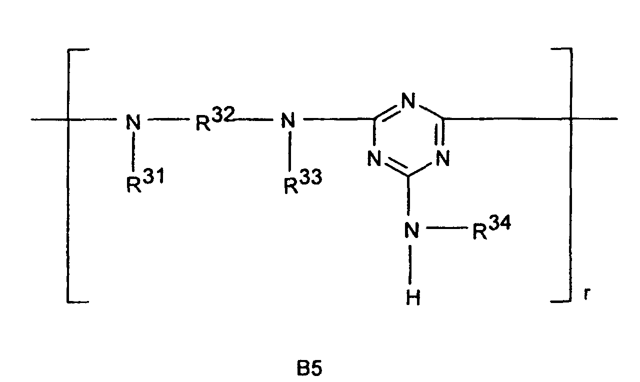 where r is as defined for formula B1,
R³¹, R³³ and R³⁴ independently of one another are hydrogen, C₁-C₁₂-alkyl, C₅-C₁₂-cycloalkyl, C₁-C₄-alkyl-substituted C₅-C₁₂-cycloalkyl, phenyl, -OH- and/or C₁-C₁₀-alkyl-substituted phenyl, C₇-C₉-phenylalkyl, C₇-C₉-phenylkalkyl substituted on the phenyl radical by -OH and/or C₁-C₁₀-alkyl, or a group of the formula B5a 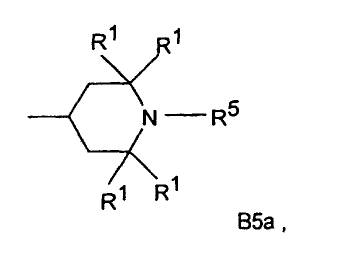 where R¹ and R⁵ are as defined in claim 1, and
R³¹ and R³³ in addition, independently of one another, are hydrogen,
R³² is C₂-C₁₈-alkylene, C₅-C₇-cycloalkylene or C₁-C₄-alkylenedi(C₅-C₇-cycloalkylene) or the radicals R³¹, R³² and R³³, together with the nitrogen atoms to which they are attached, form a 5- to 10-membered heterocyclic ring, and where at least one of the radicals R³¹, R³³ and R³⁴ is a group of the formula B5a; 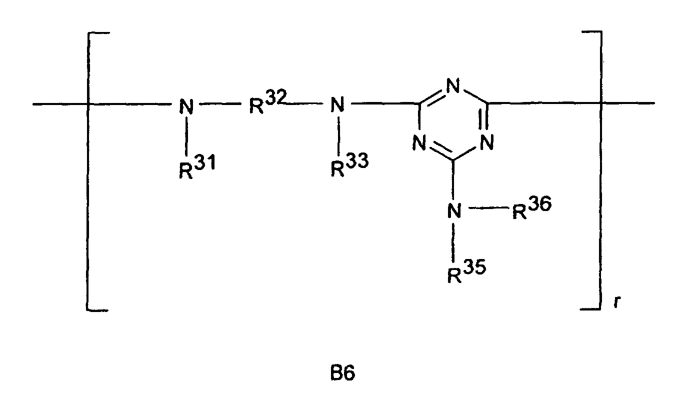 in which R³¹ , R³², R³³ and r are as defined above,
R³⁵ and R³⁶ independently of one another can have the definition of R³⁴, or
R³⁵ and R³⁶ together with the nitrogen atom to which they are attached, form a 5-to 10-membered heterocyclic ring which may in addition to the nitrogen heteroatom contain one or more other heteroatoms, preferably an oxygen atom, and at least one of the radicals R³¹, R³³, R³⁵ and/or R³⁶ is a group of the formula (B5a); 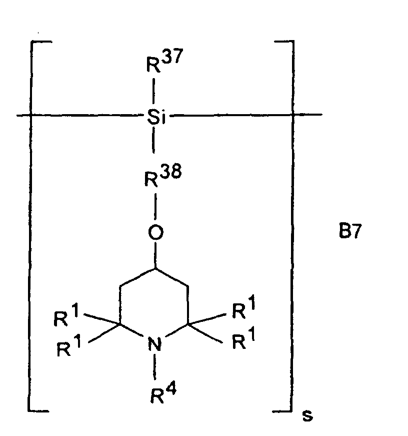 where R¹ and R⁴ are as defined in claim 1,
s is as defined for formula B3,
R³⁷ is C₁-C₁₀-alkyl, C₅-C₁₂-cycloalkyl, C₁-C₄-alkyl-substituted C₅-C₁₂-cycloalkyl, phenyl or C₁-C₁₀-alkyl-substituted phenyl, and
R³⁸ is C₃-C₁₀-alkylene.

14. A mixture as claimed in claim 13, wherein n and m independently of one another are a number from 0 to 10, but n and m cannot both be 0,
R¹ is hydrogen or a C₁-C₄-alkyl group,
R² and R³ independently of one another are a hydrogen atom or a C₁-C₈-alkyl group or, together with the carbon atom connecting them, are a 6- to 12-membered ring or, together with the carbon atom connecting them, are a group of the formula (IV),
R⁴ and R⁵ independently of one another are either hydrogen or a C₁-C₅-alkyl group, an oxygen radical O*, -OH, -NO, -CH₂CN, benzyl, allyl, a C₁-C₁₀-alkyloxy group, a C₅-C₆-cycloalkyloxy group,a C₆-C₇-aryloxy group in which additionally the aryl radical may also be substituted, a C₇-C₁₀-arylalkyloxy group in which additionally the aryl radical may also be substituted, a C₃-C₆-alkenyl group, a C₃-C₆-alkynyl group, a C₁-C₄-acyl group, halogen, or C₇-C₉-phenylalkyl which is unsubstituted or substituted on the phenyl ring by C₁-C₂-alkyl,
R¹³ is hydrogen or methyl,
R¹⁴ is C₁-C₅-alkylene,
R¹⁷, R²¹ are hydrogen or C₁-C₄-alkyl,
R¹⁵, R¹⁸ are a direct bond,
R¹⁶, R²⁰ are C₁-C₂₅-alkyl, phenyl,
R¹⁹ is hydrogen, C₁-C₁₂-alkyl or a group of the formula B2a,
R²⁵, R²⁶, R²⁷, R²⁸ and R²⁹ independently of one another are a direct bond or C₁-C₅-alkylene,
R³⁰ is hydrogen, C₁-C₄-alkyl, C₅-C₆-cycloalkyl or phenyl,
R³¹, R³³ and R³⁴ independently of one another are hydrogen, C₁-C₁₀-alkyl, C₅-C₆-cycloalkyl or a group of the formula B5a,
R³² is C₂-C₁₀-alkylene, C₅-C₆-cycloalkylene,
R³⁵ and R³⁶ independently of one another are as defined for R³⁴ , or
R³⁵ and R³⁶ together with the nitrogen atom to which they are attached, form a 5-to 7-membered heterocyclic ring which may also contain one or more heteroatoms, preferably an oxygen atom, and at least one of the radicals R³¹,R³³,R³⁵ and/or R³⁶ is a group of the formula B5a,
R³⁷ is C₁-C₅-alkyl, C₅-C₆-cycloalkyl or phenyl,
R³⁸ is C₃-C₅-alkylene and
n_{5'},n_{5"},n_{5"'} are 2 to 4.

15. A mixture as claimed in claim 13, wherein n and m independently of one another are a number from 0 to 5, but n and m cannot both be 0,
R¹ is methyl,
R² and R³ together with the carbon atom connecting them, are a 12-membered ring or, together with the carbon atom connecting them, a group of the formula (IV),
R⁴ and R⁵ independently of one another are hydrogen, acetyl, methyl, octyloxy or cyclohexyloxy,
R¹³ is hydrogen,
R¹⁴ is ethylene,
R¹⁷, R²¹ are hydrogen or methyl,
R¹⁵, R¹⁸ are a direct bond,
R¹⁶, R²⁰ are C₁-C₂₅-alkyl, phenyl,
R¹⁹ is hexadecyl or a group of the formula B2a,
R²⁵, R²⁷ are methylene,
R²⁶ is a direct bond,
R²⁸ is 2,2-dimethylethylene,
R²⁹ is 1,1-dimethylethylene,
R³⁰ is n-butyl,
R³¹, R³³ and R³⁴ independently of one another are isooctyl, cyclohexyl or 2,2,6,6-tetramethylpiperid-4-yl, and at least one of the radicals R³¹, R³³ and R³⁴ must be 2,2,6,6-tetramethylpiperid-4-yl,
R³² is hexamethylene,
R³⁵ and R³⁶ independently of one another are as defined for R³⁴ , or
R³⁵ and R³⁸ together with the nitrogen atom to which they are attached, form a 6-membered heterocyclic ring which also contains an oxygen atom and so is morpholine, and at least one of the radicals R³¹,R³³,R³⁵ and/or R³⁶ must a radical 2,2,6,6-tetramethylpiperid-4-yl,
R³⁷ is methyl,
R³⁸ is trimethylene,
n_{5'},n_{5"},n_{5"'} are 2 to 4.

16. A mixture as claimed in claim 13, wherein the polymeric HALS compounds in combination with compounds of the formula (III) are the following substances: 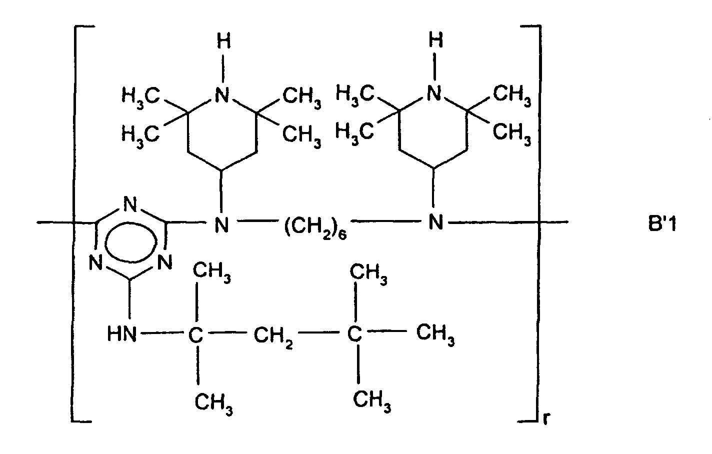 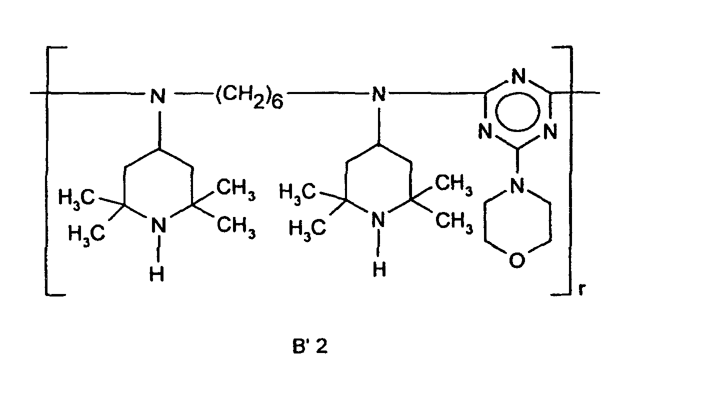 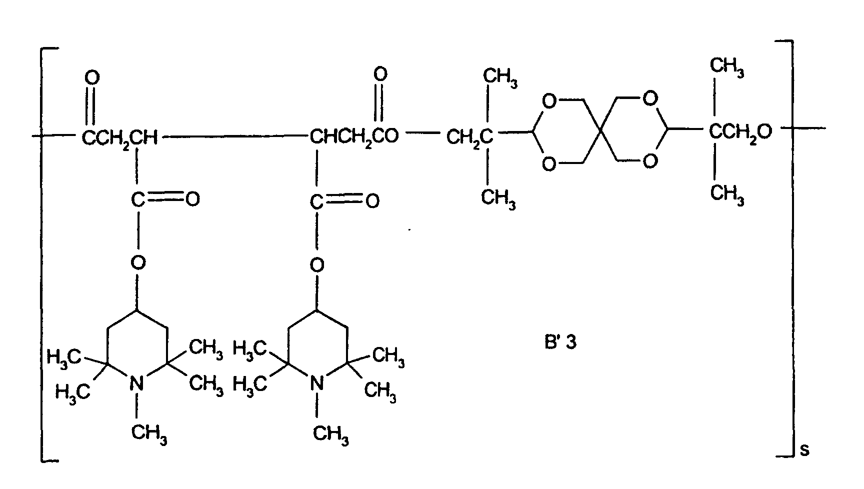 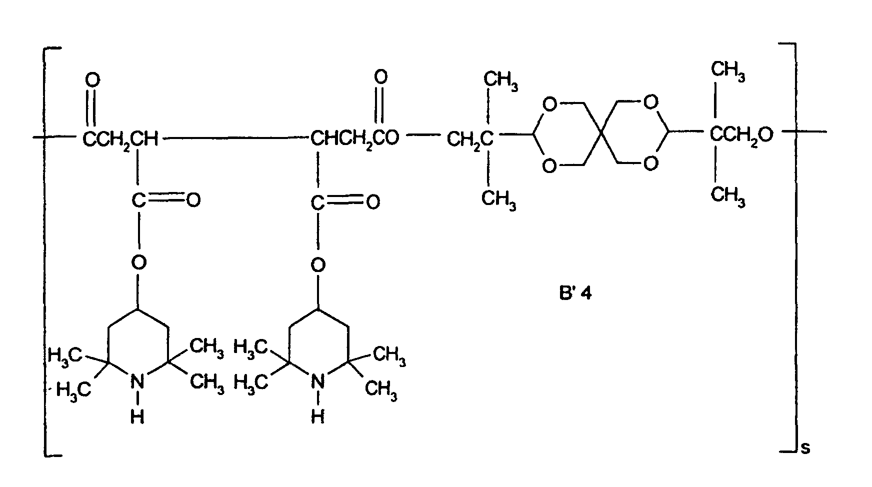 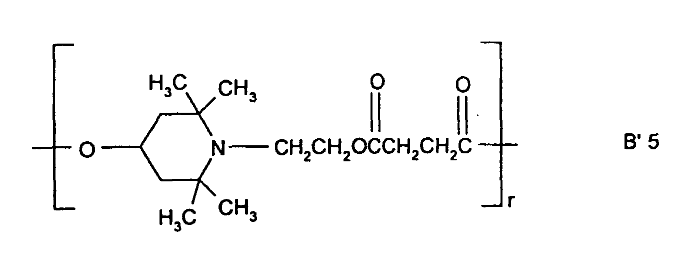 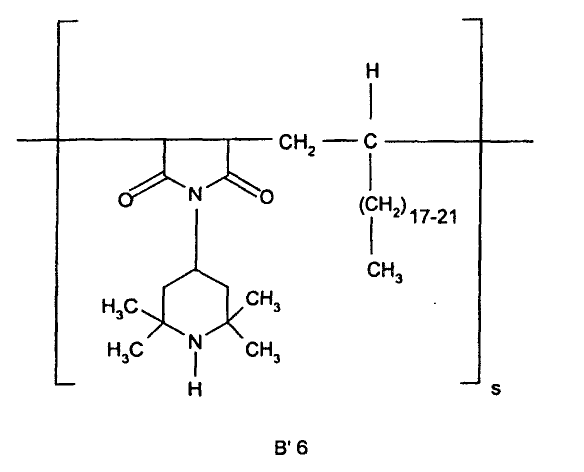 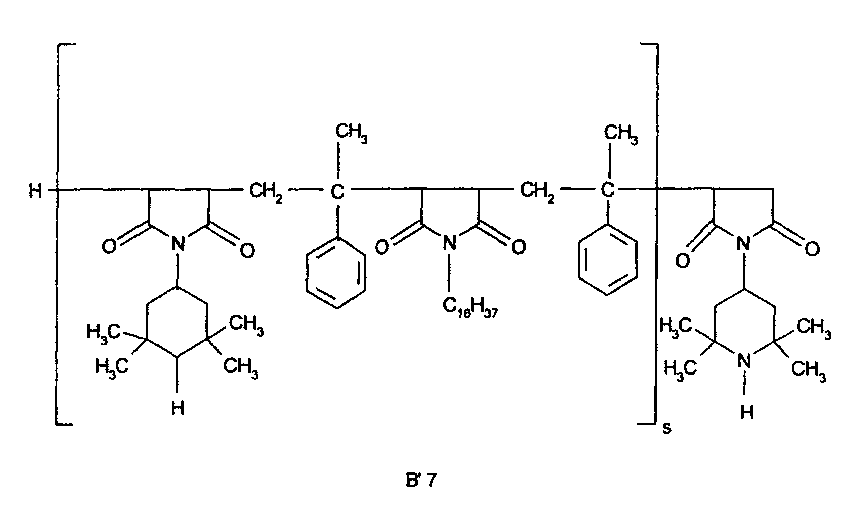 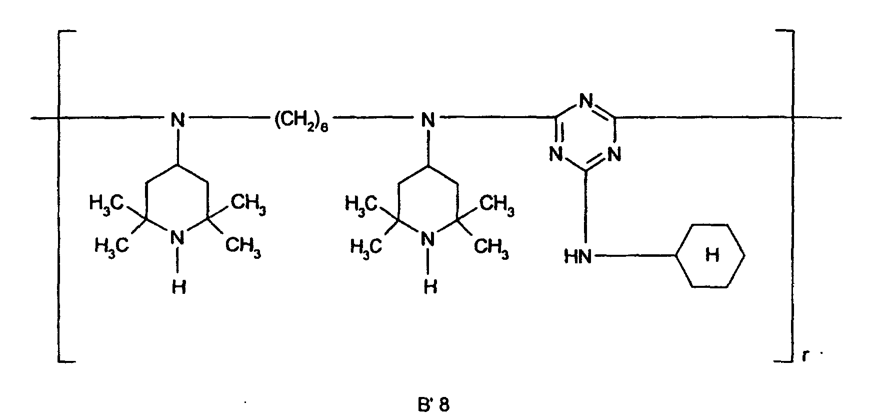 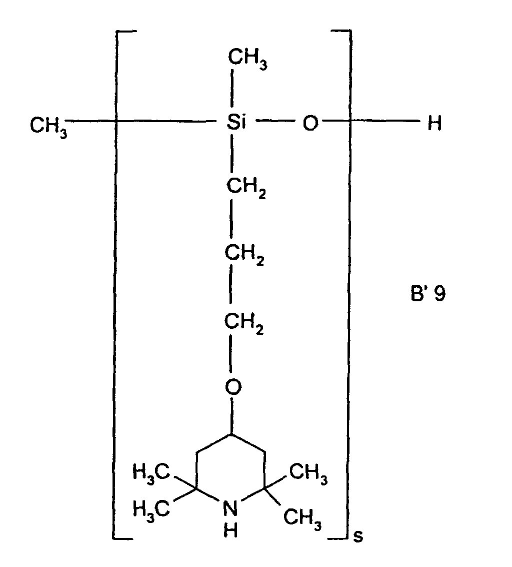 a product B'10 obtainable by reacting a polyamine of the formula B'10a
H₂N-(CH₂)₃-NH-(CH₂)₂-NH-(CH₂)₃-NH₂ (B'10a)
with cyanuric chloride and then reacting the resulting product with a compound of the formula (B'10b) 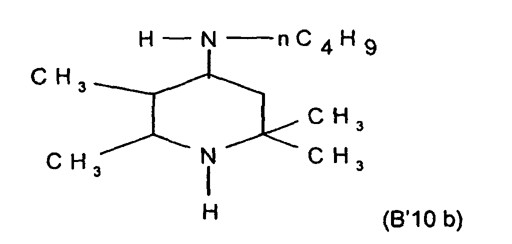 where B'10 is a compound of the formula B4-1' ,B4-2' , B4-3' 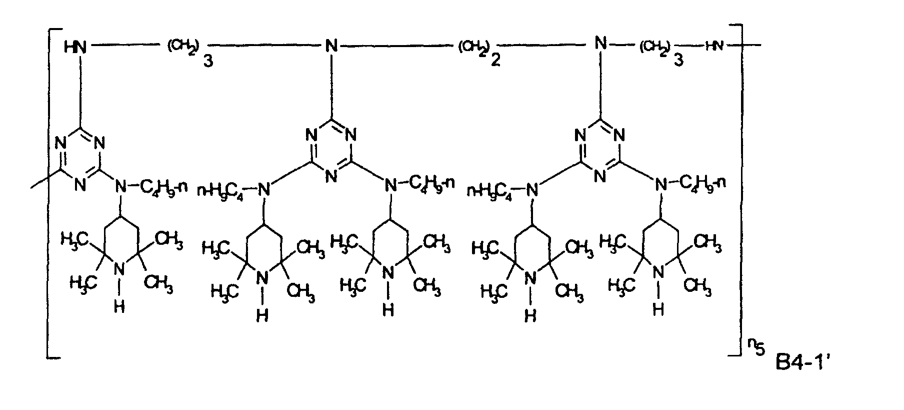 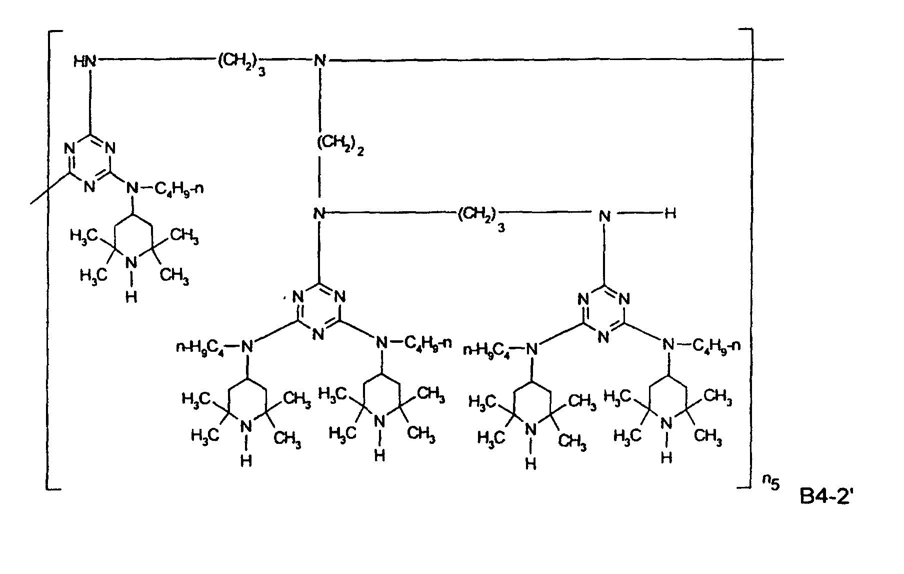 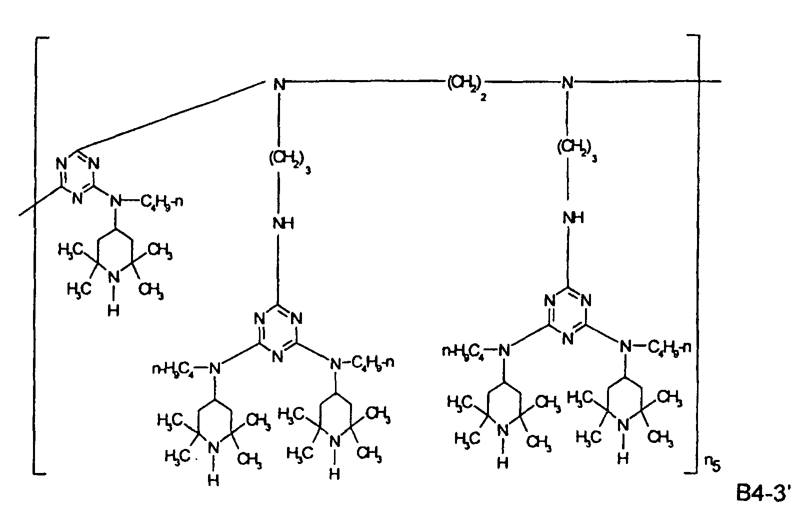 or a mixture thereof, where n₅ is 1 to 20.

17. A mixture as claimed in claim 13, wherein in mixtures comprising compounds of the formula(III) the other co-component(s) is (are) ®Chimassorb 944, ®Tinuvin 622, ®Dastib 1082, ®Uvasorb HA 88, ®Uvinul 5050, ®Lowilite 62, ®Uvasil 299, ®Cyasorb 3346, ®MARK LA 63, ®MARK LA 68 or ®Luchem B 18.

18. A mixture of a compound of the formula (III) as claimed in claim 1 and one or more phosphorus stabilizers of the formulae C1 to C7 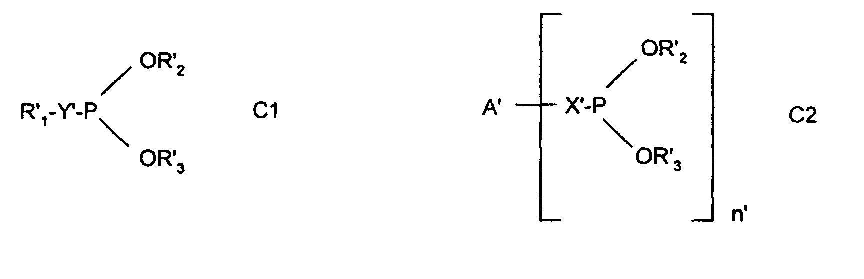 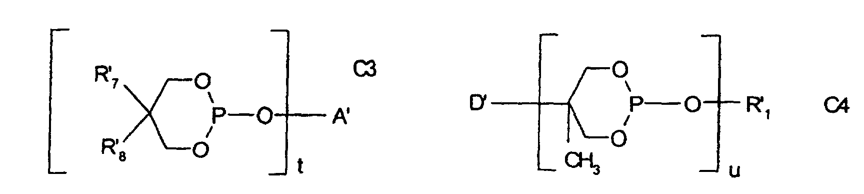 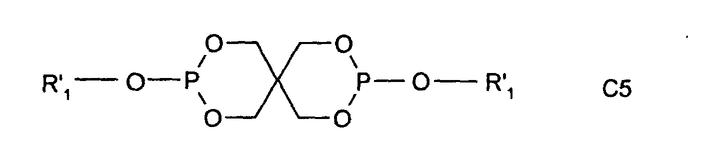 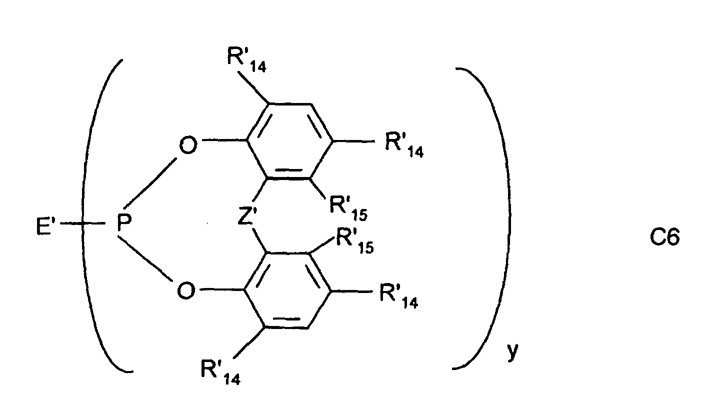 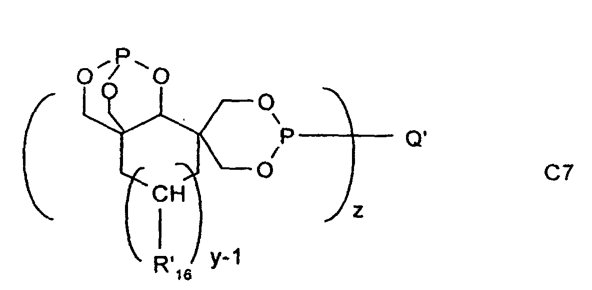 in which the indices are integral and
n' is 2, 3 or 4;
u is 1 or 2;
t is 2 or 3;
y is 1, 2 or 3; and
z is 1 to 6;
A' if n' is 2, is alkylene of 2 to 18 carbon atoms, -S-, -O- or -NR'₄-interrupted alkylene of 2 to 12 carbon atoms; a radical of one of the formulae 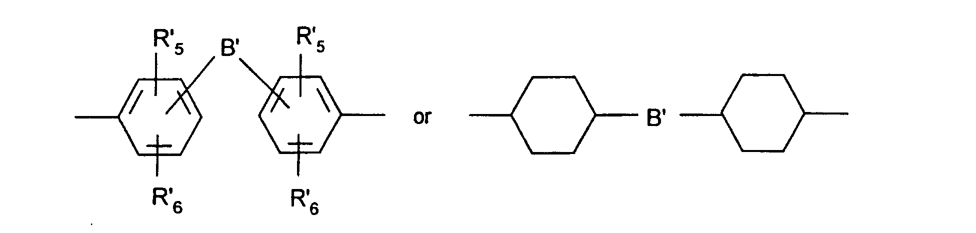 or phenylene;
A' if n' is 3, is a radical of the formula -CᵣH_{2r-1;}
A' if n' is 4, is the radical of the formula 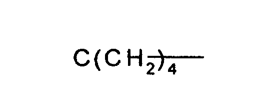 A" is as defined for A' if n' is 2;
B' is a radical of the formula -CH₂-; -CHR'₄-; -CR'₁R'₄-; -S- or a direct bond; or is C₅-C₇-cycloalkylidene; or is cyclohexylidene substituted in position 3, 4 and/or 5 by 1 to 4 C₁-C₄-alkyl radicals,
D' if u is 1, is methyl and, if u is 2, is -CH₂OCH₂-;
E' if y is 1, is alkyl of 1 to 18 carbon atoms, phenyl, a radical of the formula -OR'₁ or halogen;
E' if y is 2, is a radical of the formula O-A"-O-;
E' if y is 3, is a radical of the formula 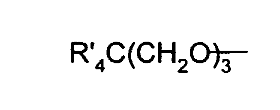 or
N(CH₂-CH₂-O-)₃,
Q' is the radical of an at least z-valent alcohol or phenol which is attached to the phosphorus atom(s) via the alcoholic and/or phenolic oxygen atom(s);
R'₁, R'₂ and R'₃ independently of one another are alkyl of 1 to 30 carbon atoms; halogen-, -COOR'₄-, -CN- or -CONR'₄R'₄-substituted alkyl of 1 to 18 carbon atoms; -S-, -O- or -NR'₄-interrupted alkyl of 2 to 18 carbon atoms; phenyl-C₁-C₄-alkyl; cycloalkyl of 5 to 12 carbon atoms; phenyl or naphthyl; phenyl or naphthyl substituted by halogen, 1 to 3 alkyl radicals or alkoxy radicals having in total 1 to 18 carbon atoms or phenyl-C₁-C₄-alkyl; or a radical of the formula 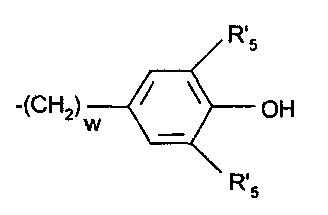 in which w is an integer from the range 3 to 6;
R'₄ or the radicals R'₄ independently of one another is or are hydrogen, alkyl of 1 to 18 carbon atoms, cycloalkyl of 5 to 12 carbon atoms, or phenylalkyl of 1 to 4 carbon atoms in the alkyl moiety;
R'₅ and R'₆ independently of one another are hydrogen, alkyl of 1 to 8 carbon atoms or cycloalkyl of 5 or 6 carbon atoms;
R'₇ and R'₈ if t = 2, independently of one another are C₁-C₄-alkyl or together are a 2,3-dehydropentamethylene radical; and
R'₇ and R'₈ if t = 3, are methyl;
the substituents R'₁₄ independently of one another are hydrogen, alkyl of 1 to 9 carbon atoms or cyclohexyl;
the substituents R'₁₅ independently of one another are hydrogen or methyl; and R'₁₆ is hydrogen or C₁-C₄-alkyl and, if two or more radicals R'₁₆ are present, the radicals R'₁₆ are identical or different;
X' and Y' are each a direct bond or -O-; and
Z' is a direct bond; -CH₂-; -C(R'₁₆)₂- or -S-.

19. A stabilizer mixture as claimed in claim 18 comprising one or more phosphorus compounds of the formulae C'1 to C'12

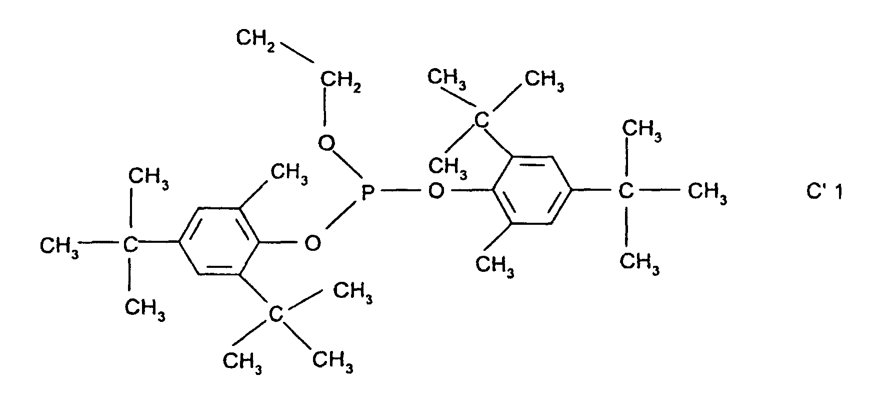

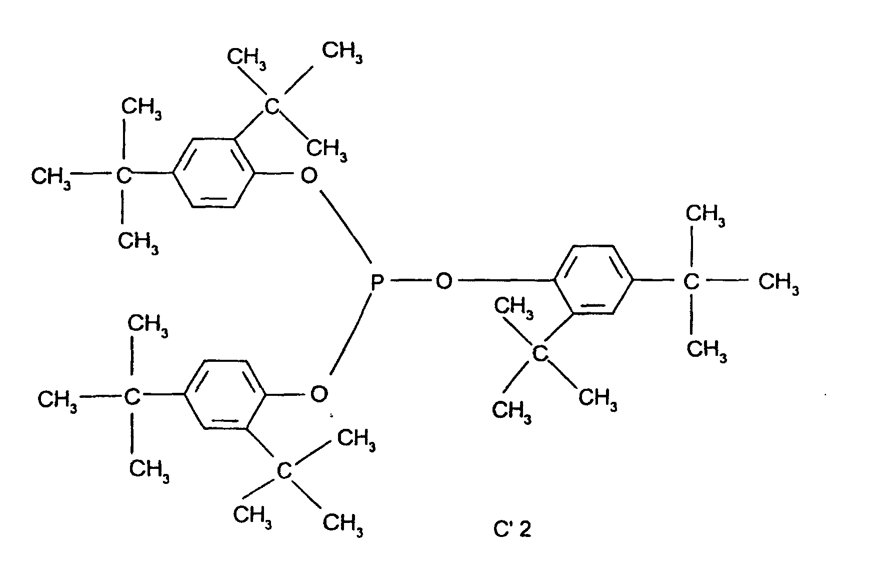

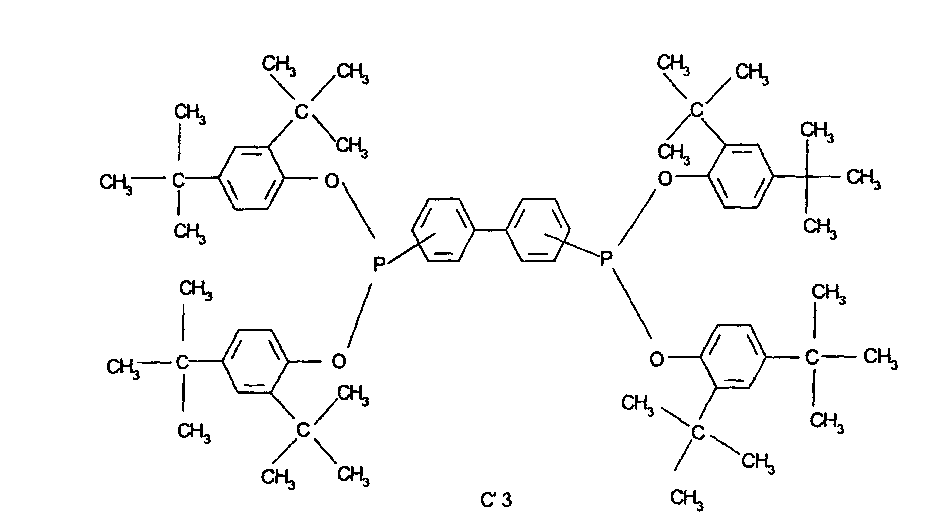

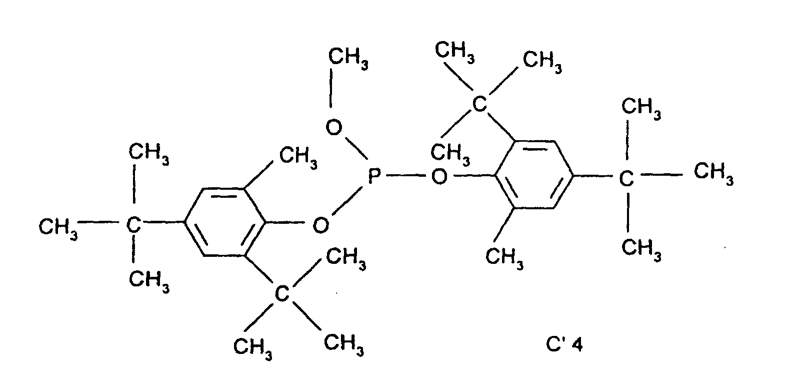

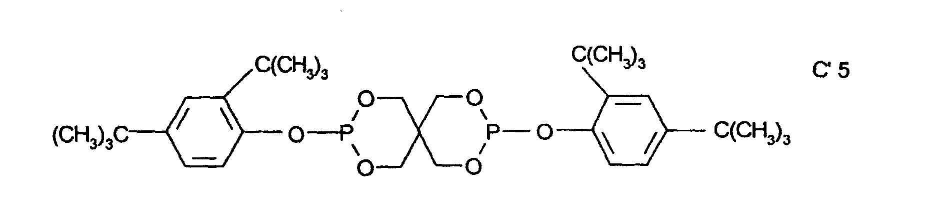

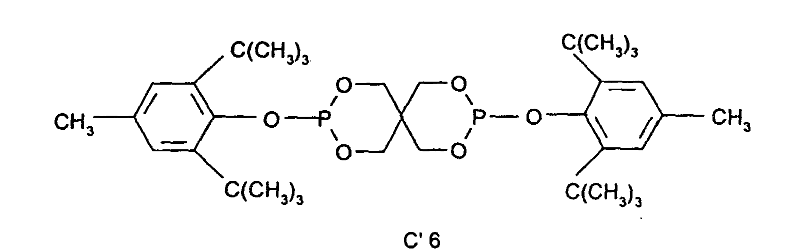

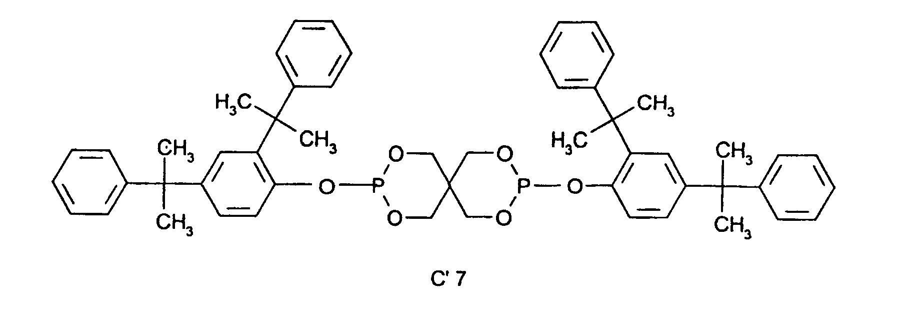

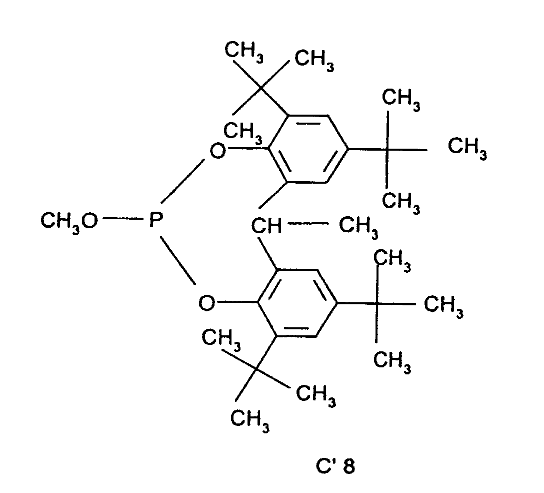

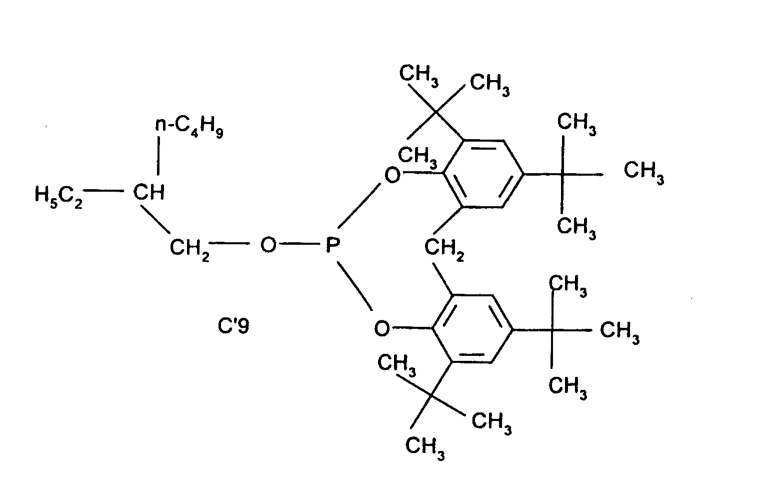

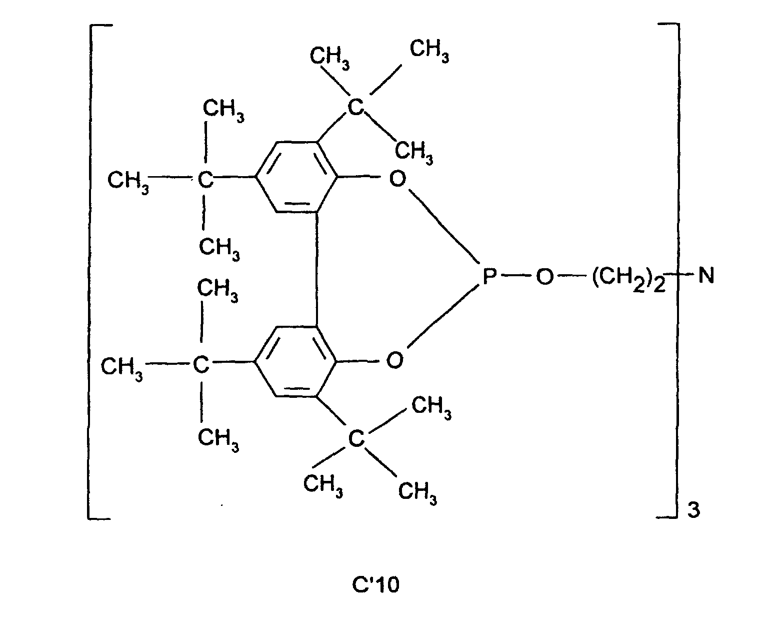

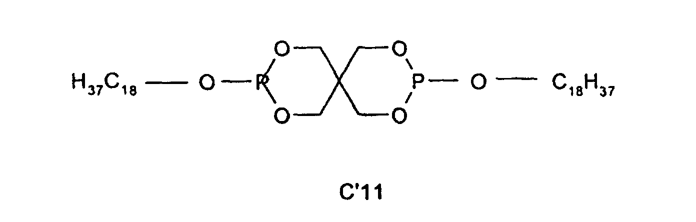

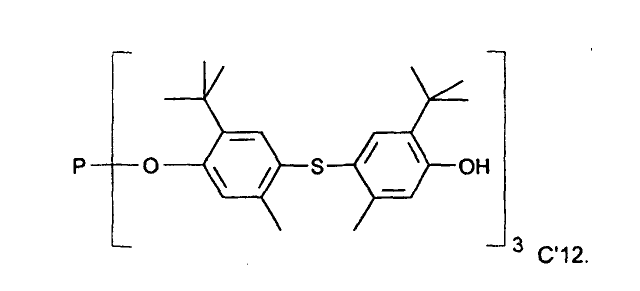

20. A mixture as claimed in claim 18, wherein the phosphorus compounds comprise ®Irgafos 38, ®Irgafos 12, ®Hostanox PAR 24, ®Hostanox OSP 1, ®Irgafos P-EPQ, ®Ultranox 626, ®Ultranox 618, ®Mark PEP-36 , ®Mark HP10, ®Doverphos 9228.

21. A mixture of a compound of the formula (III) as claimed in claim 1 and UV absorber, it being possible for said UV absorbers to be 2-hydroxybenzophenones, 2-hydroxyphenylbenzotriazoles, cinnamic acid derivatives and/or oxyanilides.

22. A mixture as claimed in claims 8 to 21, wherein the proportion of compound of the formula (III) can be between 1 and 99% by weight.

23. A blend of a mixture as claimed in claims 8 to 21 and light stabilizers from the class of the UV absorbers.

24. A blend of compounds of the formula (III) as claimed in claim 1, alone or in mixtures as claimed in claims 8 to 21, and one or more N,N-dialkyl-substituted hydroxylamines.

25. A blend as claimed in claim 24, wherein the N,N-dialkyl-substituted hydroxylamine is N,N-dioctadecylhydroxylamine.

26. A mixture of a compound of the formula (III) as claimed in claim 1 with one or more basic or other acid-binding costabilizers, wherein the latter are selected from the group consisting of metal carboxylates, oxides, hydroxides and carbonates and/or zeolites and/or hydrotalcites.

27. A mixture as claimed in claim 26, wherein the costabilizers comprise calcium stearate and/or magnesium stearate and/or magnesium oxide and zinc oxide and/or carbonate-containing zinc oxide and/or hydrotalcites.

28. A mixture as claimed in claim 26, wherein the costabilizers comprise ®Zinkoxid aktiv, ®Zinkoxid transparent and/or one of the hydrotalcites ®DHT 4A, ®DHT4 A2, ® Kyowaad 200, ®Kyowaad 300, ®Kyowaad 400, ®Kyowaad 500, ®Kyowaad 600, ®Kyowaad 700, ®Kyowaad 1000, ®Kyowaad 2000.

29. A mixture of a compound of the formula (III) as claimed in claim 1 with sterically hindered phenolic antioxidants.

30. A blend of a mixture as claimed in claims 8 to 21 with sterically hindered phenolic antioxidants.

31. A blend of a mixture as claimed in claim 30 with synergists of the 3-pyrazolidinone type.

32. A blend of a mixture as claimed in claim 30 with synergists of the 1,2,4-triazolidine-3,5-dione type.

33. A blend of a mixture as claimed in claim 30 with synergists of the 3-arylbenzofuran-2-one type.

34. A blend of a mixture as claimed in claim 30, wherein the 3-arylbenzofuran-2-one is 3-(3,4-dimethylphenyl)-5,7-di-tert-butyl-3H-benzofuran-2-one (formula D).

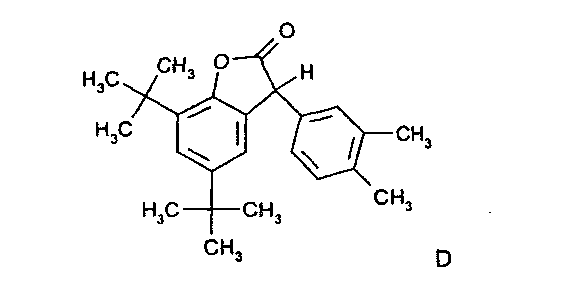

35. A blend of a mixture as claimed in claim 30 and basic or other acid-binding costabilizers.

36. A combination of a compound of the formula (III) as claimed in claim 1 and dyes or pigments based on organic or inorganic substances.

37. The use of a mixture as claimed in claims 8 to 36 for stabilizing organic material.

38. A stabilized organic material comprising a mixture as claimed in claims 8 to 36.

39. A stabilized organic material as claimed in claim 38, which is a polymer.

40. A stabilized organic material as claimed in claim 38, which comprises the compound (III) of the invention as claimed in claim 1 in a concentration of from 0.001 to 5% by weight, based on the stabilized organic material.

## Revendications

1. Composés de formule générale (III) 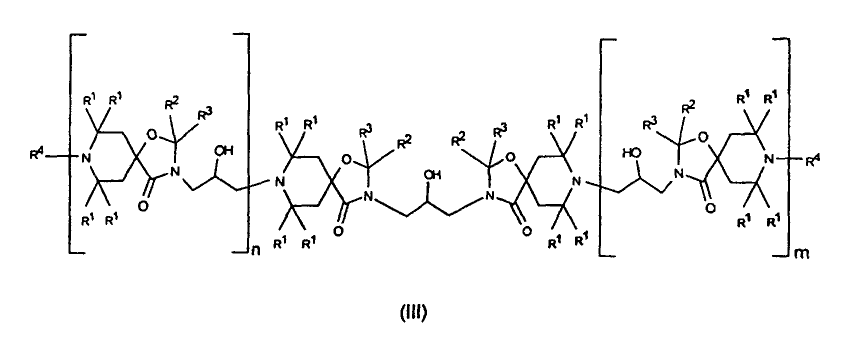 dans laquelle
n et m représentent, indépendamment l'un de l'autre, un nombre allant de 0 à 100, n et m ne pouvant pas être simultanément 0,
R¹ représente un atome d'hydrogène ou un groupe cycloalkyle en C₅-C₇ ou un groupe alkyle en C₁-C₁₂,
R² et R³ représentent, indépendamment l'un de l'autre, un atome d'hydrogène, un groupe alkyle en C₁-C₁₈ ou forment ensemble, avec l'atome de carbone auquel ils sont liés, un cycle ayant une taille de cycle de 5 à 13, ou forment ensemble, avec l'atome de carbone auquel ils sont liés, un groupe de formule (IV) 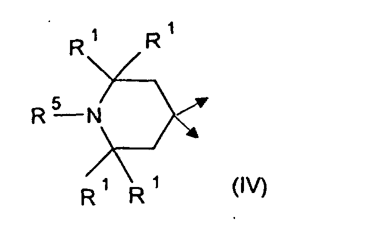 R⁴ et R⁵ représentent, indépendamment l'un de l'autre, soit un atome d'hydrogène, soit un groupe alkyle en C₁-C₂₂, un radical oxygéné O*, -OH, -NO, -CH₂CN, benzyle, allyle, un groupe alkyloxy C₁-C₃₀, un groupe cycloalkyloxy en C₅-C₁₂, un groupe aryloxy en C₆-C₁₀, le fragment aryle pouvant en outre être encore substitué, un groupe arylalkyloxy en C₇-C₂₀, le fragment aryle pouvant en outre être encore substitué, un groupe alcényle en C₃-C₁₀, un groupe alcynyle en C₃-C₆, un groupe acyle en C₁-C₁₀, un atome d'halogène, un groupe phénylalkyle en C₇-C₉ non substitué ou substitué sur le noyau phényle par un groupe alkyle en C₁-C₄.

2. Composés selon la revendication 1, **caractérisés en ce que** n et m représentent, indépendamment l'un de l'autre, un nombre allant de 0 à 10, n et m ne pouvant pas être simultanément 0,
R¹ représente un atome d'hydrogène, un groupe cycloalkyle en C₆ ou un groupe alkyle en C₁-C₄,
R² et R³ représentent, indépendamment l'un de l'autre, un atome d'hydrogène, un groupe alkyle en C₁-C₈ ou forment ensemble, avec l'atome de carbone qui les relie, un cycle ayant une taille de cycle de 6 à 12, ou forment ensemble, avec l'atome de carbone qui les relie, un groupe de formule (IV), R⁴ et R⁵ représentent, indépendamment l'un de l'autre, soit un atome d'hydrogène, soit un groupe alkyle en C₁-C₅, un radical oxygéné O*, -OH, -NO, -CH₂CN, benzyle, allyle, un groupe alkyloxy C₁-C₁₀, un groupe cycloalkyloxy en C₅-C₆, un groupe aryloxy en C₆-C₇, le radical aryle pouvant en outre être encore substitué, un groupe arylalkyloxy en C₇-C₁₀, le radical aryle pouvant en outre être encore substitué, un groupe alcényle en C₃-C₆, un groupe alcynyle en C₃-C₆, un groupe acyle en C₁-C₄, un atome d'halogène, un groupe phénylalkyte en C₇-C₉, non substitué ou substitué sur le noyau phényle par un groupe alkyle en C₁-C₂.

3. Composés selon la revendication 1, **caractérisés en ce que** n et m représentent, indépendamment l'un de l'autre, un nombre allant de 0 à 5, n et m ne pouvant pas être simultanément 0,
R¹ est le groupe méthyle,
R² et R³ forment ensemble, avec l'atome de carbone qui les relie, un cycle ayant une taille de cycle de 12, ou forment ensemble, avec l'atome de carbone qui les relie, un groupe de formule (IV),
R⁴ et R⁵ représentent, indépendamment l'un de l'autre, un atome d'hydrogène, le groupe méthyle, acétyle, octyloxy ou cyclohexyloxy.

4. Composés selon l'une quelconque des revendications 1 à 3, **caractérisés en ce qu'**il s'agit de stabilisants contre l'effet de l'oxygène, de la lumière et de la chaleur.

5. Procédé pour la préparation de composés de formule (III) selon la revendication 1, **caractérisés en ce qu'**on fait réagir des composés de formule (I) avec des composés de formule (II) 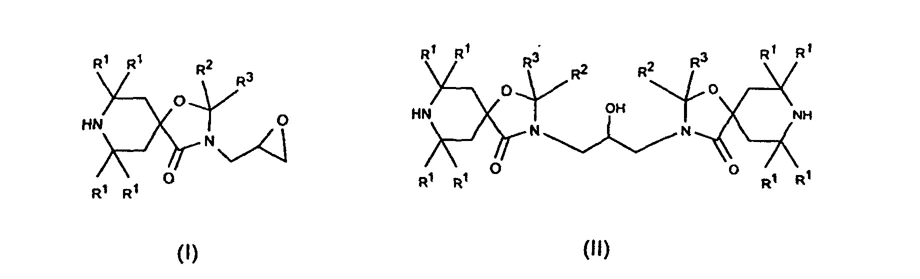 dans laquelle R¹ à R⁴ ont les significations données dans la revendication 1, avec ou sans solvant et avec ou sans catalyseur, en un rapport molaire allant de 1:1 à 100:1 et à 100-300°C.

6. Procédé selon la revendication 5, **caractérisé en ce que** la réaction a lieu sans solvant et sans catalyseur, sous vide, à 120-250°C.

7. Utilisation des composés selon l'une quelconque des revendications 1 à 4, pour la stabilisation d'un matériau organique vis-à-vis de l'endommagement par la lumière et la chaleur.

8. Mélange d'un composé de formule générale (III) selon la revendication 1 avec un ou plusieurs stabilisants à base d'amines à empêchement stérique de formules A1 à A10 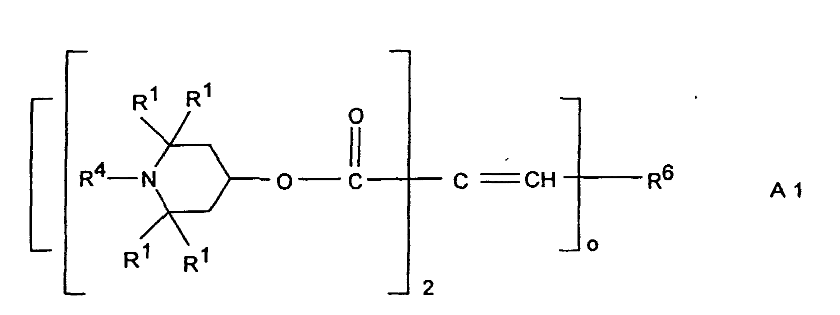 dans laquelle R¹ et R⁴ ont les significations données dans la revendication 1,
R⁶ représente un radical aromatique une ou plusieurs fois substitué par un ou des atomes d'hydrogène ou d'halogène aux groupes alkyle en C₁-C₄, alcoxy en C₁-C₄, cyano, carboxy, nitro, amino, alkyl(C₁-C₄)amino, dialkyl(C₁-C₄)amino ou acyle,
o représente 1 ou 2, 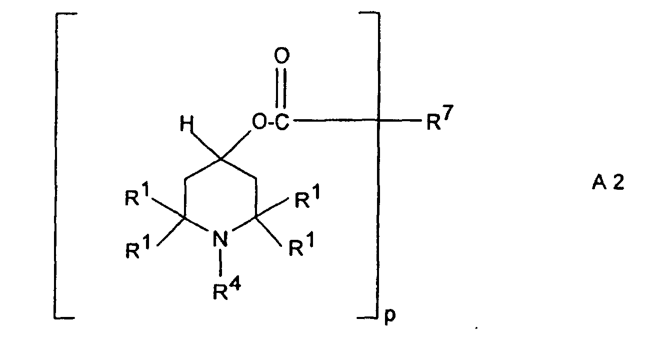 dans laquelle R¹ et R⁴ ont les significations données dans la revendication 1,
p = 1 ou 2 et pour p = 1, R⁷ représente un groupe alkyle en C₁-C₂₂, oxaalkyle en C₂-C₁₈, thiaalkyle en C₂-C₁₈, azaalkyle en C₂-C₁₈ ou alcényle en C₂-C₈, pour p = 2 R⁷ représente un groupe alkylène en C₁-C₂₂, oxaalkylène en C₂-C₁₈, thiaalkylène en C₂-C₁₈, azaalkylène en C₂-C₁₈ ou alcénylène en C₂-C₈, 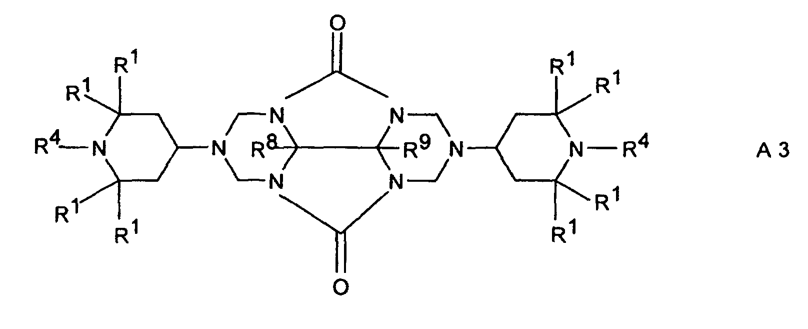 dans laquelle R¹ et R⁴ ont les significations données dans la revendication 1,
R⁸ et R⁹ représentent, indépendamment l'un de l'autre, un atome d'hydrogène ou un groupe alkyle en C₁-C₆, aralkyle en C₇-C₁₂, aryle en C₇-C₁₂ ou ester carboxylique,
R⁸ et R⁹ forment ensemble un groupe tétra- ou pentaméthyle, 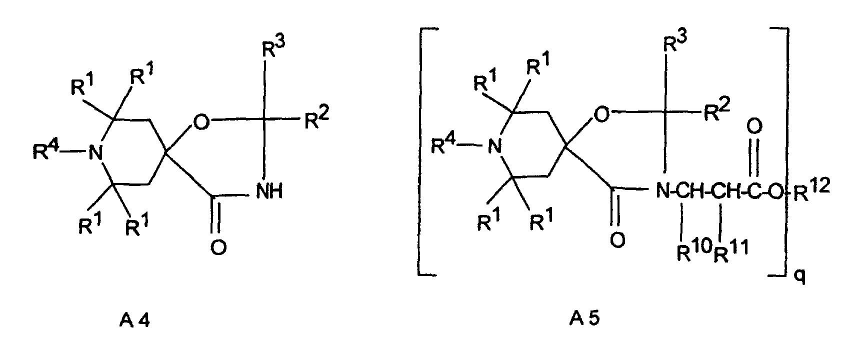 dans laquelle R¹, R², R³ et R⁴ ont les significations données dans la revendication 1,
q est le nombre 1 ou 2,
R¹⁰ représente un atome d'hydrogène ou un groupe méthyle, phényle ou carbalcoxy en C₁-C₂₁,
R¹¹ représente un atome d'hydrogène ou un groupe méthyle,
R¹² représente, pour q = 1, un atome d'hydrogène ou un groupe alkyle en C₁-C₂₁, alcényle en C₂-C₂₂, cycloalkyle en C₅-C₁₂, un radical de formule 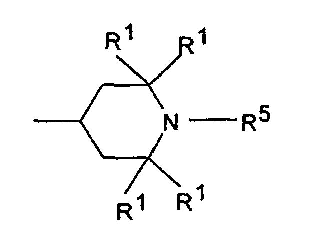 R¹ et R⁵ ayant les significations données dans la revendication 1, et
R¹² représente, pour q = 2, un groupe alkylène en C₁-C₁₈, cycloalkylène en C₅-C₉, ou arylène, 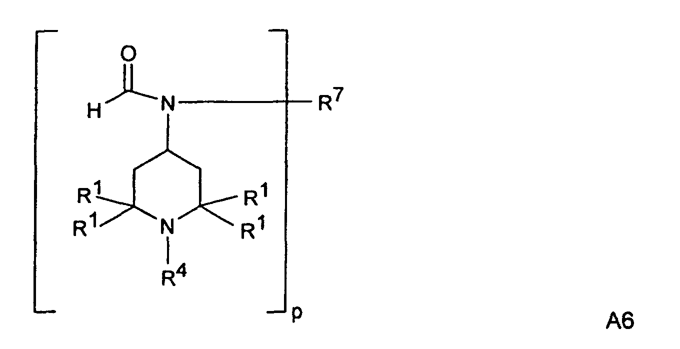 R¹, R⁴, R⁷ et p ayant les définitions indiquées plus haut, 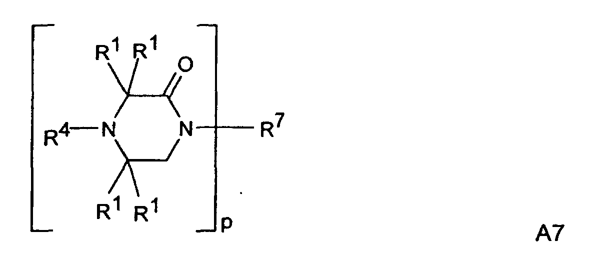 R¹, R⁴, R⁷ et p ayant les définitions indiquées plus haut, 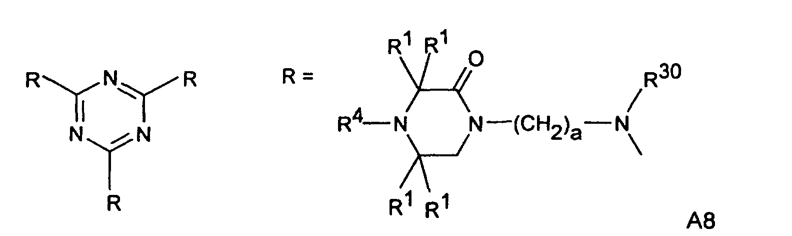 R¹, R⁴ ayant les définitions indiquées plus haut,
R³⁰ représentant un atome d'hydrogène ou un groupe alkyle en C₁-C₁₂, cycloalkyle en C₅-C₁₂, phényle ou phénylalkyle en C₇-C₉, et a représentant un nombre allant de 1 à 10, 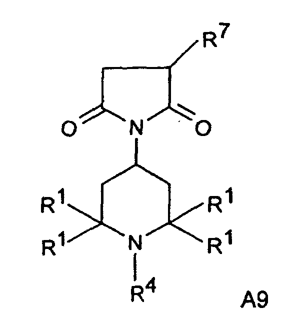 R¹, R⁴ ayant les significations données plus haut, et R⁷ ayant la signification donnée dans la formule A2 pour R⁷ et p = 1; un produit A10 pouvant être obtenu par la réaction d'une polyamine de formule A10a avec une polyamine de formule A10b: 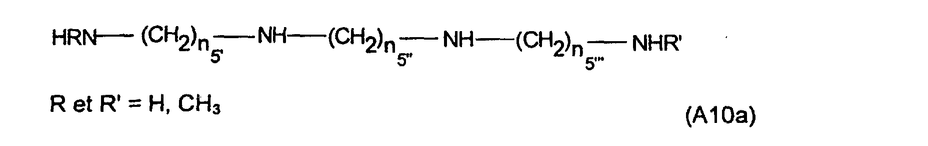 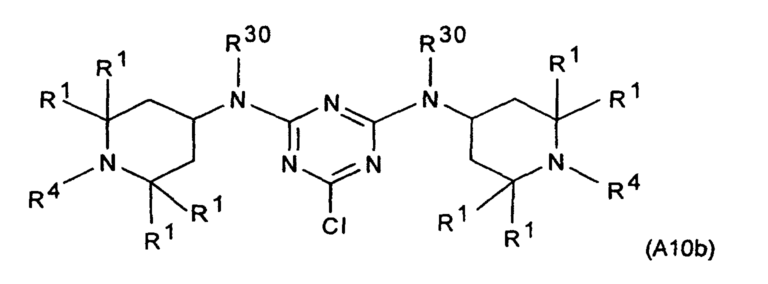 R¹, R⁴ et R³⁰ ayant les significations données plus haut,
n_{5'}, n_{5"}, et n_{5"'} représentant, indépendamment les uns des autres, un nombre allant de 2 à 12.

9. Mélange selon la revendication 8, **caractérisé en ce que** n et m représentent, indépendamment l'un de l'autre, un nombre allant de 0 à 10, n et m ne pouvant pas être simultanément 0,
R¹ représente un atome d'hydrogène ou un groupe alkyle en C₁-C₄,
R² et R³ représentent, indépendamment l'un de l'autre, un atome d'hydrogène, un groupe alkyle en C₁-C₈, ou forment ensemble, avec l'atome de carbone qui les relie, un cycle ayant une taille de cycle de 6 à 12, ou forment ensemble, avec l'atome de carbone qui les relie, un groupe de formule (IV),
R⁴ et R⁵ représentent, indépendamment l'un de l'autre, soit un atome d'hydrogène, soit un groupe alkyle en C₁-C₅, un radical oxygéné O*, -OH, -NO, -CH₂CN, benzyle, allyle, un groupe alkyloxy C₁-C₁₀, un groupe cycloalkyloxy en C₅-C₆, un groupe aryloxy en C₆-C₇, le radical aryle pouvant en outre être encore substitué, un groupe arylalkyloxy en C₇-C₁₀, le radical aryle pouvant en outre être encore substitué, un groupe alcényle en C₃-C₆, un groupe alcynyle en C₃-C₆, un groupe acyle en C₁-C₄, un atome d'halogène, un groupe phénylalkyle en C₇-C₉ non substitué ou substitué sur le noyau phényle par un groupe alkyle en C₁-C₂,
R⁷ représente un groupe alkylène en C₁-C₁₀ à chaîne droite (pour p = 2); un groupe alkyle en C₁-C₁₂ (pour p = 1),
R⁸ et R⁹ représentent, indépendamment l'un de l'autre, un atome d'hydrogène, un groupe alkyle en C₁-C₂, un groupe arylalkyle en C₇-C₈, aryle ou ester d'acide carboxylique,
R¹⁰ représente un atome d'hydrogène, un groupe méthyle, phényle ou alcoxy en C₁-C₂,
R¹¹ représente un atome d'hydrogène ou un groupe méthyle,
R¹² représente, pour q = 1, un atome d'hydrogène, un groupe alkyle en C₁-C₁₆, alcényle en C₂-C₁₆, cycloalkyle en C₅-C₆, un radical de formule 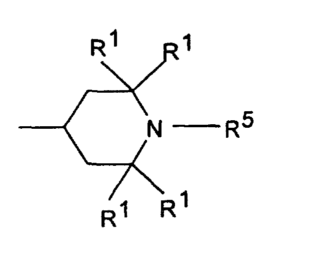 R¹² représente, pour q = 2, un groupe alkylène en C₁-C₁₆, cycloalkylène en C₅-C₆, ou arylène,
R³⁰ représente un atome d'hydrogène, un groupe alkyle en C₁-C₈, cycloalkyle en C₅-C₇, phényle ou phénylalkyle en C₇-C₈,
a va de 1 à 5,
o est égal à 1 et
p va de 2 à 5.

10. Mélange selon la revendication 8, **caractérisé en ce que** n et m représentent, indépendamment l'un de l'autre, un nombre allant de 0 à 5, n et m ne pouvant pas être simultanément 0,
R¹ représente le groupe méthyle,
R² et R³ forment ensemble, avec l'atome de carbone qui les relie, un cycle ayant une taille de cycle de 12, ou forment ensemble, avec l'atome de carbone qui les relie, un groupe de formule (IV),
R⁴ et R⁵ représentent, indépendamment l'un de l'autre, un atome d'hydrogène ou le groupe méthyle, acétyle, octyloxy ou cyclohexyloxy,
R⁶ représente le groupe p-méthoxyphényle,
R⁷ représente un groupe octaméthylène, hexaméthylène ou éthylène (pour p = 2), dodécyle (pour p = 1),
R⁸ et R⁹ représentent un atome d'hydrogène,
R¹⁰ représente un atome d'hydrogène,
R¹¹ représente un atome d'hydrogène,
R¹² représente un groupe dodécaméthylène ou tétradécaméthylène,
R³⁰ représente le groupe cyclohexyle ou n-butyle,
a est égal à 2,
o est égal à 1,
p est égal à 2, et
q est égal à 1.

11. Mélange selon la revendication 8, **caractérisé en ce que** les composés HALS du mélange avec des composés de formule (III) consistent en les substances suivantes: 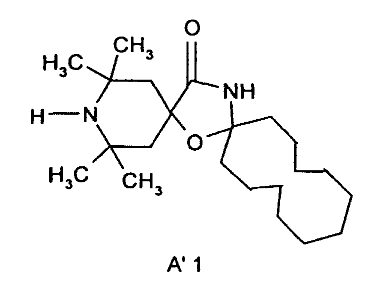 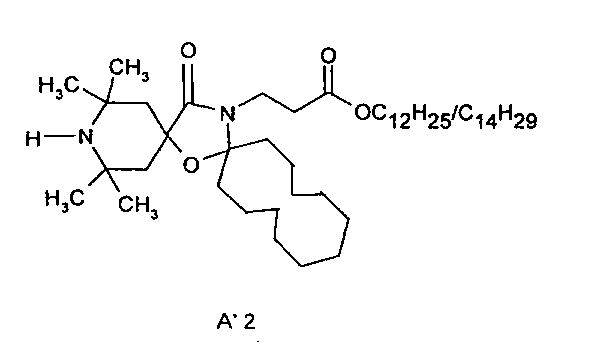 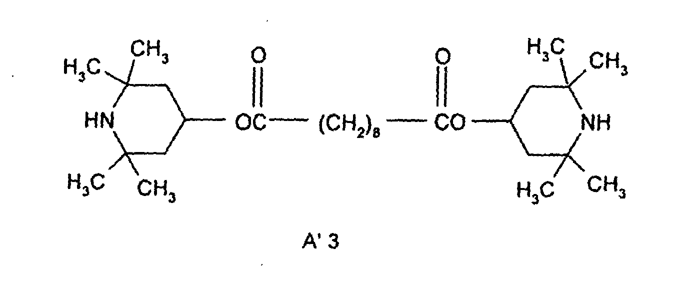 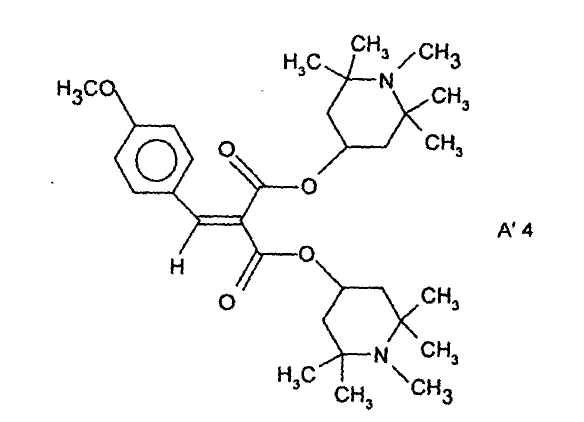 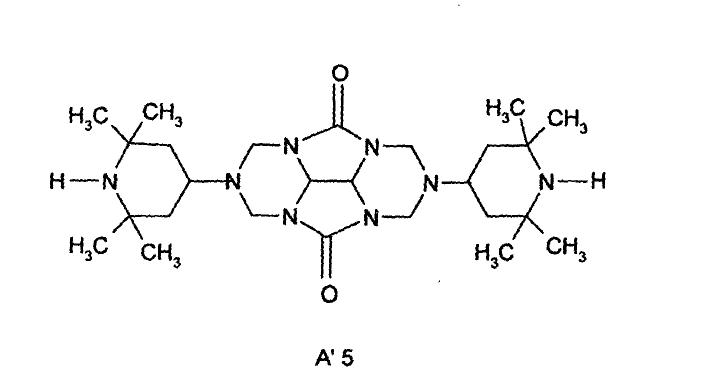 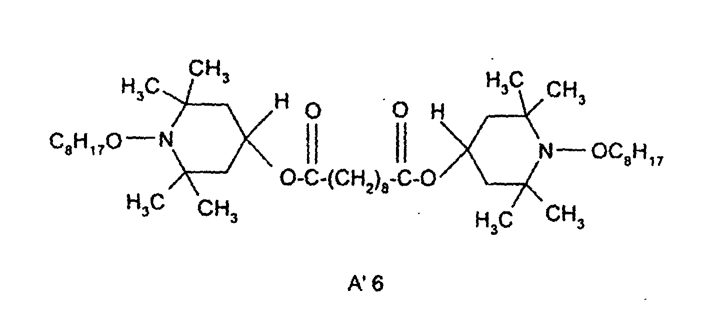 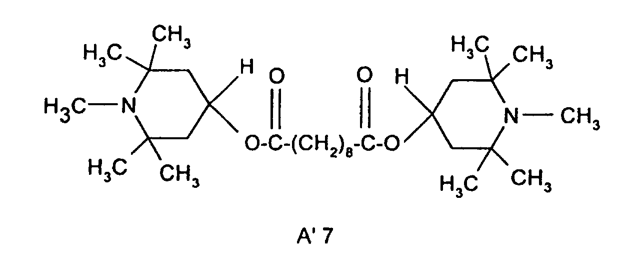 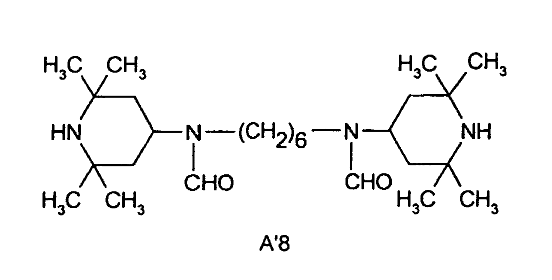 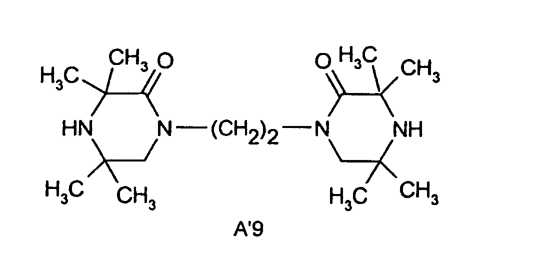 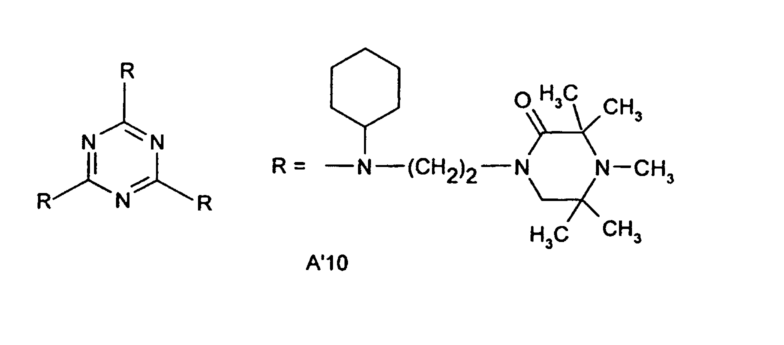 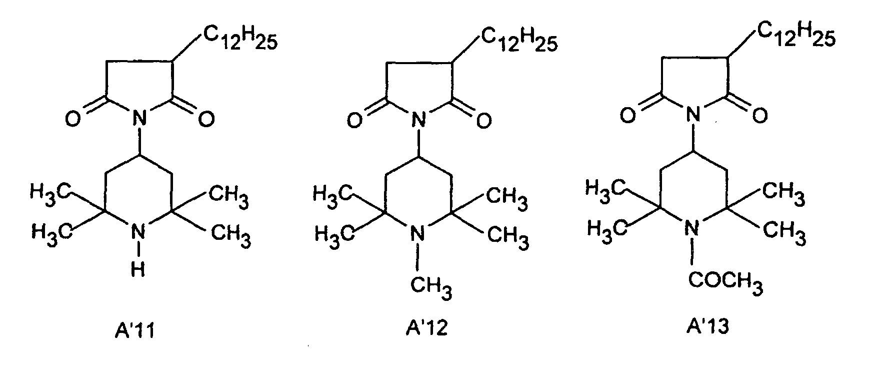 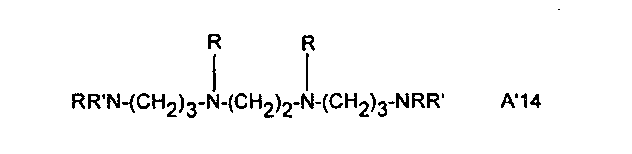 où 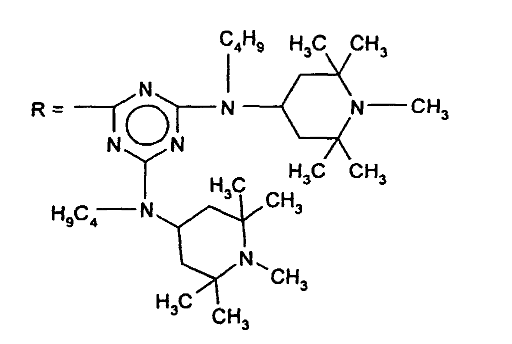 et
R' = H, CH₃

12. Mélange selon la revendication 8, **caractérisé en ce que** le(s) composant(s) de mélange consistent en Tinuvin® 770, Tinuvin® 765, Tinuvin® 123, Hostavin® N 20, Hostavin® N 24, Uvinul® 4049, Sanduvor® PR31, Uvinul® 4050, Good-rite® UV 3034 ou Good-rite® 3150, Sanduvor® 3055, Sanduvor® 3056, Sanduvor® 3058, Chimassorb® 119 et Chimassorb® 905.

13. Mélange d'un composé de formule générale (III) selon la revendication 1 avec un ou plusieurs composés HALS polymères de formules B1 à B7: 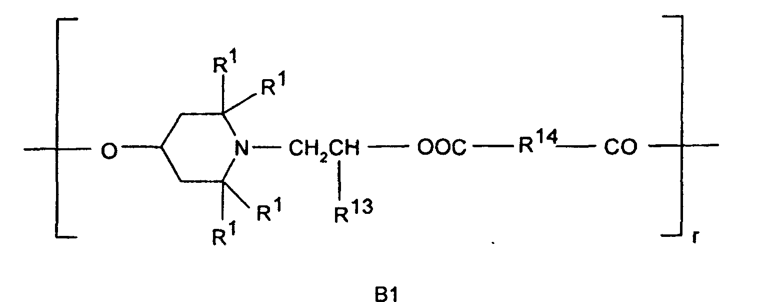 dans laquelle R¹ a la signification donnée dans la revendication 1,
R¹³ représente un atome d'hydrogène ou le groupe méthyle,
R¹⁴ représente une liaison directe ou un groupe alkylène en C₁-C₁₀ et
r représente un nombre allant de 2 à 50, 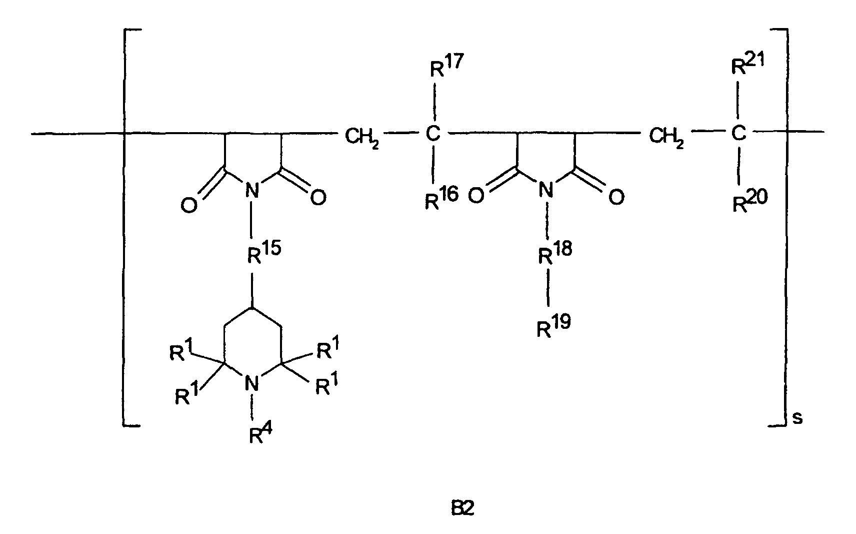 R¹ et R⁴ ayant les significations données dans la revendication 1,
R¹⁵ et R¹⁸ représentant, indépendamment l'un de l'autre, une liaison directe ou un groupe -N(R²²)-CO-R²³-CO-N(R²⁴)-,
R²² et R²⁴ représentant, indépendamment l'un de l'autre, un atome d'hydrogène, un groupe alkyle en C₁-C₈, cycloalkyle en C₅-C₁₂, phényle, phénylalkyle en C₇-C₉ ou un groupe de formule 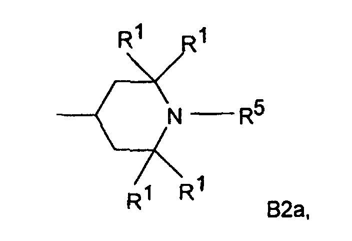 R²³ représentant une liaison directe ou un groupe alkylène en C₁-C₄,
R¹⁶, R¹⁷, R²⁰, R²¹ représentant, indépendamment les uns des autres, un atome d'hydrogène, un groupe alkyle en C₁-C₃₀, cycloalkyle en C₅-C₁₂, phényle ou un groupe de formule B2a,
R¹⁹ représentant un atome d'hydrogène, un groupe alkyle en C₁-C₃₀, cycloalkyle en C₅-C₁₂, phénylalkyle en C₇-C₉, phényle, ou un groupe de formule B2a, et
s étant un nombre allant de 1 à 50, 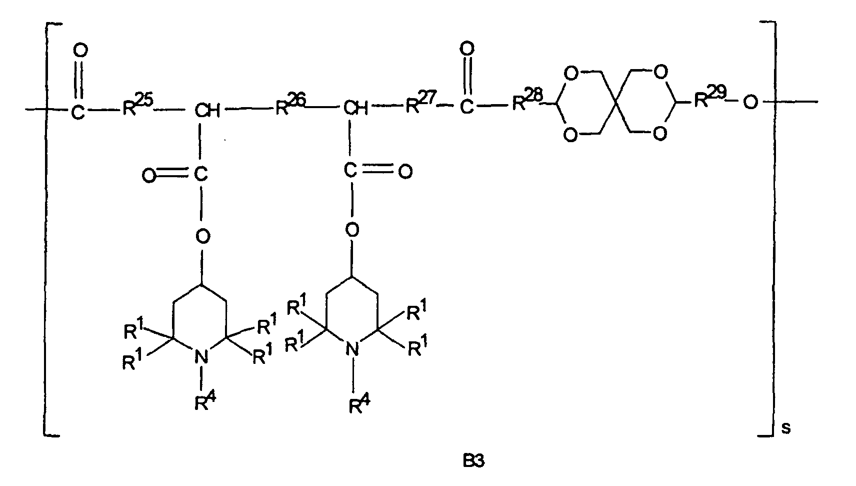 R¹, R⁴ et s ayant les significations données plus haut,
R²⁵, R²⁶, R²⁷, R²⁸ et R²⁹ représentant, indépendamment les uns des autres, une liaison directe ou un groupe alkylène en C₁-C₁₀,
un produit B4 pouvant être obtenu par la réaction d'un composé de formule B4b avec un produit obtenu par la réaction d'une polyamine de formule B4a avec du chlorure de cyanuryle 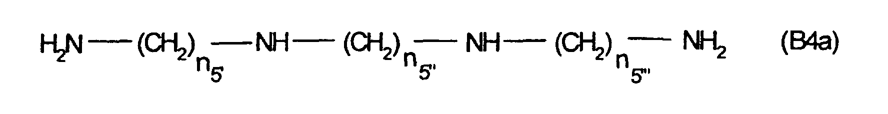 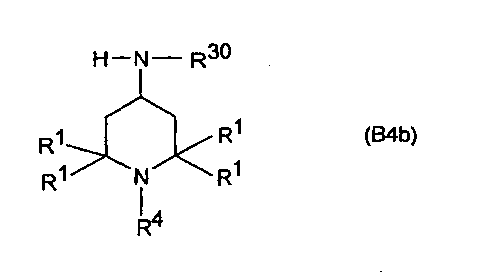 R¹ et R⁴ ayant les significations données dans la revendication 1, n_{5'}, n_{5"}, n_{5"'} représentant, indépendamment les uns des autres, un nombre allant de 2 à 12,
R³⁰ représentant un atome d'hydrogène ou un groupe alkyle en C₁-C₁₂, cycloalkyle en C₅-C₁₂, phényle ou phénylalkyle en C₇-C₉; B4 étant un composé de formule B4-1, B4-2, B4-3 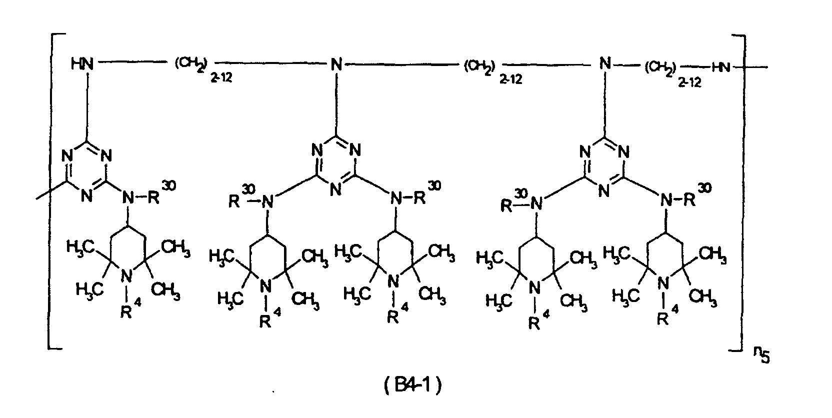 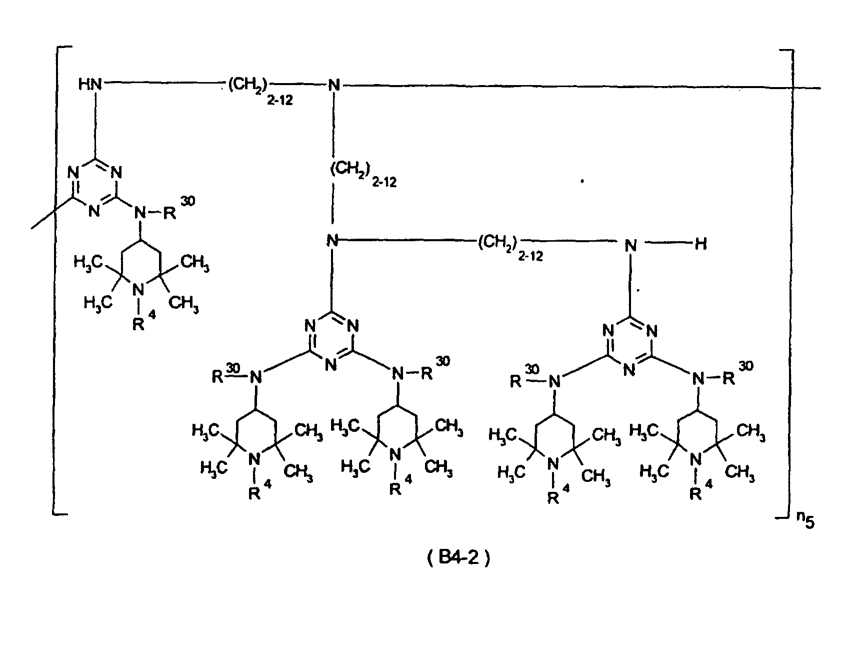 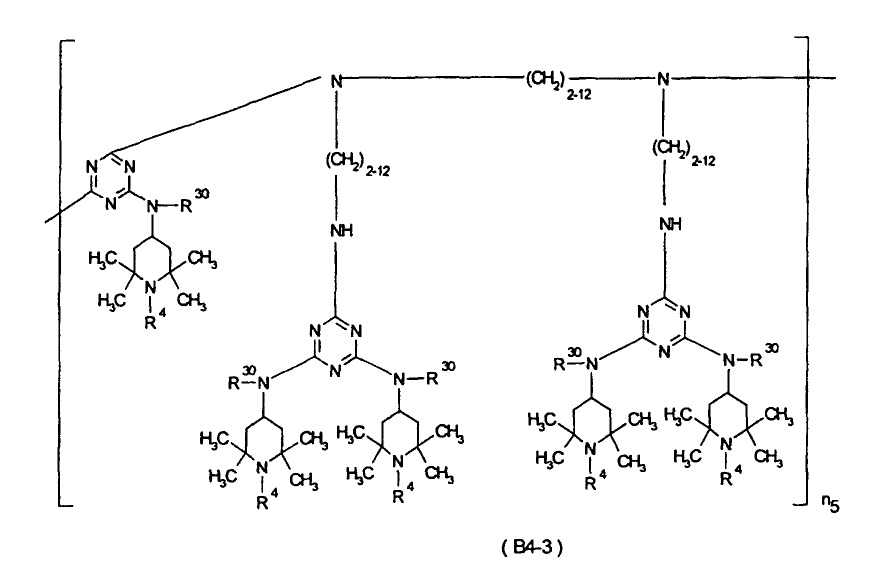 ou un mélange de tels composés, dans lesquels, n₅ va de 1 à 20,
R⁴ et R³⁰ ont les significations données plus haut, 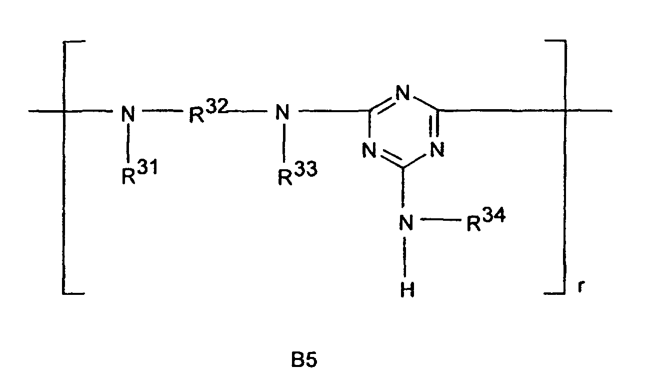 r ayant la signification donnée dans la formule B1,
R³¹, R³³ et R³⁴ représentant, indépendamment les uns des autres, un atome d'hydrogène, un groupe alkyle en C₁-C₁₂, cycloalkyle en C₅-C₁₂, un groupe cycloalkyle en C₅-C₁₂ substitué par un groupe alkyle en C₁-C₄, le groupe phényle, un groupe phényle substitué par -OH et/ou par un groupe alkyle en C₁-C₁₀, un groupe phénylalkyle en C₇-C₉, un groupe phénylalkyle en C₇-C₉ substitué sur le reste phényle par-OH et/ou par un groupe alkyle en C₁-C₁₀ ou un groupe de formule B5a 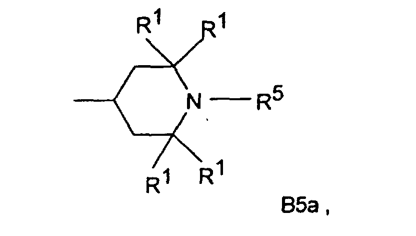 R¹ et R⁵ ayant les significations données dans la revendication 1, et
R³¹ et R³³ représentant additionnellement, indépendamment l'un de l'autre, un atome d'hydrogène,
R³² représentant un groupe alkylène en C₂-C₁₈, cycloalkylène en C₅-C₇ ou alkylène(C₁-C₄)-dicycloalkylène(C₅-C₇) ou les radicaux R³¹, R³² et R³³ formant ensemble, avec les atomes d'azote auxquels ils sont fixés, un cycle hétérocyclique à 5-10 chaînons, et au moins l'un des radicaux R³¹, R³³ et R³⁴ représentant un groupe de formule B5a 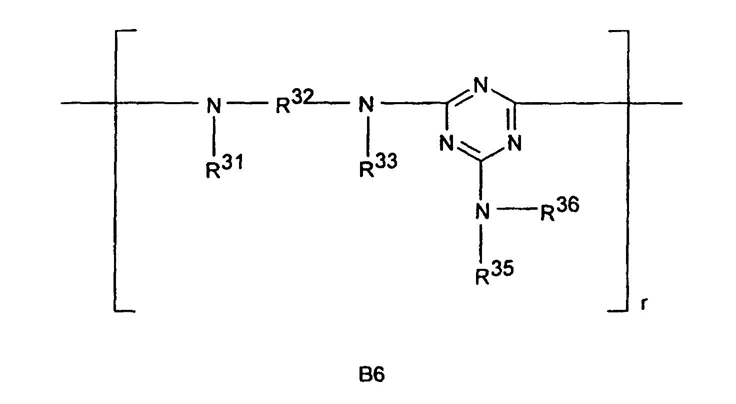 dans laquelle R³¹, R³² et R³³ et r ont les significations données plus haut,
R³⁵ et R³⁶ ont indépendamment l'un de l'autre la définition de R³⁴, ou
R³⁵ et R³⁶ forment ensemble, avec l'atome d'azote auquel ils sont fixés, un cycle hétérocyclique à 5-10 chaînons, ce demier pouvant comporter, en plus de l'hétéroatome d'azote, encore un ou plusieurs hétéroatomes, de préférence un atome d'oxygène, et au moins l'un des radicaux R³¹, R³³, R³⁵ et/ou R³⁶ représentant un groupe de formule (B5a); 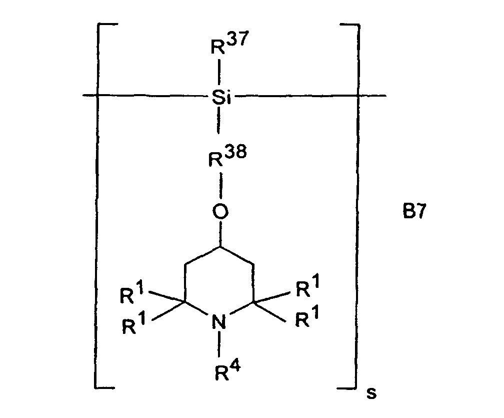 R¹ et R⁴ ayant les significations données dans la revendication 1,
s ayant la signification indiquée dans la formule B3,
R³⁷ étant un groupe alkyle en C₁-C₁₀, cycloalkyle en C₅-C₁₂, cycloalkyle en C₅-C₁₂ substitué par un groupe alkyle en C₁-C₄, le groupe phényle, ou un groupe phényle substitué par un groupe alkyle en C₁-C₁₀, et
R³⁸ représentant un groupe alkylène en C₃-C₁₀.

14. Mélange selon la revendication 13, **caractérisé en ce que** n et m représentent, indépendamment l'un de l'autre, un nombre allant de 0 à 10, n et m ne pouvant pas être simultanément 0,
R¹ représente un atome d'hydrogène ou un groupe alkyle en C₁-C₄,
R² et R³ représentent, indépendamment l'un de l'autre, un atome d'hydrogène, un groupe alkyle en C₁-C₈, ou forment ensemble, avec l'atome de carbone qui les relie, un cycle ayant une taille de cycle de 6 à 12, ou forment ensemble, avec l'atome de carbone qui les relie, un groupe de formule (IV),
R⁴ et R⁵ représentent, indépendamment l'un de l'autre, soit un atome d'hydrogène, soit un groupe alkyle en C₁-C₅, un radical oxygéné O*, -OH, -NO, -CH₂CN, benzyle, allyle, un groupe alkyloxy en C₁-C₁₀, un groupe cycloalkyloxy en C₅-C₆, un groupe aryloxy en C₆-C₇, le radical aryle pouvant en outre être encore substitué par un groupe arylalkyloxy en C₇-C₁₀, le radical aryle pouvant en outre être encore substitué par un groupe alcényle en C₃-C₆, un groupe alcynyle en C₃-C₆, un groupe acyle en C₁-C₄, un atome d'halogène, un groupe phénylalkyle en C₇-C₉ non substitué ou substitué sur le noyau phényle par un groupe alkyle en C₁-C₂,
R¹³ représente un atome d'hydrogène ou un groupe méthyle,
R¹⁴ représente un groupe alkylène en C₁-C₅,
R¹⁷, R²¹ représentent un atome d'hydrogène ou un groupe alkyle en C₁-C₄,
R¹⁵, R¹⁸ représentent une liaison directe,
R¹⁶, R²⁰ représentent un groupe alkyle en C₁-C₂₅, le groupe phényle,
R¹⁹ représente un atome d'hydrogène, un groupe alkyle en C₁-C₁₂, ou un groupe de formule B2a,
R²⁵, R²⁶, R²⁷, R²⁸ et R²⁹ représentent, indépendamment les uns des autres, une liaison directe ou un groupe alkylène en C₁-C₅,
R³⁰ représente un atome d'hydrogène ou un groupe alkyle en C₁-C₄, cycloalkyle en C₅-C₆, phényle,
R³¹, R³³ et R³⁴ représentent, indépendamment les uns des autres, un atome d'hydrogène, un groupe alkyle en C₁-C₁₀, un groupe cycloalkyle en C₅-C₆, ou un groupe de formule B5a,
R³² représente un groupe alkylène en C₂-C₁₀, cycloalkylène en C₅-C₆, R³⁵ et R³⁶ ont indépendamment l'un de l'autre la signification de R³⁴, ou
R³⁵ et R³⁶ forment ensemble, avec l'atome d'azote auquel ils sont fixés, un cycle hétérocyclique à 5-7 chaînons, ce dernier pouvant contenir encore un ou plusieurs hétéroatomes, de préférence un atome d'oxygène, et au moins l'un des radicaux R³¹, R³³, R³⁵ et/ou R³⁶ représentant un groupe de formule B5a,
R³⁷ représente un groupe alkyle en C₁-C₅, cycloalkyle en C₅-C₆ ou phényle,
R³⁸ représente un groupe alkylène en C₃-C₅ et
n_{5'}, n_{5"}, n_{5'''} vont de 2 à 4.

15. Mélange selon la revendication 13, **caractérisé en ce que** n et m représentent, indépendamment l'un de l'autre, un nombre allant de 0 à 5, n et m ne pouvant pas être simultanément 0,
R¹ représente le groupe méthyle,
R² et R³ forment ensemble, avec l'atome de carbone auquel ils sont fixés, un cycle ayant une taille de cycle de 12, ou forment ensemble, avec l'atome de carbone auquel ils sont fixés, un groupe de formule (IV),
R⁴ et R⁵ représentent, indépendamment l'un de l'autre, un atome d'hydrogène, le groupe acétyle, méthyle, octyloxy ou cyclohexyloxy,
R¹³ représente un atome d'hydrogène,
R¹⁴ représente le groupe éthylène,
R¹⁷, R²¹ représentent un atome d'hydrogène ou un groupe méthyle,
R¹⁵, R¹⁸ représentent une liaison directe,
R¹⁶, R²⁰ représentent un groupe alkyle en C₁-C₂₅, le groupe phényle,
R¹⁹ représente le groupe hexadécyle ou un groupe de formule B2a,
R²⁵, R²⁷ représentent le groupe méthylène,
R²⁶ représente une liaison directe,
R²⁸ représente le groupe 2,2-diméthyléthylène,
R²⁹ représente le groupe 1,1-diméthyléthylène,
R³⁰ représente le groupe n-butyle,
R³¹, R³³ et R³⁴ représentent, indépendamment les uns des autres, le groupe isooctyle, cyclohexyle ou 2,2,6,6-tétraméthyl-pipérid-4-yle, au moins l'un des radicaux R³¹, R³³ et R³⁴ devant représenter le groupe 2,2,6,6-tétraméthyl-pipérid-4-yle,
R³² représente un groupe hexaméthylène,
R³⁵ et R³⁶ correspondent, indépendamment l'un de l'autre à la définition de R³⁴, ou
R³⁵ et R³⁶ forment ensemble, avec l'atome d'azote auquel ils sont fixés, un cycle hétérocyclique à 6 chaînons, ce dernier contenant encore un atome d'oxygène et étant par suite le groupe morpholino, au moins l'un des radicaux R³¹, R³³, R³⁵ et/ou R³⁶ devant représenter un radical 2,2,6,6-tétraméthyl-pipérid-4-yle,
R³⁷ représente un groupe méthyle,
R³⁸ représente le groupe triméthylène,
n_{5'}, n_{5"}, n_{5"'} vont de 2 à 4.

16. Mélange selon la revendication 13, **caractérisé en ce que** les composés HALS polymères en association avec des composés de formule (III) consistent en les substances suivantes: 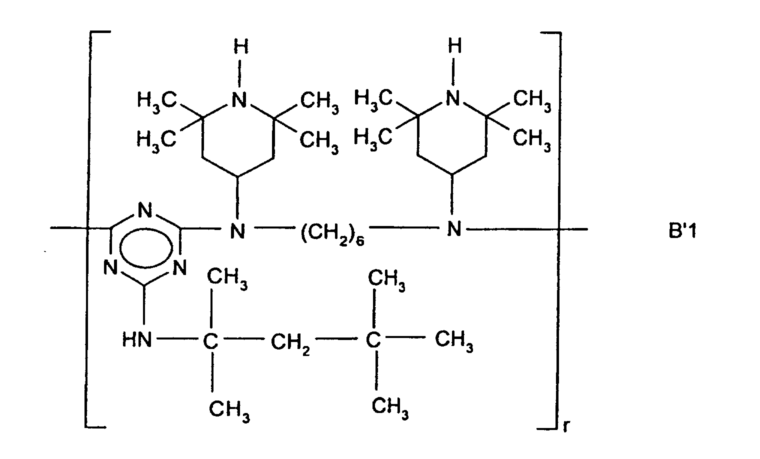 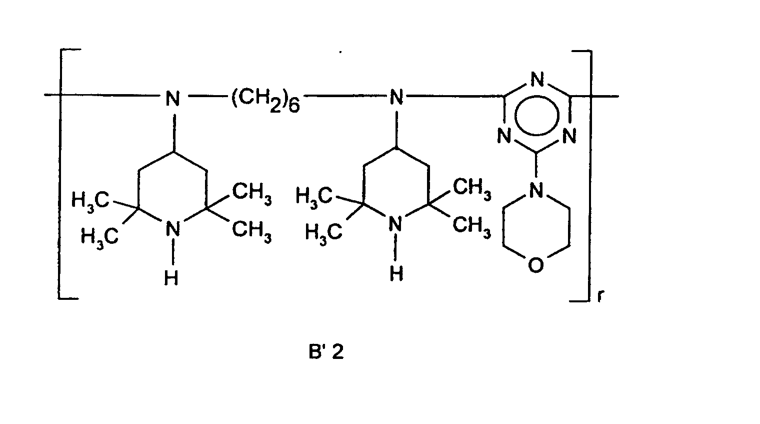 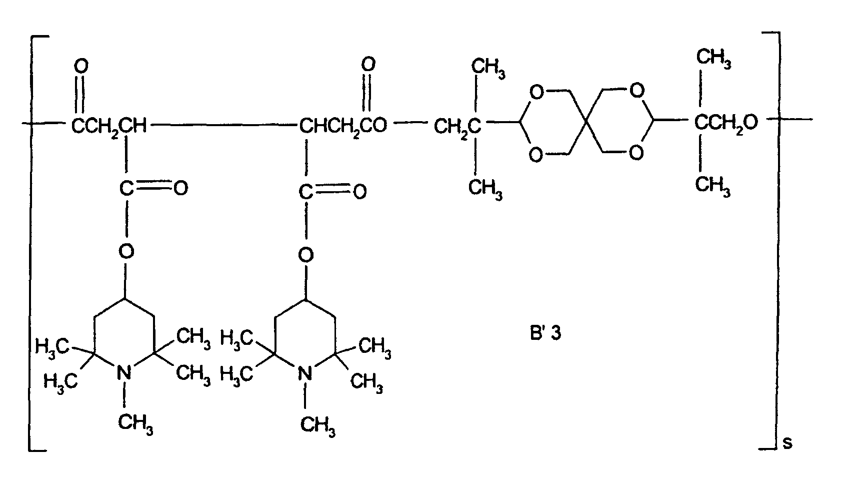 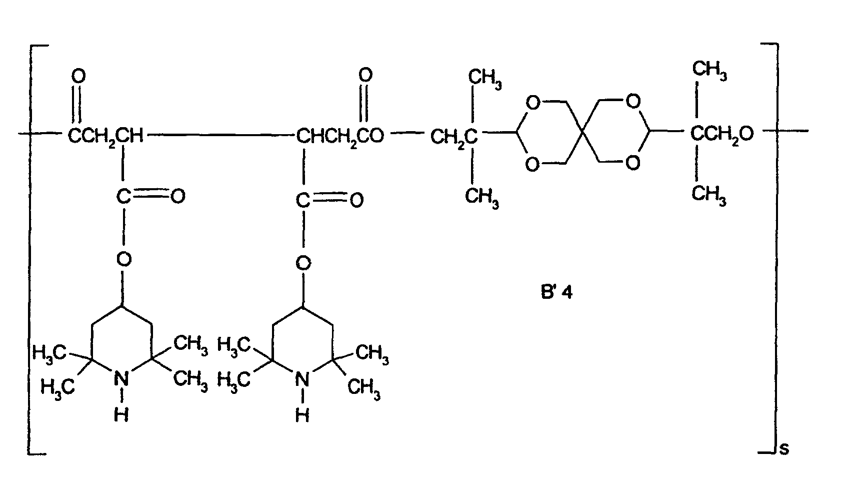 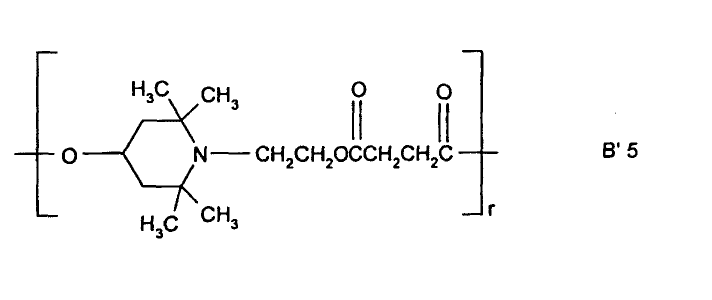 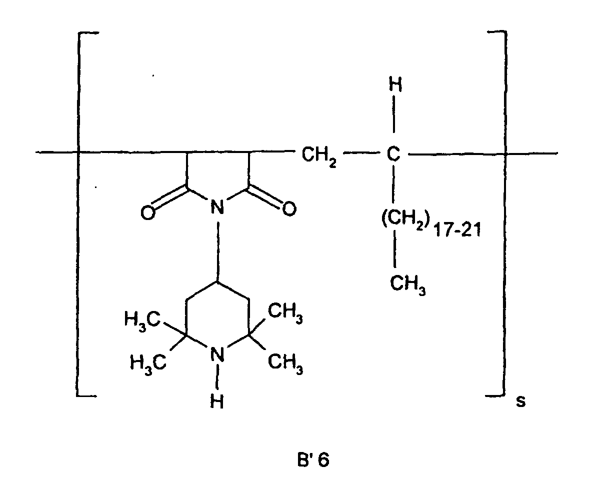 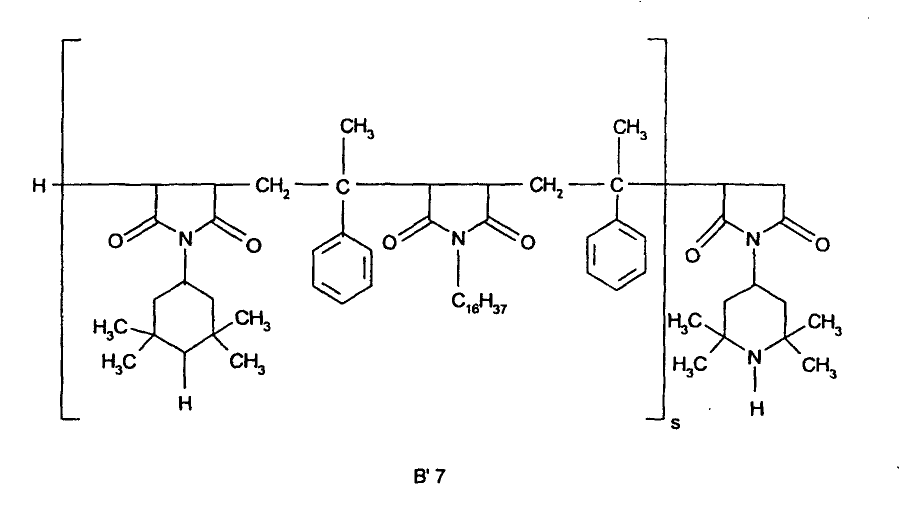 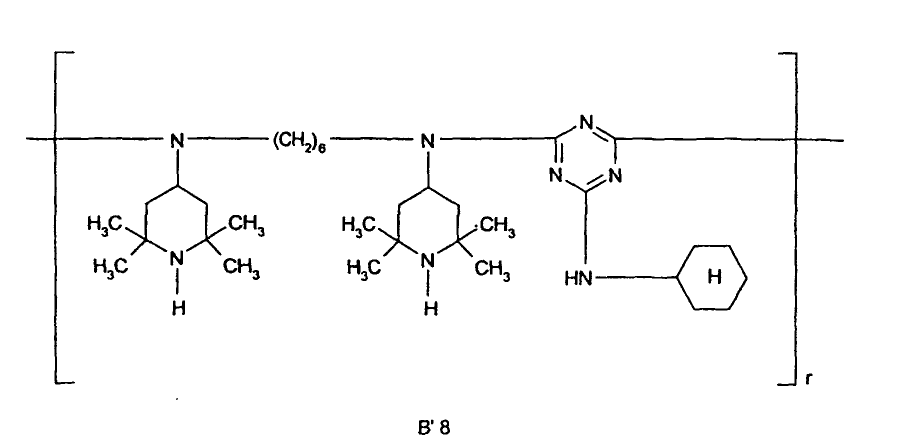 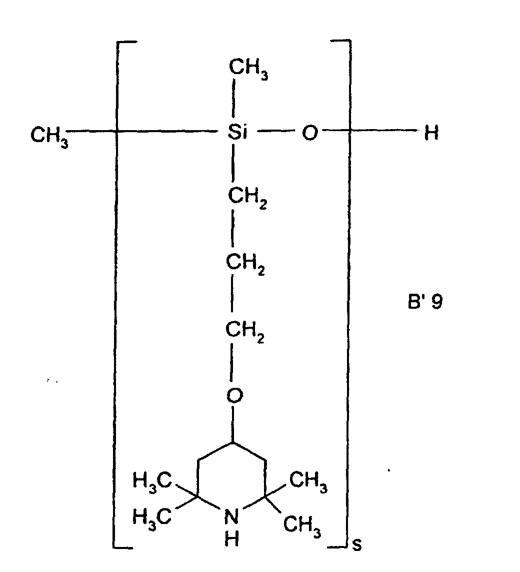 un produit B'10 pouvant être obtenu par la réaction d'un produit obtenu par la réaction d'une polyamine de formule B'10a
H₂N-(CH₂)₃-NH-(CH₂)₂-NH-(CH₂)₃-NH₂ (B'10a)
avec du chlorure de cyanuryle, avec un composé de formule (B'10b) 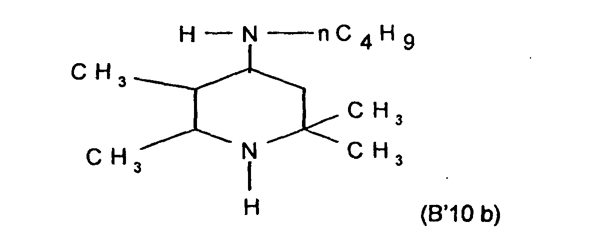 B'10 représentant un composé de formule B4-1', B4-2', B4-3' 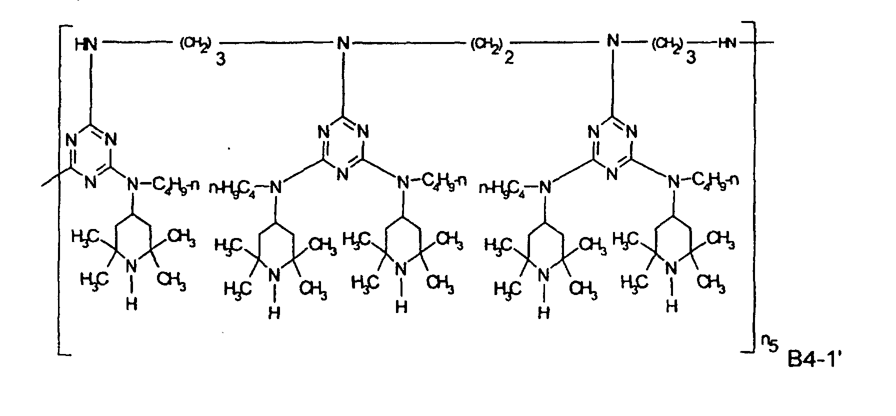 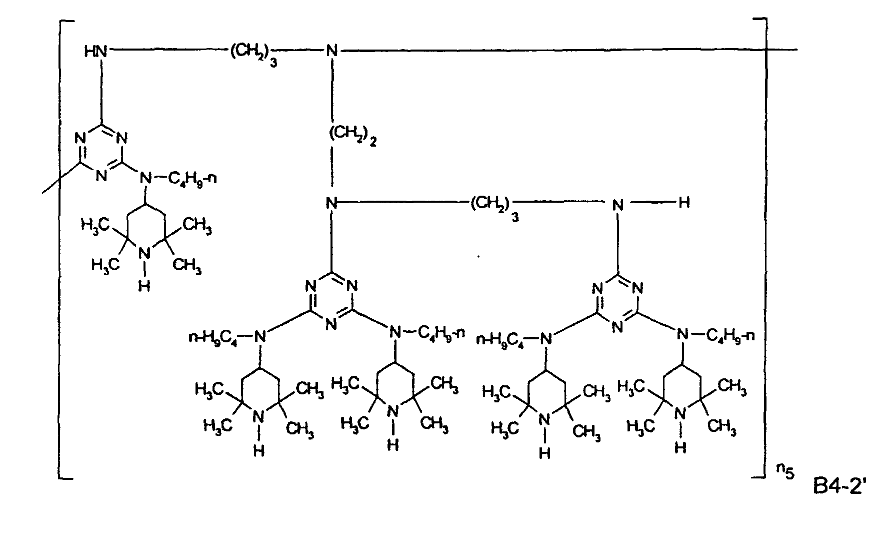 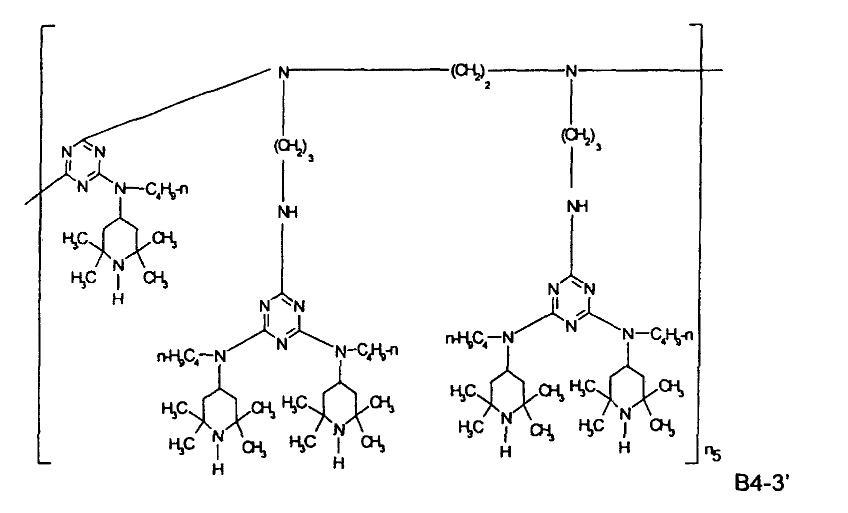 ou un mélange de ceux-ci, n₅ allant de 1 à 20.

17. Mélange selon la revendication 13, **caractérisé en ce que** dans les mélanges avec des composants de formule (III) (les autres) composant(s) de mélange consistent en Chimassorb® 944, Tinuvin® 622, Dastib® 1082, Uvasorb® HA88, Uvinul® 5050, Lowilite® 62, Uvasil® 299, Cyasorb® 3346, MARK LA® 63, MARK LA® 68 ou Luchem® B18.

18. Mélange à base d'un composé de formule générale (III) selon la revendication 1 et d'un ou plusieurs stabilisants phosphorés de formules générales C1 à C7 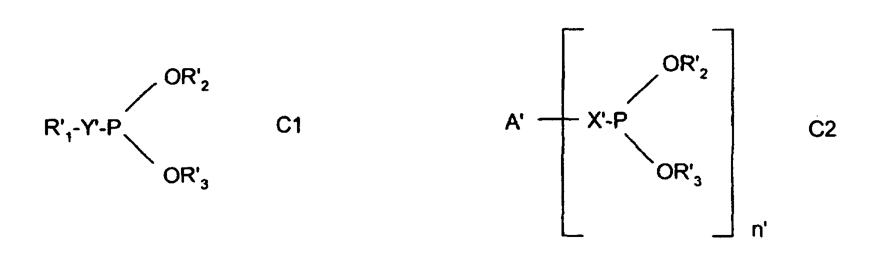 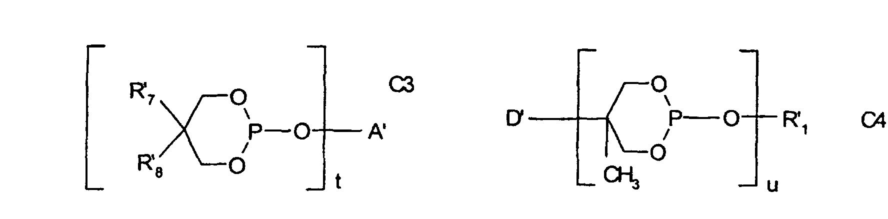 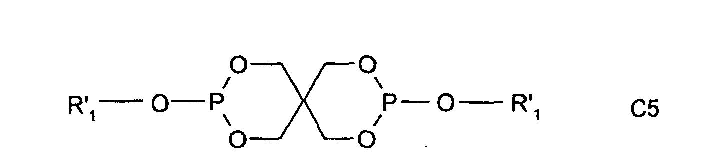 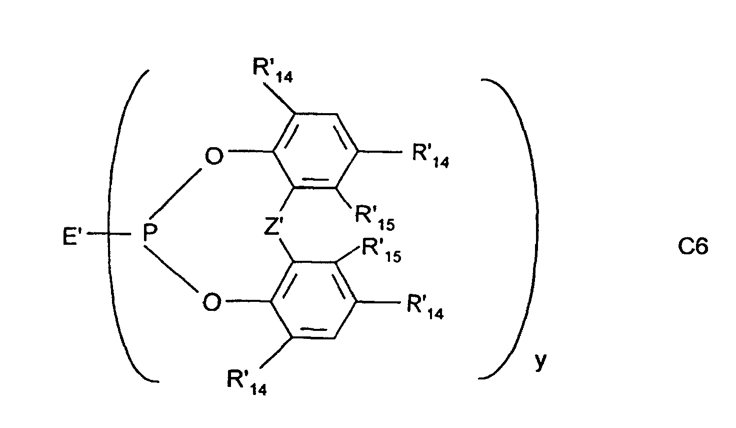 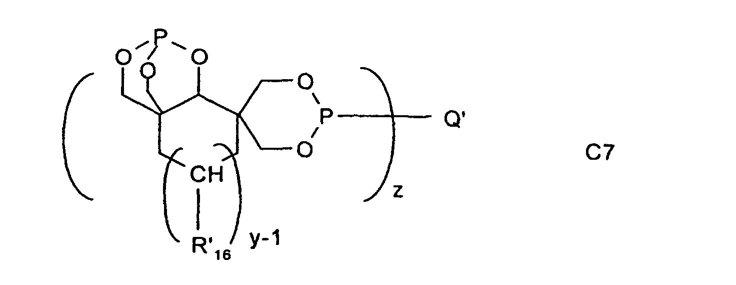 dans lesquelles les indices sont des nombres entiers, et
n' représente 2, 3 ou 4;
u représente 1 ou 2;
t représente 2 ou 3;
y représente 1, 2 ou 3; et
z représente un nombre allant de 1 à 6;
A', lorsque n' est égal à 2, représente un groupe alkylène ayant de 2 à 18 atomes de carbone; un groupe alkylène ayant de 2 à 12 atomes de carbone, interrompu par -S-, -O- ou -NR'₄-; un radical de l'une des formules 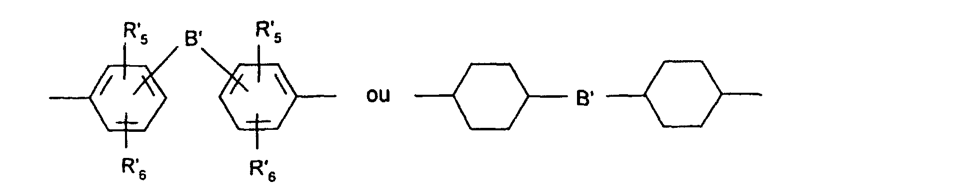 ou le groupe phénylène;
A', lorsque n' est égal à 3, est un radical de formule -CᵣH₂ᵣ₋₁;
A', lorsque n' est égal à 4, représente le radical de formule 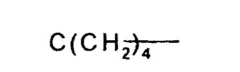 A" a la signification de A' lorsque n' est égal à 2;
B' représente un radical de formule -CH₂-; -CHR'_{4-;} -CR'₁R'₄; -S- ou une liaison directe; ou un groupe cycloalkylidène en C₅-C₇ ou un groupe cyclohexylidène substitué par 1 à 4 radicaux alkyle en C₁-C₄ en position 3, 4 et/ou 5,
D' représente, lorsque u est égal à 1, le groupe méthyle, et, lorsque u est égal à 2, -CH₂OCH₂-;
E', représente, lorsque y est égal à 1, un groupe alkyle ayant de 1 à 18 atomes de carbone, le groupe phényle, un radical de formule -OR'₁, ou un atome d'halogène;
E' représente, lorsque y est égal à 2, un radical de formule -O-A"-O-;
E', lorsque y est égal à 3, représente un radical de formule 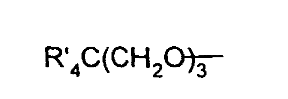 ou
N(CH₂-CH₂-O-)₃;
Q' représente le reste d'un alcool au moins z-hydroxylé ou du phénol, ce dernier étant lié à l'atome (aux atomes) de phosphore par l'atome (les atomes) d'oxygène alcooliques(s) ou phénolique(s).
R'₁, R'₂ et R'₃ représentent, indépendamment les uns des autres, un groupe alkyle en C₁-C₃₀, un groupe alkyle en C₁-C₁₈ substitué par un atome d'halogène ou par un groupe -COOR₄', -CN ou -CONR₄'R₄', un groupe alkyle en C₂-C-₁₈ interrompu par -S-, -O- ou -NR'₄-; un groupe phényl-alkyle(C₁-C₄); un groupe cycloalkyle en C₅-C₁₂, le groupe phényle ou naphtyle, un groupe phényle ou naphtyle substitué par des atomes d'halogène ou par 1 à 3 radicaux alkyle ou radicaux alcoxy ayant au total de 1 à 18 atomes de carbone, ou par un groupe phényl-alkyle(C₁-C₄); ou un radical de formule 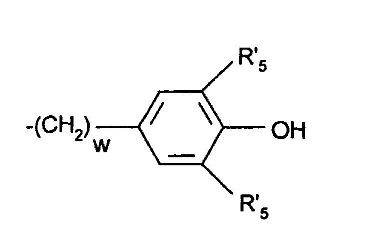 dans laquelle w représente un nombre entier dans la plage de 3 à 6;
R'₄, ou respectivement, les radicaux R'₄, indépendamment les uns des autres, représentent un atome d'hydrogène, un groupe alkyle en C₁-C₁₈, un groupe cycloalkyle en C₅-C₁₂ ou un groupe phénylalkyle ayant de 1 à 4 atomes de carbone dans le fragment alkyle;
R'₅ et R'₆ représentent, indépendamment l'un de l'autre, un atome d'hydrogène, un groupe alkyle en C₁-C₈ ou cycloalkyle en C₅-C₆;
R'₇ et R'₈, dans le cas où t = 2, représentent indépendamment l'un de l'autre un groupe alkyle en C₁-C₄ ou forment ensemble un radical 2,3-déhydropentaméthylène; et
R'₇ et R'₈, dans le cas où t = 3, représentent le groupe méthyle;
R'₁₄ représentent, indépendamment les uns des autres, un atome d'hydrogène, un groupe alkyle en C₁-C₉ ou cyclohexyle;
R'₁₅ représentent, indépendamment les uns des autres, un atome d'hydrogène ou le groupe méthyle; et
R'₁₆ représente un atome d'hydrogène ou un groupe alkyle en C₁-C₄ et, dans le cas où plusieurs radicaux R'₁₆ sont présents, les radicaux R'₁₆ sont identiques ou différents;
X' et Y' représentent chacun une liaison directe ou -O-; et
Z' est une liaison directe, -CH₂-, -C(R'₁₆)₂- ou -S-.

19. Mélange de stabilisants selon la revendication 18 avec un ou plusieurs composés phosphorés de formules C'1 à C'12

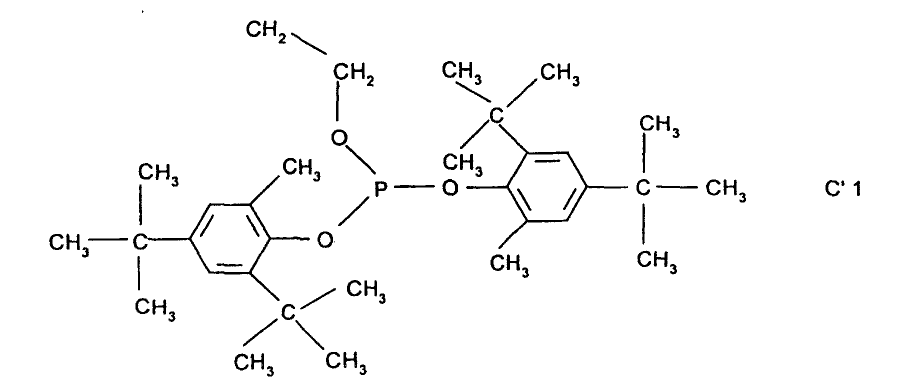

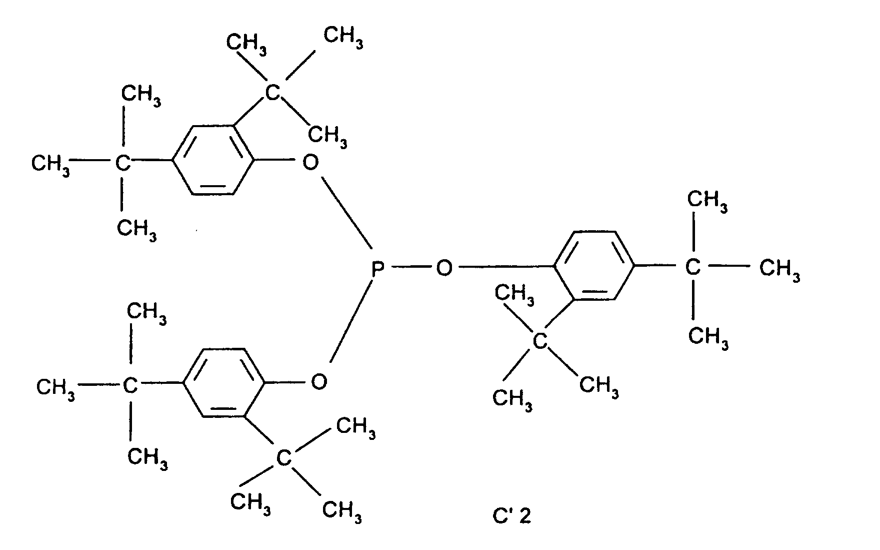

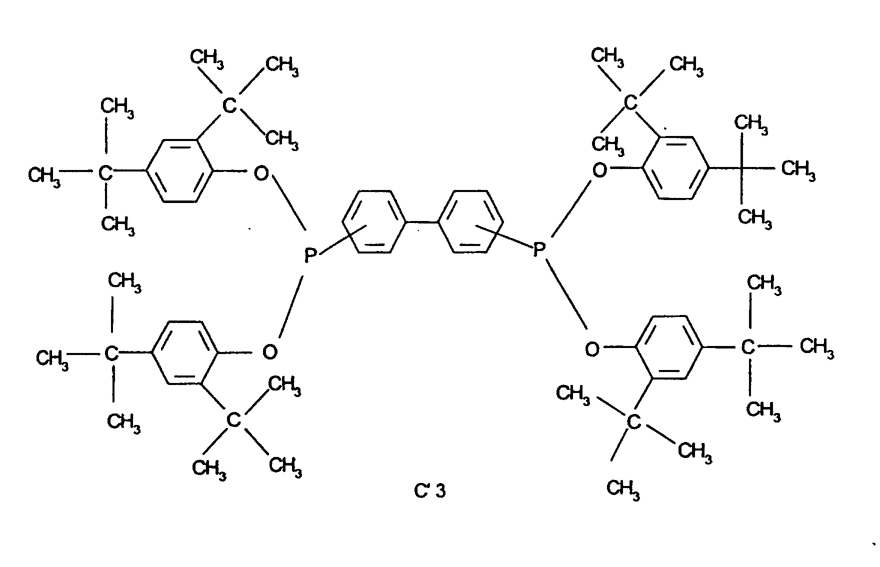

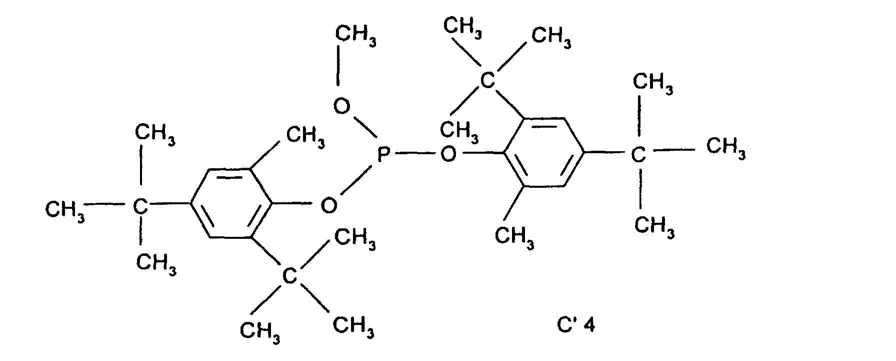

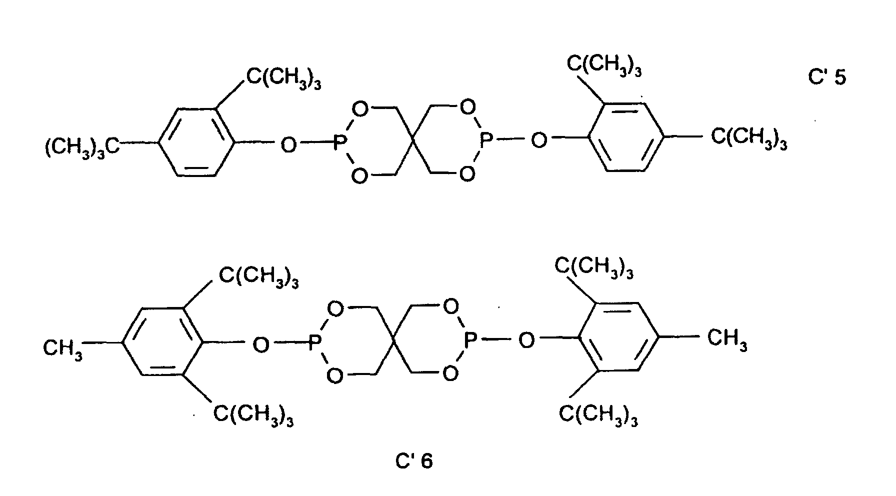

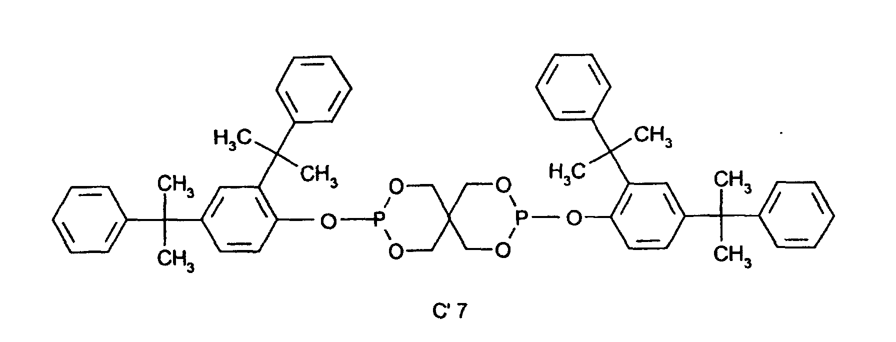

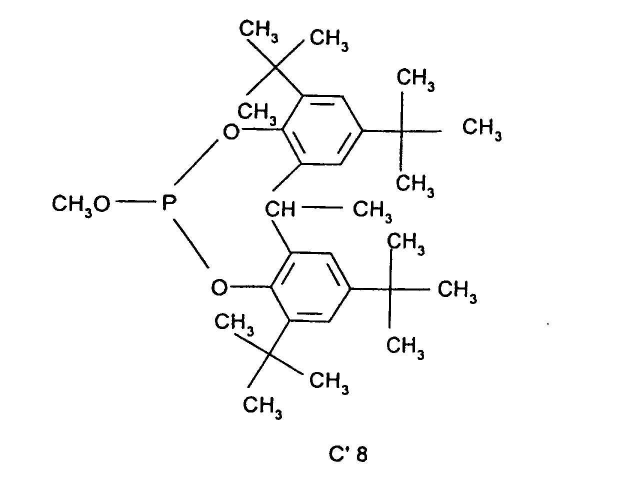

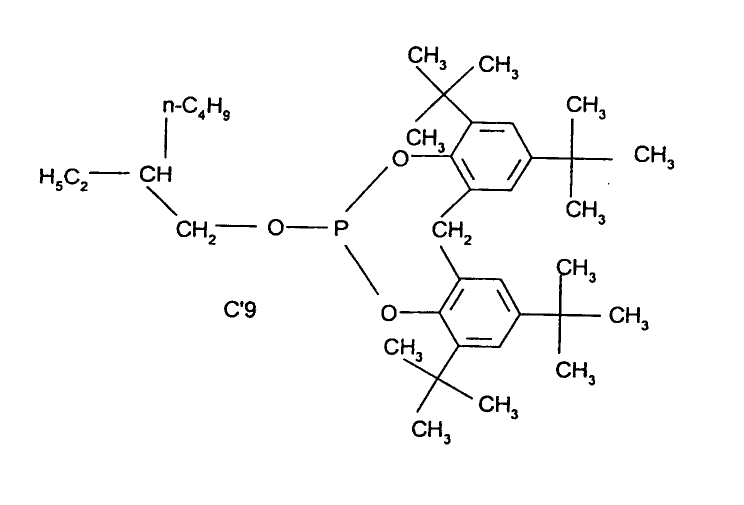

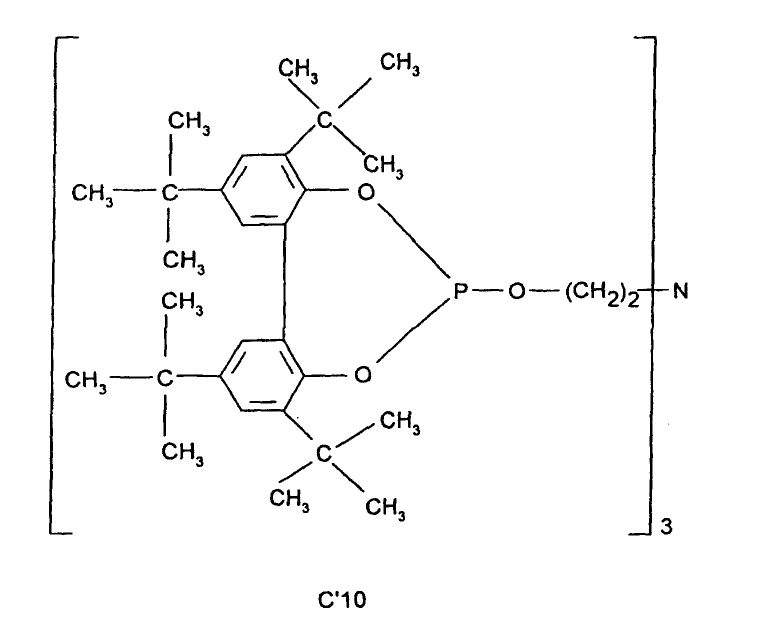

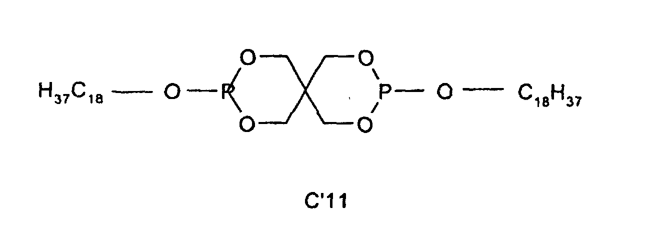

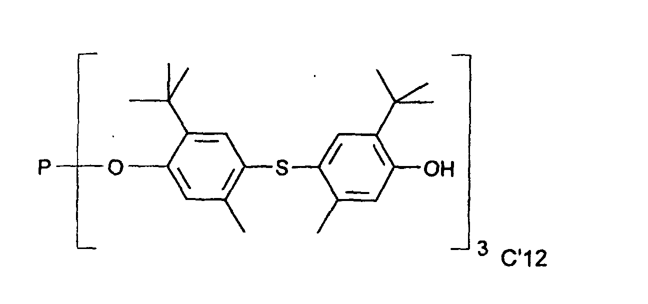

20. Mélange selon la revendication 18, **caractérisé en ce que** les composés phosphores consistent en Irgafos® 38, Irgafos® 12, Hostanox® PAR 24, Hostanox® OSP 1, Irgafos® P-EPQ, Ultranox® 626, Ultranox® 618, Mark® PEP-36, Mark® HP10, Doverphos® 9228.

21. Mélange de composés de formule générale (III) selon la revendication 1 et d'absorbeurs UV, ces absorbeurs UV pouvant être des composés choisis dans la classe des 2-hydroxybenzophénones, des 2-hydroxyphénylbenzotriazoles, des dérivés d'acide cinnamique, des oxanilides.

22. Mélange selon l'une quelconque des revendications 8 à 21, **caractérisé en ce que** la proportion du composé de formule (III) peut être comprise entre 1 et 99 % en poids.

23. Mélange d'un composé selon l'une quelconque des revendications 8 à 21 et de photoprotecteurs choisis dans la classe des absorbeurs UV.

24. Mélange de composés de formule (III) selon la revendication 1, seuls ou dans des mélanges selon les revendications 8 à 21 et d'une ou plusieurs hydroxylamines N,N-dialkyle-substituées.

25. Mélange selon la revendication 24, **caractérisé en ce que** l'hydroxylamine N,N-dialkyle-substituée consiste en la N,N-dioctadécylhydroxylamine.

26. Mélange des composés de formule générale (III) selon la revendication 1 avec un ou plusieurs co-stabilisants basiques ou autres capteurs d'acide, **caractérisé en ce que** ces derniers sont choisis dans le groupe des carboxylates, oxydes, hydroxydes, carbonates métalliques et/ou zéolithes et/ou hydrotalcites.

27. Mélange selon la revendication 26, **caractérisé en ce que** les co-stabilisants consistent en stéarate de calcium et/ou stéarate de magnésium et/ou oxyde de magnésium et/ou oxyde de zinc et/ou oxyde de zinc carbonaté et/ou en hydrotalcites.

28. Mélange selon la revendication 26, **caractérisé en ce que** les co-stabilisants consistent en Zinkoxid® aktiv, le Zinkoxid® transparent et/ou l'une des hydrotalcites DHT®4A, DHT® 4A2, Kyowaad® 200, Kyowaad® 300, Kyowaad® 400, Kyowaad® 500, Kyowaad® 600, Kyowaad® 700, Kyowaad® 1000, Kyowaad® 2000.

29. Mélange des composés de formule générale (III) selon la revendication 1 avec des antioxydants phénoliques à empêchement stérique.

30. Mélange composé d'un mélange selon les revendications 8 à 21 avec des antioxydants phénoliques à empêchement stérique.

31. Mélange composé d'un mélange selon la revendication 30 avec des agents synergiques du type des 3-pyrazolidinones.

32. Mélange composé d'un mélange selon la revendication 30 avec des agents synergiques du type des 1,2,4-triazolidine-3,5-diones.

33. Mélange composé d'un mélange selon la revendication 30 avec des agents synergiques du type des 3-arylbenzofurane-2-ones.

34. Mélange composé d'un mélange selon la revendication 30, **caractérisé en ce que** la 3-arylbenzofurane-2-one consiste en la 3-(3,4-diméthylphényl)-5,7-di-tert-butyl-3H-benzofuran-2-one (formule D).

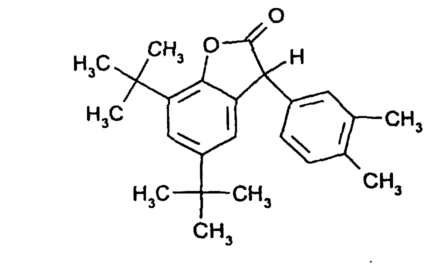

35. Mélange composé d'un mélange selon la revendication 30 et de co-stabilisants basiques ou autres capteurs d'acides.

36. Association d'un composé de formule (III) selon la revendication 1 et de colorants ou pigments à base organique ou minérale.

37. Utilisation de mélanges selon les revendications 8 à 36, pour la stabilisation de matériau organique.

38. Matériau organique stabilisé contenant des mélanges selon les revendications 8 à 36.

39. Matériau organique stabilisé selon la revendication 38, **caractérisé en ce qu'**il s'agit d'un polymère.

40. Matériau organique stabilisé selon la revendication 38, **caractérisé en ce qu'**il contient le composé (III) selon l'invention, conforme à la revendication 1, à une concentration de 0,001 à 5 % en poids, par rapport au matériau organique stabilisé.
